# EUROPEAN PATENT SPECIFICATION

(11) **EP 3 856 747 B1**
(45) Date of publication and mention of the grant of the patent: **19.07.2023**
(21) Application number: 19783228.0
(22) Date of filing: 24.09.2019
(51) Int. Cl.: C07D 519/00, C07F 9/09, A61K 31/4375, A61P 31/04

(54) **SUBSTITUTED PYRIDOINDOLES FOR THE TREATMENT AND PROPHYLAXIS OF BACTERIAL INFECTION**
SUBSTITUIERTE PYRIDOINDOLE ZUR BEHANDLUNG UND PROPHYLAXE EINER BAKTERIELLEN INFEKTION
PYRIDOINDOLES SUBSTITUÉS POUR LE TRAITEMENT ET LA PROPHYLAXIE D'UNE INFECTION BACTÉRIENNE

(30) Priority: 26.09.2018 WO PCT/CN2018/107584
(43) Date of publication of application: 04.08.2021
(73) Proprietor: F. Hoffmann-La Roche AG, 4070 Basel (CH)
(72) Inventor: DING, Xiao, Shanghai, 201203 (CN); LIU, Yongqiang, Shanghai, 201203 (CN); SHEN, Hong, Shanghai, 201203 (CN); SHI, Houguang, Shanghai, 201203 (CN); TAN, Xuefei, Shanghai, 201203 (CN); ZHOU, Chengang, Shanghai, 201203 (CN); ZHOU, Mingwei, Shanghai, 201203 (CN)
(74) Representative: Jochnowitz, Evan
(86) International application number: PCT/EP2019/075583
(87) International publication number: WO 2020/064661

(56) References cited:
- WO-A1-2010/136817
- WO-A1-2012/125746
- WO-A1-2018/178041
- JP-A- 2003 012 670

## Description

The present invention relates to organic compounds useful for therapy and/or prophylaxis in a mammal, and in particular to inhibitors of DNA gyrase and/or topoisomerase IV useful for treatment and/or prophylaxis of bacterial infection.

### FIELD OF THE INVENTION

Bacterial infections pose a continuing medical problem because anti-bacterial drugs eventually engender resistance in the bacteria on which they are used. Bacterial resistance against virtually all current antibiotic drugs are increasing. Many forms of antibiotic resistance can even cross international boundaries and spread with remarkable speed. Thus novel classes of antibacterial compounds are urgently needed.

One target for development of anti-bacterial drugs has been DNA gyrase and topoisomerase IV (bacterial type IIA topoisomerases), which are essential to cell life, that solve DNA topological problems resulting from the replication, transcription, and recombination of DNA. DNA Gyrase controls DNA supercoiling and relieves topological stress that occurs when the DNA strands are untwisted such as during replication. Topoisomerase IV primarily resolves linked chromosome dimers at the conclusion of DNA replication. Both enzymes can introduce double stranded DNA breaks; pass a second DNA strand through the break and rejoining the broken strands. The activity of both enzymes is driven by the binding and hydrolysis of ATP. Bacterial DNA gyrase consists of two A (GyrA) and two B (GyrB) subunits. Binding and cleavage of the DNA is associated with GyrA, whereas ATP is bound and hydrolyzed by GyrB. Bacterial Topoisomerase IV is also a hetero-tetramer that consists of two C (ParC) and two E (ParE) subunits. The latter subunits bind ATP like GyrB in order to supply energy necessary for catalytic turnover of the enzymes.

Inhibition of DNA gyrase and topoisomerase IV has potential for the development of broad-spectrum antibiotics. The enzymes are highly conserved across a broad range of gram-positive and gram-negative pathogens. There are two classes of antibiotics that demonstrated such mechanism of action. The first, well-represented by the quinolones, inhibits GyrA and ParC subunits by stabilizing the cleaved DNA-enzyme complex, thus inhibiting overall gyrase function, leading to cell death. Novobiocin, the only marketed drug in the second class, exerts its effect by blocking the ATPase activity of the enzymes. Novobiocin was identified in 1950s. But its use declined rapidly and it was eventually withdrawn from the market, mainly due to its low permeability in many bacteria strains, rise of spontaneous resistance development, and the development of more effective drugs, such as penicillinase-stable penicillins and the first cephalosporins in 1960s and 1970s.

Recently, strong inhibition of DNA gyrase and/or topoisomerase IV has been recognized to be important for low resistance development in bacterial strains treated by inhibitors of the enzymes. Inhibitors of bacterial DNA gyrase and/or topoisomerase IV with different mechanism of action compare to the widely used quinolones will exhibit minimal cross resistance, and will be potentially useful in combating quinolone resistance that has increased significantly in the past few years.

On the other hand, during both drug discovery and development, about 5-7% of drugs approved worldwide can be classified as prodrugs. Prodrugs are bioreversible derivatives of drug molecules that undergo an enzymatic and/or chemical transformation in vivo to release the active parent drug, which can then exert the desired pharmacological effect. For antibiotic research, most of the time, due to the high dose needed for clinical effect, the parent antibiotic drug often does not possess the good aqueous solubility needed for formulation delivery, the prodrug that can provide improved aqueous solubility is then pursued. "Prodrug strategies to overcome poor water solubility", Advanced Drug Delivery Reviews 59 (2007) 677-694. In the present invention, new types of prodrugs were disclosed with greatly improved aqueous solubility, which can be suitable for different routes of administration WO2010136817 describes heterocyclic urea derivatives for the treatment of bacterial infections.

### SUMMARY OF THE INVENTION

The present invention relates to novel compounds of formula (I), 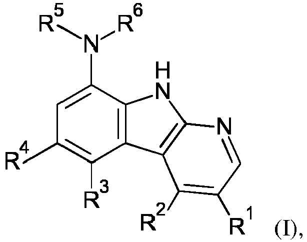 wherein
R¹ is 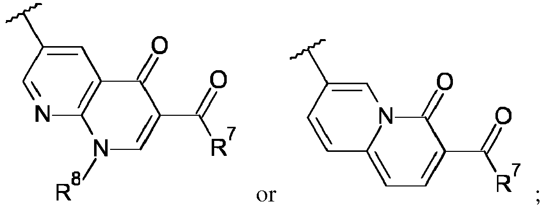 wherein R⁷ is selected from the group consisting of OH; (C₁₋₆alkyl-2-oxo-1,3-dioxol-4-yl)C₁₋₆alkoxy; aminoC₁₋₆alkylcarbonyloxyC₁₋₆alkoxy; C₁₋₆alkoxy; C₁₋₆alkoxycarbonyloxyC₁₋₆alkoxy; C₁₋₆alkylcarbonyloxyC₁₋₆alkoxy; C₁₋₆alkylsulfonylamino; carboxyC₁₋₆alkylaminocarbonylC₁₋₆alkylamino and phenylC₁₋₆alkoxy;
   R⁸ is C₁₋₆alkyl;
R² is selected from 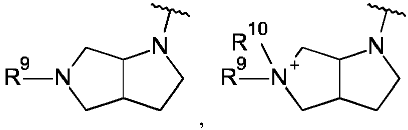 and -N(R⁹)₂; wherein R⁹ is C₁₋₆alkyl;
   R¹⁰ is selected from the group consisting of C₁₋₆alkyl-2-oxo-1,3-dioxol-4-yl; C₁₋₆alkoxy(hydroxy)phosphoryloxyC₁₋₆alkyl and phosphonooxyC₁₋₆alkyl;
R³ is halogen or cyano;
R⁴ is halogen;
R⁵ is selected from the group consisting of H; ((aminoC₁₋₆alkylcarbonyl)aminoC₁₋₆alkylcarbonyl; ((aminoC₁₋₆alkylcarbonyl)aminoC₁₋₆alkylcarbonyl)aminoC₁₋₆alkylcarbonyl; (C₁₋₆alkoxy(hydroxy)phosphoryl)C₁₋₆alkoxycarbonyl; (C₁₋₆alkoxy)₂phosphoryl; aminoC₁₋₆alkylcarbonyloxyC₁₋₆alkoxycarbonyl; C₁₋₆alkylcarbonyloxyC₁₋₆alkoxycarbonyl; carboxyC₂₋₆alkenylcarbonyloxyC₁₋₆alkoxycarbonyl; formyl; phenylC₁₋₆alkoxy(hydroxy)phosphoryl; phosphonooxyC₁₋₆alkoxycarbonyl and sulfo;
R⁶ is C₁₋₆alkyl;
with the proviso that when R⁵ is H and R⁷ is OH, R² is not 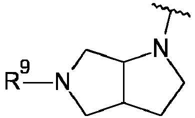 or -N(R⁹)₂;
or a pharmaceutically acceptable salt thereof.

The present invention also relates to novel compounds of formula (II), 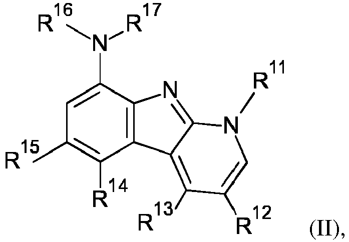 wherein
R¹¹ is selected from the group consisting of C₁₋₆alkyl-2-oxo-1,3-dioxol-4-yl; C₁₋₆alkoxy(hydroxy)phosphoryloxyC₁₋₆alkyl and phosphonooxyC₁₋₆alkyl;
R¹² is 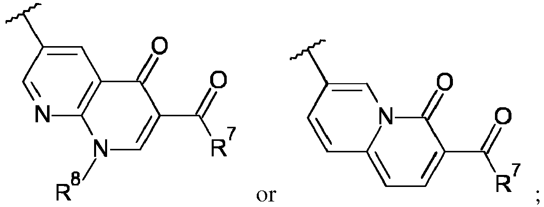 wherein R⁷ is OH;
   R⁸ is C₁₋₆alkyl;
R¹³ is 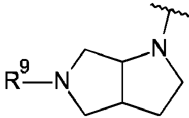 or -N(R⁹)₂; wherein R⁹ is C₁₋₆alkyl;
R¹⁴ is halogen or cyano;
R¹⁵ is halogen;
R¹⁶ is H;
R¹⁷ is C₁₋₆alkyl;
or a pharmaceutically acceptable salt thereof.

Further objects of the present invention are novel compounds of formula (I) or (II), their manufacture, medicaments based on a compound in accordance with the invention and their production as well as the use of compounds of formula (I) or (II) for the treatment or prophylaxis of bacterial infection. The use of compounds of formula (I) or (II) as DNA gyrase and/or topoisomerase IV inhibitors is also one of the objections of present invention. The compounds of formula (I) or (II) showed superior solubility compared to parent compounds, good CC₅₀ profiles, microsomal stability and/or PK profile.

### DETAILED DESCRIPTION OF THE INVENTION

Unless otherwise defined, all technical and scientific terms used herein have the same meaning as commonly understood by one of ordinary skill in the art to which this invention belongs. Furthermore, the following definitions are set forth to illustrate and define the meaning and scope of the various terms used to describe the invention. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### DEFINITIONS

The term "C₁₋₆alkyl" denotes a saturated, linear or branched chain alkyl group containing 1 to 6, particularly 1 to 4 carbon atoms, for example methyl, ethyl, propyl, isopropyl, *n*-butyl, isobutyl, *tert*-butyl and the like. Particular "C₁₋₆alkyl" groups are methyl, ethyl and propyl.

The term "C₁₋₆alkoxy" denotes C₁₋₆alkyl-O-, wherein the "C₁₋₆alkyl" is as defined above; for example methoxy, ethoxy, propoxy, *iso*-propoxy, *n*-butoxy, *iso*-butoxy, 2-butoxy, tert-butoxy, pentoxy, hexyloxy and the like. Particular "C₁₋₆alkoxy" groups are methoxy, ethoxy and propoxy.

The term "C₂₋₆alkenyl" denotes an unsaturated, linear or branched chain alkenyl group containing 2 to 6, particularly 2 to 4 carbon atoms, for example vinyl, propenyl, allyl, butenyl and the like. Particular "C₂₋₆alkenyl" group is propenyl.

The term "halogen" and "halo" are used interchangeably herein and denote fluoro, chloro, bromo, or iodo.

The term "carbonyloxy" denotes -C(O)-O-.

The term "cis-isomers" and "*trans*-isomers" denote the relative stereochemistry of the molecule or moiety. For example: Intermediate C1 ( 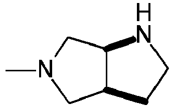 ) as the "*cis*-isomers" refers to a mixture of 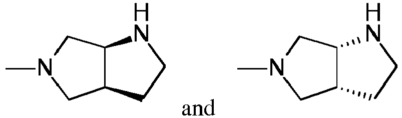 . The way of showing relative stereochemistry also applies to the final compounds.

The term "enantiomer" denotes two stereoisomers of a compound which are non-superimposable mirror images of one another.

The term "diastereomer" denotes a stereoisomer with two or more centers of chirality and whose molecules are not mirror images of one another. Diastereomers have different physical properties, e.g. melting points, boiling points, spectral properties, and reactivities.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and *tert*iary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### INHIBITORS OF DNA GYRASE AND/OR TOPOISOMERASE IV

The present invention relates to a compound of formula (I), 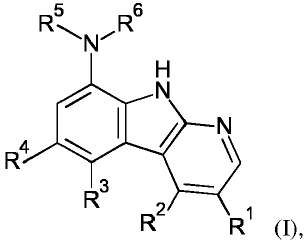 wherein
R¹ is 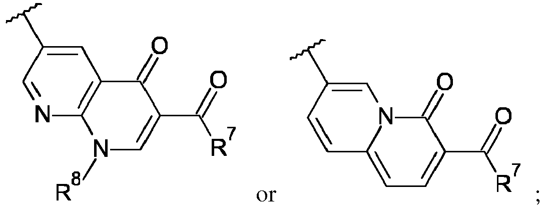 wherein R⁷ is selected from the group consisting of OH; (C₁₋₆alkyl-2-oxo-1,3-dioxol-4-yl)C₁₋₆alkoxy; aminoC₁₋₆alkylcarbonyloxyC₁₋₆alkoxy; C₁₋₆alkoxy; C₁₋₆alkoxycarbonyloxyC₁₋₆alkoxy; C₁₋₆alkylcarbonyloxyC₁₋₆alkoxy; C₁₋₆alkylsulfonylamino; carboxyC₁₋₆alkylaminocarbonylC₁₋₆alkylamino and phenylC₁₋₆alkoxy;
   R⁸ is C₁₋₆alkyl;
R² is selected from 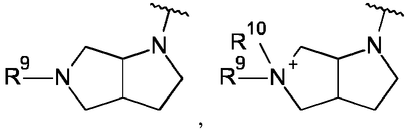 and -N(R⁹)₂; wherein R⁹ is C₁₋₆alkyl;
   R¹⁰ is selected from the group consisting of C₁₋₆alkyl-2-oxo-1,3-dioxol-4-yl; C₁₋₆alkoxy(hydroxy)phosphoryloxyC₁₋₆alkyl and phosphonooxyC₁₋₆alkyl;
R³ is halogen or cyano;
R⁴ is halogen;
R⁵ is selected from the group consisting of H; ((aminoC₁₋₆alkylcarbonyl)aminoC₁₋₆alkylcarbonyl; ((aminoC₁₋₆alkylcarbonyl)aminoC₁₋₆alkylcarbonyl)aminoC₁₋₆alkylcarbonyl; (C₁₋₆alkoxy(hydroxy)phosphoryl)C₁₋₆alkoxycarbonyl; (C₁₋₆alkoxy)₂phosphoryl; aminoC₁₋₆alkylcarbonyloxyC₁₋₆alkoxycarbonyl; C₁₋₆alkylcarbonyloxyC₁₋₆alkoxycarbonyl; carboxyC₂₋₆alkenylcarbonyloxyC₁₋₆alkoxycarbonyl; formyl; phenylC₁₋₆alkoxy(hydroxy)phosphoryl; phosphonooxyC₁₋₆alkoxycarbonyl and sulfo;
R⁶ is C₁₋₆alkyl;
with the proviso that when R⁵ is H and R⁷ is OH, R² is not 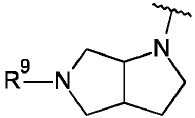 or -N(R⁹)₂;
or a pharmaceutically acceptable salt thereof.

A further embodiment of present invention is (ii) a compound of formula (I) according to (i), wherein
R¹ is 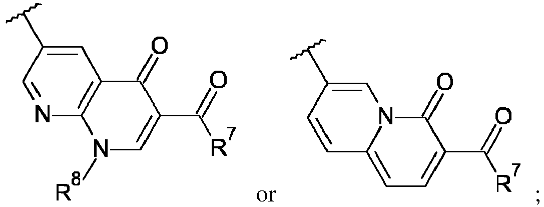 wherein R⁷ is selected from the group consisting of OH; (5-methyl-2-oxo-1,3-dioxol-4-yl)methoxy; acetoxyethoxy; amino(methyl)butanoyloxyethoxy; benzyloxy; carboxymethylaminocarbonylmethylamino; ethoxy; isopropoxycarbonyloxyethoxy and methylsulfonylamino;
   R⁸ is methyl;
R² is selected from 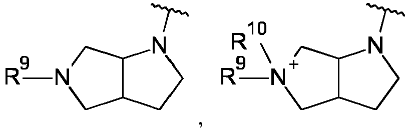 and -N(R⁹)₂; wherein R⁹ is methyl;
   R¹⁰ is selected from the group consisting of 5-methyl-2-oxo-1,3-dioxol-4-ylmethyl; *tert*-butoxy(hydroxy)phosphoryloxymethyl and phosphonooxymethyl;
R³ is fluoro of cyano;
R⁴ is fluoro;
R⁵ is selected from the group consisting of H; ((aminoacetyl)aminoacetyl)aminoacetyl; (aminoacetyl)aminoacetyl; (tert-butoxy(hydroxy)phosphoryloxy)methoxycarbonyl; acetoxyethoxycarbonyl; aminoacetyloxymethoxycarbonyl; amino(methyl)butanoyloxyethoxycarbony; benzyloxy(hydroxy)phosphoryl; carboxypropenoyloxymethoxycarbonyl; diethoxyphosphoryl; formyl; phosphonooxymethoxycarbonyl and sulfo;
R⁶ is methyl;
with the proviso that when R⁵ is H and R⁷ is OH, R² is not 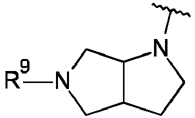 or -N(R⁹)₂;
or a pharmaceutically acceptable salt thereof.

A further embodiment of present invention is (iii) a compound of formula (I) according to (i), wherein
R¹ is 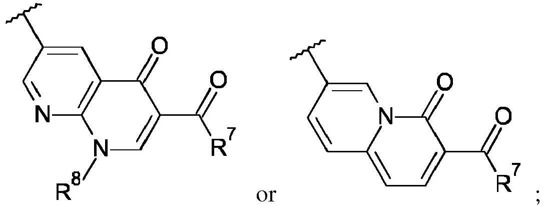 wherein R⁷ is selected from the group consisting of (C₁₋₆alkyl-2-oxo-1,3-dioxol-4-yl)C₁₋₆alkoxy; aminoC₁₋₆alkylcarbonyloxyC₁₋₆alkoxy; C₁₋₆alkoxy; C₁₋₆alkoxycarbonyloxyC₁₋₆alkoxy; C₁₋₆alkylcarbonylC₁₋₆alkoxy; C₁₋₆alkylsulfonylamino; carboxyC₁₋₆alkylaminocarbonylC₁₋₆alkylamino and phenylC₁₋₆alkoxy;
   R⁸ is C₁₋₆alkyl;
R² is 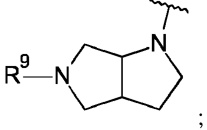 wherein R⁹ is C₁₋₆alkyl;
R³ is halogen or cyano;
R⁴ is halogen;
R⁵ is H;
R⁶ is C₁₋₆alkyl;
or a pharmaceutically acceptable salt thereof.

A further embodiment of present invention is (iv) a compound of formula (I) according to (iii), or a pharmaceutically acceptable salt thereof,wherein R⁷ is selected from the group consisting of (C₁₋₆alkyl-2-oxo-1,3-dioxol-4-yl)C₁₋₆alkoxy; aminoC₁₋₆alkylcarbonyloxyC₁₋₆alkoxy; C₁₋₆alkoxycarbonyloxyC₁₋₆alkoxy and C₁₋₆alkylcarbonyloxyC₁₋₆alkoxy.

A further embodiment of present invention is (v) a compound of formula (I) according to (iii) or (iv), or a pharmaceutically acceptable salt thereof, wherein R⁷ is selected from the group consisting of (5-methyl-2-oxo-1,3-dioxol-4-yl)methoxy; acetoxyethoxy; amino(methyl)butanoyloxyethoxy and isopropoxycarbonyloxyethoxy.

A further embodiment of present invention is (vi) a compound of formula (I) according to any one of (iii) to (v), or a pharmaceutically acceptable salt thereof, wherein R³ is fluoro or cyano; R⁴ is fluoro; R⁶ is methyl; R⁸ is methyl; R⁹ is methyl.

A further embodiment of present invention is (vii) a compound of formula (I), or a pharmaceutically acceptable salt thereof, according to (i), wherein
R¹ is 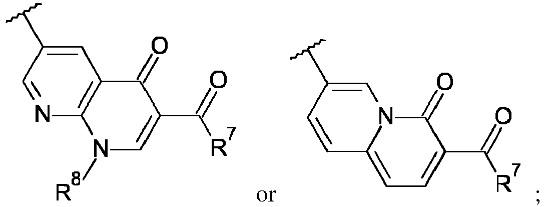 wherein R⁷ is OH;
   R⁸ is C₁₋₆alkyl;
R² is 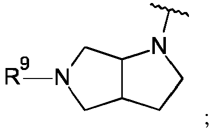 wherein R⁹ is C₁₋₆alkyl;
R³ is halogen or cyano;
R⁴ is halogen;
R⁵ is selected from the group consisting of ((aminoC₁₋₆alkylcarbonyl)aminoC₁₋₆alkylcarbonyl; ((aminoC₁₋₆alkylcarbonyl)aminoC₁₋₆alkylcarbonyl)aminoC₁₋₆alkylcarbonyl; (C₁₋₆alkoxy(hydroxy)phosphoryl)C₁₋₆alkoxycarbonyl; (C₁₋₆alkoxy)₂phosphoryl; aminoC₁₋₆alkylcarbonyloxyC₁₋₆alkoxycarbonyl; C₁₋₆alkylcarbonyloxyC₁₋₆alkoxycarbonyl; carboxyC₂₋₆alkenylcarbonyloxyC₁₋₆alkoxycarbonyl; formyl; phenylC₁₋₆alkoxy(hydroxy)phosphoryl; phosphonooxyC₁₋₆alkoxycarbonyl and sulfo;
R⁶ is C₁₋₆alkyl;
or a pharmaceutically acceptable salt thereof.

A further embodiment of present invention is (viii) a compound of formula (I) according to (vii), or a pharmaceutically acceptable salt thereof, wherein R⁵ is selected from the group consisting of C₁₋₆alkylcarbonyloxyC₁₋₆alkoxycarbonyl; carboxyC₂₋₆alkenylcarbonyloxyC₁₋₆alkoxycarbonyl and phosphonooxyC₁₋₆alkoxycarbonyl.

A further embodiment of present invention is (ix) a compound of formula (I) according to (vii) or (viii), or a pharmaceutically acceptable salt thereof, wherein R⁵ is selected from the group consisting of acetoxyethoxycarbonyl; carboxypropenoyloxymethoxycarbonyl and phosphonooxymethoxycarbonyl.

A further embodiment of present invention is (x) a compound of formula (I) according to any one of (vii) to (ix), or a pharmaceutically acceptable salt thereof, wherein R³ is fluoro or cyano; R⁴ is fluoro; R⁶ is methyl; R⁸ is methyl; R⁹ is methyl.

An another embodiment of present invention is (xi) a compound of formula (II), 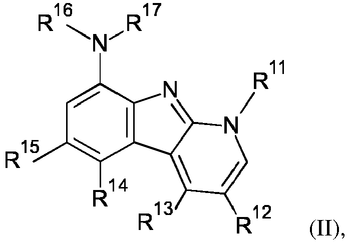 wherein
R¹¹ is selected from the group consisting of C₁₋₆alkyl-2-oxo-1,3-dioxol-4-yl; C₁₋₆alkoxy(hydroxy)phosphoryloxyC₁₋₆alkyl and phosphonooxyC₁₋₆alkyl;
R¹² is 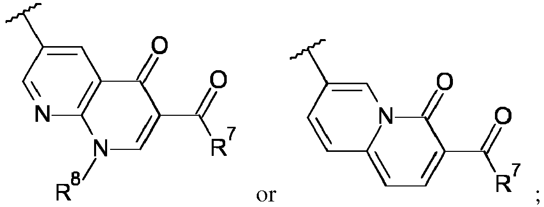 wherein R⁷ is OH;
   R⁸ is C₁₋₆alkyl;
R¹³ is 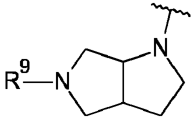 or -N(R⁹)₂; wherein R⁹ is C₁₋₆alkyl;
R¹⁴ is halogen or cyano;
R¹⁵ is halogen;
R¹⁶ is H;
R¹⁷ is C₁₋₆alkyl;
or a pharmaceutically acceptable salt thereof.

A further embodiment of present invention is (xii) a compound of formula (II) according to (xii), or a pharmaceutically acceptable salt thereof, wherein R¹¹ is selected from the group consisting of 5-methyl-2-oxo-1,3-dioxol-4-ylmethyl; *tert-*butoxy(hydroxy)phosphoryloxymethyl and phosphonooxymethyl;
R¹² is 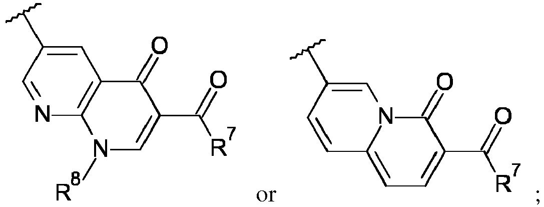 wherein R⁷ is OH; R⁸ is methyl;
R¹³ is 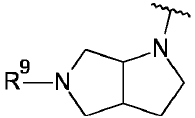 or -N(R⁹)₂; wherein R⁹ is methyl;
R¹⁴ is fluoro or cyano;
R¹⁵ is fluoro;
R¹⁶ is H;
R¹⁷ is methyl;
   or a pharmaceutically acceptable salt thereof.

Any of the above embodiments may be combined.

Another embodiment of present invention is that compounds of formula (I) or (II) are selected from:
6-[5-Cyano-6-fluoro-8-[methyl(phosphonooxymethoxycarbonyl)amino]-4-[(3aS,6aS)-5-methyl-2,3,3a,4,6,6a-hexahydropyrrolo [2,3-c]pyrrol-1-yl]-9H-pyrido [2,3-b]indol-3-yl]-1-methyl-4-oxo-1,8-naphthyridine-3-carboxylic acid;
6-[5-Cyano-6-fluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5-Cyano-6-fluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5-Cyano-6-fluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5-Cyano-6-fluoro-8-(methylamino)-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5-Cyano-6-fluoro-8-(methylamino)-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid;
6-[5-Cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid;
2-[(2S)-2-Amino-3-methyl-butanoyl]oxyethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate;
2-[[2-[[6-[5-Cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carbonyl]amino]acetyl]amino]acetic acid;
6-[5-Cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-N-methylsulfonyl-4-oxo-1,8-naphthyridine-3-carboxamide;
1-Acetoxyethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate;
1-Isopropoxycarbonyloxyethyl 6-[6-fluoro-5-methyl-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate;
(5-Methyl-2-oxo-1,3-dioxol-4-yl)methyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate;
6-[8-[(2-Aminoacetyl)oxymethoxycarbonyl-methyl-amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[8-[1-Acetoxyethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[8-[Diethoxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[8-[[Benzyloxy(hydroxy)phosphoryl]-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5,6-Difluoro-8-[methyl(phosphonooxymethoxycarbonyl)amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[8-[[*tert*-Butoxy(hydroxy)phosphorylloxymethoxycarbonyl-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
(E)-4-[[[3-(6-Benzyloxycarbonyl-8-methyl-5-oxo-1,8-naphthyridin-3-yl)-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-methyl-carbamoyl]oxymethoxy]-4-oxo-but-2-enoic acid;
6-[5,6-Difluoro-8-[methyl-[[(E)-3-carboxyprop-2-enoyl]oxymethoxycarbonyl]amino]-4- [(3aR,6aR)-5-methyl-2,3,3a,4,6,6a-hexahydropyrrolo[2,3-c]pyrrol- 1-yll-9H-pyrido[2,3-b]indol-3-yl]-1-methyl-4-oxo-1,8-naphthyridine-3-carboxylic acid;
6-[1-[[*tert*-Butoxy(hydroxy)phosphoryl]oxymethyl]-5,6-difluoro-8-(methylamino)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5,6-Difluoro-8-(methylamino)-1-(phosphonooxymethyl)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5,6-Difluoro-8-(methylamino)-4-[(3aR,6aR)-5-[[*tert-*butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid;
6-[5,6-Difluoro-8-(methylamino)-4-[(3aR,6aR)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid;
6-[5,6-Difluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5,6-Difluoro-8-[methyl(sulfo)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5,6-Difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5,6-Difluoro-8-[methyl(sulfo)amino]-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[8-[[2-[(2-Aminoacetyl)amino]acetyl]-methyl-amino]-5,6-difluoro-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
Ethyl 6-[5,6-difluoro-8-(methylamino)-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate;
7-[5,6-Difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3 -carboxylic acid;
7-[5,6-Difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid;
7-[8-[[2-[[2-[(2-Aminoacetyl)amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid;
7-[8-[[2-[(2-Aminoacetyl)amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3 -carboxylic acid;
7-[5,6-Difluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid;
2-[(2S)-2-Amino-3-methyl-butanoyl]oxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate;
2-[[2-[[7-[5,6-Difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetic acid;
1-Isopropoxycarbonyloxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate;
1-Acetoxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate;
7-[5,6-Difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-N-methylsulfonyl-4-oxo-quinolizine-3-carboxamide;
6-[5,6-Difluoro-8-(methylamino)-4-[*cis*-5-methyl-2,3,3a,4,6,6a-hexahydropyrrolo[2,3-c]pyrrol-1-yl]-9H-pyrido [2,3-b]indol-3-yl]-1-methyl-N-methylsulfonyl-4-oxo-1,8-naphthyridine-3-carboxamide;
6-[5,6-Difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[4-(Dimethylamino)-5,6-difluoro-8-(methylamino)-1-(phosphonooxymethyl)pyrido[2,3-b]indol-3-yl]-1-methyl-4-oxo-1,8-naphthyridine-3-carboxylic acid; and
6-[5-Cyano-6-fluoro-8-[methyl-[1-[(2S)-2-amino-3-methyl-butanoyl]oxyethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
or a pharmaceutically acceptable salt.

### SYNTHESIS

The compounds of the present invention can be prepared by any conventional means. Suitable processes for synthesizing these compounds as well as their starting materials are provided in the schemes below and in the examples. All substituents, in particular, R¹ to R⁷ are as defined above unless otherwise indicated. Furthermore, and unless explicitly otherwise stated, all reactions, reaction conditions, abbreviations and symbols have the meanings well known to a person of ordinary skill in organic chemistry. 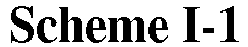 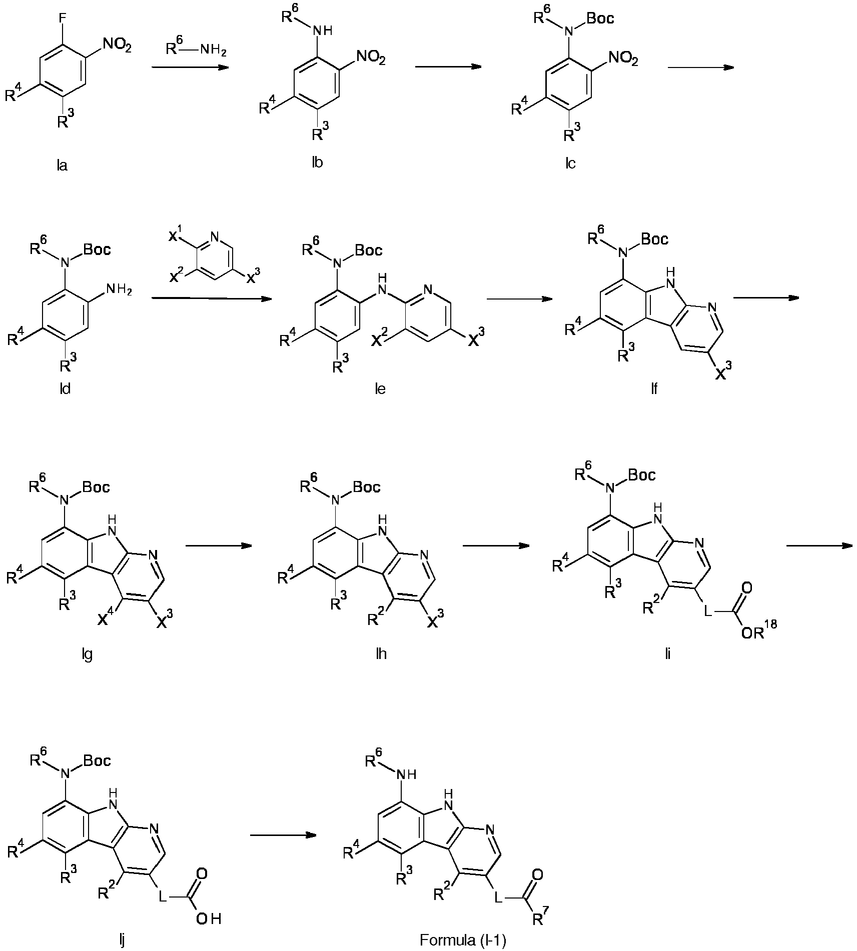 wherein X¹, X², X³ and X⁴ are independently halogen, boronic ester, or boronic acid; L is unsubstituted or substituted 4-oxo-1,8-naphthyridinyl, or 4-oxoquinolizinyl; R¹⁸ is C₁₋₆alkyl or Bn.

In one embodiment, the compound of formula (I-1) can be prepared according to **Scheme I-1.** Nucleophilic substitution of ortho-fluoro nitrobenzene (Ia) with amine R⁶-NH₂ affords aniline (Ib). The aniline (Ib) can be protected with di-*tert*-butyl carbonate to give the protected aniline (Ic). The nitro group in aniline (Ic) can be reduced by a reducing agent, such as H₂ with palladium catalysts, to give the compound of formula (Id). Coupling of the compound of formula (Id) with tri-halogenated pyridine can be achieved using palladium catalysts and phosphine ligands to give the compound of formula (Ie). Cyclization of the compound of formula (Ie) using palladium catalysts and phosphine ligands gives the compound of formula (If). The compound of formula (Ig) can be obtained from the compound of formula (If) through oxidation of the pyridine followed by halogenation, such as treatment of POCl₃ or POBr₃. Alternatively, the compound of formula (Ig) can also be obtained by additional halogenation of the compound of formula (Ig) (when R³ is H) with halogenating reagent, such as NCS, followed by di-*tert*-butyl carbonate re-protection.

Coupling of the compound of formula (Ig) to introduce R² can be achieved through either nucleophilic substitution with an amine, in the presence of a base for certain C-N bond formation (with R² bearing a nucleophilic N), or a Buchwald-Hartwig cross coupling reaction for certain C-N bond formation (with R² bearing a basic N). Further coupling of the compound of formula (Ih) to introduce R¹ can be achieved using a palladium catalyzed Suzuki coupling to give the compound of formula (Ii). Chiral separation can be carried out for the compound of formula (Ih) or the compound of formula (Ii). Alternatively, the compound of formula (Ii) can also be obtained by converting the halide X³ of compound of formula (Ih) into a boronic ester or boronic acid, then coupling with the R¹ halide. Converting the ester of the compound of formula (Ii) into a carboxylic acid in the presence of a base, such as NaOH or LiOH, affords the compound of formula (Ij).

The compound of formula (I-1) can be prepared by the ester or the amide bond formation via coupling the compound of formula (Ij) carboxylic acid with an alcohol or amine, in the presence of a coupling reagent, such as HATU, followed by the deprotection of Boc group with an acid, such as TFA/DCM. Alternatively, the compound of formula (I) can also be prepared by the amide bond formation via first deprotection of Boc group, followed by the activation of the carboxylic acid into an acid chloride and reaction with amide, such as methanesulfonamide. Alternatively, the compound of formula (I-1) can also be prepared by the ester bond formation via reacting the carboxylic acid with an electrophile halide in the presence of a base such as K₂CO₃, in a solvent such as DMF. 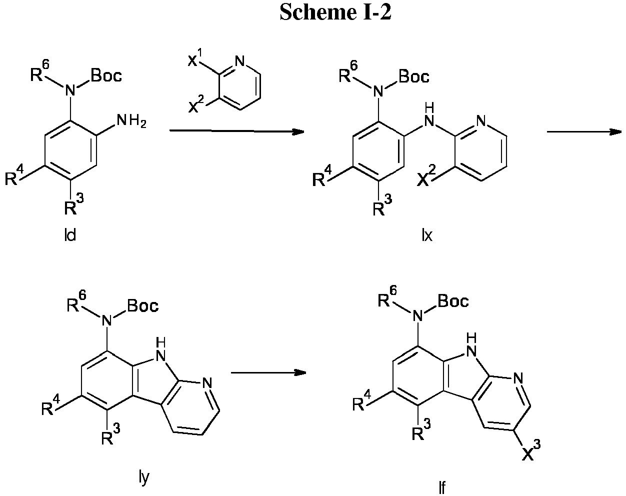 wherein X¹, X², X³ and X⁴ are independently halogen, boronic ester, or boronic acid.

In another embodiment, the compound of formula (If) can be prepared according to **Scheme I-2.** Coupling of the compound of formula (Id) with di-halogenated pyridine can be achieved using palladium catalysts and phosphine ligands to give the compound of formula (Ix). Cyclization of the compound of formula (Ix) using palladium catalysts and phosphine ligands gives the compound of formula (Iy). The compound of formula (Iy) can be subject to halogenation using halogenating reagent, such as NCS, NBS, or NIS, to give the compound of formula (If). 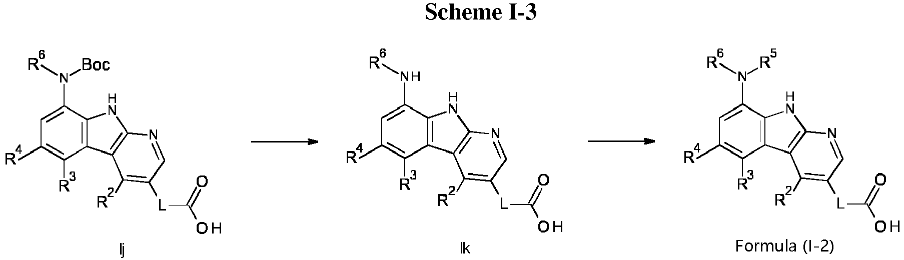 wherein L is unsubstituted or substituted 4-oxo-1,8-naphthyridinyl, or 4-oxoquinolizinyl.

In another embodiment, the compound of formula (I-2) can be prepared according to **Scheme I-3.** The compound of formula (Ij) is deprotected to give the of compound of formula (Ik). The compound of formula (Ik) can then be derivatized into the compound of formula (I) by reaction with acylating or sulfonylating reagents, such as formic acid or sulfur trioxide. 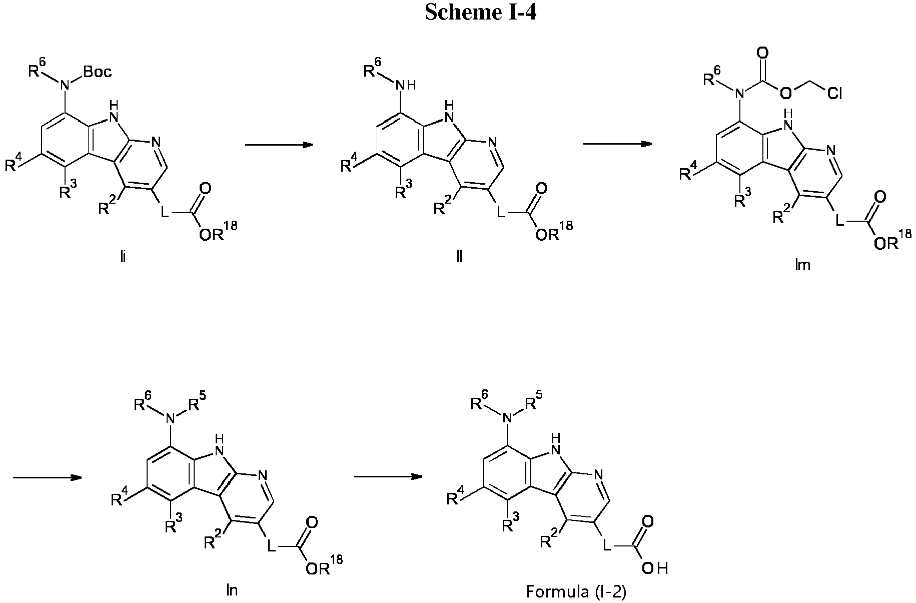 wherein L is unsubstituted or substituted 4-oxo-1,8-naphthyridinyl, or 4-oxoquinolizinyl; R¹⁸ is C₁₋₆alkyl or Bn.

In another embodiment, the compound of formula (I) can be prepared according to **Scheme I-4.** Deprotection the compound of formula (Ii) affords the compound of formula (Il). A carbamate linker formation by reaction of the compound of formula (Il) with a chlorformate such as chloromethyl chloroformate gives the compound of formula (Im). SN2 nucleophilic displacement of halide of the compound of formula (Im) with agents such as potassium di*-tert-*butylphosphate or sodium hydrogen fumarate gives the compound of formula (In). Compound of formula (In) can be converted to compound of formula (I-2) via hydrogenation (when R¹⁸ is Bn) in the presence of Pd/C or hydrolysis in the presence of a base, such as NaOH or LiOH (when R¹⁸ is C₁₋₆alkyl). 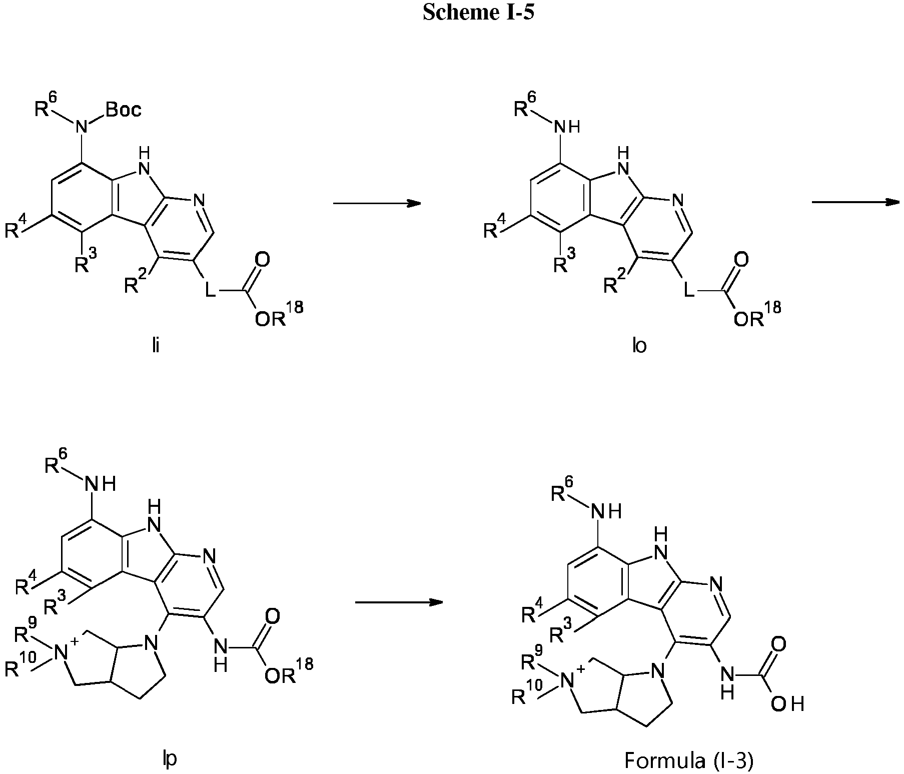 wherein R² is 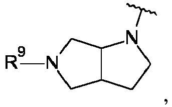 R¹⁸ is C₁₋₆alkyl or Bn.

In another embodiment, compound of formula (I-3) can be prepared according to **Scheme I-5.** Deprotection of the compound of formula (Ii) affords the compound of formula (Io). Reaction of the compound of formula (Io) with halide such as di-*tert*-butyl chloromethyl phosphate or 4-(bromomethyl)-5-methyl-1,3-dioxol-2-one, in the presence of a base such as KOH, a phase transfer catalyst such as tetraethylammonium iodide gives the compound of formula (Ip) of a quaternary ammonium salt. The compound of formula (I) can then be obtained by converting the ester of compound of formula (Ip) into a carboxylic acid via hydrogenation (when R¹⁸ is Bn) in the presence of Pd/C or hydrolysis in the presence of a base, such as NaOH or LiOH (when R¹⁸ is C₁₋₆alkyl). 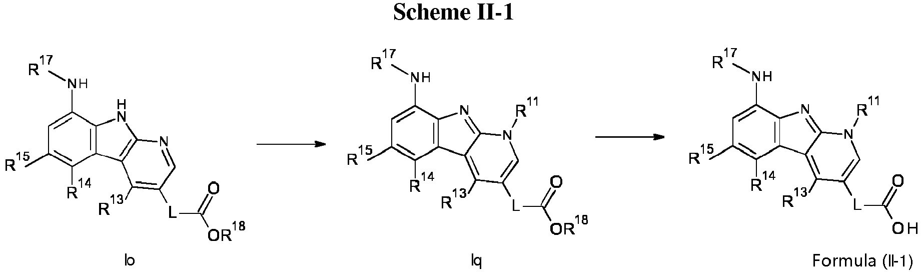 wherein L is unsubstituted or substituted 4-oxo-1,8-naphthyridinyl, or 4-oxoquinolizinyl; R¹⁸ is C₁₋₆alkyl or Bn.

In another embodiment, the compound of formula (II-1) can be prepared according to **Scheme II-1.** The compound of formula (Io) reacts with a halide such as di-*tert*-butyl chloromethyl phosphate, in the presence of a base such as Cs₂CO₃, in a solvent such as NMP to give the compound of formula (Iq). The compound of formula (II-1) can then be obtained by converting the ester of compound of formula (Iq) into a carboxylic acid via hydrogenation (when R¹⁸ is Bn) in the presence of Pd/C or hydrolysis in the presence of a base, such as NaOH or LiOH (when R¹⁸ is C₁₋₆alkyl).

This invention also relates to a process for the preparation of a compound of formula (I) or (II) comprising any of the following steps:
a) the coupling reaction of compound of formula (Ij), 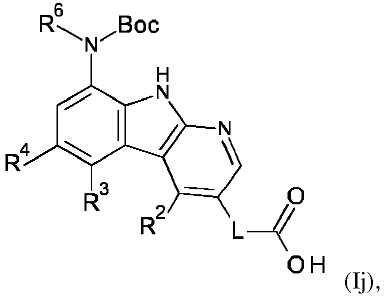 with an alcohol or amine in the presence of a coupling reagent followed by the deprotection of Boc group with an acid;
b) reaction of compound of formula (Ik), 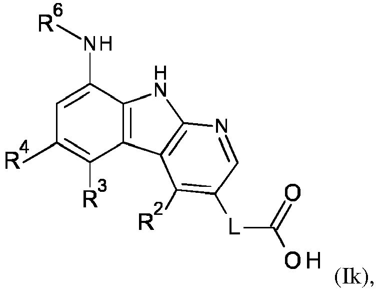 with acylating or sulfonylating reagents;
c) hydrolysis or hydrogentation of compound of formula (In), 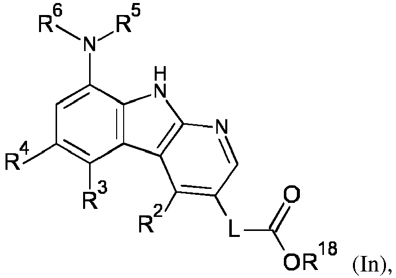 in the presence of a base or a catalyst;
d) hydrolysis or hydrogentation of compound of formula (Ip), 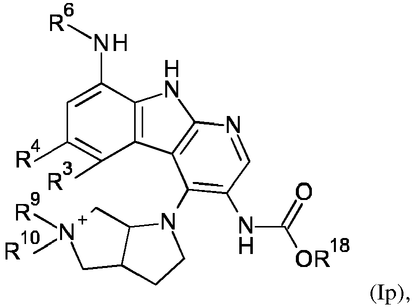 in the presence of a base or a catalyst;
e) hydrolysis or hydrogentation of compound of formula (Iq), 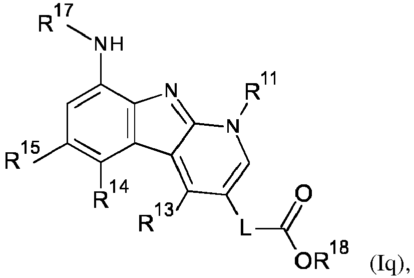
   in the presence of a base or a catalyst;
   wherein R¹ to R¹⁷ are defined above; L is unsubstituted or substituted 4-oxo-1,8-naphthyridinyl, or 4-oxoquinolizinyl; R¹⁸ is C₁₋₆alkyl or Bn;
   in step a), the coupling reagent can be, for example, HATU; the acid can be, for example, TFA;
   in step b), the acylating reagent can be, for example, formic acid; the sulfonylating reagent can be, for example, sulfur trioxide;
   in step c), d) and e), the base can be, for example, NaOH and LiOH; the catalyst can be, for example, Pd/C.

A compound of formula (I) or (II) when manufactured according to the above process is also an object of the invention.

### PHARMACEUTICAL COMPOSITIONS AND ADMINISTRATION

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) or (II) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) or (II) is formulated in an acetate buffer, at pH 5. In another embodiment, the compounds of formula (I) or (II) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to reduced bacterial load or improve host survival through the inhibition of bacterial DNA gyrase and/or Topoisomerase IV. For example, such amount may be below the amount that is toxic to normal cells, or the mammal as a whole.

In one example, the pharmaceutically effective amount of the compound of the invention administered parenterally per dose will be in the range of about 0.1 to 1000 mg/kg, alternatively about 1 to 100 mg/kg of patient body weight per day. In another embodiment, oral unit dosage forms, such as tablets and capsules, preferably contain from about 5 to about 5000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 10 to 500 mg of the compound of the invention compounded with about 40 to 400mg anhydrous lactose, about 5 to 50 mg sodium croscarmellose, about 5 to 50 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 1000 mg) of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of formula (I) or (II) , or a stereoisomer or pharmaceutically acceptable salt thereof. In a further embodiment includes a pharmaceutical composition comprising a compound of formula (I) or (II) , or a stereoisomer or pharmaceutically acceptable salt thereof, together with a pharmaceutically acceptable carrier or excipient.

Another embodiment includes a pharmaceutical composition comprising a compound of formula (I) or (II) for use in the treatment and/or prophylaxis of bacterial infections.

In some embodiments, the compounds of this invention may be administered, as part of a single or multiple dosage regimen, orally, parenterally, by inhalation spray, topically, rectally, nasally, buccally, vaginally, or via an implanted reservoir. The term parenteral as used includes subcutaneous, intracutaneous, intravenous, intramuscular, intra-articular, intrasynovial, intrasternal, intrathecal, intralesional and intracranial injection or infusion techniques. Typically, the pharmaceutical compositions of the invention will be administered from about 1 to 5 times per day or alternatively, as a continuous infusion upon improvement of a patient's condition.

### INDICATIONS AND METHODS OF TREATMENT

The compounds of the invention are useful for treatment and/or prophylaxis of bacterial infection in humans or other animals by administering to the subject in need of a therapeutically effective amount of compound of formula (I) or (II) , or a pharmaceutically acceptable salt, or enantiomer or diastereomer thereof. The compounds and methods of the invention are particularly well suited for human patients infected by pathogens that include *Staphylococcus aureus, Escherichia coli, Klebsiella pneumoniae, Acinetobacter baumannii* and *Pseudomonas aeruginosa.* Examples of bacterial organisms that may also be controlled by the compounds of the invention include, but not limited to, the following Gram-Positive and Gram-Negative organisms: *Streptococcus pneumoniae, Streptococcus pyogenes, Enterococcus faecalis, Enterococcus faecium, Enterobacter spp.* species, *Proteus spp.* species, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides spp.* species *Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii, E. coli and Mycobacterium ulcerans.*

Examples of bacterial infections may include, but not limited to, upper respiratory infections, lower respiratory infections, ear infections, pleuropulmonary and bronchial infections, complicated urinary tract infections, uncomplicated urinary tract infections, intra-abdominal infections, cardiovascular infections, a blood stream infection, sepsis, bacteremia, CNS infections, skin and soft tissue infections, GI infections, bone and joint infections, genital infections, eye infections, or granulomatous infections. Examples of specific bacterial infections include, but not limited to, uncomplicated skin and skin structure infections (uSSSI), complicated skin and skin structure infections (cSSSI), catheter infections, pharyngitis, sinusitis, otitis extema, otitis media, bronchitis, empyema, pneumonia, community-acquired bacterial pneumoniae (CABP), hospital-acquired pneumonia (HAP), hospital-acquired bacterial pneumonia, ventilator-associated pneumonia (VAP), diabetic foot infections, vancomycin resistant enterococci infections, cystitis and pyelonephritis, renal calculi, prostatitis, peritonitis, complicated intra-abdominal infections (cIAI) and other inter-abdominal infections, dialysis-associated peritonitis, visceral abscesses, endocarditis, myocarditis, pericarditis, transfusion-associated sepsis, meningitis, encephalitis, brain abscess, osteomyelitis, arthritis, genital ulcers, urethritis, vaginitis, cervicitis, gingivitis, conjunctivitis, keratitis, endophthalmitisa, an infection in cystic fibrosis patients or an infection of febrile neutropenic patients.

Furthermore, the invention relates to the use of a compound of formula (I) or (II) for the treatment and/or prophylaxis of bacterial infection. The invention relates to the use of a compound of formula (I) or (II) for the preparation of a medicament for the treatment and/or prophylaxis of bacterial infection. Another embodiment includes a method for the treatment or prophylaxis of bacterial infection which method comprises administering an effective amount of a compound of formula (I) or (II), or pharmaceutically acceptable salt thereof.

### EXAMPLES

The invention will be more fully understood by reference to the following examples. They should not, however, be construed as limiting the scope of the invention.

### ABBREVIATIONS

Abbreviations used herein are as follows:
- ACN or MeCN: acetonitrile
- AcOK: potassium acetate
- [α]D₂₀: optical rotation at 20 degrees Celsius
- B₂Pin₂: bis(pinacolato)diboron
- BINAP: 2,2' -bis(diphenylphosphino) -1,1' -binaphthalene
- Boc₂O: di-*tert*-butyl dicarbonate
- CC₅₀: concentration results in the death of 50 percent of the cells (To remove?)

- DBU: 1,8-diazabicyclo[5.4.0]undec-7-ene
- DIC: N,N'-diisopropylcarbodiimide
- DIPA: diisopropylamine
- DIPEA: N,N-diisopropylethylamine
- DMA: dimethylacetamide
- DMAP: 4-dimethylaminopyridine
- EDCI: 1-ethyl-3-(3-dimethylaminopropyl)carbodiimide
- EtOAc: ethyl acetate
- Ex: example
- h: hour
- HATU:: 1 - [bis(dimethylamino)methylene] -1H-1,2,3-triazolo [4,5 -b]pyridinium 3-oxid hexafluorophosphate
- HPLC:: high performance liquid chromatography
- HPLC-UV:: high performance liquid chromatography with ultraviolet detector
- IPA: isopropyl alcohol
- IV: intravenous
- LAH: lithium aluminum hydride
- LC-MS: liquid chromatography-mass spectrometry
- m-CPBA: 3-chloroperoxybenzoic acid
- NBS: N-bromosuccinimide
- NMP: N-methyl-2-pyrrolidone
- OD: optical density
- Pd-Ad₂nBuP: biphenyl chloro[(di(1-adamantyl)-N-butylphosphine)-2-(2-aminobiphenyl)]palladium(II)
- Pd₂(dba)₃: tris(dibenzylideneacetone)dipalladium(0)
- PPA: polyphosphoric Acid
- prep-HPLC: preparative high performance liquid chromatography
- r.t.: room temperature
- Rt: retention time
- SFC: supercritical fluid chromatography
- TBME: methyl *tert*-butyl ether
- TEA: triethylamine
- TFA: trifluoroacetic acid
- TsOH: p-toluenesulfonic acid
- TLC: Thin Layer Chromatography
- UV: ultraviolet detector
- x-phos: 2-dicyclohexylphosphino-2',4',6'-triisopropylbiphenyl
- wt: weight

### GENERAL EXPERIMENTAL CONDITIONS

Intermediates and final compounds were purified by flash chromatography using one of the following instruments: i) Biotage SP1 system and the Quad 12/25 Cartridge module, ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

Chiral Separation was conducted on Thar 350 preparative SFC using ChiralPak AD-10µ (200 × 50 mm I.D.) with mobile phase A for CO₂ and B for ethanol. LC/MS spectra were obtained using a Waters UPLC-SQD Mass. Standard LC/MS conditions were as follows (running time: 3 minutes):
Acidic condition: A: 0.1% formic acid (or 0.1% TFA) and 1% acetonitrile in H₂O; B: 0.1% formic acid (or 0.1% TFA) in acetonitrile;
Basic condition: A: 0.05% NH₃·H₂O in H₂O; B: acetonitrile;
Neutral condition: A: 0.1% NH₄HCO₃ in H₂O; B: acetonitrile.
Mass spectra (MS): generally only ions which indicate the parent mass are reported, and unless otherwise stated the mass ion quoted is the positive mass ion (M+H)⁺.
NMR Spectra were obtained using Bruker Avance 400MHz.

All reactions involving air-sensitive reagents were performed under an nitrogen/or argon atmosphere. Reagents were used as received from commercial suppliers without further purification unless otherwise noted. In this specification the following non-SI unit is used: pound/square inch (psi): Conversion factor into SI unit (Pa): 6.894757x103.

### PREPARATIVE EXAMPLES

### Intermediate A1

### tert-Butyl N-(3,4-dichloro-5,6-difluoro-9H-pyrido[2,3-b]indol-8-yl)-N-methyl-carbamate

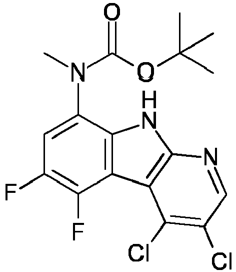

The title compound was synthesized according to the following scheme:

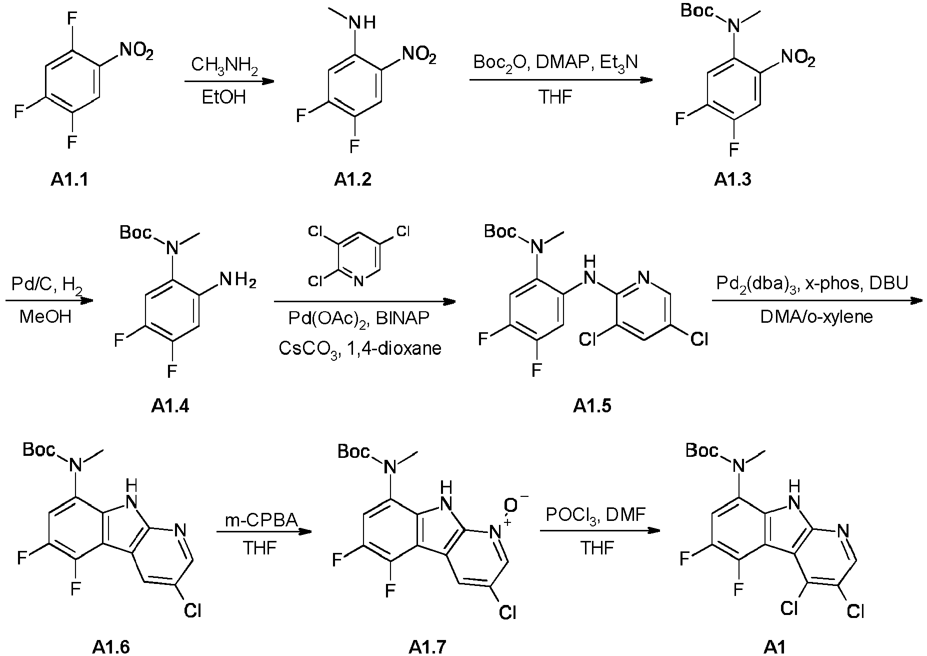

### Step (a) Preparation of 4,5-difluoro-N-methyl-2-nitro-aniline (compound A1.2)

To a solution of 1,2,4-trifluoro-5-nitro-benzene (1.0 kg, 5.65 mol) in EtOH (2.0 L) was added methylamine solution (1.06 kg, 11.29 mmol, 33% in EtOH) dropwise at 0 °C over 3 h. After completion of the addition, the reaction mixture was stirred at 0 °C for additional1 h before poured into ice-water (15.0 L). The resulting precipitates were collected by filtration, and resuspended in petroleum ether / EtOAc (10.0 L, v/v=4:1). The by-products were removed by filtration and the filtrate was dried over anhy. Na₂SO₄, filtered, and concentrated *in vacuo* to give a crude product. It was then purified by silica gel flash chromatography (petroleum ether:EtOAc = 100:1∼20:1) to give 4,5-difluoro-N-methyl-2-nitro-aniline(250.0 g, 23.5% yield) as a yellow solid. MS (ESI): 189.1 ([M+H]⁺).

### Step (b) Preparation of tert-butyl N-(4,5-difluoro-2-nitro-phenyl)-N-methyl-carbamate (compound A1.3)

To a mixture solution of 4,5-difluoro-N-methyl-2-nitro-aniline (250.0 g, 1.33 mol), BoczO (580.0 g, 2.66 mol), and Et₃N (403.0 g, 3.99 mol) in THF (1.0 L) was added DMAP (32.5 g, 0.266 mmol) and the resulting reaction mixture was stirred at 60 °C for 3 h. After TLC (petroleum ether:EtOAc = 10:1) showed the reaction was completed, MeOH (100.0 mL) was added. The mixture solution was concentrated *in vacuo* to give a crude product, which was purified by silica gel flash chromatography (petroleum ether:EtOAc = 100:1∼10:1) to give *tert-*butyl N-(4,5-difluoro-2-nitro-phenyl)-N-methyl-carbamate (305.0 g, 79.6% yield) as a white solid. MS (ESI): 189.1 ([M-Boc+H]⁺), 233.1 ([M-*^{t}*Bu+H]⁺), 311.1 ([M+Na]⁺).

### Step (c) Preparation of tert-butyl N-(2-amino-4,5-difluoro-phenyl)-N-methyl-carbamate (compound A1.4)

To a solution of *tert*-butyl N-(4,5-difluoro-2-nitro-phenyl)-N-methyl-carbamate (104.0 g, 360.8 mmol) in MeOH (1.2 L) was added Pd/C (10.0 g, 50% H₂O) under N₂. After the suspension was degassed under vacuum and purged with H₂ several times, the reaction mixture was stirred at 30 °C for 18 h under H₂ (50 psi). The reaction mixture was filtered through Celite and the filterate was concentrated *in vacuo* to give a crude product of *tert*-butyl N-(2-amino-4,5-difluoro-phenyl)-N-methyl-carbamate (92.0 g, 98.7% yield) as a white solid. MS (ESI): 159.1 ([M-Boc+H]⁺), 203.1 ([M-*^{t}*Bu+H]⁺), 281.1 ([M+Na]⁺). It was used directly in the next step without further purification.

### Step (d) Preparation of tert-butyl N-[2-[(3,5-dichloro-2-pyridyl)aminol-4,5-difluoro-phenyl]-N-methyl-carbamate (compound A1.5)

To a solution of *tert*-butyl N-(2-amino-4,5-difluoro-phenyl)-N-methyl-carbamate (90.0 g, 348.5 mmol) and 2,3,5-trichloropyridine (64.2 g, 352.0 mmol) in 1,4-dioxane (1.5 L) was added Cs₂CO₃ (227.0 g, 697.0 mmol). The mixture was degassed with nitrogen for 2 min before Pd(OAc)₂ (7.82 g, 34.85 mmol) and BINAP (32.55 g, 52.28 mmol, CAS: 98327-87-8) were added under nitrogen. The resulting suspension was heated to 110 °C and stirred for 3 h. After TLC (petrolieum ether:EtOAc =10 : 1) showed the reaction was completed, the mixture was cooled back to room temperature and diluted with EtOAc (1 L) and filtered. The filtrate was concentrated *in vacuo* to give a crude product, which was purified by silica gel flash chromatography (petroleum ether:EtOAc = 500:1~20:1) to give *tert*-butyl N-[2-[(3,5-dichloro-2-pyridyl)amino]-4,5-difluoro-phenyl]-N-methyl-carbamate (124.0 g, 88.0% yield) as a white solid. MS (ESI): 404.0 ([{³⁵Cl}M+H]⁺), 406.0 ([{³⁷Cl}M+H]⁺), 348.0 ([{³⁵Cl}[M-*^{t}*Bu+H]⁺), 350.0 ([{³⁷Cl}M-*^{t}*Bu+H]⁺).

### Step (e) Preparation of tert-butyl N-(3-chloro-5,6-difluoro-9H-pyrido[2,3-b]indol-8-yl)-N-methyl-carbamate (compound A1.6)

A mixture of *tert*-butyl N-[2-[(3,5-dichloro-2-pyridyl)amino]-4,5-difluoro-phenyl]-N-methyl-carbamate (123.0 g, 304.3 mmol), Pd₂(dba)₃ (55.73 g, 60.86 mmol, CAS: 51364-51-3), x-phos (58.03 g, 121.72 mmol, CAS: 564483-18-7) and DBU (92.7 g, 608.6 mmol, CAS: 6674-22-2) were dissolved in o-xylene / DMA (v/v=1:1, 220.0 mL). After being degassed with nitrogen for 5 min, the reaction mixture was heated at 130 °C for 1 h. After TLC (petrolieum ether:EtOAc = 5:1) showed the reaction was completed, the reaction mixture was cooled back to room temperature and diluted with EtOAc (800 mL). The mixture was filtered and the filtrate was poured into water (500 mL) and extracted with EtOAc (1.2 L) three times. Combined organics were washed with aq. CaClz solution (1N, 400 mL) three times, brine (400 mL) twice, dried over anhy. Na₂SO₄ and concentrated *in vacuo* to give a crude product, which was recrystallized in MeOH (800 mL) to give *tert*-butyl N-(3-chloro-5,6-difluoro-9H-pyrido[2,3-b]indol-8-yl)-N- methyl-carbamate (68.0 g, 60.8% yield) as a white solid. MS (ESI): 368.1 ([{³⁵Cl}M+H]⁺), 370.1([{³⁷Cl}M+H]⁺).

### Step (f) Preparation of tert-butyl N-(3-chloro-5,6-difluoro-1-oxido-9H-pyrido[2,3-b]indol-1ium-8-yl)-N-methyl-carbamate (compound A1.7)

To a solution of *tert*-butyl N-(3-chloro-5,6-difluoro-9H-pyrido[2,3-b]indol-8-yl)-N-methyl-carbamate (32.0 g, 87.0 mmol) in THF (400.0 mL) was added *m*-CPBA (26.5 g, 130.5 mmol), and the resulting reaction mixture was stirred at 50 °C for 2 h. After TLC (DCM:EtOH = 40:1) showed the reaction was completed, the mixture was cooled back to room temperature, poured into aq. Na₂SO₃ solution (3.0 L, 10%) and stirred at room temperature for 1 h. The resulting precipitate was then collected by filtration, washed with aq. Na₂SO₃ solution (1.0 L, 5%), and dried under vacuum to give *tert*-butyl N-(3-chloro-5,6-difluoro-1-oxido-9H-pyrido[2,3-b]indol-1ium-8-yl)-N-methyl-carbamate (32.0 g, 95.8% yield) as a yellow solid. MS (ESI): 384.1 ([{³⁵Cl}M+H]⁺), 386.0 ([{³⁷Cl}M+H]⁺).

### Step (g) Preparation of tert-Butyl N-(3,4-dichloro-5,6-difluoro-9H-pyrido[2,3-b]indol-8-vl)-N-methyl-carbamate (Intermediate A1)

To a stirred solution of *tert*-butyl N-(3-chloro-5,6-difluoro-1-oxido-9H-pyrido[2,3-b]indol-1ium-8-yl)-N-methyl-carbamate (60.0 g, 0.156 mol) in DMF / THF (2.0 L, v/v = 1:1) was added POCl₃ (267.42 g, 1.744 mol) dropwise under ice-salt bath. The resulting mixture was stirred at -5 °C ~ 0 °C for 3 h before poured into satd. aq. solution of Na₂CO₃ (3.0 L) at 0 °C and extracted with EtOAc (1.0 L) four times. Combinded organics was then washed with satd. aq. Na₂CO₃ solution (500 mL) twice, aq. CaClz solution (1N, 500 mL) four times and brine (300 mL) twice. The separated organic layer was dried over anhy. Na₂SO₄ and concentrated *in vacuo* to give a crude product, which was recrystallized in MeOH (600 mL) to give *tert*-Butyl N-(3,4-dichloro-5,6-difluoro-9H-pyrido[2,3-b]indol-8-yl)-N-methyl-carbamate (57.3 g, 91.3% yield) as a white solid. ¹H NMR (400 MHz, DMSO-d6) δ: 12.624 (br. s., 1 H), 8.652 (s, 1 H), 7.572~7.629 (dd, J=11.6, 6.8 Hz, 1 H), 3.247 (s, 3 H), 1.343 (s, 9 H). MS (ESI): 402.1 ([{³⁵Cl} M+H]⁺), 404.1 ([{³⁷Cl} M+H]⁺).

### Intermediate A2

### tert-Butyl N-(3-bromo-4-chloro-5,6-difluoro-9H-pyrido[2,3-b]indol-8-yl)-N-methyl-carbamate

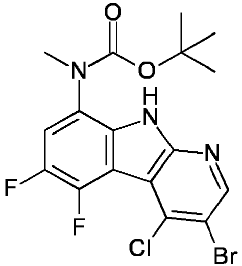

The title compound was synthesized according to the following scheme:

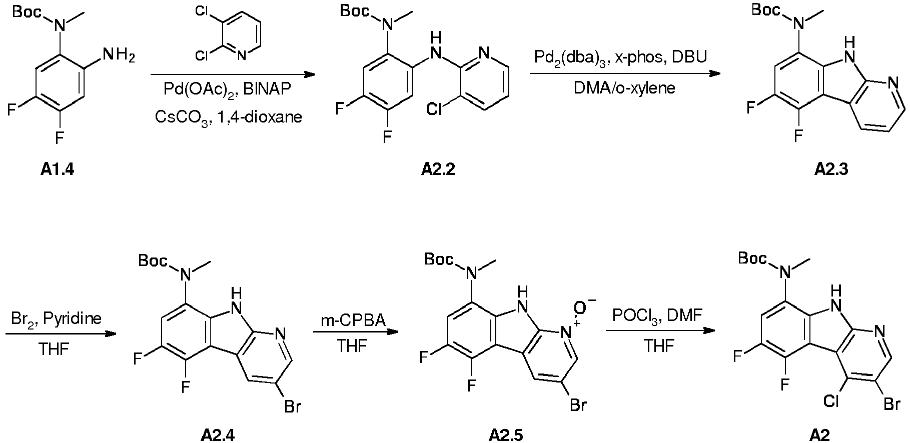

### Step (a) Preparation of tert-butyl N-[2-[(3-chloro-2-pyridyl)amino]-4,5-difluoro-phenyl]-N-methyl-carbamate (compound A2.2)

To a stirred solution of *tert*-butyl N-(2-amino-4,5-difluoro-phenyl)-N-methyl-carbamate (142.0 g, 0.55 mol, compound A1.4) and 2,3-dichloropyridine (81.4 g, 0.55 mol) in dioxane (1.5 L) was added cesium carbonate (358.6 g, 1.10 mol). The mixture was degassed with nitrogen for 2 min before palladium(II) acetate (12.3 g, 0.055 mol) and BINAP (68.5 g, 0.110 mmol, CAS: 98327-87-8) were added under nitrogen. The resulting suspension was then heated to 110°C and stirred for 3 h. The reaction mixture was then cooled back to r.t., diluted with EtOAc (1.0 L) and filtered. The filtrate was concentrated *in vacuo* to give a crude product, which was purified by silica gel flash chromatography (petroleum ether:EtOAc = 500:1∼20:1) to give *tert-butyl* N-[2-[(3-chloro-2-pyridyl)amino]-4,5-difluoro-phenyl]-N-methyl-carbamate (175.0 g, 86.1% yield) as a white solid. MS (ESI): 370.1 ([{³⁵Cl}M+H]⁺), 372.1 ([{³⁷Cl}M+H]⁺).

### Step (b) Preparation of tert-butyl N-(5,6-difluoro-9H-pyrido[2,3-b]indol-8-yl)-N-methyl-carbamate (compound A2.3)

*tert*-Butyl N-[2-[(3-chloro-2-pyridyl)amino]-4,5-difluoro-phenyl]-N-methyl-carbamate (175.0g, 473.24 mmol), Pd₂(dba)₃ (65.0 g, 71.0 mmol, CAS: 51364-51-3), x-phos (67.7 g, 142.0 mmol, CAS: 564483-18-7), and DBU (144 g, 946.5 mmol, CAS: 6674-22-2) were dissolved in a mixture solution of o-xylene / DMA (v/v=1:1, 260.0 mL). After the mixture solution was degassed with nitrogen for 5 min, it was heated at 130°C for 3 h before cooled back to r.t. and diluted with EtOAc (500 mL). The mixture was filtered to remove part of catalyst and ligand, and the filtrate was poured into water (1.0 L) and extracted with EtOAc (2.0 L) three times. Combined organics was then washed with aq. Ca₂Cl₂ solution (1N, 500 mL) four times, brine (500 mL) twice, dried over anhy. Na₂SO₄, filtered, and concentrated *in vacuo* to give a crude product. It was then recrystallized in MeOH (800 mL) to give *tert*-butyl N-(5,6-difluoro-9H-pyrido[2,3-b]indol-8-yl)-N-methyl-carbamate (110 g, 69.7% yield) as a white solid. The mother liquid was purified by silica gel flash chromatography (petroleum ether: EtOAc = 50:1~1:1) to give additional *tert*-butyl N-(5,6-difluoro-9H-pyrido[2,3-b]indol-8-yl)-N-methyl-carbamate (35.0 g, 22.2% yield) as a white solid. In total, 145g of compound A2.3 (91.9% yield) was obtained. MS (ESI): 334.1 ([M+H]⁺).

### Step (c) Preparation of tert-butyl N-(3-bromo-5,6-difluoro-9H-pyrido[2,3-b]indol-8-yl)-N-methyl-carbamate (compound A2.4)

To a stirred solution of *tert*-butyl N-(5,6-difluoro-9H-pyrido[2,3-b]indol-8-yl)-N-methyl-carbamate (140.0 g, 0.420 mol) and pyridine (166.1 g, 2.1 mol) in THF (2.0 L) was added bromine (335.6 g, 2.1 mol) at -60 °C. After the addition, the mixture was warmed up and stirred at 15 °C for 2 h. After LC-MS showed the reaction was completed, the mixture was added dropwise into aq. sodium sulfite solution (5.0 L, 10%) and adjusted to pH = 8.0 with aq. sodium carbonate solution at 0 °C and stirred at 25 °C for additional 1 h. The mixture was then extracted by EtOAc (2.0 L) three times. Combined organics was then washed with aq. sodium carbonate solution (1.0 L) twice and brine (1.0 L) three times. Separated organic layer was dried over anhy. Na₂SO₄, filtered, and concentrated *in vacuo* to give a crude product, which was recrystallized in MeOH (1.0 L) to give *tert*-butyl N-(3-bromo-5,6-difluoro-9H-pyrido[2,3-b]indol-8-yl)-N-methyl-carbamate (150.0 g, 86.6% yield) as a yellow solid. MS (ESI): 412.1 ([{⁷⁹Br}M+H]⁺), 414.1 ([{⁸¹Br}M+H]⁺).

### Step (d) Preparation of tert-butyl N-(3-bromo-5,6-difluoro-1-oxido-9H-pyrido[2,3-b]indol-1-ium-8-yl)-N-methyl-carbamate (compound A2.5)

To a stirred solution of *tert*-butyl N-(3-bromo-5,6-difluoro-9H-pyrido[2,3-b]indol-8-yl)-N-methyl-carbamate (150.0 g, 363.9 mmol) in THF (1.2 L) was added 3-chlorobenzoperoxoic acid (148.0 g, 727.8 mmol, 85% wt). The mixture was degassed with nitrogen for 2 min before stirred at 50 °C for 2 h. After TLC (petroleum ether:EtOAc = 2:1) showed the starting material was consumed completely, the reaction was cooled back to r.t. and the mixture was poured into aq. sodium sulfite solution (6.0 L, 10%) and stirred for 1 h. The resulting precipitate was collected by filtration and washed by aq. sodium sulfite solution (1.0 L, 5%). The filter cake was evaporated to dryness under the reduced pressure to give *tert-butyl* N-(3-bromo-5,6-difluoro-1-oxido-9H-pyrido[2,3-b]indol-1-ium-8-yl)-N-methyl-carbamate (150.0 g, 96.3% yield) as a yellow solid. MS (ESI): 428.1 ([{⁷⁹Br}M+H]⁺), 430.1 ([{⁸¹Br}M+H]⁺).

### Step (e) Preparation of tert-Butyl N-(3-bromo-4-chloro-5,6-difluoro-9H-pyrido[2,3-b]indol-8-yl)-N-methyl-carbamate (Intermediate A2)

To a stirred solution of *tert*-butyl N-(3-bromo-5,6-difluoro-1-oxido-9H-pyrido[2,3-b]indol-1-ium-8-yl)-N-methyl-carbamate (150 g, 0.35 mol) in DMF / THF (2.5 L, v/v = 1:1) was added phosphorus(V) oxychloride (421.3 g, 2.75 mol) dropwise under ice-salt bath. After the addition, the mixture was stirred at -5 °C~ 0 °C for 4 h until TLC (petroleum ether: EtOAc = 2:1) showed the starting material was consumed completely. The mixture was then poured into satd. aq. solution of sodium carbonate (5.0 L) at 0 °C and extracted with EtOAc (3.0 L) twice. Combined organics was then washed with satd. aq. solution of Na₂CO₃ (1.0 L) twice, aq. CaClz solution (1N, 1.5 L) four times, and brine (1.0 L) twice. The organic layer was dried over anhy. Na₂SO₄, filtered, and concentrated *in vacuo* to give a crude product, which was recrystallized in MeOH (1.2 L) to give *tert*-Butyl N-(3-bromo-4-chloro-5,6-difluoro-9H-pyrido[2,3-b]indol-8-yl)-N-methyl-carbamate (142.0 g, 90.8% yield) as a white solid. ¹H NMR (400 MHz, DMSO-d6) δ: 12.85 (br. s., 1 H), 8.75 (s, 1 H), 7.69 ~ 7.74 (dd, *J*=11.6, 6.8 Hz, 1 H), 3.22 (s, 3 H), 1.22 ~ 1.50 (m, 9 H). MS (ESI): 446.0 ([{⁷⁹Br} M+H]⁺), 448.0 ([{⁸¹Br} M+H]⁺).

### Intermediate A3

### tert-Butyl N-(3,4-dichloro-5-cyano-6-fluoro-9H-pyrido[2,3-b]indol-8-yl)-N-methyl-carbamate

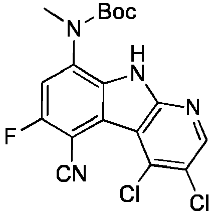

The titled compound was synthesized according to the following scheme:

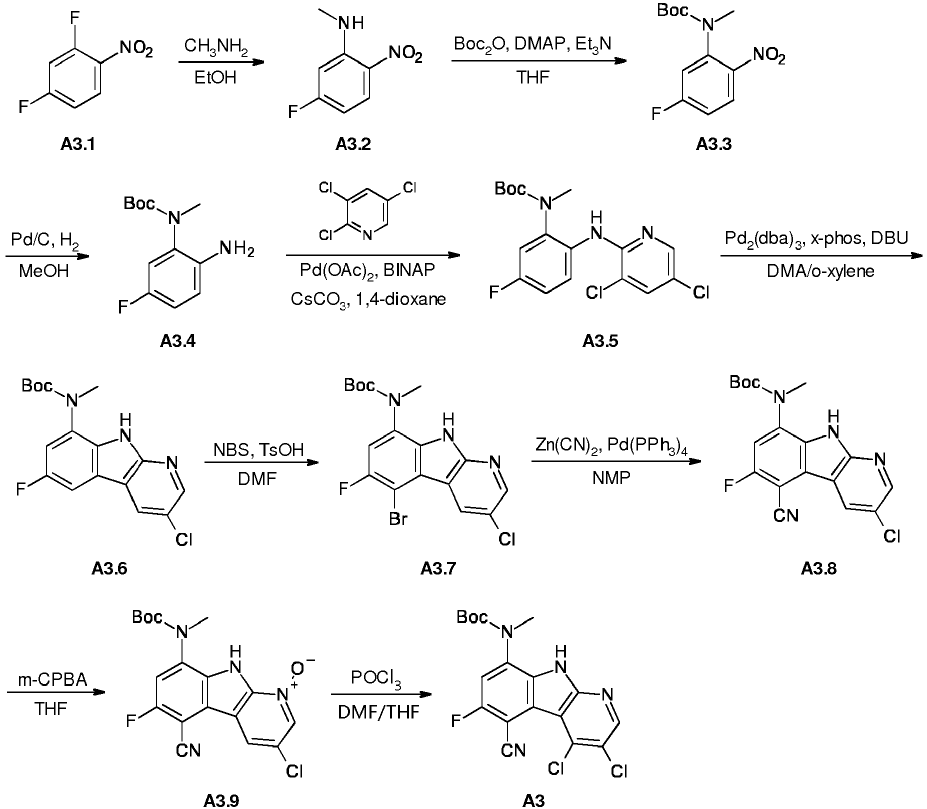

### Step (a) Preparation of 5-fluoro-N-methyl-2-nitro-aniline (compound A3.2)

To a stirred solution of 2,4-difluoro-1-nitro-benzene (130.0 g, 0.817 mol) in EtOH (500.0 mL) was added methyl amine (230.7 g, 2.45 mmol, 33% in EtOH) dropwise under ice-water bath. After completion of the addition, the reaction mixture was stirred at 0 °C for 1.5 h and then poured into ice-water (4.0 L). The resulting crystal precipitate was collected by filtration and dried under vacuum to give 5-fluoro-N-methyl-2-nitro-aniline (132.0 g, 94.9% yield) as a yellow solid. MS (ESI): 171.1 (M+H]⁺).

### Step (b) Preparation of tert-butyl N-(5-fluoro-2-nitro-phenyl)-N-methyl-carbamate (compound A3.3)

To a mixture solution of 5-fluoro-N-methyl-2-nitro-aniline (132.0 g, 0.776 mol), BoczO (338.0 g, 1.551 mol) and DIPEA (200.5 g, 1.551 mol) in THF (1.0 L) was added DMAP (19.0 g, 0.155 mmol) and the resulting reaction mixture was stirred at 70 °C for 12 h. After TLC (petrolieum ether:EtOAc = 20 : 1) showed the starting material was consumed, the reaction mixture was concentrated *in vacuo* to give a crude product, which was purified by silica gel flash chromatography (petroleum ether:EtOAc = 100:1 ~ 10:1) to give *tert*-butyl N-(5-fluoro-2-nitrophenyl)-N-methyl-carbamate (190.0 g, 90.6% yield) as a white solid. MS (ESI): 171.1 ([M-Boc+H]⁺).

### Step (c) Preparation of tert-butyl N-(2-amino-5-fluoro-phenyl)-N-methyl-carbamate (compound A3.4)

To a solution of *tert*-butyl N-(5-fluoro-2-nitro-phenyl)-N-methyl-carbamate (190.0 g, 0.703 mol) in MeOH (2 L) was added Pd/C (18.0 g, 50% H₂O) under N₂. After the suspension was degassed under vacuum and purged with H₂ several times, the reaction mixture was stirred at 40 °C for 5 h under H₂ atmosphere (50 psi). After TLC (petrolieum ether:EtOAc = 5 : 1) showed the reaction was completed, the reaction mixture was cooled back to r.t. and filtered through Celite to remove catalyst. The filtrate was then concentrated under *in vacuo* to give a crude product of *tert*-butyl N-(2-amino-5-fluoro-phenyl)-N-methyl-carbamate (162.0 g, 95.9% yield) as a white solid. MS (ESI): 141.2 ([M-Boc+H]⁺), 185.1 ([M-*^{t}*Bu+H]⁺). It was used directly in the next step without further purification.

### Step (d) Preparation of tert-butyl N-[2-[(3,5-dichloro-2-pyridyl)amino]-5-fluoro-phenyl]-N-methyl-carbamate (compound A3.5)

To a solution of *tert-butyl* N-(2-amino-5-fluoro-phenyl)-N-methyl-carbamate (142.0 g, 0.591 mol) and 2,3,5-trichloropyridine (108.0 g, 0.591 mol) in dioxane (2.0 L) was added Cs₂CO₃ (385.0 g, 1.18 mol). The mixture was degassed with nitrogen for 2 min before Pd(OAc)₂ (13.3 g, 0.059 mol) and BINAP (73.5 g, 0.118 mol) were added under nitrogen. After the charging was completed, the suspension was heated to 110 °C and stirred for 3 h. After TLC (petrolieum ether:EtOAc = 20 : 1) showed the reaction was completed, the reaction mixture was cooled back to r.t., diluted with EtOAc (3.0 L) and filtered. The filtrate was concentrated *in vacuo* to give a crude product, which was purified by silica gel flash chromatography (petroleum ether:EtOAc = 500 : 1 ~ 20 : 1) to give *tert*-butyl N-[2-[(3,5-dichloro-2-pyridyl)amino]-5-fluorophenyl]-N-methyl-carbamate (190.0 g, 83.2% yield) as a white solid. MS (ESI): 385.9 ([M{³⁵Cl}+H]⁺), 329.9 ([M{³⁵Cl}-*^{t}*Bu+H]⁺), 331.9 ([M{³⁷Cl}-*^{t}*Bu+H]⁺).

### Step (e) Preparation of tert-butyl N-(3-chloro-6-fluoro-9H-pyridor2,3-blindol-8-yl)-N-methyl-carbamate (compound A3.6)

A mixture of *tert*-butyl N-[2-[(3,5-dichloro-2-pyridyl)amino]-5-fluoro-phenyl]-N-methyl-carbamate (184.0 g, 476.4 mmol), Pd₂(dba)₃ (87.3 g, 95.3 mmol), x-phos (90.8 g, 190.6 mmol) and DBU (145.0 g, 953.0 mmol) were dissolved in a mixture of o-xylene / DMA (v/v = 1:1, 300.0 mL). After the mixture was degassed with nitrogen for 5 min, it was heated at 130 °C for 2 h. The mixture was then cooled back to r.t., diluted with EtOAc (2.0 L), and filtered. The filtrate was poured into water (1.0 L) and extracted with EtOAc (1.0 L) three times. Combined organics was washed with aq. CaClz solution (1N, 500 mL) three times, and brine (500 mL) twice. The separated organic layer was dried over anhy. Na₂SO₄, filtered, and concentrated *in vacuo* to give a crude product, which was recrystallized in MeOH (1.5 L) to give *tert*-butyl N-(3-chloro-6-fluoro-9H-pyrido[2,3-b]indol-8-yl)-N-methyl-carbamate (93.0, 55.8% yield) as a yellow solid. MS (ESI): 350.2 ([M{³⁵Cl}+H]⁺).

### Step (f) Preparation of tert-butyl N-(5-bromo-3-chloro-6-fluoro-9H-pyrido[2,3-b]indol-8-yl)-N-methyl-carbamate (compound A3.7)

A suspension of *tert*-butyl N-(3-chloro-6-fluoro-9H-pyrido[2,3-b]indol-8-yl)-N-methyl-carbamate (88.0 g, 251.6 mmol) in DMF (700.0 mL) was heated to 100 °C for full dissolvation. Afterwards, the mixture was cooled back to r.t. and added NBS (53.8 g, 302.0 mmol) and TsOH•H₂O (7.2 g, 37.7 mmol). The resulting mixture solution was then re-heated to 45 °C and stirred for 2 h. After LC-MS indicated the starting material was completely consumed, the mixture was cooled back to r.t., poured into aq. Na₂SO₃ solution (4.0 L, 10%) and stirred at r.t. for another 0.5 h. The resulting crystal precipitate was then collected by filtration and dried under vacuum to give a crude product of *tert*-butyl N-(5-bromo-3-chloro-6-fluoro-9H-pyrido[2,3-b]indol-8-yl)-N-methyl-carbamate (96.0 g, 89.0% yield) as a yellow solid. MS (ESI): 430.1 ([M{⁷⁹Br}+H]⁺). It was used directly in the next step without further purification.

### Step (g) Preparation of tert-butyl N-(3-chloro-5-cyano-6-fluoro-9H-pyrido[2,3-b]indol-8-yl)-N-methyl-carbamate (compound A3.8)

To a stirred solution of *tert*-butyl N-(5-bromo-3-chloro-6-fluoro-9H-pyrido[2,3-b]indol-8-yl)-N-methyl-carbamate (80.0 g, 186.6 mmol) and Zn(CN)₂ (131.0 g, 1.12 mol) in NMP (300.0 mL) was added Pd(PPh₃)₄ (21.6 g, 18.7 mmol) in glovebox under argon. The resulting reaction mixture was stirred at 120 °C for 3 h under argon. After LC-MS showed the starting material was completely consumed, the reaction mixture was cooled back to r.t., diluted with EtOAc (2.5 L) and filtered. The filtrate was washed with aq. CaClz solution (400 mL) four times, and brine (400 mL) three times. The organic layer was then dried over anhy. Na₂SO₄, filtered, and concentrated *in vacuo* to give a crude product, which was recrystallized in MeOH (250 mL) to give tert-butyl N-(3-chloro-5-cyano-6-fluoro-9H-pyrido[2,3-b]indol-8-yl)-N-methyl-carbamate (74.0 g, 86.3% yield) as a yellow solid. MS (ESI): 375.2 ([M{³⁵Cl}+H]⁺), 377.2 ([M{³⁷Cl}+H]⁺).

### Step (h) Preparation of tert-butyl N-(3-chloro-5-cyano-6-fluoro-1-oxido-9H-pyrido[2,3-b]indol-1-ium-8-yl)-N-methyl-carbamate (compound A3.9)

To a solution of tert-butyl N-(3-chloro-5-cyano-6-fluoro-9H-pyrido[2,3-b]indol-8-yl)-N-methyl-carbamate (27.0 g, 72.04 mmol) in THF (150.0 mL) was added *m*-CPBA (29.3 g, 114.08 mmol, 85% wt). The reaction mixture was degassed with nitrogen for 2 min and stirred at 45 °C for 2 h. After TLC (petrolieum ether:EtOAc = 2:1) showed the starting material was consumed completely, the mixture was cooled back to r.t., poured into 10% aq. Na₂SO₃ solution (2.0 L, 10%) and stirred for 1 h. The resulting precipitate was collected by filtration and washed by aq. Na₂SO₃ solution (100 mL). The filter cake was evaporated to dryness under the reduced pressure to give *tert*-butyl N-(3-chloro-5-cyano-6-fluoro-1-oxido-9H-pyrido[2,3-b]indol-1-ium-8-yl)-N-methyl-carbamate (27.0 g, 95.9% yield) as a yellow solid. MS (ESI): 391.2 ([M{³⁵Cl}+H]⁺), 335.1 ([M{³⁵Cl}-*^{t}*Bu+H]⁺).

### Step (i) Preparation of tert-butyl N-(3,4-dichloro-5-cyano-6-fluoro-9H-pyrido[2,3-b]indol-8-yl)-N-methyl-carbamate (Intermediate A3)

To a solution of *tert*-butyl N-(3-chloro-5-cyano-6-fluoro-1-oxido-9H-pyrido[2,3-b]indol-1-ium-8-yl)-N-methyl-carbamate (27.0 g, 69.09 mmol) in DMF / THF (500.0 mL, v/v = 1:1) was added POCl₃ (15.9 g, 103.6 mmol) dropwise under ice-salt bath. The resulting reaction mixture was stirred at -40 °C~ -30 °C for 5 h. After TLC (petrolieum ether:EtOAc = 2 : 1) showed the starting material was consumed completely, the mixture was poured into satd. aq. NaHCOs solution (1.0 L) at 0 °C and subsequently extracted by EtOAc (1.0 L) twice. Combined organics was washed with aq. NaHCOs solution (300.0 mL) twice, aq. CaCh solution (300.0 mL) four times, and brine (300.0 mL) twice. The organic layer was then dried over Na₂SO₄, filtered, and concentrated *in vacuo* to give a crude product, which was recrystallized in THF / MeOH (150.0 mL, v/v=1:10) to give *tert*-butyl N-(3,4-dichloro-5-cyano-6-fluoro-9H-pyrido[2,3-b]indol-8-yl)-N-methyl-carbamate (18.0 g, 63.7% yield) as a yellow solid. MS (ESI): 409.2 ([{³⁵Cl}M+H]⁺), 411.2 ([{³⁷Cl}M+H]⁺). ¹H NMR (400 MHz, DMSO-*d*₆) δ ppm 13.15 (s, 1 H), 8.75 (s, 1 H), 7.85 (d, 7=10.8 Hz, 1 H), 3.28 (s, 3 H), 1.25 (br s, 9 H).

### Intermediate B1

### Ethyl 1-methyl-4-oxo-6-(4,4,5,5-tetramethyl-1,3,2-dioxaborolan-2-yl)-1,8-naphthyridine-3-carboxylate

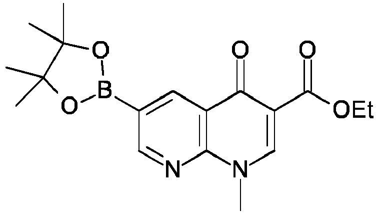

The titled compound was synthesized according to the following scheme:

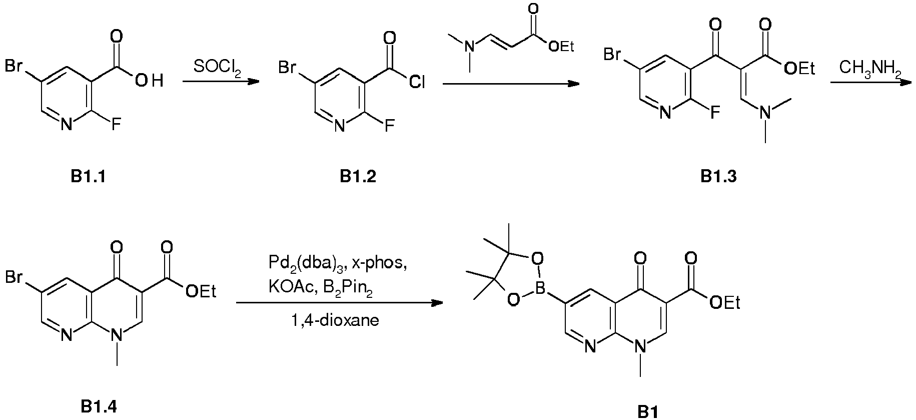

### Step (a) Preparation of 5-bromo-2-fluoro-pyridine-3-carbonyl chloride (compound B1.2)

A solution of 5-bromo-2-fluoro-pyridine-3-carboxylic acid (40.0 g, 181.82 mmol) in SOCh (200.0 mL) was stirred 80 °C for 2 h. Afterwards, the mixture solution was cooled back to r.t. and concentrated under the reduced pressure to give a crude product of 5-bromo-2-fluoro-pyridine-3-carbonyl chloride (42.0 g, 96.9% yield). It was used directly in the next step without further purification.

### Step (b) Preparation of ethyl (Z)-2-(5-bromo-2-fluoro-pyridine-3-carbonyl)-3-(dimethylamino)prop-2-enoate (compound B1.3)

To a solution of 5-bromo-2-fluoro-pyridine-3-carbonyl chloride (42.0 g, 176.14 mmol) and Et₃N (35.6 g, 352.3 mmol) in THF (150.0 mL) was added ethyl (E)-3-(dimethylamino)prop-2-enoate (30.3 g, 211.37 mmol), and the resulting reaction mixture was stirred at 60 °C for 2 h. After LC-MS showed the starting material was fully consumed, the mixture was cooled back to r.t., diluted with EtOAc (400 mL) and filtered. The filtrate was concentrated *in vacuo* to give a crude product of ethyl (Z)-2-(5-bromo-2-fluoro-pyridine-3-carbonyl)-3-(dimethylamino)prop-2-enoate (58.0 g, 95.4% yield). MS (ESI): 345.0 ([{⁷⁹Br}M+H]⁺), 347.1([{⁸¹Br}M+H]⁺). It was used directly in the next step without further purification.

### Step (c) Preparation of ethyl 6-bromo-1-methyl-4-oxo-1,8-naphthyridine-3-carboxylate (compound B1.4)

To a solution of ethyl (Z)-2-(5-bromo-2-fluoro-pyridine-3-carbonyl)-3-(dimethylamino)prop-2-enoate (58.0 g, 0.168 mol) in THF (170 mL) was added methyl amine (800.0 mL, 1.6 mol, 2M in THF), and the resulting reaction mixture was stirred at 60 °C for 0.5 h. After cooling down to r.t., the mixture solution was evaporated to dryness under the reduced pressure. The residue was re-dissolved in EtOAc (300 mL) and washed with brine (300 mL). The organic layer was dried over anhy. Na₂SO₄, filtered, and concentrated *in vacuo* to give a crude product, which was recrystallized in MeOH (300 mL) to give ethyl 6-bromo-1-methyl-4-oxo-1,8-naphthyridine-3-carboxylate (49.5 g, 94.7% yield) as a white solid. MS (ESI): 311.0 ([{⁷⁹Br}M+H]⁺), 313.0 ([{⁸¹Br}M+H]⁺).

### Step (d) Preparation of ethyl 1-methyl-4-oxo-6-(4,4,5,5-tetramethyl-1,3,2-dioxaborolan-2-yl)-1,8-naphthyridine-3-carboxylate (Intermediate B1)

A mixture of ethyl 6-bromo-1-methyl-4-oxo-1,8-naphthyridine-3-carboxylate (24.0 g, 77.14 mmol), B₂Pin₂ (39.2 g, 154.28 mmol), Pd₂(dba)₃ (7.0 g, 7.71 mmol), x-phos (7.34 g, 15.4 mmol), and KOAc (22.7 g, 231.4 mmol) were dissolved in dioxane (250.0 mL). The solution was degassed with argon for 5 min before stirred at 100 °C for 2 h. The reaction mixture was cooled back to room temperature and precipitate was removed by filtration. The filtrate was concentrated under the reduced pressure to give a crude product, which was then recrystallized in petrolieum ether / MTBE (500 mL) to give ethyl 1-methyl-4-oxo-6-(4,4,5,5-tetramethyl-1,3,2-dioxaborolan-2-yl)-1,8-naphthyridine-3-carboxylate (27.0 g, yield: 97.7% yield) as a yellow solid. MS (ESI): 277.0 ([M-82+H]⁺). ¹HNMR (400 MHz, DMSO-d6) δ: 8.890~8.894 (d, J=1.6 Hz, 1 H), 8.820 (s, 1 H), 8.691~8.694 (d, *J*=1.2 Hz, 1 H), 4.211~4.265 (q, *J*=7.2 Hz, 2 H), 3.922 (s, 3H), 1.232-1.311 (m, 15 H).

### Intermediate B2

### Ethyl 1-[tert-butoxycarbonyl(methyl)amino]-4-oxo-6-(4,4,5,5-tetramethyl-1,3,2-dioxaborolan-2-yl)-1,8-naphthyridine-3-carboxylate

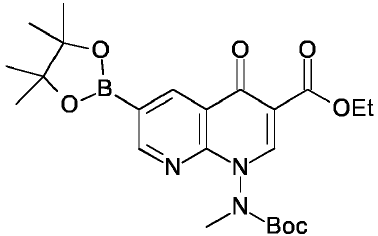

The titled compound was synthesized according to the following scheme:

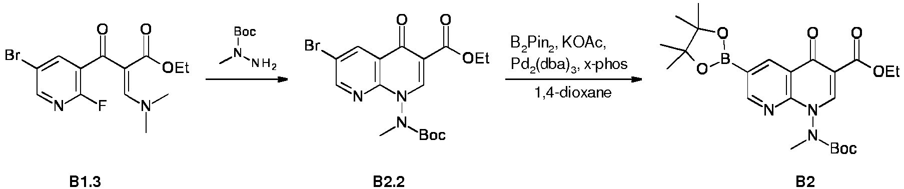

### Step (a) Preparation of ethyl 6-bromo-1-[tert-butoxycarbonyl(methyl)amino]-4-oxo-1,8-naphthyridine-3-carboxylate (compound B2.2)

To a mixture of ethyl (Z)-2-(5-bromo-2-fluoro-pyridine-3-carbonyl)-3-(dimethylamino)prop-2-enoate (42.3 g, 123.0 mmol, compound B1.3) in THF (400.0 mL) was added *tert*-butyl N-amino-N-methyl-carbamate (32.3 g, 221.3 mmol), and the resulting reaction mixture was stirred at 60 °C for 2 h. After being cooled back to r.t., the mixture solution was evaporated to dryness under the reduced pressure to give a crude product, which was recrystallized in MeOH (300 mL) to give ethyl 6-bromo-1-[*tert*-butoxycarbonyl(methyl)amino]-4-oxo-1,8-naphthyridine-3-carboxylate (50.9 g, 97.3% yield) as a white solid. MS (ESI): 428.0 ([{⁸¹Br}M+H]⁺), 426.0 ([{⁷⁹Br}M+H]⁺). ¹H NMR (400 MHz, CDCl₃) δppm: 8.77∼8.76 (m, 1 H), 8.67∼8.62 (m, 1 H), 8.57∼8.56 (m, 1 H), 4.36∼4.29 (m, 2 H), 3.38∼3.36 (m, 3 H), 1.48 (s, 3 H), 1.37∼1.31 (m, 3H), 1.17 (s, 6 H).

### Step (b) Preparation of ethyl 1-[tert-butoxycarbonyl(methyl)amino1-4-oxo-6-(4,4,5,5-tetramethyl-1,3,2-dioxaborolan-2-yl)-1,8-naphthyridine-3-carboxylate (Intermediate B2)

A mixture solution of ethyl 6-bromo-1-[*tert*-butoxycarbonyl(methyl)amino]-4-oxo-1,8-naphthyridine-3-carboxylate (35.0 g, 82.3 mmol2), B₂Pin₂ (41.9 g, 164.7 mmol), KOAc (20.3 g, 246.9 mmol), x-phos (3.9 g, 8.2 mmol) and Pd₂(dba)₃ (7.5 g, 8.2 mmol) in dioxane (400.0 mL) was stirred at 100 °C for 2 h under N₂. After being cooled back to r.t., the mixture was diluted with DCM (500 mL) and filtered. The filterate was concentrated *in vacuo* to give a crude product, which was recrystallized in petroleum ether (400 mL) to give ethyl 1-[*tert-*butoxycarbonyl(methyl)amino]-4-oxo-6-(4,4,5,5-tetramethyl-1,3,2-dioxaborolan-2-yl)-1,8-naphthyridine-3-carboxylate (34.2 g, 87.7 % yield) as yellow solid. MS (ESI): 392.1 ([M-82+H]⁺). ¹H NMR (400 MHz, DMSO-d₆) δppm: 8.99∼8.91 (m, 1 H), 8.79 (m, 1 H), 8.72∼8.66 (m, 1 H), 4.32∼4.26 (m, 2 H), 3.40 (S, 3 H), 1.49∼1.12 (m, 24 H).

### Intermediate B3

### Benzyl 1-methyl-4-oxo-6-(4,4,5,5-tetramethyl-1,3,2-dioxaborolan-2-yl)-1,8-naphthyridine-3-carboxylate

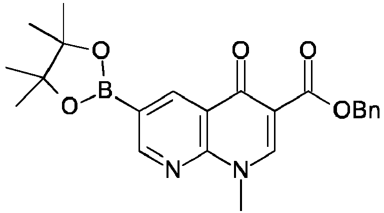

The titled compound was synthesized according to the following scheme:

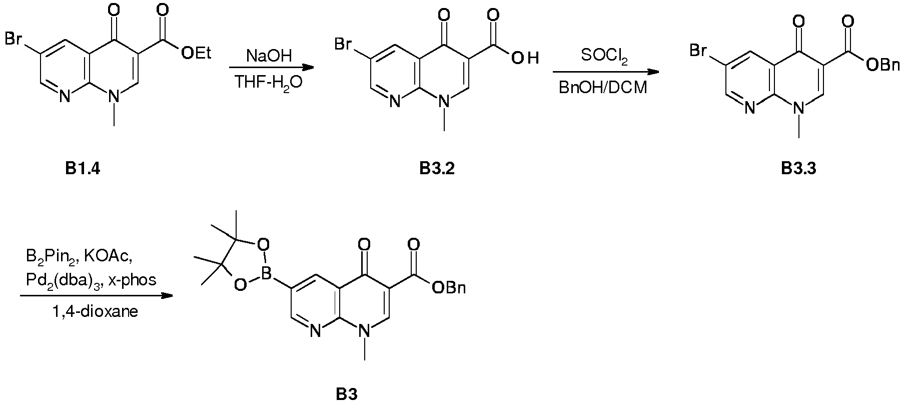

### Step (a) Preparation of 6-bromo-1-methyl-4-oxo-1,8-naphthyridine-3-carboxylic acid (compound B3.2)

To a suspension of ethyl 6-bromo-1-methyl-4-oxo-1,8-naphthyridine-3-carboxylate_(5.0 g, 16.07 mmol, compound B1.4) in THF (100 mL) and water (30 mL) was added NaOH (6.43 g, 160.7 mmol). The reaction mixture was stirred at 15 °C for 1 h until LC-MS showed the starting material was consumed completely. The mixture was acidified to pH 3~4 by aq. HCl solution (2M) and the precipitate formed was collected by filtration. The filter cake was washed with water (50 mL) and dried under vacuum to give 6-bromo-1-methyl-4-oxo-1,8-naphthyridine-3-carboxylic acid (4.4 g, 96.7% yield) as an off-white solid. MS (ESI): 283.0 ([{⁷⁹Br}M+H]⁺), 285.0 ([{⁸¹Br}+H]⁺). It was used directly in the next step without further purification.

### Step (b) Preparation of benzyl 6-bromo-1-methyl-4-oxo-1,8-naphthyridine-3-carboxylate (compound B3.3)

A suspension of 6-bromo-1-methyl-4-oxo-1,8-naphthyridine-3-carboxylic acid (4.3 g, 15.19 mmol) in SOCh (30.0 mL) was stirred at 80 °C for 0.5 h under N₂. Afterwards, the mixture was cooled back to r.t. and concentrated *in vacuo.* The residue was re-dissolved in DCM (15 mL) under N₂, followed by the addition of benzyl alcohol (4.93 g, 45.57 mmol), Et₃N (6.35 mL, 45.57 mmol) at 0 °C. The mixture solution was allowed to stirred at 15 °C for 1 h until LC-MS showed the intermediate was consumed completely. The mixture was then washed with aq. HCl solution (50 mL, 0,5 M), and the separated organic layer was concentrated *in vacuo* to give a crude product, which was purified by silica gel flash chromatography (EtOAc, 100%) to give benzyl 6-bromo-1-methyl-4-oxo-1,8-naphthyridine-3-carboxylate (5.5 g, 95.1% yield) as an off-white solid. MS (ESI): 373.1 ([{⁷⁹Br}M+H]⁺), 375.1 ([{⁸¹Br}+H]⁺).

### Step (c) Preparation of benzyl 1-methyl-4-oxo-6-(4,4,5,5-tetramethyl-1,3,2-dioxaborolan-2-yl)-1,8-naphthyridine-3-carboxylate (Intermediate B3)

A mixture of benzyl 6-bromo-1-methyl-4-oxo-1,8-naphthyridine-3-carboxylate (5.0 g, 13.4 mmol3), B₂Pin₂ (10.21 g, 40.19 mmol), tris(dibenzylideneacetone)dipalladium (0) (368.05 mg, 0.400 mmol), x-phos (383.22 mg, 0.800 mmol), and AcOK (3.94 g, 40.19 mmol) were dissolved in 1,4-dioxane (100 mL) under Ar. The resulting reaction mixture was stirred at 100 °C for 2 h until LC-MS showed the starting material was consumed completely. The mixture was cooled back to r.t, diluted with DCM (100 mL) and filtered. The filtrate was concentrated *in vacuo,* and the residue was treated with TBME (960 mL). The precipitate was collected by filtration and the filter cake was dried under vacuum to give benzyl 1-methyl-4-oxo-6-(4,4,5,5-tetramethyl-1,3,2-dioxaborolan-2-yl)-1,8-naphthyridine-3-carboxylate (5.6 g, 96.5% yield) as a yellow solid. MS (ESI): 339.2 ([M-82+H]⁺). ¹H NMR (400 MHz, CHCl₃-d) δ ppm 9.17 (d, J=1.71 Hz, 1 H), 9.02 (d, J=1.59 Hz, 1 H), 8.62 (s, 1 H), 7.52 (d, J=7.34 Hz, 2 H), 7.36 - 7.42 (m, 2 H), 7.33 (br d, J=7.21 Hz, 1 H), 5.40 (s, 2 H), 3.96 (s, 3 H), 1.37 (s, 12 H).

### Intermediate B4

### Benzyl 4-oxo-7-(4,4,5,5-tetramethyl-1,3,2-dioxaborolan-2-yl)quinolizine-3-carboxylate

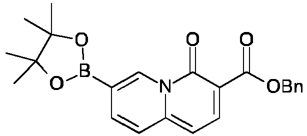

The titled compound was synthesized according to the following scheme:

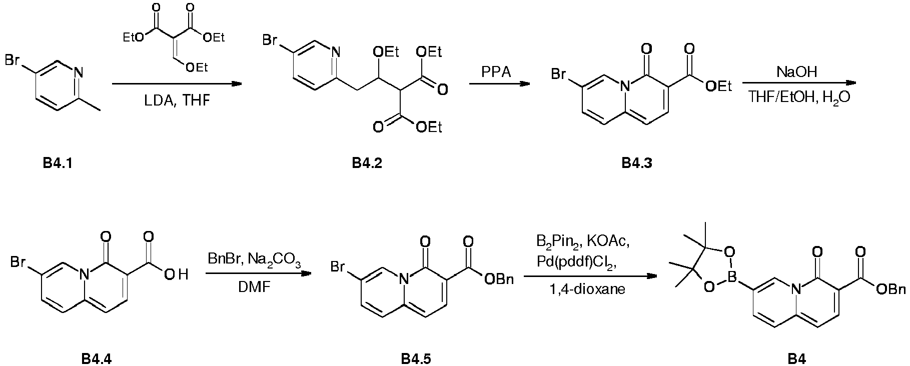

### Step (a) Preparation of diethyl 2-[2-(5-bromo-2-pyridyl)-1-ethoxyy-ethyl]propanedioate (compound B4.2)

To a solution of DIPA (52.90 g, 523.19 mmol) in THF (600 mL) was added n-BuLi (0.39 mL, 0.970 mmol) dropwise at -78 °C. After the mixture solution was stirred at -30 °C for 1 h, 5-bromo-2-methylpyridine (90.0 g, 523.19 mmol) in THF (600 mL) was added dropwise at -78 °C and stirred for another 1 h. Afterwards, diethyl ethoxymethylenemalonate (113.13 g, 523.19 mmol) was added and the resulting reaction mixture was stirred at -78 °C for 2 h until LC-MS showed the starting material was consumed completely. Aqueous NH₄Cl solution was introduced to quench the reaction and the solution was extracted with EtOAc (500 mL) three times. Combined organics was then washed with brine, dried over anhy. Na₂SO₄, filtered, and concentrated *in vacuo* to give a crude product, which was purified by silica gel flash chromatography (petroleum ether:EtOAc = 100:1 to 5:1) to give diethyl 2-[2-(5-bromo-2-pyridyl)-1-ethoxy-ethyl]propanedioate (20 g, 9.9% yield) as yellow oil. MS (ESI): 388.0 ([{⁷⁹Br}M+H]⁺), 390.1 ([{⁸¹Br}M+H]⁺).

### Step (b) Preparation of ethyl 7-bromo-4-oxo-quinolizine-3-carboxylate (compound B4.3)

A solution of diethyl 2-[2-(5-bromo-2-pyridyl)-1-ethoxy-ethyl]propanedioate_(42.0 g, 108.18 mmol) in PPA (40.0 mL, 0.200 mmol) was stirred at 130 °C for 2 h. After LC-MS showed the starting material was consumed, the reaction mixture was cooled back to r.t. and basified with aq. Na₂CO₃ solution to pH 9 and extracted with DCM (30 mL) three times. Combined organics was washed with brine, dried over anhy. Na₂SO₄, filtered and concentrated *in vacuo* to give a crude product of ethyl 7-bromo-4-oxo-quinolizine-3-carboxylate (4.5 g, 14.1% yield) as a yellow solid. MS (ESI): 296.0 ([{⁷⁹Br}M+H]⁺), 298.0 ([{⁸¹Br}M+H]⁺). It was used directly in the next step without further purification.

### Step (c) Preparation of 7-bromo-4-oxo-quinolizine-3-carboxylic acid (compound B4.3)

To a solution of ethyl 7-bromo-4-oxo-quinolizine-3-carboxylate (8.5 g, 28.7 mmol) in EtOH (66.94 mL) and water (13.39 mL) was added NaOH (0.89 mL, 1.78 mmol), and the resulting reaction mixture was stirred at 15 °C for 2 h. After LC-MS showed the starting material was consumed, the mixture solution was concentrated under the reduced pressure and acidified with aq. HCl solution (1N) to pH 6 and extracted with DCM (30 mL) three times. Combined organics was then washed with brine, dried over anhy. Na₂SO₄, filtered and concentrated *in vacuo* to give a crude product of 7-bromo-4-oxo-quinolizine-3-carboxylic acid (8.0 g, 100% yield) as a yellow solid. MS (ESI): 290.0 ([{⁷⁹Br}M+H]⁺), 291.9 ([{⁸¹Br}M+Na]⁺). It was used directly in the next step without further purification.

### Step (d) Preparation of benzyl 7-bromo-4-oxo-quinolizine-3-carboxylate (compound B4.4)

To a solution of 7-bromo-4-oxo-quinolizine-3-carboxylic acid (8.0 g, 29.84 mmol) in DMF (80 mL) was added BnBr (14.92 mL, 29.84 mmol) and Na₂CO₃ (1.0 mL, 29.84 mmol), and the resulting reaction mixture was stirred at 30 °C for 18 h. After LC-MS showed the starting material was consumed, the reaction mixture was concentrated *in vacuo* to give a crude product, which was purified by prep-HPLC (TFA as additive) to give benzyl 7-bromo-4-oxo-quinolizine-3-carboxylate (10 g, 93.6% yield) as a yellow solid. MS (ESI): 358.0 ([{⁷⁹Br}M+H]⁺), 360.0 ([{⁸¹Br}M+H]⁺).

### Step (e) Preparation of benzyl 4-oxo-7-(4,4,5,5-tetramethyl-1,3,2-dioxaborolan-2-yl)quinolizine-3-carboxylate (Intermediate B4)

To a solution of benzyl 7-bromo-4-oxo-quinolizine-3-carboxylate (1.0 g, 2.79 mmol) in THF (10 mL) was added Pd(dppf)Cl₂ (166.64 mg, 0.280 mmol, 0.100 eq), KOAc (547.2 mg, 5.58 mmol, 2 eq) and B₂Pin₂ (2.161 g, 8.38 mmol). After the mixture solution was degassed and purged with argon several times, the reaction mixture was stirred at 90 °C for 16 h. After LC-MS showed the starting material was consumed completely, the reaction mixture was cooled back to r.t and concentrated *in vacuo* to give a crude product, which was purified by prep-HPLC (TFA as additive) to give benzyl 4-oxo-7-(4,4,5,5-tetramethyl-1,3,2-dioxaborolan-2-yl)quinolizine-3-carboxylate (1.0 g, 88.4% yield) as a yellow solid. MS (ESI): 324.0 ([M+H]⁺).

### Intermediate C1

### cis-5-Methyl-2,3,3a,4,6,6a-hexahydro-1H-pyrrolo[2,3-c]pyrrole

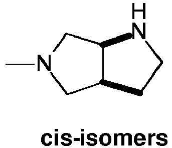

The titled compound was synthesized according to the following scheme:

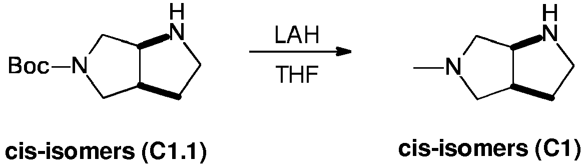

### Preparation of cis-5-Methyl-2,3,3a,4,6,6a-hexahydro-1H-pyrrolo[2,3-c]pyrrole (Intermediate C1)

To a stirred solution of LAH (4.02 g, 105.99 mmol) in THF (50 mL) was added *cis-tert-*butyl 2,3,3a,4,6,6a-hexahydro-1H-pyrrolo[2,3-c]pyrrole-5-carboxylate (4.5 g, 21.2 mmol, CAS: 132414-81-4) at 0 °C. After the completion of addition, the resulting reaction mixture was allowed to warm up and stirred at 70 °C for 2 h under N₂. After TLC (DCM:MeOH = 10:1) showed the starting material was consumed completely, the reaction mixture was cooled back to 0 °C and quenched by the addition of H₂O (5 mL), and followed by the addition of aq. NaOH solution (5 mL, 15%), H₂O (15 mL), and MgSO₄ (10 g). After the mixture solution was stirred at 25°C for 1 h, it was filtered and the filtrate was concentrated *in vacuo* to give a crude product of cis-5-methyloctahydropyrrolo[2,3-c]pyrrole (2.6 g, 20.6 mmol, 97.2% yield) as yellow oil. It was used directly in the next step without further purification. ¹H NMR (400 MHz, CHCl₃-d) δ ppm 3.66 - 3.79 (m, 1 H), 2.89 - 3.02 (m, 1 H), 2.75 (dt, J=11.19, 6.50 Hz, 1 H), 2.65 (dtd, J=12.40, 8.35, 8.35, 4.39 Hz, 1 H), 2.46 - 2.54 (m, 2 H), 2.38 - 2.45 (m, 1 H), 2.34 (dd, J=9.28, 4.14 Hz, 1 H), 2.27 (s, 3 H), 1.85 (ddt, J=12.42, 8.78, 6.33, 6.33 Hz, 1 H), 1.45 - 1.56 (m, 1 H), 1.43 (s, 1 H), 1.21 - 1.31 (m, 1 H).

### Example 1.01

### 6-[5-Cyano-6-fluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

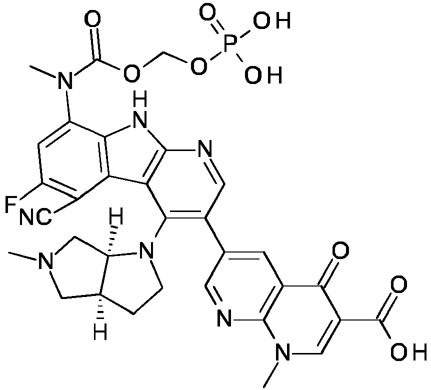

The titled compound was synthesized according to the following scheme:

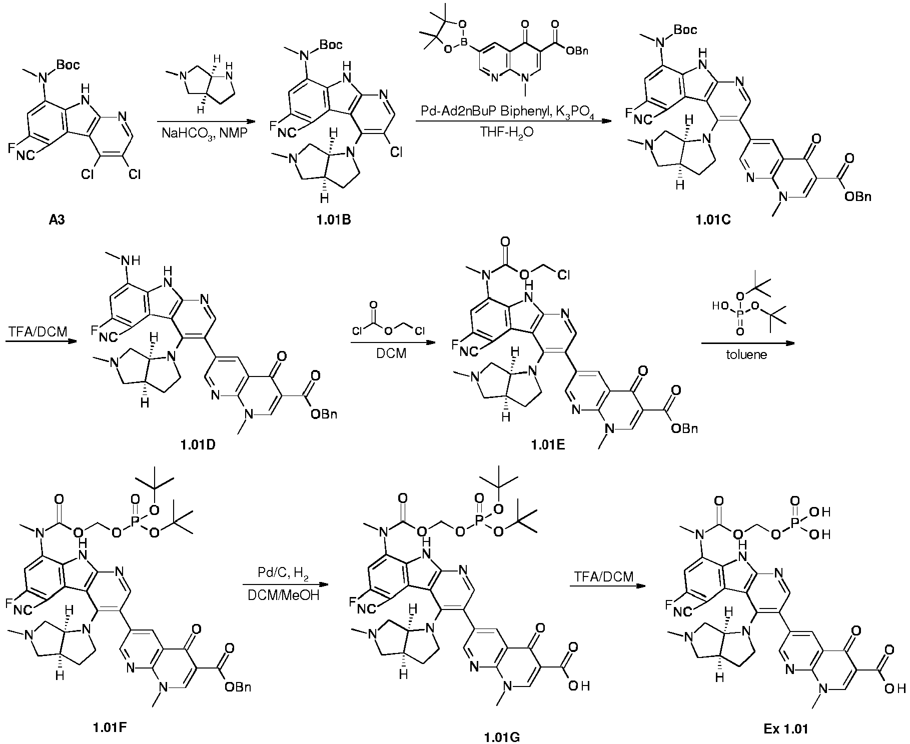

### Step (a) Preparation of tert-butyl N-[3-chloro-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.01B)

A mixture solution of *tert*-butyl N-(3,4-dichloro-5-cyano-6-fluoro-9H-pyrido[2,3-b]indol-8-yl)-N-methyl-carbamate (3.3 g, 8.06 mmol, Intermediate A3), (3aS,6aS)-5-methyl-2,3,3a,4,6,6a-hexahydro-1H-pyrrolo[2,3-c]pyrrole (1.53 g, 12.1 mmol, CAS: 1176846-84-6) and NaHCO₃ (3.39 g, 40.32 mmol) in NMP (12 mL)) was stirred at 120 °C for 48 h. After TLC (petrolieum ether:EtOAc = 2: 1, 2% of TEA as additive) showed the starting material was consumed completely, the mixture was cooled back to r.t. and poured into EtOAc (500 mL). It was washed with aq. CaCh solution (100 mL) three times and brine (100 mL) twice. The separated organic layer was dried over anhy. Na₂SO₄, filtered and concentrated *in vacuo* to give a crude product, which was purified by silica gel flash chromatography (petroleum ether:EtOAc = 10:1-1:1, 2% of TEA as additive) to give *tert-butyl* N-[3-chloro-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (add optical rotation value here, 3 g, 72.8% yield) as a light yellow solid. MS (ESI): 499.3 ([{³⁵Cl}M+H]⁺).

### Step (b) Preparation of benzyl 6-[8-[tert-butoxycarbonyl(methyl)aminol-5-cyano-6-fluoro-4-[(3aS,6aS)-5-methyl-2,3,3a,4,6,6a-hexahydropyrrolo[2,3-c]pyrol-1-yl]-9H-pyrido[2,3-b]indol-3-yl]-1-methyl-4-oxo-1,8-naphthyridine-3-carboxylate (compound 1.01C)

A mixture of benzyl 1-methyl-4-oxo-6-(4,4,5,5-tetramethyl-1,3,2-dioxaborolan-2-yl)-1,8-naphthyridine-3-carboxylate (2.95 g, 7.01 mmol, Intermediate B3), *tert*-butyl N-[4-[(3aS,6aS)-5-methyl-2,3,3a,4,6,6a-hexahydropyrrolo[2,3-c]pyrrol-1-yl]-3-chloro-5-cyano-6-fluoro-9H-pyrido[2,3-b]indol-8-yl]-N-methyl-carbamate (2.0 g, 4.01 mmol), potassium phosphate (3.4 g, 16.03 mmol), and Pd-Ad2nBuP Biphenyl (536.0 mg, 0.800 mmol, cataCXium A-Pd-G2, CAS: 1375477-29-4) were dissolved in 1,4-dioxane (150 mL) and water (18 mL) under argon. The resulting reaction mixture was stirred at 70 °C for 12 h until LC-MS showed the starting material was consumed completely. The mixture was cooled back to r.t., and filtered. The filtrate was diluted with EtOAc (500 mL) and washed with brine (100 mL) three times. The separated organic layer was then dried over anhy. Na₂SO₄, filtered, and concentrated *in vacuo* to give a crude product, which was purified by prep-HPLC to give benzyl 6-[8-[*tert-*butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (1.85 g, 60.5% yield) ) as a light yellow solid. MS (ESI): 757.4 ([M+H]⁺).

### Step (c) Preparation of benzyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01D)

To a solution of benzyl 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (1.85 g, 2.44 mmol) in DCM (20 mL) was added TFA (8.0 mL, 103.84 mmol) and the resulting mixture was stirred at 25 °C for 1 h. After LC-MS showed the starting material was consumed completely, the mixture was concentrated *in vacuo* to give a crude product, which was purified by prep-HPLC to give benzyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (1.5 g, 1.95 mmol, 79.6% yield) as a yellow solid. MS (ESI): 657.3 ([M+H]⁺).

### Step (d) Preparation of benzyl 6-[8-[chloromethoxycarbonyl(methyl)aminol-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01E)

To a solution of benzyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (1.0 g, 1.52 mmol) in DCM (30 mL) was added chloromethyl chloroformate (60.0 g, 465.33 mmol) and the resulting mixture was stirred at r.t. for 18 h. After LC-MS showed the starting material was consumed completely, the mixture solution was evaporated under the reduced pressure to dryness. The residue was re-dissolved in DCM and basified with aq. NH₄HCO₃ solution to pH = 7. The solution was filtered and the filtrate was concentrated *in vacuo* to give a crude product, which was purified by prep-HPLC (formic acid as additive). The collected eluents were then basified with aq. NH₄HCO₃ solution to pH = 7 and extracted with DCM (300 mL) twice. Combined organics was then washed with brine (200 mL) twice, dried by anhy. Na₂SO₄, filtered and concentrated *in vacuo* to give benzyl 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (580 mg, 46.6% yield) as a yellow solid. MS (ESI): 749.3 ([{³⁵Cl}M+H]⁺).

### Step (e) Preparation of benzyl 6-[5-cyano-8-[di-tert-butoxyphosphoryloxymethoxycarbonyl(methyl)amino]-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01F)

To a solution of benzyl 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (330.0 mg, 0.440 mmol) in toluene (7 mL) was added potassium di-*tert*-butylphosphate (437.13 mg, 1.76 mmol). The resulting reaction mixture was stirred at 90 °C for 2 h until LC-MS showed the starting material was consumed completely. The mixture was then cooled back to r.t., poured into water (50 mL) and extracted with DCM (50 mL) three times. The organic layer was dried over anhy. Na₂SO₄, filtered, and concentrated *in vacuo* to give a crude product, which was purified by prep-HPLC (formic acids as additive). The collected eluents were then basified with aq. NH₄HCO₃ solution to pH 7~8 and extracted with DCM (200 mL) twice. Combined organics was then dried over anhy. Na₂SO₄, filtered, and concentrated *in vacuo* to give benzyl 6-[5-cyano-8-[di-*tert-*butoxyphosphoryloxymethoxycarbonyl(methyl)amino]-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (250 mg, 57.9% yield) as a light yellow solid. MS (ESI): 923.4 ([M+H]⁺).

### Step (g) Preparation of 6-[5-cyano-8-[di-tert-butoxyphosphoryloxymethoxycarbonyl(methyl)amino]-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.01G)

To a solution of benzyl 6-[5-cyano-8-[di-*tert-*butoxyphosphoryloxymethoxycarbonyl(methyl)amino]-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (240.0 mg, 0.260 mmol) in DCM (4 mL) and MeOH (8 mL) was added palladium on carbon (100 mg). After the suspension was degassed under vacuum and purged with H₂ several times, the reaction mixture was stirred at 25 °C for 3 h under H₂ atmosphere (15 psi). After LC-MS showed the starting material was consumed completely, the reaction mixture was filtered through Celite. The filtrate was then concentrated *in vacuo* to give a crude product of 6-[5-cyano-8-[di-*tert*-butoxyphosphoryloxymethoxycarbonyl(methyl)amino]-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (190 mg, 0.230 mmol, 87.8% yield) as a light yellow solid. MS (ESI): 833.3 ([M+H]⁺). It was used directly in the next step without further purification.

### Step (h) Preparation of 6-[5-cyano-6-fluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-blindol-3-yl]-1-methyl-4-oxo-1,8-naphthyridine-3-carboxylic acid (Example 1.01)

A mixture solution of 6-[4-[(3aS,6aS)-5-methyl-2,3,3a,4,6,6a-hexahydropyrrolo[2,3-c]pyrrol-1-yl]-5-cyano-8-[di-*tert*-butoxyphosphoryloxymethoxycarbonyl(methyl)amino]-6-fluoro-9H-pyrido[2,3-b]indol-3-yl]-1-methyl-4-oxo-1,8-naphthyridine-3-carboxylic acid (161.36 mg, 0.190 mmol) and TFA in DCM (5.0 mL) (v/v, DCM:TFA = 20:1) was stirred at 25 °C for 2 h under nitrogen. After LC-MS showed the starting material was consumed completely, the solution was adjust to pH > 7 by aq. NH₄HCO₃ solution. The mixture was concentrated *in vacuo* to give a crude product, which was purified by prep-HPLC (NH₄HCO₃ as additive) to give 6-[5-cyano-6-fluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (80.7 mg, 0.110 mmol, 49.8% yield) as a light yellow solid. MS (ESI): 721.3 ([M+H]⁺). ¹H NMR (400 MHz, DMSO-*d*₆) δ ppm 12.53 - 14.43 (m, 1 H), 9.10 - 9.36 (m, 2 H), 8.74 (br s, 1 H), 7.93 - 8.15 (m, 1 H), 7.11 - 7.77 (m, 2 H), 4.51 - 5.70 (m, 3 H), 4.11 - 4.27 (m, 3 H), 3.31 (br s, 4 H), 2.93 - 3.09 (m, 3 H), 2.54 - 2.64 (m, 1 H), 2.12 - 2.46 (m, 6 H), 1.48 (br s, 1 H).

### Example 1.02

### 6-[5-Cyano-6-fluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

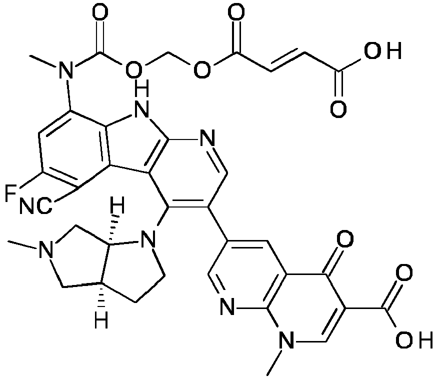

The titled compound was synthesized according to the following scheme:

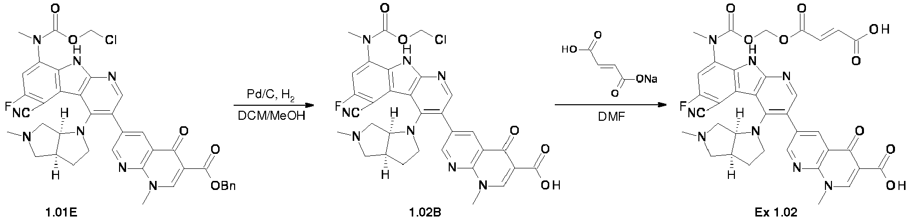

### Step (a) Preparation of 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.02B)

To a solution of benzyl 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (230mg, 0.31 mmol, compound 1.01E) in DCM (2 mL) and MeOH (2 mL) was added palladium on carbon (115 mg). After the suspension was degassed under vacuum and purged with H₂ several times, the reaction mixture was stirred at 25 °C for 3 h under H₂ atmosphere (15 psi). After LC-MS showed the starting material was consumed completely, the reaction mixture was filtered through Celite. The filtrate was concentrated *in vacuo* to give a crude product of 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (180 mg, 73.2% yield) as a light yellow solid. MS (ESI): 659.3 (([{³⁵Cl}M+H]⁺). It was used directly in the next step without further purification.

### Step (b) Preparation of 6-[5-cyano-6-fluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.02)

To a solution of 6-[4-[(3aS,6aS)-5-methyl-2,3,3a,4,6,6a-hexahydropyrrolo[2,3-c]pyrrol-1-yl]-8-[chloromethoxycarbonyl(methyl)amino]-5-cyano-6-fluoro-9H-pyrido[2,3-b]indol-3-yl]-1-methyl-4-oxo-1,8-naphthyridine-3-carboxylic acid (180.0 mg, 0.270 mmol) in DMF (4 mL) was added sodium hydrogen fumarate (150.81 mg, 1.09 mmol, CAS: 5873-57-4). The resulting mixture was stirred at 90 °C for 2 h until LC-MS showed the starting material was consumed completely. The reaction mixture was cooled back to r.t. and filtered. The residue was subject to prep-HPLC purification (NH₄HCO₃ as additive) to give 6-[5-cyano-6-fluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (24.5 mg, 11.8% yield) as a light yellow solid. MS (ESI): 739.3 ([M+H]⁺). ¹H NMR (400 MHz, DMSO-d6) δ ppm 14.81 (br s, 1 H), 12.24 - 12.96 (m, 1 H), 9.27 (s, 1 H), 9.17 (br s, 1 H), 8.81 (br s, 1 H), 8.00 - 8.19 (m, 1 H), 7.74 (br d, J=10.3 Hz, 1 H), 6.21 - 6.96 (m, 2 H), 5.47 - 6.01 (m, 2 H), 4.59 (br s, 1 H), 4.18 (s, 3 H), 3.07 - 3.20 (m, 3 H), 2.95 - 3.02 (m, 2 H), 2.79 - 2.85 (m, 1 H), 2.83 (br d, J=7.3 Hz, 1 H), 2.39 - 2.47 (m, 1 H), 1.97 - 2.29 (m, 7 H), 1.75 (br s, 1 H), 1.49 (br s, 1 H).

### Example 1.03

### 6-[5-Cyano-6-fluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

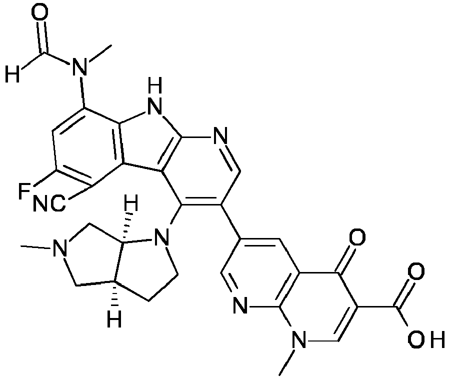

The titled compound was synthesized according to the following scheme:

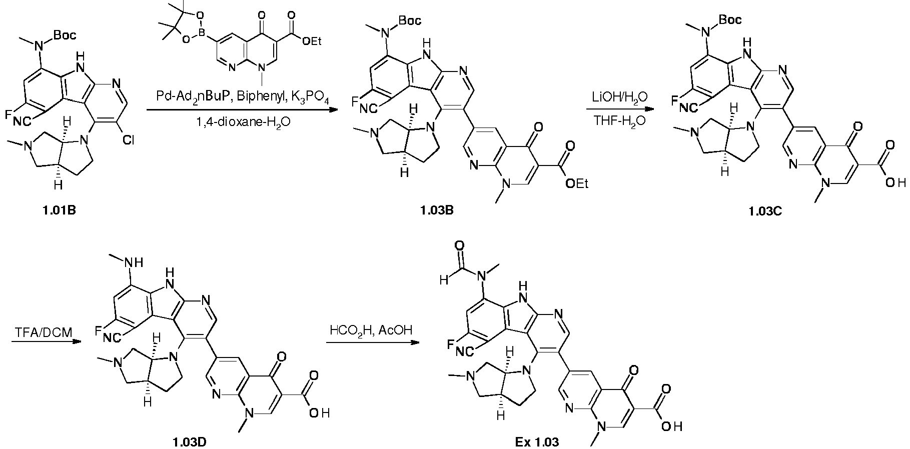

### Step (a) Preparation of ethyl 6-[8-[tert-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.03B)

To a mixture solution of *tert*-butyl N-[3-chloro-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methylcarbamate (20.0 g, 40.1 mmol, compound 1.01B), ethyl 1-methyl-4-oxo-6-(4,4,5,5-tetramethyl-1,3,2-dioxaborolan-2-yl)-1,8-naphthyridine-3-carboxylate (21.5 g, 60.1 mmol, Intermediate B1), and K₃PO₄ (34.1 g, 160.4 mmol) in dioxane / H₂O (500.0 mL, v/v=5:1) was added Pd-Ad₂nBuP Biphenyl (4.02 g, 6.01mmol) in a glove box under argon. The resulting reaction mixture was stirred at 90 °C for 12 h until TLC (petrolieum ether:EtOAc = 1:2, 3% TEA as additive) showed the starting material was consumed completely. The mixture was cooled back to r.t. and filtered. The filtrate was diluted with EtOAc (1.2 L) and washed by brine (300 mL) twice. The organic layer was dried with anhy. Na₂SO₄, filtered, and concentrated *in vacuo* to give a crude product, which was purified by silica gel flash chromatography (petroleum ether:EtOAc = 100:1-1:5, 3% TEA as additive) to give ethyl 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (21.2 g, 76.3% yield) as a yellow solid. MS (ESI): 695.4 ([M+H]⁺).

### Step (b) Preparation of 6-[8-[tert-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.03C)

To a stirred solution of ethyl 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (20.8 g, 29.94 mmol) in THF / EtOH, 120.0 mL, v/v=3:1) was added aq. LiOH solution (44.91 mL, 89.92 mmol, 2.0 N). The resulting reaction mixture was stirred at 15 °C for 3 h until LC-MS showed the starting material was consumed completely. After dilution with EtOAc (300 mL) and H₂O (50 mL), the mixture was adjusted to pH = 6 with 1 N HCl aqueous solution and extracted with EtOAc (300 mL) twice. Combined organics was washed with brine (100.0 mL) twice, dried over anhy. Na₂SO₄, filtered, and concentrated *in vacuo* to give 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (19.0 g, 95.2% yield) as a yellow solid. MS (ESI): 667.4 ([M+H]⁺). It was used directly in the next step without further purification.

### Step (c) Preparation of 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.03D)

To a stirred solution of 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (18.9 g, 28.35 mmol) in DCM (50.0 mL) was added TFA (20.0 mL). The reaction mixture was stirred at 15 °C for 2 h until LC-MS showed the starting material was consumed completely. The mixture was evaporated to dryness under the reduced pressure. The residue was re-dissolved in MeOH (150 mL) and adjusted to pH = 6 with NaHCO₃ solid. The mixture was filtered and the filtrate was concentrated *in vacuo* to give a crude product, which was purified by prep-HPLC (NH₃•H₂O as additive) to give 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (11.6 g, 72.2% yield) as a yellow solid. MS (ESI): 567.4 ([M+H]⁺), 284.3 ([M/2+H]⁺). ¹H NMR (400 MHz, DMSO-d6) δ ppm 14.82 (br s, 1 H), 11.89 (br s, 1 H), 9.25 (s, 1 H), 9.16 (d, *J*=2.3 Hz, 1 H), 8.76 (d, *J*=2.3 Hz, 1 H), 8.03 (s, 1 H), 6.83 (br d, *J*=3.3 Hz, 1 H), 6.65 (d, *J*=13.1 Hz, 1 H), 4.60 ~ 4.74 (m, 1 H), 4.17 (s, 3 H), 2.89 ~ 3.05 (m, 5 H), 2.71 ~ 2.81 (m, 1 H), 2.46 (br d, 7=9.0 Hz, 1 H), 2.07 ~ 2.31 (m, 6 H), 1.66 (dd, 7=9.7, 5.1 Hz, 1 H), 1.42 (br s, 1 H).

### Step (d) Preparation of 6-[5-cyano-6-fluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.03)

A solution of 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (30.0 mg, 0.050 mmol) in acetic acid (2.19 mL) and HCOOH (2.0 mL, 0.530 mmol) was stirred at 15 °C for 2 h. After LC-MS showed the starting material was consumed completely, the mixture was concentrated *in vacuo* to give a crude product, which was purified by prep-HPLC (TFA as additive) to give 6-[5-cyano-6-fluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (8 mg, 23.9% yield) as a yellow solid. MS (ESI): 595.3 ([M+H]⁺). ¹H NMR (400 MHz, DMSO-*d*₆) δ ppm: ¹H NMR (400MHz, DMSO-*d*₆) δ = 12.91 (br s, 1H), 12.54 (br s, 1H), 9.34 - 9.19 (m, 2H), 8.76 (d, *J*=2.0 Hz, 1H), 8.50 (s, 1H), 8.53 - 8.36 (m, 1H), 8.39 (s, 1H), 8.19 (br s, 1H), 7.84 - 7.73 (m, 1H), 4.73 (br s, 1H), 4.19 (s, 3H), 3.59 - 3.56 (m, 2H), 3.35 (s, 3H), 3.23 (br s, 3H), 2.88 - 2.61 (m, 5H), 2.30 - 2.07 (m, 1H), 1.65 (br s, 1H).

### Example 1.04

### 6-[5-Cyano-6-fluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid

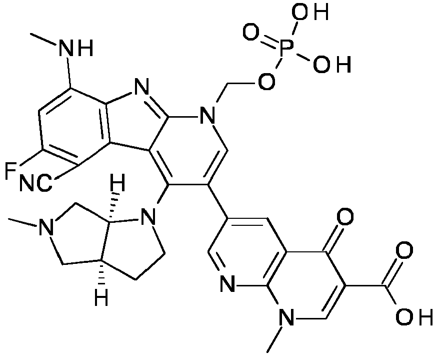

The titled compound was synthesized according to the following scheme:

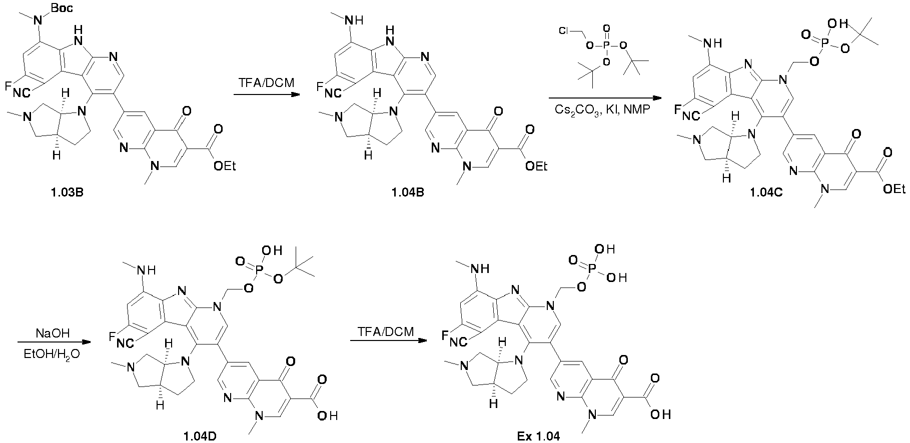

### Step (a) Preparation of ethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.04B)

To a solution of ethyl 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (510 mg, 0.73 mmol, compound 1.03B) in DCM (5 mL) was added trifluoroacetic acid (2 mL, 25.9 mmol), and the reaction mixture was stirred at 20 °C for 1 h. After LC-MS showed the starting material was consumed, the mixture was diluted with DCM (50 mL), poured into water (50 mL), adjusted to pH = 8 by aq. NaOH solution (2 N), and extracted with DCM (100 mL). The organic layer was washed with brine (100 mL), dried over anhy. Na₂SO₄, filtered, and concentrated *in vacuo* to give a crude product of ethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (410 mg, 93.9% yield) as a yellow solid. MS (ESI): 595.3 ([M+H]⁺). It was used directly in the next step without further purification.

### Step (b) Preparation of ethyl 6-[1-[[tert-butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylate (compound 1.04C)

To a mixture solution of ethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (350 mg, 0.59 mmol), cesium carbonate (553.3 mg, 1.7 mmol), and potassium iodide (0.09 mL, 1.67 mmol) in NMP (5.0 mL) was added di*-tert-*butyl chloromethyl phosphate (345.8 mg, 1.34 mmol), and the reaction mixture was stirred at 20 °C for 4 h. After LC-MS showed the starting material was consumed, the mixture was diluted with DCM (100 mL), poured into water (100 mL), and extracted with DCM (100 mL) twice. Combined organics was then washed with brine (100 mL), dried over anhy. Na₂SO₄, filtered, concentrated *in vacuo* to give a crude product, which was purified by prep-HPLC (NH₄HCO₃ as additive) to give ethyl 6-[1-[[*tert*-butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylate (65.0 mg, 14.5% yield) as a yellow solid. MS (ESI): 761.4 ([M+H]⁺).

### Step (c) Preparation of 6-[1-[[tert-butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (compound 1.04D)

To a solution of ethyl 6-[1-[[*tert*-butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylate (60 mg, 0.08 mmol) in EtOH (3.0 mL) and water (0.5 mL) was added lithium hydroxide (0.21 mL, 1.67 mmol), and the reaction mixture was stirred at 20 °C for 1 h. After LC-MS showed the starting material was consumed, the mixture was adjusted to pH = 6 with aq. HCl solution (1M), and purified by prep-HPLC to give 6-[1-[[*tert*-butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (55.0 mg, 95.2% yield) as a yellow solid. MS (ESI): 733.3 ([M+H]⁺).

### Step (d) Preparation of 6-[-cyano-6-fluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (Example 1.04)

To a solution of 6-[1-[[*tert*-butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo [2,3-c]pyrrol-1-yl]pyrido[2,3-b]indol-3-yl]-1-methyl-4-oxo-1,8-naphthyridine-3-carboxylic acid (50.0 mg, 0.07 mmol) in DCM (5.0 mL) was added TFA / DCM (3.0 mL, 0.07 mmol, v/v = 1:3), and the reaction mixture was stirred at 20 °C for 1 h. After LC-MS showed the starting material was consumed, the mixture was concentrated *in vacuo* to give a crude product, which was purified by prep-HPLC (TFA as additive) to give 6-[5-cyano-6-fluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (14.5 mg, 26.4% yield) as a yellow solid. MS (ESI): 677.3 ([M+H]⁺). ¹H NMR (400 MHz, DMSO-d6) δ ppm 9.30 (br d, J=10.88 Hz, 2 H), 8.98 (br s, 1 H), 8.49 (br s, 1 H), 7.23~7.25 (m, 1 H), 6.66~6.73 (m, 1 H), 6.65~6.66 (m, 1 H), 6.07~6.17 (m, 2 H), 4.17 (br s, 4 H), 3.01~3.39 (m, 3 H), 2.94 (br s, 3 H), 2.68 (br d, J=1.83 Hz, 4 H), 2.30~2.37 (m, 1 H), 2.11 (br s, 1 H), 1.73 (br s, 2H).

### Example 1.05

### 6-[5-Cyano-6-fluoro-8-(methylamino)-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid

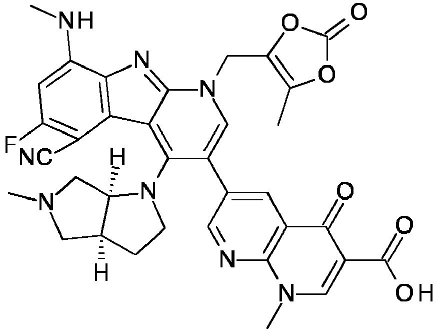

The titled compound was synthesized according to the following scheme:

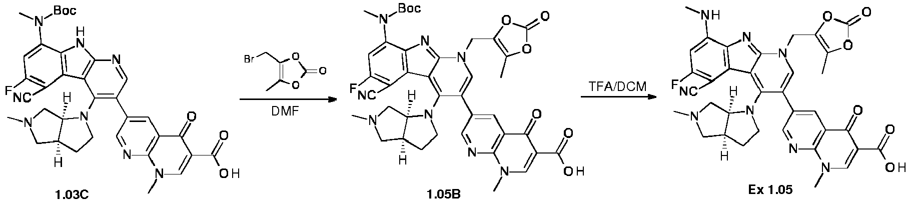

### Step (a) Preparation of 6-[8-[tert-butoxycarbonyl(methyl)aminol-5-cyano-6-fluoro-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (compound 1.05B)

To a solution of 6-[8-[tert-butoxycarbonyl(methyl)amino]-5-cyano-6-fluoro-4-[(3aS,6aS)-5-methyl-2,3,3a,4,6,6a-hexahydropyrrolo [2,3-c]pyrrol-1-yl]-9H-pyrido [2,3-b]indol-3-yl]-1-methyl-4-oxo-1,8-naphthyridine-3-carboxylic acid (190 mg, 0.28 mmol, compound 1.03C) in DMF (3 mL) was added 4-(bromomethyl)-5-methyl-1,3-dioxol-2-one (95.0 mg, 0.490 mmol) at 0 °C, and the resulting reaction mixture was stirred at 20 °C for 12 h. After LC-MS showed the starting material was consumed, the reaction mixture was then concentrated *in vacuo* to give a crude product, which was further purified by prep-HPLC (TFA as additive) to give 6-[8-[*tert-*butoxycarbonyl(methyl)amino]-5-cyano-6-fluoro-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (45 mg, 20.3% yield) as a yellow solid. MS (ESI): 779.2 ([M+H]⁺). Analytical HPLC Rt = 2.97 min (faster eluted compared to compound 1.06B).

### Step (b) Preparation of 6-[5-cyano-6-fluoro-8-(methylamino)-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (Example 1.05)

To a solution of 6-[8-[*tert*-butoxycarbonyl(methyl)amino]-5-cyano-6-fluoro-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido [2,3-b]indol-3-yl]-1-methyl-4-oxo-1,8-naphthyridine-3-carboxylic acid (40.0 mg, 0.05 mmol) in DCM (3.0 mL) was added TFA (1.0 mL, 13 mmol), and the resulting reaction mixture was stirred at 20 °C for 1 h. After LC-MS showed the starting material was consumed, the mixture solution was concentrated *in vacuo* to give a crude product, which was purified by prep-HPLC (TFA as additive) to give 6-[5-cyano-6-fluoro-8-(methylamino)-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (22.3 mg, 47.1% yield) as a yellow solid. MS (ESI): 679.2 ([M+H]⁺). ¹H NMR (400 MHz, DMSO-d6) δ ppm 14.65 (br s, 1 H), 12.09 (br s, 1 H), 9.29~9.37 (m, 1 H), 9.16~9.28 (m, 1 H), 8.91 (br s, 1 H), 8.73 (br s, 1 H), 8.24 (br s, 1 H), 8.11 (s, 1 H), 6.61~6.74 (m, 1 H), 5.67 (br d, J=11.62 Hz, 2 H), 4.86~5.21 (m, 1 H), 4.19 (s, 3 H), 3.25 (br d, J=7.83 Hz, 2 H), 3.03 (s, 5 H), 2.59~2.93 (m, 6 H), 2.29 (s, 3 H), 1.97~2.15 (m, 1 H), 1.63~1.79 (m, 1 H).

### Example 1.06

### 6-[5-Cyano-6-fluoro-8-(methylamino)-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid

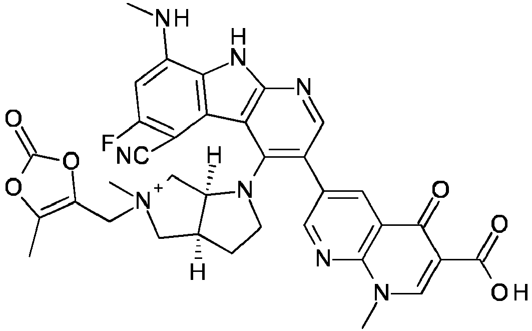

The titled compound was synthesized according to the following scheme:

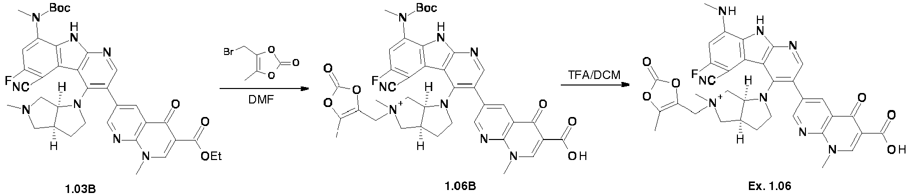

### Step (a) Preparation of 6-[8-[tert-butoxycarbonyl(methyl)amino]-5-cyano-6-fluoro-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido[2,3-b]indol-3-yl-1-methyl-4-oxo-1,8-naphthyridine-3-carboxylic acid (compound 1.06B)

During the synthesis of Example 1.05B, 6-[8-[*tert*-butoxycarbonyl(methyl)amino]-5-cyano-6-fluoro-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid was also obtained as another yellow solid (46 mg, 20.8% yield) after prep-HPLC purification. MS (ESI): 779.3 ([M+H]⁺). Analytical HPLC Rt = 3.37 min (slower eluted compared to compound 1.05B).

### Step (b) Preparation of 6-[5-cyano-6-fluoro-8-(methylamino)-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid (Example 1.06)

To a solution of 6-[8-[*tert*-butoxycarbonyl(methyl)amino]-5-cyano-6-fluoro-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido [2,3-b]indol-3-yl]-1-methyl-4-oxo-1,8-naphthyridine-3-carboxylic acid (50 mg, 0.06 mmol) in DCM (3.0 mL) was added TFA (1.0 mL, 12.9 mmol), and the resulting reaction mixture was stirred at 20 °C for 1 h. After LC-MS showed the starting material was consumed, the mixture solution was concentrated *in vacuo* to give a crude product, which was purified by prep-HPLC (TFA additive) to give 6-[5-cyano-6-fluoro-8-(methylamino)-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid (2.0 mg, 3.1% yield) as a yellow solid. MS (ESI): 679.2 ([M+H]⁺). ¹H NMR (400 MHz, DMSO-d6) δ ppm 14.56~14.85 (m, 1 H), 12.09 (br s, 1 H), 9.16~9.33 (m, 2 H), 8.67~8.74 (m, 1 H), 8.04~8.17 (m, 1 H), 7.01~7.05 (m, 1 H), 6.86~6.98 (m, 1 H), 6.65~6.73 (m, 1 H), 4.84~5.02 (m, 1 H), 4.32~4.43 (m, 1 H), 4.10~4.22 (m, 3 H), 3.81~4.06 (m, 2 H), 3.48~3.53 (m, 1 H), 3.34~3.45 (m, 2 H), 3.21~3.31 (m, 2 H), 2.93~3.20 (m, 6 H), 2.67~2.87 (m, 2 H), 2.52~2.56 (m, 1 H), 2.43 (br s, 1 H), 2.17~2.40 (m, 2 H), 2.07~2.15 (m, 1 H), 1.82~1.95 (m, 1 H), 1.50~1.79 (m, 3 H).

### Example 1.07

### 6-[5-Cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid

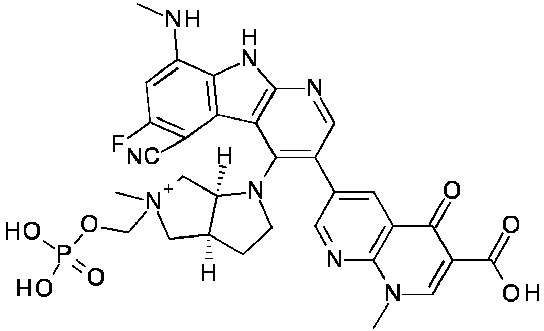

The titled compound was synthesized according to the following scheme:

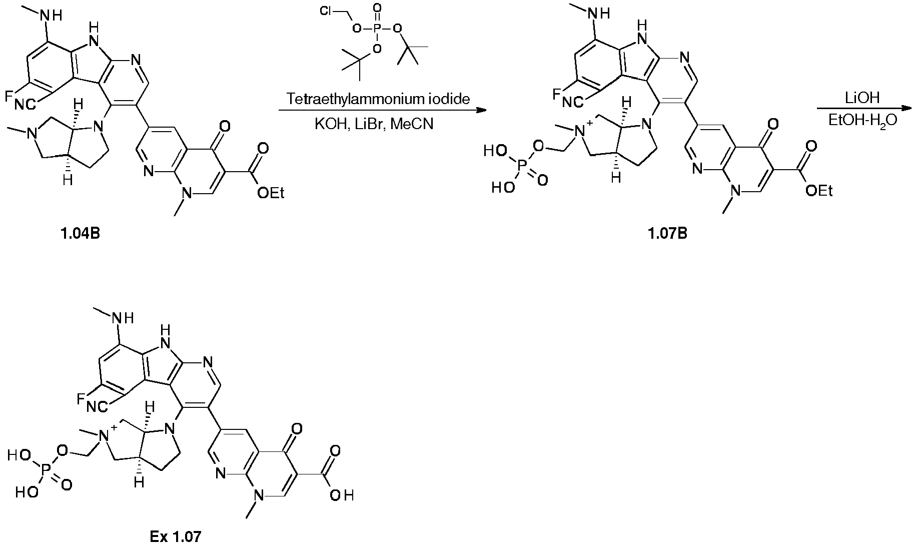

### Step (a) Preparation of ethyl 6-[5-cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methvy-4-oxo-1,8-naphthyridine-3-carboxylate (compound 1.07B)

A mixture solution of ethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (120.0 mg, 0.2 mmol, compound 1.04B), di-*tert*-butyl chloromethyl phosphate (49.0 mg, 0.2 mmol), lithium bromide (17.5 mg, 0.2 mmol), potassium hydroxide (11.3 mg, 0.2 mmol), and tetraethylammonium iodide (52.0 mg, 0.2 mmol) in MeCN (6 mL) was stirred at 20 °C for 12 h. Afterwards, the mixture was diluted with DCM (100 mL), poured into water (100 mL), and extracted with DCM (100 mL). The organic layer was washed with brine (100 mL), dried over anhy. Na₂SO₄, filtered, and concentrated *in vacuo* to give a crude product, which was purified prep-HPLC (first with 0.1% NH₄HCO₃ in water as additive and then 0.1% TFA in water as additive) to give ethyl 6-[5-cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylate (20 mg, 14.0% yield) as a yellow solid. MS (ESI): 705.3 ([M+H]⁺).

### Step (b) Preparation of 6-[5-cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-blindol-3-yl]-1-methyl-4-oxo-1,8-naphthyridine-3-carboxylic acid (Example 1.07)

To a solution of ethyl 6-[5-cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylate (13.0 mg, 0.02 mmol) in EtOH (3.2 mL) and water (0.65 mL) was added lithium hydroxide monohydrate (6.5 mg, 0.15 mmol), and the reaction mixture was stirred at 20 °C for 1 h. After LC-MS showed the starting material was consumed, the mixture was purified by prep-HPLC (TFA as additive) to give 6-[5-cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid (3.8 mg, 14.5% yield) as a yellow solid. MS (ESI): 677.3 ([M+H]⁺). ¹H NMR (400 MHz, Solvent) δ ppm 9.05 (br s, 1 H), 8.96 (s, 1 H), 8.69 (br s, 1 H), 7.98 (br s, 1 H), 6.44 (br d, J=13.08 Hz, 1 H), 4.05 (m, 3 H), 3.83 (br s, 2 H), 3.26 (br s, 6 H), 2.95 (br s, 3 H), 2.90 (s, 3 H), 1.81~1.91 (m, 1H), 1.10~1.12 (m, 1H).

### Example 1.08

### 2-[(2S)-2-Amino-3-methyl-butanoyl]oxyethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate

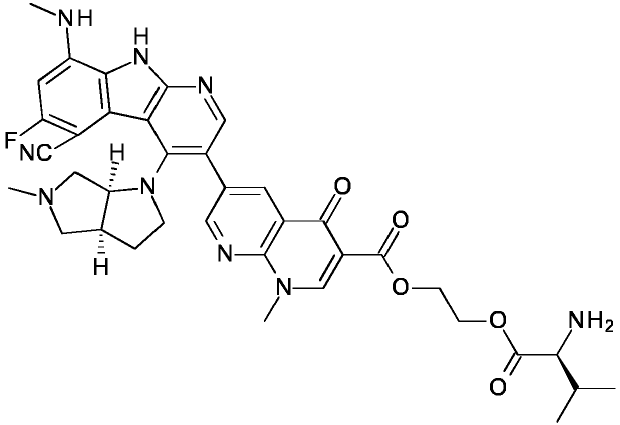

The titled compound was synthesized according to the following scheme:

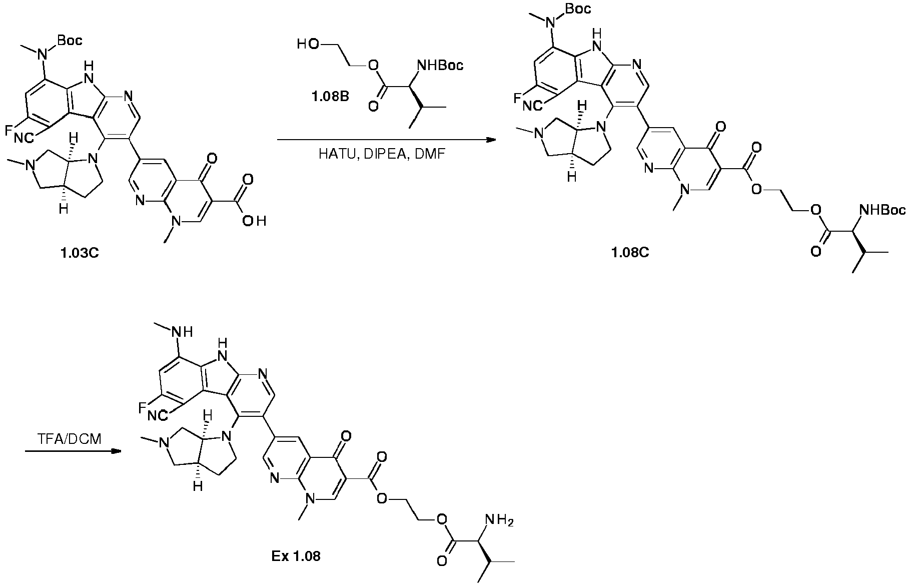

### Step (a) Preparation of (S)-2-hydroxyethyl 2-(tert-butoxycarbonylamino)-3-methyl-butanoate (compound 1.08B)

To a solution of (2S)-2-(*tert*-butoxycarbonylamino)-3-methyl-butanoic acid (500.0 mg, 2.3 mmol) and ethylene glycol (0.13 mL, 2.3 mmol) in DCM (4.72 mL) was added DMAP (281.16 mg, 2.3 mmol) and DIC (1.15 mL, 2.3 mmol), and the resulting reaction mixture was stirred at 25 °C for 1 h. After LC-MS showed the starting material was consumed, the mixture was concentrated *in vacuo* to give a crude product, which was purified by prep-HPLC (TFA as additive) to give (S)-2-hydroxyethyl 2-(*tert*-butoxycarbonylamino)-3-methyl-butanoate (370 mg, 61.5% yield) as colorless oil. MS (ESI): 284.1 ([M+Na]⁺).

### Step (b) Preparation of 2-[(2S)-2-(tert-butoxycarbonylamino)-3-methyl-butanoyl]oxyethyl 6-[8-[tert-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.08C)

To a solution of 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (100.0 mg, 0.150 mmol, compound 1.03C) and (S)-2-hydroxyethyl 2-(*tert*-butoxycarbonylamino)-3-methyl-butanoate (58.8 mg, 0.22 mmol) in DMF (5.11 mL) was added HATU (68.4 mg, 0.18 mmol) and DIPEA (0.1 mL, 0.6 mmol). The resulting reaction mixture was stirred at 25 °C for 12 h before it was poured into water (100 mL) and extracted with EtOAc (100 mL) three times. Combined organics was then washed with aq. CaCh solution (100 mL) three times, brine (100 mL), dried with anhy. Na₂SO₄, filtered, and concentrated *in vacuo.* The residue was purified first by prep-TLC (DCM: MeOH=20:1) and then prep-HPLC (TFA as additive) to give 2-[(2S)-2-(*tert-*butoxycarbonylamino)-3-methyl-butanoyl]oxyethyl 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (50 mg, 36.6% yield) as a yellow solid. MS (ESI): 910.5 ([M+H]⁺).

### Step (c) Preparation of 2-[(2S)-2-Amino-3-methyl-butanovl]oxvethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (Example 1.08)

To a solution of 2-[(2S)-2-(*tert*-butoxycarbonylamino)-3-methyl-butanoylloxyethyl 6-[8-[*tert-*butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (30.0 mg, 0.030 mmol) in DCM (3 mL) was added TFA (0.01 mL, 0.160 mmol), and the resulting mixture solution was stirred at 25 °C for 1 h. After LC-MS showed the starting material was consumed, the mixture solution was adjust to pH = 6~8 by aq. NH₄HCO₃ solution and evaporated to dryness under the reduced pressure. The residue was then purified by prep-HPLC (NH₄HCO₃ as additive) to give 2-(((S)-2-amino-3-methylbutanoyl)oxy)ethyl 6-(5-cyano-6-fluoro-8-(methylamino)-4-((3aS,6aS)-5-methylhexahydropyrrolo [2,3-c]pyrrol-1(2H)-yl)-9H-pyrido[2,3-b]indol-3-yl)-1-methyl-4-oxo-1,4-dihydro-1,8-naphthyridine-3-carboxylate (10.1 mg, 41.7% yield) as a yellow solid. MS (ESI): 710.2 ([M+H]⁺). ¹H NMR (400 MHz, DMSO-*d*₆) δ ppm: 11.89 (br s, 1 H), 8.97 (d, *J*=2.4 Hz, 1 H), 8.88 (s, 1 H), 8.54 (d, *J*=2.4 Hz, 1 H), 7.98 (s, 1 H), 6.85 (br s, 1 H), 6.64 (d, *J*=13.1 Hz, 1 H), 4.64 (br d, *J*=6.2 Hz, 1 H), 4.40 - 4.47 (m, 4 H), 4.35 - 4.39 (m, 1 H), 4.00 (s, 3 H), 2.99 (d, *J*=4.6 Hz, 3 H), 2.88 - 2.95 (m, 2 H), 2.74 (br d, *J*=6.5 Hz, 2 H), 2.38 (br d, *J*=8.4 Hz, 1 H), 2.21 (br d, *J*=8.9 Hz, 3 H), 2.08 (s, 3 H), 1.88 - 1.94 (m, 1 H), 1.69 (br dd, *J*=9.4, 5.0 Hz, 1 H), 1.42 (br s, 1 H), 0.86 (dd, *J*=12.1, 6.8 Hz, 6 H).

### Example 1.09

### 2-[[2-[[6-[5-Cyano-6-fluoro-8-(methylamino)-4-[(3aS,6aS)-5-methyl-2,3,3a,4,6,6a-hexahydropyrrolo[2,3-c]pyrrol-1-yll-9H-pyrido[2,3-b]indol-3-yl]-1-methyl-4-oxo-1,8-naphthyridine-3-carbonyl]amino]acetyl]amino]acetic acid

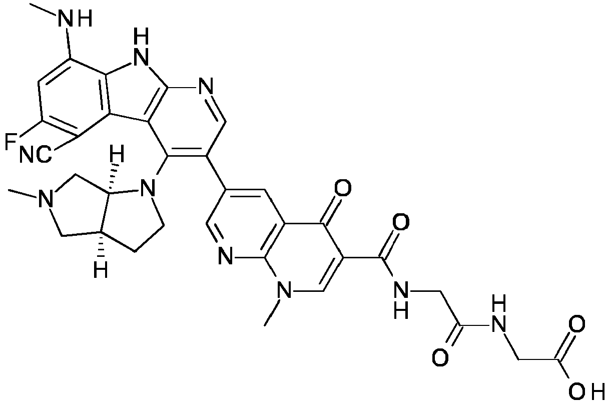

The titled compound was synthesized according to the following scheme:

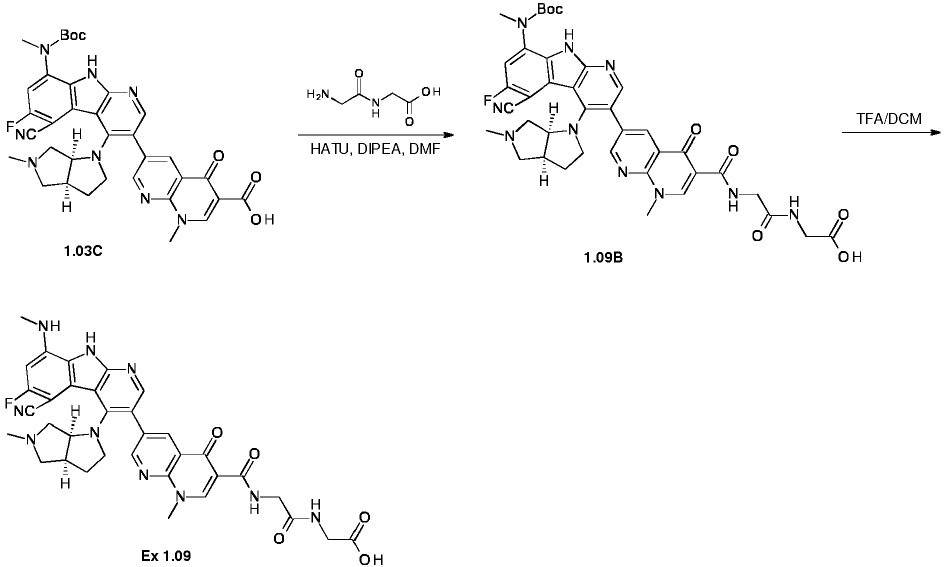

### Step (a) Preparation of 2-[[2-[[6-[8-[tert-butoxycarbonyl(methyl)aminol-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-v1]-1-methyl-4-oxo-1,8-naphthyridine-3-carbonyl]aminolacetyl]amino]acetic acid (compound 1.09B)

To a solution of 6-[8-[*tert-*butoxycarbonyl(methyl)amino]-5-cyano-6-fluoro-4-[(3aS,6aS)-5-methyl-2,3,3a,4,6,6a-hexahydropyrrolo [2,3-c]pyrrol-1-yl]-9H-pyrido [2,3-b]indol-3-yl]-1-methyl-4-oxo-1,8-naphthyridine-3-carboxylic acid (30.0 mg, 0.04 mmol, compound 1.03C) in DMF (1.5 mL) was added HATU (20.5 mg, 0.050 mmol) and DIPEA (0.02 mL, 0.130 mmol) under N₂. The mixture was stirred at 25 °C for 0.5 h before glycylglycine (11.9 mg, 0.09 mmol) was added and the resulting reaction mixture was stirred at 25 °C for another 2 h. After LC-MS showed the starting material was consumed completely, the mixture was subject to prep-HPLC purification (TFA as additive) to give 2-[[2-[[6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carbonyl]amino]acetyl]amino]acetic acid (16 mg, 44.2% yield) as a yellow solid. MS (ESI): 781.4 ([M+H]⁺).

### Step (b) Preparation of 2-[[2-[[6-[8-[tert-butoxycarbonyl(methyl)amino1-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carbonyl]amino]acetyl]amino]acetic acid (Example 1.09)

To a solution of 2-[[2-[[6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carbonyl]amino]acetyl]amino]acetic acid (14.0 mg, 0.020 mmol) in DCM (5 mL) was added TFA (0.04 mL, 0.540 mmol), and the resulting mixture was stirred at 25 °C for 1 h. After LC-MS showed the starting material was consumed completely, the mixture solution was concentrated *in vacuo* to give a crude product, which was purified by prep-HPLC (TFA as additive) to give 2-[[2-[[6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl] -1-methyl-4-oxo-1,8-naphthyridine-3-carbonyl]amino] acetyl]amino] acetic acid (13.6 mg, 94.6% yield) as a yellow solid. MS (ESI): 681.2 ([M+H]⁺). ¹H NMR (400 MHz, DMSO-d6) δ ppm 11.99 - 12.16 (m, 1 H), 9.99 - 10.16 (m, 1 H), 9.34 (s, 1 H), 9.07 - 9.19 (m, 2 H), 8.67 (d, J=2.32 Hz, 1 H), 8.41 (q, J=5.87 Hz, 1 H), 8.06 - 8.25 (m, 1 H), 6.94 (s, 1 H), 6.66 - 6.79 (m, 1 H), 4.64 (s, 1 H), 4.06 - 4.17 (m, 5 H), 3.81 (d, J=5.87 Hz, 2 H), 3.02 (d, J=2.45 Hz, 4 H), 2.66 - 2.89 (m, 5 H), 2.55 (s, 2 H), 2.06 - 2.28 (m, 1 H).

### Example 1.10

### 6-[5-Cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-N-methylsulfonyl-4-oxo-1,8-naphthyridine-3-carboxamide

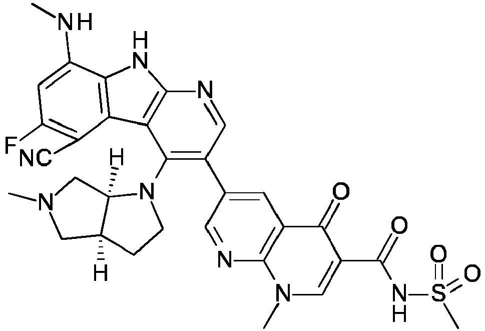

To a solution of 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (80.0 mg, 0.14 mmol, compound 1.03D) in DCM (5 mL) was added thionyl chloride (33.6 mg, 0.280 mmol) at 0 °C. Afterwards, the reaction mixture was allowed to warm up to r.t. and stirred at 25 °C for 2 h before it was added into a solution of methanesulfonamide (67.2 mg, 0.71 mmol) in DCM (5 mL). The resulting mixture solution was stirred at 25 °C for addition 16 h and then concentrated under the reduced pressure. The residue was subject to prep-HPLC (NH₃H₂O as additive) purification to give 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-N-methylsulfonyl-4-oxo-1,8-naphthyridine-3-carboxamid (8.1 mg, 9.0% yield) as a yellow solid. MS (ESI): 644.1 ([M+H]⁺). ¹H NMR (400 MHz, DMSO-*d*₆) δ ppm: 11.87 (br s, 1 H), 9.18 (s, 1 H), 9.10 (d, *J*=2.2 Hz, 1 H), 8.72 (d, *J*=2.3 Hz, 1 H), 8.00 (s, 1 H), 6.82 (br d, *J*=4.2 Hz, 1 H), 6.65 (d, *J*=13.0 Hz, 1 H), 4.14 (s, 3 H), 3.39 (br s, 3 H), 2.99 (br d, *J*=4.5 Hz, 3 H), 2.93 (br d, *J*=10.8 Hz, 2 H), 2.73 - 2.79 (m, 2 H), 2.41 (br d, *J*=5.0 Hz, 2 H), 2.19 - 2.23 (m, 2 H), 2.11 (s, 3 H), 1.69 (br dd, 7=9.8, 5.2 Hz, 1 H), 1.42 (br s, 1 H).

### Example 1.11

### 1-Acetoxyethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate

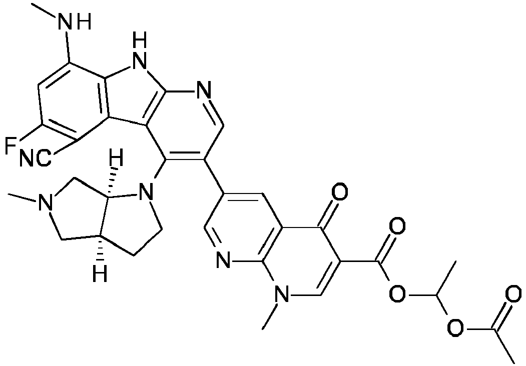

To a solution of 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (100mg, 0.18 mmol, compound 1.03D) in DMF (1 mL) was added potassium carbonate (48.1 mg, 0.35 mmol), and the mixture was stirred at 20 °C for 0.5 h before 1-bromoethyl acetate (65.5 mg, 0.39 mmol) was added. The resulting mixture was stirred at 20 °C for another 16 h before concentrated under reduced pressure. The residue was subject to Prep-HPLC (TFA as additive) purification to give 1-acetoxyethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (4.5 mg, 4.0% yield) as a yellow solid. MS (ESI): 653.3 ([M+H]⁺). ¹H NMR (400 MHz, DMSO-*d*₆) δ ppm: 12.08 (br s, 1H), 9.60 (br s, 1H), 9.07 - 8.95 (m, 2H), 8.53 (br s, 1H), 8.20 - 8.02 (m, 1H), 6.95 (q, *J*=5.3 Hz, 2H), 6.69 (br d, *J*=13.1 Hz, 1H), 4.86 - 4.41 (m, 1H), 4.04 (s, 3H), 3.29 - 3.05 (m, 4H), 3.01 (s, 4H), 2.74 - 2.65 (m, 4H), 2.26 - 2.13 (m, 1H), 2.07 (s, 4H), 1.66 (br s, 1H), 1.53 (d, *J*=5.4 Hz, 3H).

### Example 1.12

### 1-Isopropoxycarbonyloxyethyl 6-[6-fluoro-5-methyl-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate

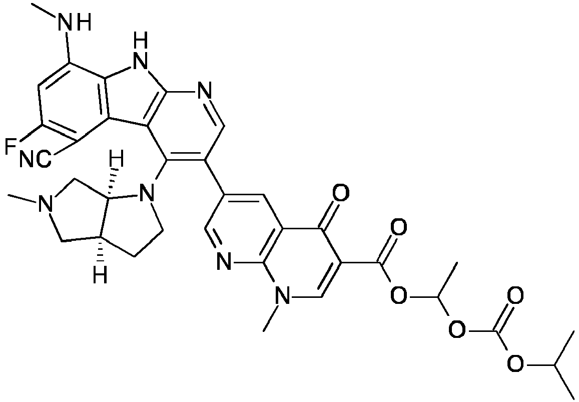

To a solution of 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (100 mg, 0.18 mmol, compound 1.03D) in DMSO (2 mL) was added potassium carbonate (48.1 mg, 0.35 mmol), and the reaction mixture was stirred at 20°C for 0.5 h before 1-chloroethyl isopropyl carbonate (67.3 mg, 0.40 mmol) was added. The resulting mixture was stirred at 20°C for another 16 h and then concentrated under the reduced pressure. The residue was subject to prep-HPLC (TFA as additive) purification to give 1-isopropoxycarbonyloxyethyl 6-[6-fluoro-5-methyl-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (11.5 mg, 9.0% yield) as a yellow solid. MS (ESI): 697.4 ([M+H]⁺). ¹H NMR (400 MHz, DMSO-*d*₆) δ ppm: 12.14 (br s, 1H), 9.74 (br s, 1H), 9.08 - 8.89 (m, 2H), 8.53 (s, 1H), 8.20 - 8.00 (m, 1H), 7.21 - 7.03 (m, 1H), 6.90 - 6.79 (m, 1H), 6.68 (br d, *J*=12.8 Hz, 1H), 4.87 - 4.74 (m, 1H), 4.05 (s, 3H), 3.00 (s, 4H), 2.74 - 2.67 (m, 4H), 1.56 (d, *J*=5.4 Hz, 3H), 1.24 (d, *J*=6.2 Hz, 6H).

### Example 1.13

### (5-Methyl-2-oxo-1,3-dioxol-4-yl)methyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate

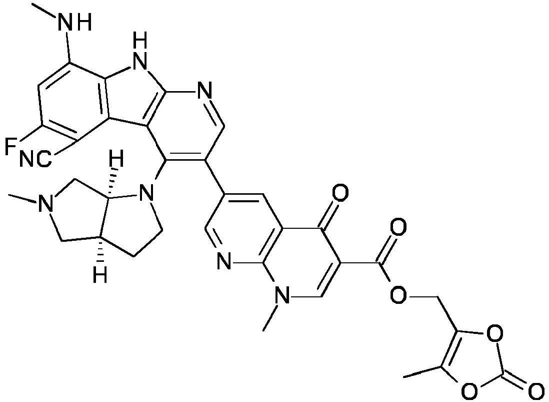

The titled compound was synthesized according to the following scheme:

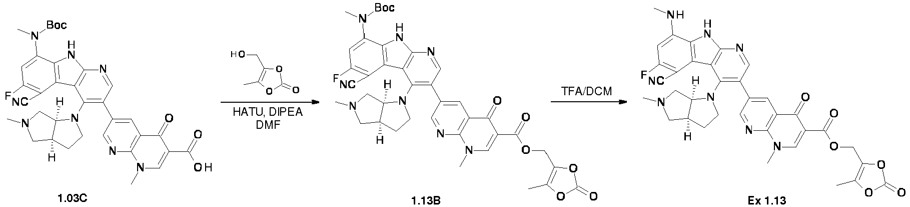

### Step (a) Preparation of (5-methyl-2-oxo-1,3-dioxol-4-yl)methyl 6-[8-[tert-butoxycarbonyl(methyl)amino1-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.13B)

A mixture of 6-[8-[*tert*-butoxycarbonyl(methyl)amino]-5-cyano-6-fluoro-4-[(3aS,6aS)-5-methyl-2,3,3a,4,6,6a-hexahydropyrrolo [2,3-c]pyrrol-1-yl]-9H-pyrido [2,3-b]indol-3-yl]-1-methyl-4-oxo-1,8-naphthyridine-3-carboxylic acid (100 mg, 0.15 mmol. compound 1.03C), 4-(hydroxymethyl)-5-methyl-1,3-dioxol-2-one (100 mg, 0.77 mmol), DCC (100 mg, 0.48 mmol), and DMAP (30 mg, 0.25 mmol) in DMF (3.0 mL) was stirred at 20 °C for 12 h. After LC-MS showed the starting material was consumed, the mixture solution was concentrated *in vacuo* to give a crude product, which was purified by prep-HPLC (TFA as additive) to give (5-methyl-2-oxo-1,3-dioxol-4-yl)methyl 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (45.0 mg, 38.5% yield) as an off-white solid. MS (ESI): 779.3 ([M+H]⁺).

### Step (b) Preparation of (5-methyl-2-oxo-1,3-dioxol-4-yl)methyl 6-[5-cyano-6-fluoro-8-(methylamino)-4-[(3aS,6aS)-5-methyl-2,3,3a4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (Example 1.13)

To a solution of (5-methyl-2-oxo-1,3-dioxol-4-yl)methyl 6-[8-[*tert-*butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (45.0 mg, 0.06 mmol) in DCM (3.0 mL) was added TFA (1.0 mL, 12.9 mmol), and the resulting reaction mixture was stirred at 20 °C for 1 h. After LC-MS showed the starting material was consumed, the mixture solution was concentrated *in vacuo* to give a crude product, which was purified by prep-HPLC (TFA as additive) to give (5-methyl-2-oxo-1,3-dioxol-4-yl)methyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (11.1 mg, 18.7% yield) as a yellow solid. MS (ESI): 679.3 ([M+H]⁺). ¹H NMR (400 MHz, DMSO-d6) δ ppm 12.03~12.14 (m, 1 H), 9.53 (br s, 1 H), 8.95~9.10 (m, 2 H), 8.54 (d, J=2.32 Hz, 1 H), 8.07~8.20 (m, 1 H), 8.07 - 8.20 (m, 1 H), 6.97 (br s, 1 H), 6.67~6.78 (m, 1 H), 5.16 (s, 2 H), 4.68 (br s, 1 H), 4.05 (s, 3 H), 3.67 (br s, 3 H), 3.42 (br s, 1 H), 3.23 (br s, 2 H), 3.07~3.15 (m, 1 H), 3.02 (s, 3 H), 2.65~2.87 (m, 4 H), 2.31~2.36 (m, 1 H), 2.24 (s, 3 H), 2.04~2.15 (m, 1 H), 1.68 (br s, 1 H).

### Example 1.14

### 6-[8-[(2-Aminoacetyl)oxymethoxycarbonyl-methyl-amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

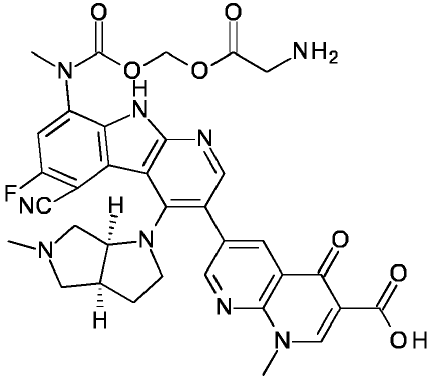

The titled compound was synthesized according to the following scheme:

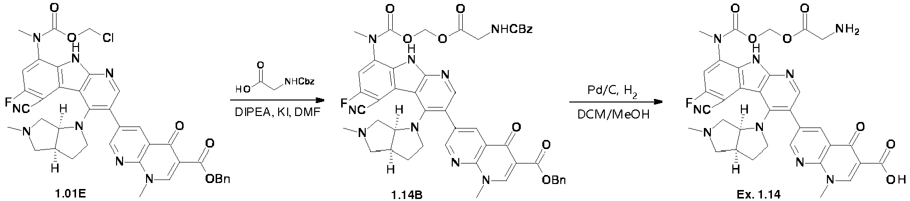

### Step (a) Preparation of benzyl 6-[8-[[2-(benzyloxycarbonylamino)acetyl]oxymethoxycarbonyl-methyl-amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.14B)

To a solution of benzyl 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (200.0 mg, 0.270 mmol, compound 1.01E), DIPEA (0.23 mL, 1.33 mmol), and potassium iodide (0.07 mL, 1.33 mmol) in DMF (12 mL) was added N-benzyloxycarbonylglycine (279.23 mg, 1.33 mmol, CAS: 1138-80-3). The resulting reaction mixture was stirred at 50 °C for 2 h until LC-MS showed the starting material was consumed completely. The mixture solution was diluted with EtOAc (300.0 mL) and washed with water (100.0 mL) three times, and brine (100 mL) three times. The organic layer was dried over anhy. Na₂SO₄, filtered, and concentrated *in vacuo* to give a crude product, which was purified by prep-HPLC (formic acid as additive) purification. The collected eluents was basified to pH > 7 with NH₄HCO₃ solid and extracted with EtOAc (150.0 mL) three times. Combined organics was dried over anhy. Na₂SO₄, filtered, and evaporated to dryness to give benzyl 6-[8-[[2-(benzyloxycarbonylamino)acetyl]oxymethoxycarbonyl-methyl-amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (150 mg, 55.6% yield) as a yellow solid. MS (ESI): 922.4 (([{³⁵C1}M+H]⁺).

### Step (b) Preparation of 6-[8-[(2-aminoacetyl)oxymethoxycarbonyl-methyl-aminol-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.14)

A solution of benzyl 6-[8-[[2-(benzyloxycarbonylamino)acetyl]oxymethoxycarbonyl-methyl-amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (130.0 mg, 0.140 mmol) in DCM (0.919 mL) and MeOH (0.919 mL) was added palladium on carbon (130 mg). After the suspension was degassed under vacuum and purged with H₂ several times, the mixture solution was stirred at 25 °C for 3 h under H₂ atmosphere (15 psi). After LC-MS showed the starting material was consumed completely, the reaction mixture was filtered through Celite. The filtrate was then concentrated *in vacuo* to give a crude product of 6-[8-[(2-aminoacetyl)oxymethoxycarbonyl-methyl-amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (50 mg, 20.6% yield) as a yellow solid. MS (ESI): 698.3 ([M+H]⁺).

### Example 1.15

### 6-[8-[1-Acetoxyethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

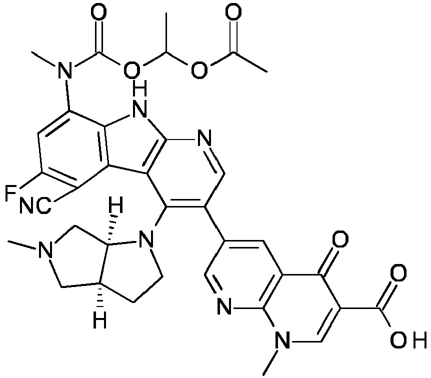

The titled compound was synthesized according to the following scheme:

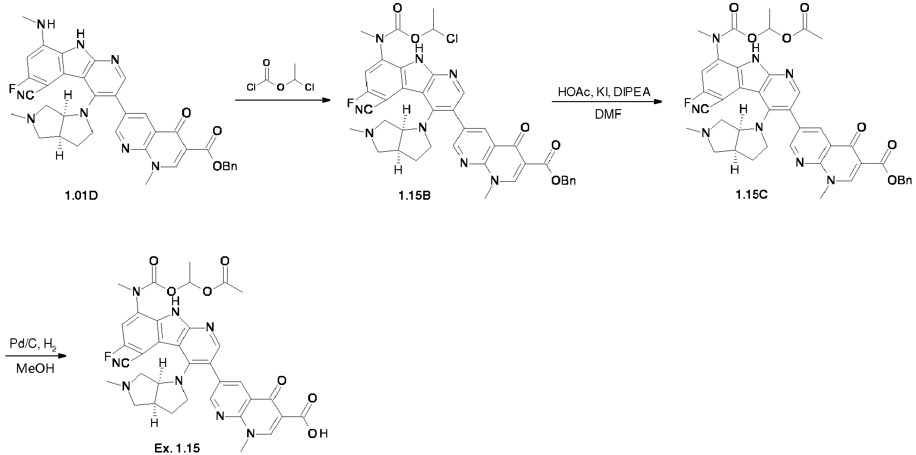

### Step (a) Preparation of benzyl 6-[8-[1-chloroethoxycarbonyl(methyl)amino]-5-cyano-6-fluoro-4-[(3aS,6aS)-5-methyl-2,3,3a,4,6,6a-hexahydrot)yrrolo[2,3-c]pyrrol-1-yl]-9H-pyrido[2,3-b]indol-3-yl]-1-methyl-4-oxo-1,8-naphthyridine-3-carboxylate (compound 1.15B)

A mixture solution of benzyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (200 mg, 0.30 mmol, compound 1.01D) and 1-chloroethyl chloroformate (40.0 g, 279.8 mmol) was stirred at 25 °C for 12 h. After LC-MS showed the starting material was consumed completely, the mixture was concentrated *in vacuo* to give a crude product of benzyl 6-[8-[1-chloroethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (180 mg, 77.4% yield) as a light yellow solid. MS (ESI): 763.2 ([M+H]⁺). It was used directly in the next step without further purification.

### Step (b) Preparation of benzyl 6-[8-[1-acetoxyethoxycarbonyl(methyl)aminol-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.15C)

To a solution of benzyl 6-[8-[1-chloroethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (150 mg, 0.20 mmol), DIPEA (0.17 mL, 0.98 mmol), and potassium iodide (0.05 mL, 0.980 mmol) in DMF (15 mL) was added acetic acid (59.0 mg, 0.98 mmol). The resulting reaction mixture was stirred at 50 °C for 2 h until LC-MS showed the starting material was consumed. The mixture solution was diluted with EtOAc (400.0 mL) and washed with water (100.0 mL) three times and brine (100 mL) three times. Combined organics was dried over anhy. Na₂SO₄, filtered, and concentrated *in vacuo* to give a crude product, which was purified by prep-TLC (EtOAc:MeCN = 1:1) to give benzyl 6-[8-[1-acetoxyethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (90 mg, 46.0% yield) as a yellow solid. MS (ESI): 787.3 ([M+H]⁺).

### Step (c) Preparation of 6-[8-[1-acetoxyethoxycarbonyl(methyl)amino1-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.15)

To a solution of benzyl 6-[8-[1-acetoxyethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (80.0 mg, 0.10 mmol) in THF (4 mL) was added palladium on carbon (80.0 mg). After the suspension was degassed under vacuum and purged with H₂ several times, the mixture solution was stirred at 25 °C for 1 h under H₂ atmosphere (15 psi). After LC-MS showed the starting material was consumed completely, the reaction mixture was filtered through Celite. The filtrate was then concentrated *in vacuo* to give a crude product, which was purified by prep-HPLC (NH₄HCO₃ as additive) to give 6-[8-[1-acetoxyethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (30.3 mg, 42.4% yield) as a light yellow solid. MS (ESI): 697.2 ([M+H]⁺). ¹H NMR (400 MHz, DMSO-d₆) δ ppm 12.65 (br s, 1 H), 9.08 - 9.39 (m, 2 H), 8.75 (br s, 1 H), 8.12 (br s, 1 H), 7.72 (br d, J=10.8 Hz, 1 H), 6.77 (br s, 1 H), 4.58 (br s, 1 H), 4.16 (br s, 3 H), 3.44 - 3.60 (m, 2 H), 2.79 - 3.02 (m, 3 H), 2.53 (d, J=2.0 Hz, 2 H), 2.45 (br s, 1 H), 1.97 - 2.27 (m, 9 H), 1.43 - 1.72 (m, 3 H), 1.18 (br s, 2 H).

### Example 1.16

### 6-[8-[Diethoxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

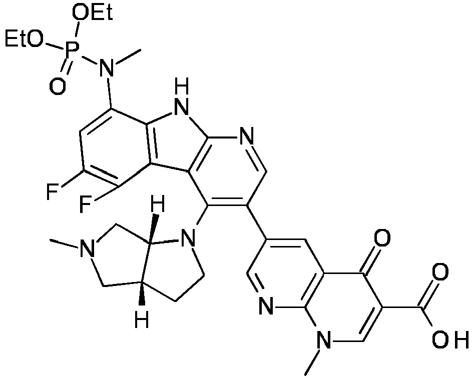

The titled compound was synthesized according to the following scheme:

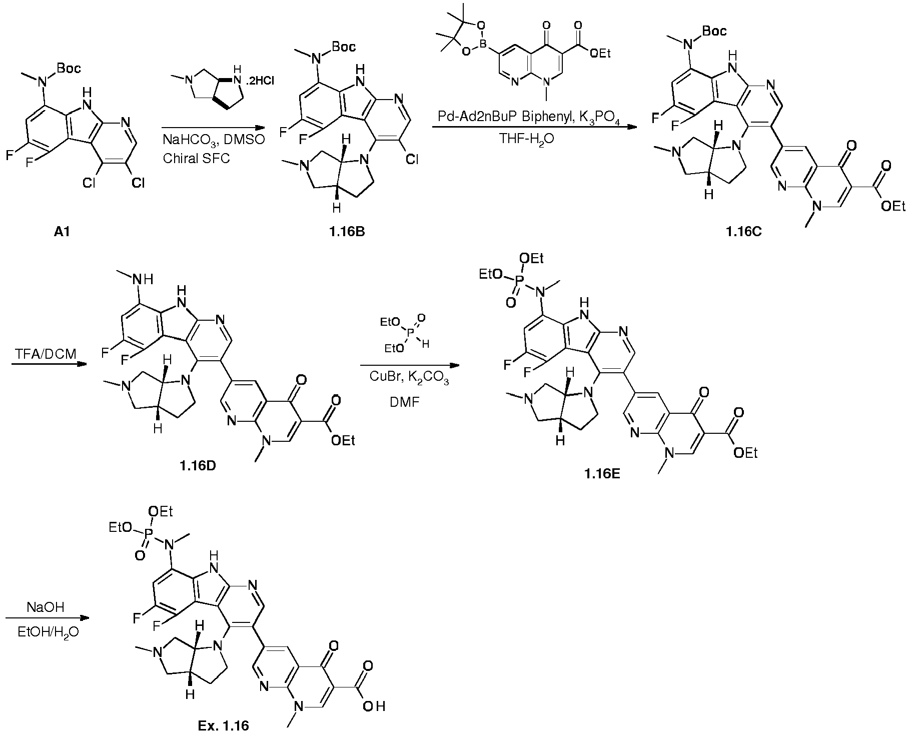

### Step (a) Preparation of tert-butyl N-[3-chloro-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.16B)

A mixture solution of *tert*-butyl N-(3,4-dichloro-5,6-difluoro-9H-pyrido[2,3-b]indol-8-yl)-N-methyl-carbamate (24.0 g, 59.67 mmol, Intermediate A1), 5-methyl-1H-hexahydropyrrolo[2,3-c]pyrrole dihydrochloride (14.9 g, 74.59 mmol, CAS:1197193-15-9) and NaHCO₃ (25.1 g, 298.4 mmol) in DMSO (40.0 mL) was stirred at 45 °C for 1 h until evolution of CO₂ had ceased. The reaction mixture was then allowed to heated at 120 °C for another 47 h until LC-MS showed the starting material was consumed completely. The mixture was cooled back to r.t., poured into water (500.0 mL), and the resulting precipitate was collected by filtration. The filter cake was then recrystallized in MeOH / EtOAc (v/v = 5:1, 400 mL) to give a racemic mixture of compound 1.16B as a white solid. It was subject to chiral SFC separation (column: Chiralcel OD, 50×4.6mm I.D., particle size 3µm (Daicel); mobile phase: A: 95% CO₂, B: 5% IPA (0.05% Et₂NH); flow rate: 3 mL/min) ) to give *tert*-butyl N-[3-chloro-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-N-methyl-carbamate (9.9 g, 41.1% yield, > 98%ee, optical rotation [α]_{D} = + 26.6°, HPLC Rf = 6.29 min) as a white solid. MS (ESI): 492.2 ([{³⁵Cl}M+H] ⁺), 494.2 ([{³⁷Cl}M+H]⁺). Another enantiomeric product obtained matched with compound 1.26 B, tert-butyl N-[3-chloro-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-N-methyl-carbamate (9.4 g, 39% yield, > 98%ee, optical rotation [α]_{D} = + 27.7°, HPLC Rf = 5.45 min).

### Step (b) Preparation of ethyl 6-[8-[tert-butoxycarbonyl(methyl)amino]-5,6-difluoro-4-[(3aR,6aR)-5-methyl-2,3,3a,4,6,6a-hexahydropyrrolo[2,3-c]pyrrol-1-yl-9H-pyrido[2,3-b]indol-3-yl]-1-methyl-4-oxo-1,8-naphthyridine-3-carboxylate (compound 1.16C)

To a solution of *tert*-butyl N-[3-chloro-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-N-methyl-carbamate (100.0 mg, 0.203 mmol), benzyl 1-methyl-4-oxo-6-(4,4,5,5-tetramethyl-1,3,2-dioxaborolan-2-yl)-1,8-naphthyridine-3-carboxylate (109 mg, 0.31 mmol, Intermediate B3), and K₃PO₄ (172 mg, 0.812 mmol) in THF / H₂O (4.0 mL, v/v=5:1) was added Pd-Ad2nBuP Biphenyl (27.1 mg, 0.04 mmol, cataCXium A-Pd-G2, CAS: 1375477-29-4) in glovebox under argon. The mixture solution was stirred at 70 °C for 12 h until LC-MS showed the starting material was consumed completely. The mixture was then cooled back to r.t., poured into water (50 mL) and extracted with EtOAc (100 mL) twice. Combined organics was washed with brine (50 mL) twice, dried with anhy. Na₂SO₄, filtered, and concentrated *in vacuo* to give a crude product, which was purified by prep-HPLC (TFA as additive) to give ethyl 6-[8-[*tert*-butoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (115.0 mg, 82.3% yield) as a yellow solid. MS (ESI): 688.4 ([M+H]⁺).

### Step (c) Preparation of ethyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.16D)

To a solution of ethyl 6-[8-[*tert*-butoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (300 mg, 0.44 mmol) in DCM (5 mL) was added TFA (18.95 mL, 75.8 mmol), and the resulting mixture solution was stirred at 20 °C for 2 h. After LC-MS showed the starting material was consumed, the mixture was concentrated *in vacuo* to give a crude product, which was purified by prep-HPLC (TFA as additive) to give ethyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (200 mg, 78.0% yield) as a yellow solid. MS (ESI): 588.3 ([M+H]⁺).

### Step (d) Preparation of ethyl 6-[8-[diethoxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.16E)

To a solution of ethyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (50.0 mg, 0.09 mmol) in DMF (2 mL) was added diethyl phosphite (23.5 mg, 0.17 mmol) and CuBr (15.9 mg, 0.11 mmol) under nitrogen. The mixture was stirred at 60 °C for 15 h until LC-MS showed the starting material was consumed completely. The mixture was cooled back to r.t. and concentrated *in vacuo* to give a crude product, which was purified by prep-HPLC (TFA as additive) to give ethyl 6-[8-[diethoxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (15 mg, 24.4% yield) as a black solid. MS (ESI): 724.3 ([M+H]⁺).

### Step (e) Preparation of 6-[8-[diethoxyphosphoryl(methyl)amino]-5,6-difluoro-4-(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.16)

To a solution of ethyl 6-[8-[diethoxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (74.0 mg, 0.10 mmol) in EtOH (2 mL) was added NaOH (41.9 mg, 1.02 mmol), and the resulting mixture was stirred at 25 °C for 2 h. After LC-MS showed the starting material was consumed completely, the solution was acidified with aq. HCl solution (1N) to pH = 6, and extracted with DCM (50 mL) three times. Combined organics was washed with brine, dried over anhy. Na₂SO₄, filtered and concentrated *in vacuo* to give a crude product, which was purified by prep-HPLC (TFA as additive). The combined eluents were evaporated to dryness under the reduced pressure, and the residue was re-dissolved in MeOH and basified with aq. NaHCO₃ solution to pH = 7. The solution was subject to another Prep-HPLC (NH₄HCO₃ as additive) purification to give 6-[8-[diethoxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (21.6 mg, 29.2% yield) as a white solid. MS (ESI): 696.4 ([M+H]⁺). ¹H NMR (400 MHz, DMSO-d6) δ12.51 (br s, 1H), 9.29 (s, 1H), 9.12 (br s, 1H), 8.69 (d, J=2.1 Hz, 1H), 8.31 (br d, J=15.5 Hz, 1H), 7.54 - 7.42 (m, 1H), 7.35 - 7.04 (m, 1H), 4.19 (s, 3H), 4.17 - 4.06 (m, 4H), 3.66 (br s, 1H), 3.57 - 3.32 (m, 3H), 3.12 (br d, J=9.3 Hz, 2H), 3.19 - 2.99 (m, 1H), 3.01 (br s, 1H), 2.94 - 2.78 (m, 2H), 2.74 - 2.63 (m, 4H), 2.21 - 1.99 (m, 1H), 1.96 - 1.75 (m, 1H), 1.35 (s, 2H), 1.29 - 1.16 (m, 7H).

### Example 1.17

### 6-[8-[[Benzyloxy(hydroxy)phosphoryl]-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

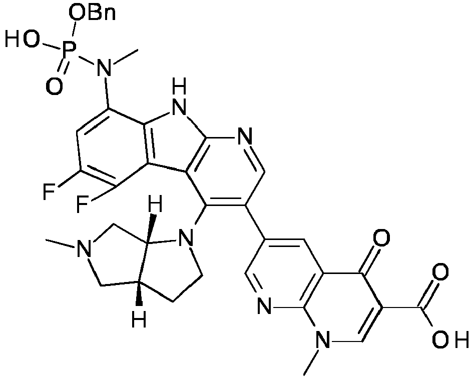

The titled compound was synthesized according to the following scheme:

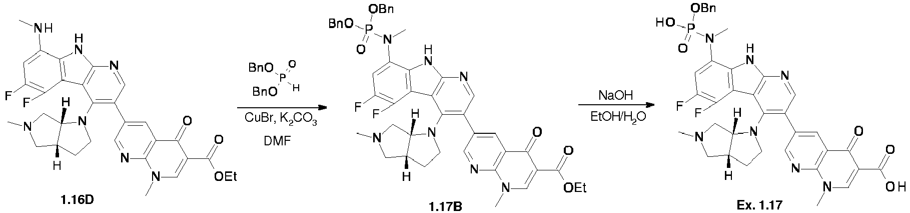

### Step (a) Preparation of ethyl 6-[8-[dibenzyloxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.17B)

To a solution of ethyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (150.0 mg, 0.260 mmol, compound 1.16D) in DMF (2 mL) was added Et₃N (129.15 mg, 1.28 mmol), dibenzyl phosphite (133.88 mg, 0.510 mmol) and CuBr (47.74 mg, 0.330 mmol). The mixture solution was stirred at 50 °C for 15 h until LC-MS showed the starting material was consumed completely. The mixture solution was cooled back to r.t., concentrated *in vacuo* to give a crude product, which was purified by prep-HPLC (TFA as additive) to give ethyl 6-[8-[dibenzyloxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (90 mg, 44.9% yield) as a yellow solid. MS (ESI): 848.4 ([M+H]⁺).

### Step (b) Preparation of 6-[8-[[benzyloxy(hydroxy)phosphoryl]-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.17)

To a solution of ethyl 6-[8-[dibenzyloxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (90.0 mg, 0.110 mmol) in EtOH (2 mL) was added aq. NaOH solution (2.0 mL, 4 mmol), and the resulting solution was stirred at 65 °C for 15 h. After LC-MS showed the starting material was consumed completely, the mixture was acidified with aq. HCl solution (1N) to pH = 7. The solution was subject to prep-HPLC (NH₃•H₂O as additive) purification to give benzyloxy(hydroxy)phosphoryl]-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (5.1 mg, 6.5% yield) as a yellow solid. MS (ESI): 730.3 ([M+H]⁺). ¹H NMR (400MHz, DMSO-d₆) δ 14.81 (br s, 1H), 14.18 (br s, 1H), 9.27 (s, 1H), 9.14 (br s, 1H), 8.71 (br s, 1H), 8.16 (br s, 1H), 7.25 (br s, 1H), 7.23 - 7.05 (m, 4H), 7.00 (br s, 1H), 7.05 - 6.93 (m, 1H), 4.78 - 4.27 (m, 3H), 4.18 (s, 3H), 3.22 (br d, J=7.0 Hz, 7H), 2.89 (br s, 1H), 2.97 - 2.76 (m, 1H), 2.46 - 2.44 (m, 1H), 2.06 (br d, J=18.3 Hz, 1H), 1.70 (br s, 2H).

### Example 1.18

### 6-[5,6-Difluoro-8-[methyl(phosphonooxymethoxycarbonyl)amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

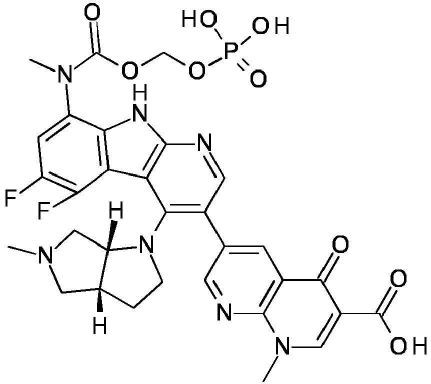

The titled compound was synthesized according to the following scheme:

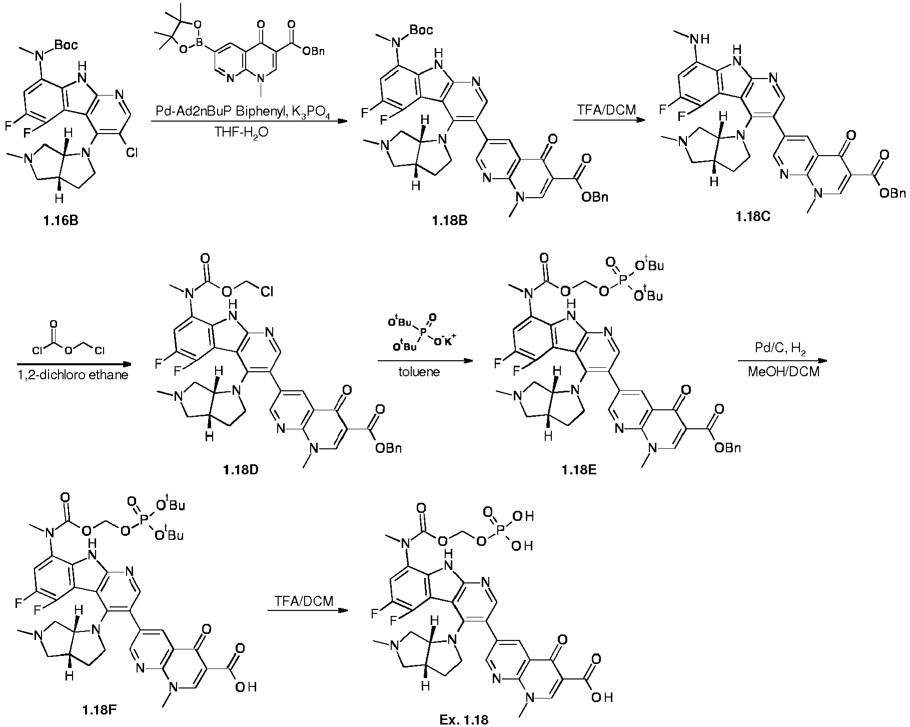

### Step (a) Preparation of benzyl 6-[8-[tert-butoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.18B)

To a solution of tert-butyl N-[3-chloro-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-N-methyl-carbamate (500 mg, 1.02 mmol, compound 1.16B) in THF (10 mL) and water (1 mL) was added benzyl 1-methyl-4-oxo-6-(4,4,5,5-tetramethyl-1,3,2-dioxaborolan-2-yl)-1,8-naphthyridine-3-carboxylate (640 mg, 1.52 mmol, Intermediate B3), Pd-Ad2nBuP Biphenyl (34 mg, 0.05 mmol, cataCXium A-Pd-G2, CAS: 1375477-29-4), and K₃PO₄ (647.21 mg, 3.05 mmol) under nitrogen. The resulting reaction mixture was stirred at 70 °C for 12 h until LC-MS showed the start material was consumed completely. The reaction mixture was then cooled back to r.t., poured into water (300 mL), and extracted with EtOAc (100 mL) three times. Combined organics was washed with brine, dried over anhy. Na₂SO₄, filtered and concentrated *in vacuo* to give a crude product, which was purified by prep-HPLC (TFA as additive) to give benzyl 6-[8-[*tert-*butoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (350 mg, 36.5% yield) as a yellow solid. MS (ESI): 750.3 (M+H)⁺.

### Step (b) Preparation of benzyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.18C)

To a solution of benzyl 6-[8-[*tert*-butoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (2.5 g, 3.33 mmol) in DCM (30 mL) was added TFA (1.03 mL, 13.3 mmol), and the resulting reaction mixture was stirred at 20 °C for 2 h. After LC-MS showed the starting material was consumed completely, the mixture was concentrated *in vacuo* to give a crude product, which was purified by prep-HPLC (TFA as additive) to give benzyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (1.8 g, 83.1% yield) as a yellow solid. MS (ESI): 650.1 (M+H)⁺.

### Step (c) Preparation of benzyl 6-[8-[chloromethoxycarbonyl(methyl)amino]-5,6-difluoro-4-[(3aR,6aR)-5-methyl-2,3,3a,4,6,6a-hexahydropyrrolo[2,3-c]pyrrol-1-yl-9H-pyrido[2,3-b]indol-3-yl]-1-methyl-4-oxo-1,8-naphthyridine-3-carboxylate (compound 1.18D)

To a solution of benzyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (1.7 g, 2.62 mmol) in 1,2-dichloro ethane (340 mL) was added chloromethyl chloroformate (1.33 g, 10.47 mmol), and the reaction mixture was stirred at 20 °C for 2 h. After LC-MS showed the starting material was consumed completely, the reaction mixture was concentrated *in vacuo* to give a crude product, which was then purified by prep-HPLC (TFA as additive). The collected eluents were adjust to pH > 7 with aq. NH₄HCO₃ solution to give benzyl 6-[8-[chloromethoxycarbonyl(methyl)amino]-5,6-difluoro-4-[(3aR,6aR)-5-methyl-2,3,3a,4,6,6a-hexahydropyrrolo [2,3-c]pyrrol-1-yl]-9H-pyrido [2,3-b]indol-3-yl]-1-methyl-4-oxo-1,8-naphthyridine-3-carboxylate (1.1 g, 56.6% yield) as a yellow solid. MS (ESI): 742.3 (M+H)⁺.

### Step (d) Preparation of benzyl 6-[8-[di-tert-butoxyphosphoryloxymethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]1-1-methyl-4-oxo-1,8-naphthyridine-3-carboxylate (compound 1.18E)

To a solution of benzyl 6-[8-[chloromethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (200 mg, 0.27 mmol) in toluene (8 mL) was added potassium di-tert-butylphosphate (201 mg, 0.81 mmol). The resulting reaction mixture was stirred at 90 °C for 2 h until LC-MS showed the starting material was consumed completely. The mixture solution was cooled back to r.t., poured into water (50 mL), and extracted with DCM (50 mL) three times. Combinded organics was dried over anhy. Na₂SO₄, filtered, and concentrated *in vacuo* to give a crude product, which was then purified by prep-HPLC (TFA as additive). The collected eluents were adjust to pH > 7 by aq. NH₄HCO₃ solution to give benzyl 6-[8-[di-tert-butoxyphosphoryloxymethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (50 mg, 20.3% yield) as a yellow solid. MS (ESI): 916.5 (M+H)⁺.

### Step (e) Preparation of 6-[8-[di-tert-butoxyphosphoryloxymethoxycarbonyl(methyl)aminol-5,6-difluoro-4-[(3aR,6aR)-5-methyl-2,3,3a,4,6,6a-hexahydropyrrolo[2,3-c]pyrrol-1yl]-9H-pyrido[2,3-b]indol-3-yl]-1-methyl-4-oxo-1,8-naphthyridine-3-carboxylic acid (compound 1. 18F)

To a solution of benzyl 6-[8-[di-tert-butoxyphosphoryloxymethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (50 mg, 0.05 mmol) in DCM (1 mL) and MeOH (1 mL) was added palladium (0.58 mg, 0.01 mmol). After degassed under vacuum and flushed with H₂ several times, the reaction mixture was stirred at 10 °C for 1 h under a H₂ balloon. After LC-MS showed the starting material was consumed, mixture was filtered through Celite. The filtrate was concentrated *in vacuo* to give a crude product of 6-[8-[di-tert-butoxyphosphoryloxymethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (30 mg, 66.6% yield) as a yellow solid. MS (ESI): 826.5 (M+H)⁺. It was used directly in the next step without further purification.

### Step (f) Preparation of 6-[5,6-difluoro-8-[methyl(phosiphonooxymethoxycarbonyl)amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.18)

To a solution of 6-[8-[di-*tert*-butoxyphosphorylmethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (29.0 mg, 0.040 mmol) in DCM (1 mL) was added TFA (0.05 mL), and the resulting mixture was stirred at 10 °C for 0.5 h. After LC-MS showed the starting material was consumed, the reaction mixture was adjust to pH > 7 with aq. NH₄HCO₃ solution. It was subject to prep-HPLC (NH₄HCO₃ as additive) purification to give 6-[5,6-difluoro-8-[methyl(phosphonooxymethoxycarbonyl)amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (19.5 mg, 74.9% yield) as a white solid. MS (ESI): 714.3 ([M+H]⁺). ¹H NMR (400 MHz, DMSO-*d*₆) δ ppm 9.26 (s, 1 H) 9.14 (br s, 1 H) 8.72 (br d, *J*=6 Hz, 1 H) 8.15 (s, 1 H) 7.53 - 7.69 (m, 1 H) 5.35 - 5.66 (m, 2 H) 4.43 (br s, 1 H) 4.17 (s, 3 H) 3.16 - 3.25 (m, 2 H) 2.98 (br s, 2 H) 2.83 (br s, 1 H) 2.53 (d, *J*=2 Hz, 1 H) 2.21 (br s, 1 H) 2.06 - 2.16 (m, 3 H) 1.99 (br s, 1 H) 1.89 (br s, 1 H) 1.65 (br s, 1 H).

### Example 1.19

### 6-[8-[[tert-Butoxy(hydroxy)phosphoryl]oxymethoxycarbonyl-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

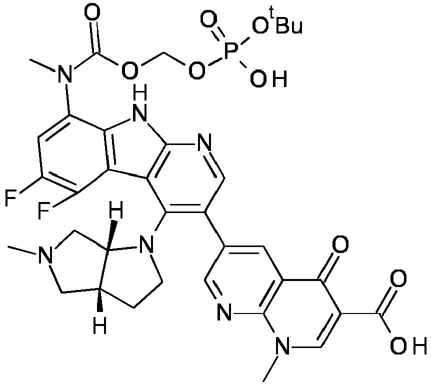

The titled compound was synthesized according to the following scheme:

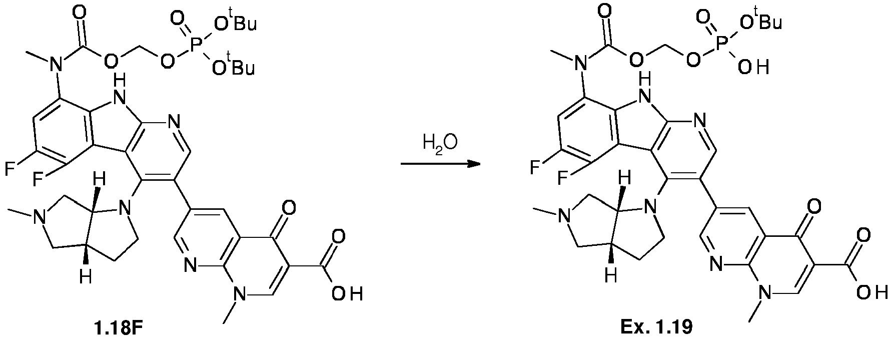

A solution of 6-[8-[di-*tert*-butoxyphosphoryloxymethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (30.0 mg, 0.040 mmol, compound 1.18F) in water (1 mL) was stirred at 60 °C for 0.5 h. After LC-MS showed the starting material was consumed, the reaction mixture was subject to prep-HPLC (NH₄HCO₃ as additive) purification to give 6-[8-[[*tert*-butoxy(hydroxy)phosphoryl]oxymethoxycarbonyl-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (11 mg, 37.8% yield) as a yellow solid. MS (ESI): 770.5 ([M+H]⁺). ¹H NMR (400 MHz, DMSO-*d*₆) δ ppm 14.57 - 14.90 (m, 1 H) 13.32 (br s, 1 H) 9.27 (br s, 1 H) 9.11 (br s, 1 H) 8.66 (br s, 1 H) 8.08 - 8.33 (m, 1 H) 8.08 - 8.33 (m, 1 H) 7.62 (br s, 1 H) 5.46 (br s, 2 H) 4.37 (br s, 1 H) 4.18 (br s, 2 H) 3.41 - 3.57 (m, 2 H) 3.25 - 3.31 (m, 3 H) 2.92 (br s, 2 H) 2.52 - 2.57 (m, 3 H) 2.36 - 2.45 (m, 2 H) 1.88 - 2.10 (m, 2 H) 1.74 (br d, *J*=15 Hz, 1 H) 0.95 - 1.46 (m, 9 H).

### Example 1.20

### (E)-4-[[[3-(6-Benzyloxycarbonyl-8-methyl-5-oxo-1,8-naphthyridin-3-yl)-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-methyl-carbamoyl]oxymethoxy]-4-oxo-but-2-enoic acid

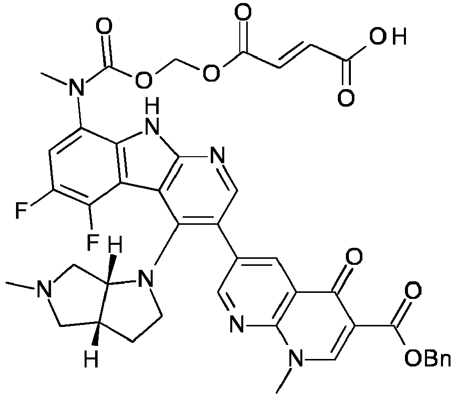

The titled compound was synthesized according to the following scheme:

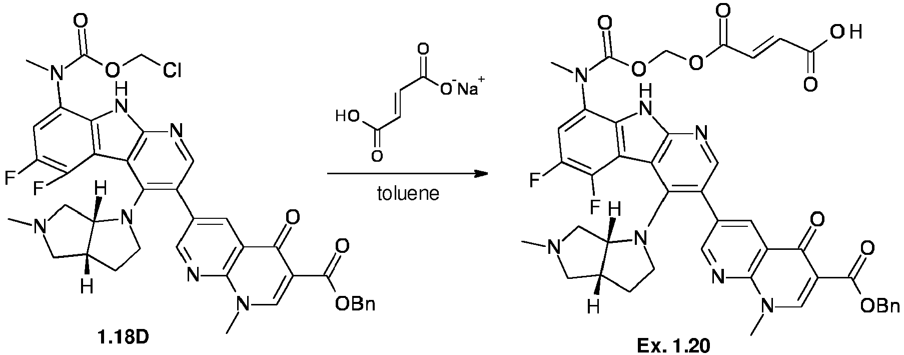

To a solution of benzyl 6-[8-[chloromethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (100 mg, 0.13 mmol, compound 1.18D) in DMF (3 mL) was added sodium hydrogen fumarate (55.8 mg, 0.40 mmol). The resulting reaction mixture was stirred at 95 °C for 2 h until LC-MS showed the starting material was consumed completely. The mixture was cooled back to r.t. and subject to prep-HPLC (NH₄HCO₃ as additive) purification to give (E)-4-[[[3-(6-Benzyloxycarbonyl-8-methyl-5-oxo-1,8-naphthyridin-3-yl)-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-methyl-carbamoyl]oxymethoxy]-4-oxo-but-2-enoic acid (25 mg, 22.2% yield) as a white solid. MS (ESI): 822.5 ([M+H]⁺). ¹H NMR (400 MHz, DMSO-d₆) δ ppm 12.42 (br s, 1 H) 8.86 - 9.04 (m, 2 H) 8.55 (br s, 1 H) 8.14 (br d, J=17 Hz, 1 H) 7.65 (br s, 1 H) 7.53 (d, J=7 Hz, 2 H) 7.28 - 7.47 (m, 3 H) 6.53 - 6.89 (m, 2 H) 6.46 (br d, J=15 Hz, 1 H) 5.93 (br s, 1 H) 5.71 (br s, 1 H) 5.34 (s, 2 H) 4.15 - 4.44 (m, 2 H) 4.03 (s, 3 H) 3.25 - 3.28 (m, 3 H) 2.99 (br s, 1 H) 2.81 (br s, 1 H) 2.46 (br s, 1 H) 2.20 - 2.40 (m, 2 H) 2.07 - 2.17 (m, 3 H) 1.96 (br s, 2 H) 1.65 (br s, 1 H).

### Example 1.21

### 6-[5,6-Difluoro-8-[methyl-[[(E)-3-carboxyprop-2-enoyl]oxymethoxycarbonyl]amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

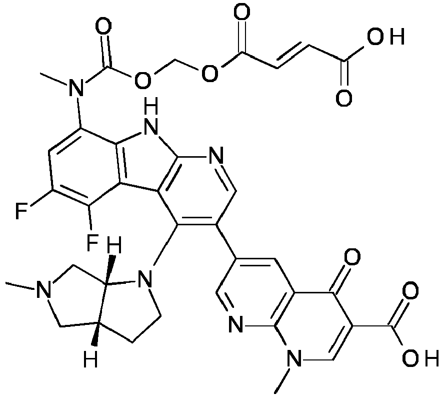

The titled compound was synthesized according to the following scheme:

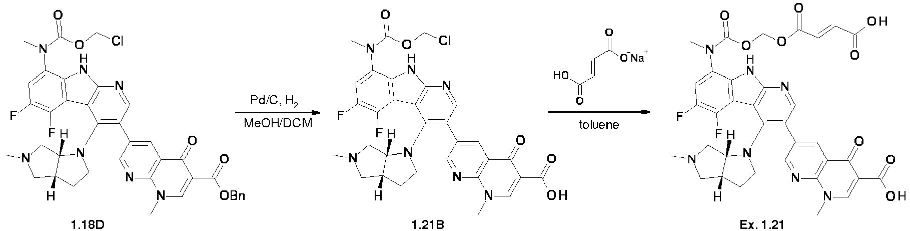

### Step (a) Preparation of 6-[8-[chloromethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.21B)

To a solution of benzyl 6-[8-[chloromethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (250 mg, 0.34 mmol, compound 1.18D) in MeOH (3 mL) was added palladium on carbon (0.03 mL, 0.34 mmol). After degassed under vacuum and flushed with H₂ several times, the reaction mixture was stirred at 20 °C for 2 h. After LC-MS showed the starting material was consumed, the reaction mixture was filtered through Celite. The filtrated was concentrated *in vacuo* to give a crude product of benzyl 6-[8-[chloromethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (168 mg, 51.3% yield) as a yellow solid. MS (ESI): 652.0 (M+H)⁺. It was used directly in the next step without further purification.

### Step (b) Preparation of 6-[5,6-difluoro-8-[methyl-[[(E)-3-carboxyprop-2-enoyl]oxymethoxycarbonyl]amino]-4-[(3aR,6aR)-5-methyl-2,3,3a,4,6,6a-hexahydropyrrolo[2,3-c]pyrrol-1-yl]-9H-pyrido[2,3-b]indol-3-yl-1-methyl-4-oxo-1,8-naphthyridine-3-carboxylic acid (Example 1.21)

To a solution of benzyl 6-[8-[chloromethoxycarbonyl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (168 mg, 0.20 mmol) in DMF (1 mL) was added sodium hydrogen fumarate (84.7 mg, 0.61 mmol). The resulting reaction mixture was stirred at 90 °C for 2 h until LC-MS showed the starting material was consumed completely. The reaction mixture was cooled back to r.t. and filtered. The filtrate was subject to prep-HPLC (NH₄HCO₃ as additive) purification to give 6-[5,6-difluoro-8-[methyl-[[(E)-3-carboxyprop-2-enoyl]oxymethoxycarbonyl]amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (10.3 mg, 6.7% yield) as a white solid. MS (ESI): 732.3 ([M+H]⁺). ¹H NMR (400 MHz, DMSO-*d*₆) δ ppm 12.32 - 12.63 (m, 1 H) 9.27 (s, 1 H) 9.09 - 9.18 (m, 1 H) 8.73 (br s, 1 H) 8.12 - 8.23 (m, 1 H) 7.58 - 7.73 (m, 1 H) 6.85 (br d, *J*=15 Hz, 1 H) 6.40 - 6.59 (m, 1 H) 5.92 (br d, *J*=3 Hz, 1 H) 5.71 (br s, 1 H) 4.41 (br s, 1 H) 4.18 (s, 3 H) 3.31 - 3.34 (m, 5 H) 2.99 (br s, 2 H) 2.84 (br s, 1 H) 2.52 - 2.56 (m, 1 H) 2.24 (br d, *J*=7 Hz, 1 H) 2.10 - 2.18 (m, 3 H) 1.87 - 2.04 (m, 2 H) 1.58 - 1.72 (m, 1 H) 1.66 (br s, 1 H).

### Example 1.22

### 6-[1-[[tert-Butoxy(hydroxy)phosphoryl]oxymethyl]-5,6-difluoro-8-(methylamino)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid

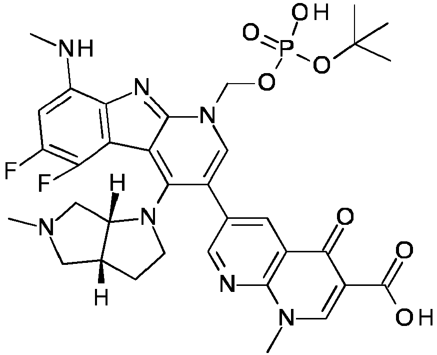

The titled compound was synthesized according to the following scheme:

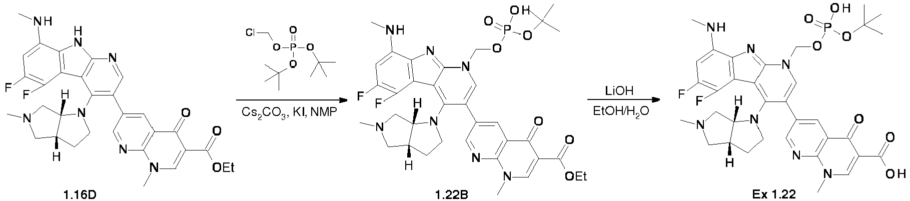

### Step (a) Preparation of ethyl 6-[1-[[tert-butoxy(hydroxy)phosphoryl]oxymethyl]-5,6-difluoro-8-(methylamino)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylate (compound 1.22B)

To a mixture solution of ethyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (460 mg, 0.78 mmol, compound 1.16D), cesium carbonate (736 mg, 2.26 mmol), and potassium iodide (368 mg, 2.22 mmol) in anhydrous NMP (5.0 mL) was added di-*tert*-butyl chloromethyl phosphate (460 mg, 1.78 mmol). The resulting reaction mixture was stirred at 20 °C for 12 h until LC-MS showed the starting material was consumed completely. The mixture was then diluted with DCM (100 mL), poured into water (100 mL), and extracted with DCM (100 mL) twice. Combined organics was washed with brine (100 mL), dried over anhy. Na₂SO₄, filtered, and concentrated *in vacuo* to give a crude product, which was purified by prep-HPLC (NH₃.H₂O as additive) to give ethyl 6-[1-[[*tert-*butoxy(hydroxy)phosphoryl]oxymethyl]-5,6-difluoro-8-(methylamino)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylate (150 mg, 25.4% yield) as an orange solid. MS (ESI): 754.4 ([M+H]⁺). ¹H NMR (400 MHz, METHANOL-d4) δ ppm 8.91-9.02 (m, 2 H), 8.46~8.83 (m, 1 H), 7.87~8.26 (m, 1 H), 6.07~6.53 (m, 3 H), 4.76 (br s, 1 H), 4.25~4.42 (m, 2 H), 4.12 (br s, 3 H), 2.96 (s, 7 H), 2.54~2.66 (m, 2 H), 2.42 (br s, 3 H), 1.91~2.09 (m, 1 H), 1.68 (br s, 1 H), 1.39 (br d, J=12.59 Hz, 12 H).

### Step (b) Preparation of 6-[1-[[tert-Butoxy(hydroxy)phosphoryl]oxymethyl]-5,6-difluoro-8-(methylamino)-4-[(3aR,6aR)-5-methyl-2,3,3a,4,6,6a-hexahydropyrrolo[2,3-c]pyrrol-1-vl]pyrido[2,3-b]indol-3-yl]-1-methyl-4-oxo-1,8-naphthyridine-3-carboxylic acid (Example 1.22)

To a solution of ethyl 6-[1-[[*tert*-butoxy(hydroxy)phosphoryl]oxymethyl]-5,6-difluoro-8-(methylamino)-4-[(3aR,6aR)-5-methyl-2,3,3a,4,6,6a-hexahydropyrrolo [2,3-c]pyrrol-1-yl]pyrido[2,3-b]indol-3-yl]-1-methyl-4-oxo-1,8-naphthyridine-3-carboxylate (150 mg, 0.2 mmol) in EtOH (2.5 mL) was added lithium hydroxide (0.5 mL, 4 mmol), and the reaction mixture was stirred at 20 °C for 1 h. After LC-MS showed the starting material was consumed, the mixture was adjusted to pH = 6 with aq. HCl solution (1N). It was then subject to prep-HPLC (NH₃.H₂O as additive) purification to give 6-[1-[[*tert*-butoxy(hydroxy)phosphoryl]oxymethyl]-5,6-difluoro-8-(methylamino)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid (35.0 mg, 23.5% yield) as a red solid. MS (ESI): 726.4 ([M+H]⁺). ¹H NMR (400 MHz, DMSO-d6) δ ppm 14.42~14.94 (m, 1 H), 9.27 (s, 1 H), 9.11~9.18 (m, 1 H), 9.11~9.18 (m, 1 H), 8.83 (s, 1 H), 8.31~8.40 (m, 1 H), 8.31~8.40 (m, 1 H), 6.80~7.60 (m, 1 H), 6.34~6.71 (m, 1 H), 5.93~6.19 (m, 2 H), 5.12~5.52 (m, 1 H), 4.78 (br s, 1 H), 4.17 (s, 3 H), 2.97~3.15 (m, 3 H), 2.90 (br s, 5 H), 2.59~2.66 (m, 1 H), 2.22~2.30 (m, 1 H), 2.13~2.21 (m, 3 H), 1.87~2.04 (m, 2 H), 1.61~1.70 (m, 1H), 1.27 (br s, 9H).

### Example 1.23

### 6-[5,6-Difluoro-8-(methylamino)-1-(phosphonooxymethyl)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid

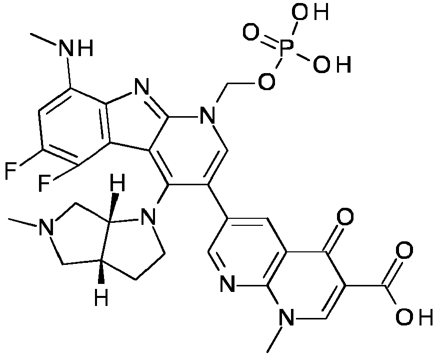

The titled compound was synthesized according to the following scheme:

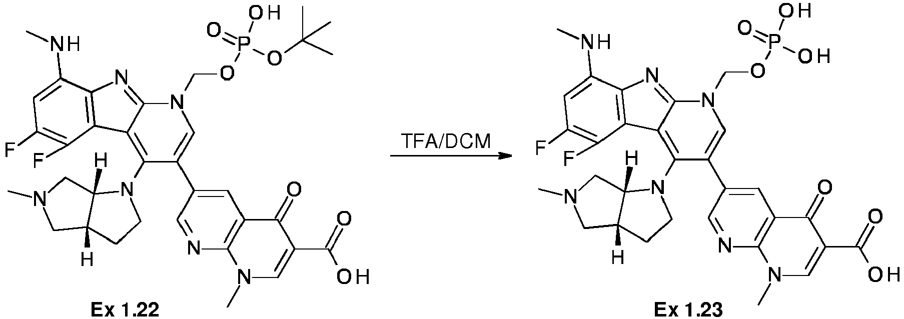

To a solution of 6-[1-[[*tert*-butoxy(hydroxy)phosphoryl]oxymethyl]-5,6-difluoro-8-(methylamino)-4-[(3aR,6aR)-5-methyl-2,3,3a,4,6,6a-hexahydropyrrolo [2,3-c]pyrrol-1-yl]pyrido[2,3-b]indol-3-yl]-1-methyl-4-oxo-1,8-naphthyridine-3-carboxylic acid (23.0 mg, 0.03 mmol) in DCM (2.0 mL) was added TFA (0.2 mL, 0.03 mmol), and the reaction mixture was stirred at 20 °C for 2 h. After LC-MS showed the starting material was consumed, the mixture was subject to prep-HPLC (NH₃.H₂O as additive) purification to give 6-[5,6-difluoro-8-(methylamino)-1-(phosphonooxymethyl)-4-[(3aR,6aR)-5-methyl-2,3,3a,4,6,6a-hexahydropyrrolo[2,3-c]pyrrol-1-yl]pyrido [2,3-b]indol-3-yl]-1-methyl-4-oxo-1,8-naphthyridine-3-carboxylic acid (12.2 mg, 56.9% yield) as a yellow solid. MS (ESI): 670.3 ([M+H]⁺). ¹H NMR (400 MHz, DMSO-d6) δ ppm 8.92~9.07 (m, 2 H), 8.66 (br s, 1 H), 8.03 (s, 1 H), 7.25~7.25 (m, 1 H), 6.34~6.47 (m, 1 H), 5.92~6.03 (m, 2 H), 4.59~4.70 (m, 1 H), 4.11~4.13 (m, 3 H), 2.86~3.01 (m, 2 H), 2.80~2.85 (m, 3 H), 2.64~2.71 (m, 1 H), 2.34 (br s, 2 H), 2.13 (br s, 4 H), 1.82~1.96 (m, 1 H), 1.50~1.64 (m, 1 H).

### Example 1.24

### 6-[5,6-Difluoro-8-(methylamino)-4-[(3aR,6aR)-5-[[tert-butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid

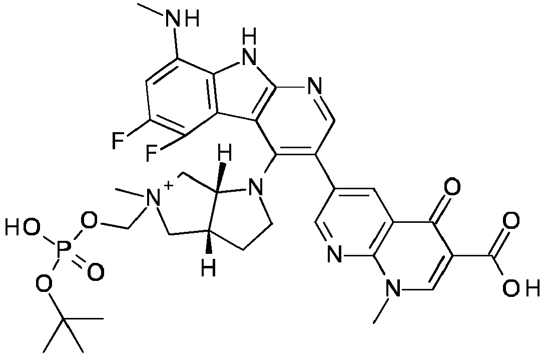

The titled compound was synthesized according to the following scheme:

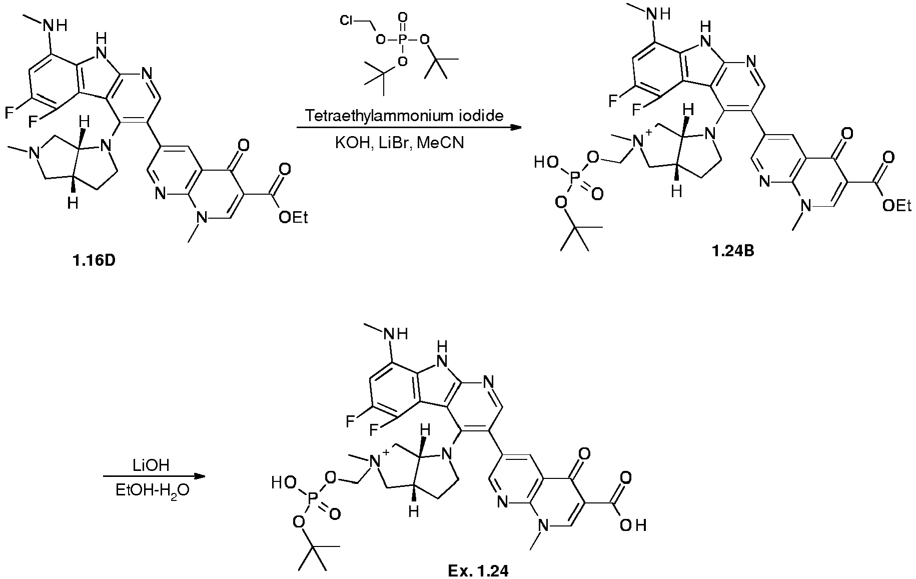

### Step (a) Preparation of ethyl 6-[5,6-difluoro-8-(methylamino)-4-[(3aR,6aR)-5-[[tert-butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylate (compound 1.24B)

To a solution of ethyl 6-[5,6-difluoro-8-(methylamino)-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (105.0 mg, 0.180 mmol, compound 1.16D), lithium bromide (46.55 mg, 0.540 mmol), potassium hydroxide (30.08 mg, 0.540 mmol), and tetraethylammonium iodide (137.85 mg, 0.540 mmol) in anhydrous ACN (2.0 mL) was added di-*tert*-butyl chloromethyl phosphate (138.67 mg, 0.540 mmol). The resulting reaction mixture was stirred at 20 °C for 12 h. After LC-MS showed the starting material was consumed completely, the mixture was poured into water (20 mL) and lyophilized to give a crude product. It was re-dissolved in MeOH and subject to prep-HPLC (NH₄HCO₃ as additive) purification to give ethyl 6-[5,6-difluoro-8-(methylamino)-4-[(3aR,6aR)-5-[[*tert-*butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylate (35.0 mg, 24.2% yield) as a grey solid. MS (ESI): 754.4 ([M+H]⁺). ¹H NMR (400 MHz, DMSO-d6) δ ppm 12.13~12.34 (m, 1 H), 8.88~8.91 (m, 1 H), 8.83~8.85 (m, 1 H), 8.37~8.40 (m, 1 H), 8.22~8.24 (m, 1 H), 6.46~6.60 (m, 1 H), 5.94~6.07 (m, 1 H), 5.94~6.07 (m, 1 H), 4.58~4.67 (m, 1 H), 4.49~4.57 (m, 1 H), 4.35~4.48 (m, 1 H), 4.26 (br d, J=7.09 Hz, 2 H), 4.00 (s, 4 H), 3.66~3.75 (m, 1 H), 3.09~3.31 (m, 4 H), 3.00 (s, 3 H), 2.84 (br s, 4 H), 1.98~2.12 (m, 1 H), 1.69~1.80 (m, 1 H), 1.28~1.35 (m, 3 H), 1.15~1.22 (m, 9 H).

### Step (b) Preparation of 6-[5,6-difluoro-8-(methylamino)-4-[(3aR,6aR)-5-[[tert-butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid (Example 1.24)

A mixture solution of ethyl 6-[5,6-difluoro-8-(methylamino)-4-[(3aR,6aR)-5-[[*tert-*butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylate (45.0 mg, 0.060 mmol) and lithium hydroxide (44.9 mg, 1.88 mmol) in EtOH (2.0 mL) and water (0.5 mL) was stirred at 20 °C for 2 h. After LC-MS showed the starting material was consumed, the mixture was diluted with MeOH (2.0 mL), and subject to prep-HPLC (NH₄HCO₃ as additive) purification to give 6-[5,6-difluoro-8-(methylamino)-4-[(3aR,6aR)-5-[[*tert-*butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid (40.0 mg, 92.3% yield) as a grey solid. MS (ESI): 726.4 ([M+H]⁺). ¹H NMR (400 MHz, MeOH-d4) δ ppm 9.37~9.61 (m, 1 H), 8.86~9.21 (m, 1 H), 8.57 (s, 1 H), 7.83~8.29 (m, 1 H), 6.26~6.70 (m, 1 H), 6.17 (br s, 1 H), 4.40~4.68 (m, 2 H), 4.08 (br s, 4 H), 3.90~4.03 (m, 3 H), 3.47~3.73 (m, 1 H), 3.37 (s, 1 H), 2.83~3.09 (m, 3 H), 2.61 (br s, 3 H), 2.06~2.42 (m, 2 H), 1.85~1.99 (m, 1 H), 1.85~1.99 (m, 1 H), 1.56~1.70 (m, 1 H), 1.23~1.40 (m, 9 H).

### Example 1.25

### 6- [5,6-Difluoro-8-(methylamino)-4-[(3aR,6aR)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid

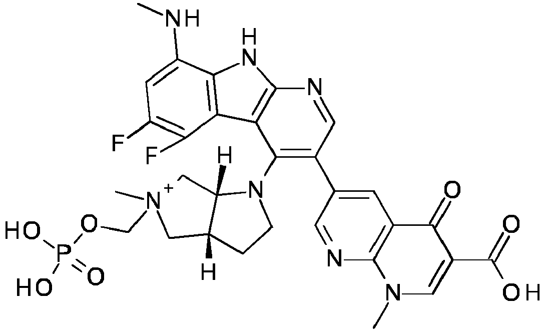

To a solution 6-[5,6-difluoro-8-(methylamino)-4-[(3aR,6aR)-5-[[*tert-*butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid (29.7 mg, 0.04 mmol, Example 1.24) in DCM (1.0 mL) was added TFA / DCM (0.4 mL, 0.01 mmol, v/v = 1:5), and the reaction mixture was stirred at 20 °C for 1 h. After LC-MS showed the starting material was consumed, the mixture was concentrated *in vacuo* to give a crude product, which was purified by prep-HPLC (NH₄HCO₃ as additive) to give 6-[5,6-difluoro-8-(methylamino)-4-[(3aR,6aR)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid (7 mg, 21.5% yield) as a yellow solid. MS (ESI): 670.3 ([M+H]⁺). ¹H NMR (400 MHz, DMSO-d6) δ ppm 9.23~9.29 (m, 1 H), 9.05~9.12 (m, 1 H), 8.61~8.68 (m, 1 H), 8.22~8.29 (m, 1 H), 6.54~6.64 (m, 1 H), 5.59~5.96 (m, 1 H), 4.43~4.61 (m, 2 H), 4.28~4.39 (m, 1 H), 4.18 (s, 4 H), 3.92~4.02 (m, 2 H), 3.56~3.69 (m, 1 H), 3.07~3.17 (m, 1 H), 2.88 (br s, 6 H) 2.64~2.71 (m, 2 H), 2.30~2.36 (m, 2 H), 2.04~2.11 (m, 1 H), 1.76 (s, 1 H).

### Example 1.26

### 6-[5,6-Difluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

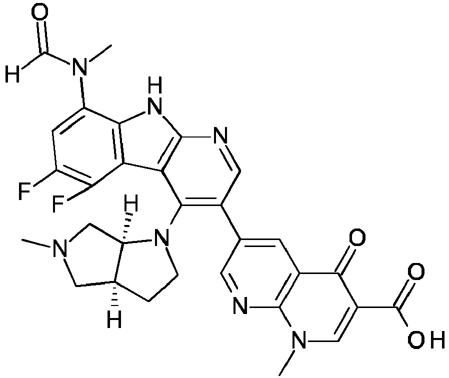

The titled compound was synthesized according to the following scheme:

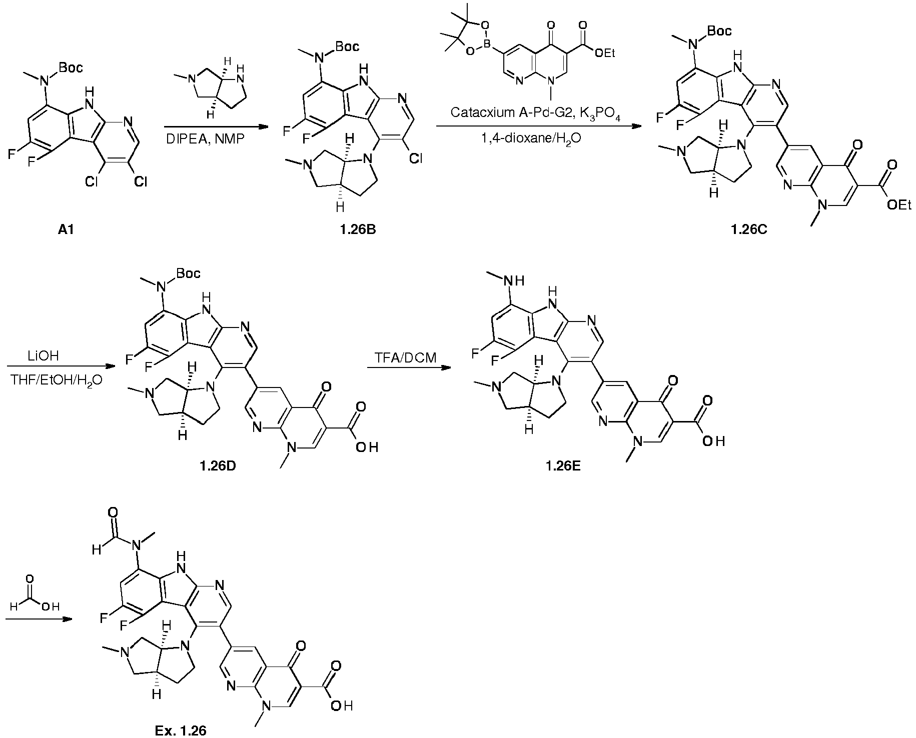

### Step (a) Preparation of tert-butyl N-[3-chloro-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.26B)

A solution of *tert*-butyl N-(3,4-dichloro-5,6-difluoro-9H-pyrido[2,3-b]indol-8-yl)-N-methyl-carbamate (10.8 g, 26.87 mmol, Intermediate A1), (3aS,6aS)-5-methyl-2,3,3a,4,6,6a-hexahydro-1H-pyrrolo[2,3-c]pyrrole (6.1g, 48.36 mmol, CAS: 1176846-84-6), and DIPEA (14.04 mL, 80.6 mmol) in NMP (15 mL) was stirred at 118 °C for 48 h. After TLC (petroleum ether:EtOAc = 2:1, 2% of Et₃N as additive) showed the starting material was consumed, the mixture was poured into EtOAc (500 mL). The separated organic layer was then washed with aq. CaCh solution (100 mL) three times, brine (100 mL) twice, dried over anhy. Na₂SO₄, filtered, and concentrated *in vacuo* to give a crude product, which was purified by silica gel flash chromatography (petroleum ether:EtOAc = 10:1~1, 2% of Et₃N as additive) to give *tert-butyl* N-[3-chloro-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (12.0 g, 88.8% yield, > 98%ee, optical rotation [α]_{D} = + 27.7°) as a white solid. MS (ESI): 492.3 ([{³⁵Cl}M+H]⁺).

### Step (b) Preparation of ethyl 6-[8-[tert-butoxycarbonyl(methyl)aminol-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.26C)

To a solution of *tert-*butyl N-[3-chloro-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (10.0 g, 20.33 mmol), ethyl 1-methyl-4-oxo-6-(4,4,5,5-tetramethyl-1,3,2-dioxaborolan-2-yl)-1,8-naphthyridine-3-carboxylate (14.6 g, 40.65 mmol, Intermediate B1), and potassium phosphate (17.3 g, 81.31 mmol) in 1,4-dioxane (300 mL) and water (40 mL) was added Pd-Ad2nBuP Biphenyl (2.71 g, 4 mmol, cataCXium A-Pd-G2, CAS: 1375477-29-4)in a glove box under argon. The reaction mixture was stirred at 90 °C for 12 h until LC-MS showed the starting material was consumed completely. The reaction mixture was cooled back to r.t., filtered and the filtrate was diluted with EtOAc (800 mL). The organic phase was washed with brine (200 mL) three times, dried over anhy. Na₂SO₄, filtered, and concentrated *in vacuo* to give a crude product. It was purified twice by silica gel flash chromatography (first with DCM:MeOH = 100:1~10:1 and then with petroleum ether:EtOAc = 100:1~1:3, 2% Et₃N as additive) to give ethyl 6-[8-[*tert-*butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (10.5 g, 65.6% yield) ) as a white solid. MS (ESI): 688.5 ([M+H]⁺).

### Step (c) Preparation of 6-[8-[tert-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.26D)

To a solution of ethyl 6-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (10.0 g, 14.54 mmol) in THF (60 mL) and EtOH (15 mL) was added aq. LiOH solution (21.8 mL, 43.6 mmol). The resulting mixture was stirred at 15 °C for 4 h until LC-MS showed the starting material was consumed completely. The mixture was diluted with EtOAc (200 mL) and H₂O (50 mL), adjusted pH to 6 with aq. HCl solution (1N), and extracted with DCM / MeOH (10:1, 400 mL) twice. Combined organics was then washed with brine (200.0 mL) twice, dried over anhy. Na₂SO₄, filtered, and concentrated *in vacuo* to give a crude product of 6-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (9.5 g, 88.5% yield) as a white solid. MS (ESI): 660.3 ([M+H]⁺). It was used directly in the next step without further purification.

### Step (d) Preparation of 6-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.26E)

To a solution of 6-[4-[(3aS,6aS)-5-methyl-2,3,3a,4,6,6a-hexahydropyrrolo[2,3-c]pyrrol-1-yl]-8-[tert-butoxycarbonyl(methyl)amino]-5,6-difluoro-9H-pyrido[2,3-b]indol-3-yl]-1-methyl-4-oxo-1,8-naphthyridine-3-carboxylic acid (9.45 g, 14.33 mmol) in DCM (80 mL) was added TFA (20.0 mL, 259.6 mmol), and the resulting reaction mixture was stirred at 15 °C for 1 h. After LC-MS showed the starting material was consumed completely, the mixture solution was evaporated to dryness under the reduced pressure. The residue was re-dissolved in MeOH (100 mL) and adjusted with aq. NaHCO₃ solution to pH = 6. The resulting suspension was then filtered and the filtrate was concentrated *in vacuo* to give a crude product, which was further purified by prep-HPLC (NH₃•H₂O as additive) to give 6-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (4.87 g, 59.5% yield) as a yellow solid. MS (ESI): 560.3 ([M+H]⁺), 280.8 ([M/2+H]⁺). ¹H NMR (400 MHz, DMSO-d6) δ ppm 14.76 (br s, 1 H), 12.09 (br s, 1 H), 9.19 (s, 1 H), 9.08 (d, J=2.2 Hz, 1 H), 8.68 (d, J=2.4 Hz, 1 H), 8.10 (s, 1 H), 6.54 (dd, J=13.6, 6.2 Hz, 1 H), 5.91 (br d, J=3.7 Hz, 1 H), 4.44 (br s, 1 H), 4.14 (s, 3 H), 2.77 - 3.01 (m, 6 H), 2.44 (br d, J=9.3 Hz, 2 H), 2.15 (br t, J=8.2 Hz, 1 H), 2.08 (s, 3 H), 1.88 - 2.02 (m, 1 H), 1.79 (br dd, J=9.7, 5.3 Hz, 1 H), 1.52 - 1.65 (m, 1 H).

### Step (e) Preparation of 6-[5,6-difluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.26)

A solution of 6-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid_(30 mg, 0.054 mmol) in formic acid (0.5 mL) was stirred at 100 °C for 10 min under microwave. Afterwards, the reaction mixture was cooled back to r.t. and the residue formic acid was removed under the reduced pressure to give a crude product. It was then subject to prep-HPLC (formic acid as additive) purification to give 6-[5,6-difluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (7.1 mg, 21.4 % yield) as a white solid. MS (ESI): 588.2 ([M+H]⁺). ¹H NMR (400 MHz, MeOD-d4) δ 9.06 (s, 1H), 8.98 (s, 1H), 8.73 (s, 1H), 8.27-8.33 (m, 1H), 8.15 (m, 1H), 7.54 - 7.42 (m, 1H), 7.39 - 7.47 (m, 1H), 4.27 (m, 1H), 4.13 (s, 3H), 3.39 (m, 2H), 3.35 (s, 3H), 3.04 (m, 2H), 2.60 - 2.90 (m, 3H), 2.47 - 2.51 (m, 3H), 2.10 (m, 1H), 1.75 (m, 1H).

### Example 1.27

### 6-[5,6-Difluoro-8-[methyl(sulfo)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

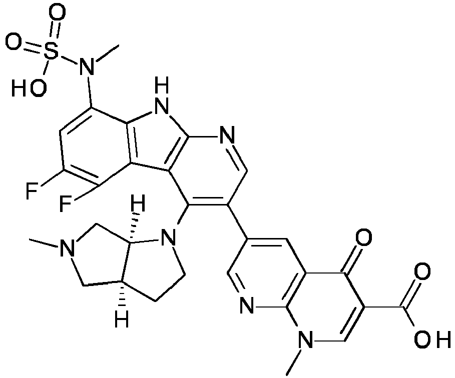

The titled compound was synthesized according to the following scheme:

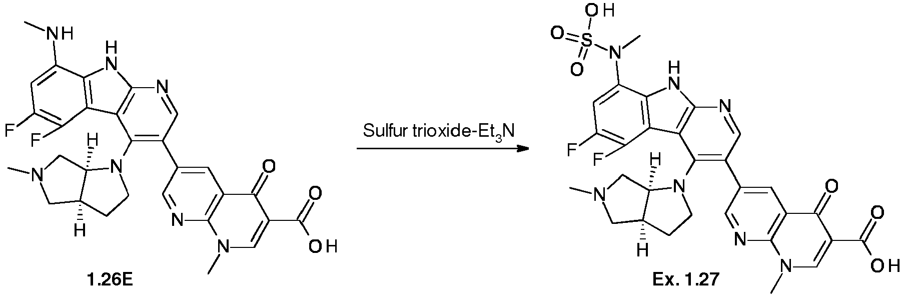

A mixture solution of 6-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (30 mg, 0.054 mmol, compound 1.26E), sulfur trioxide triethylamine complex (38 mg, 0.21mmol), and Et₃N (32.5 mg, 0.32 mmol) in DMF (2.0 mL) was stirred at 100 °C for 10 min under microwave. Afterwards, the mixture solution was cooled back to r.t., and subject to prep-HPLC (p formic acid as additive) purification to give 6-[5,6-difluoro-8-[methyl(sulfo)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (7.9 mg, 22 % yield) as a white solid. MS (ESI): 640.2 ([M+H]⁺). ¹H NMR (400 MHz, MeOD-d4) δ9.19 (s, 1H), 9.09 (s, 1H), 8.90 (d, J = 4Hz, 1H), 8.32 (s, 1H), 7.67 (m, 1H), 4.26 (s, 3H), 3.69 (m, 1H), 3.45 (m, 2H), 3.25 (s, 3H), 3.01-3.15 (m, 3H), 2.91 (s, 2H), 2.76 (s, 3H), 2.30 (m, 1H), 1.94 (m, 1H).

### Example 1.28

### 6-[5,6-Difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

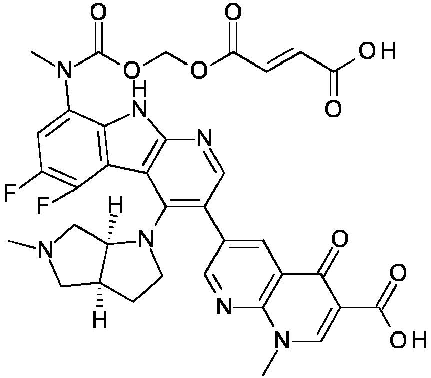

The titled compound was synthesized according to the following scheme:

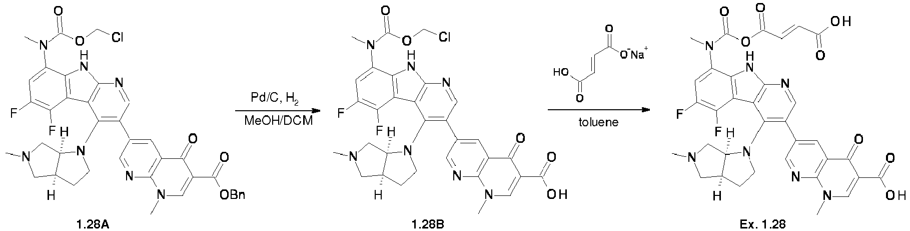

### Step (a) Preparation of benzyl 6-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.28A)

In analogy to the synthesis of compound 1.18D (steps a, b, and c), *tert*-butyl N-[3-chloro-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate was used instead of *tert*-butyl N-[3-chloro-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.16B in step a) to give benzyl 6-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate as a yellow solid. MS (ESI): 742.3 ([M+H]⁺).

### Step (b) Preparation of 6-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.28B)

To a solution of benzyl 6-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (300 mg, 0.40 mmol) in DCM (1.8 mL) and MeOH (1.8 mL) was added palladium on carbon (60.0 mg, 0.06 mmol). After the suspension was degassed under vacuum and purged with H₂ several times, the mixture solution was stirred at 20 °C for 2 h under H₂ atmosphere (15 psi). After LC-MS showed the starting material was consumed completely, the reaction mixture was filtered through Celite. The filtrate was then concentrated *in vacuo* to give a crude product of 6-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (100 mg, 37.9% yield) as a yellow solid. MS (ESI): 652.3 ([M+H]⁺). It was used directly in the next step without further purification.

### Step (c) Preparation of 6-[5,6-difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.28)

To a solution of 6-[8-[chloromethoxycarbonyl(methyl)amino]-5,6-difluoro-4-[(3aS,6aS)-5-methyl-2,3,3a,4,6,6a-hexahydropyrrolo [2,3-c]pyrrol-1-yl]-9H-pyrido [2,3-b]indol-3-yl]-1-methyl-4-oxo-1,8-naphthyridine-3-carboxylic acid (100 mg, 0.15 mmol) in DMF (2.7 mL) was added sodium hydrogen fumarate (63.5 mg, 0.46 mmol). The resulting reaction mixture was stirred at 90 °C for 1 h until LC-MS showed the starting material was consumed. The mixture was filtered and the filtrate was concentrated *in vacuo* to give a crude product, which was purified by prep-HPLC (NH₄HCO₃ as additive) to give 6-[5,6-Difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (11 mg, 3.1% yield) as a white solid. MS (ESI): 732.1 ([M+H]⁺). ¹H NMR (400 MHz, DMSO-*d*₆) δ ppm: 14.82 (br s, 1 H), 12.47 (br d, *J*=13.4 Hz, 1 H), 9.27 (s, 1 H), 9.11 - 9.15 (m, 1 H), 8.71 - 8.75 (m, 1 H), 8.10 - 8.24 (m, 1 H), 7.59 - 7.72 (m, 1 H), 6.43 - 6.91 (m, 2 H), 5.65 - 5.96 (m, 2 H), 4.41 (br s, 1 H), 4.18 (s, 3 H), 3.31 (br s, 3 H), 2.99 (br s, 2 H), 2.83 (br s, 1 H), 2.53 (d, *J*=2.0 Hz, 2 H), 2.23 - 2.30 (m, 1 H), 2.11 - 2.18 (m, 3 H), 1.90 - 2.06 (m, 2 H), 1.61 - 1.69 (m, 1 H).

### Example 1.29

### 6-[5,6-Difluoro-8-[methyl(sulfo)amino]-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

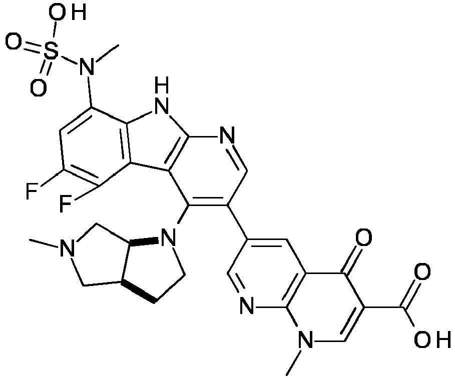

The titled compound was synthesized according to the following scheme:

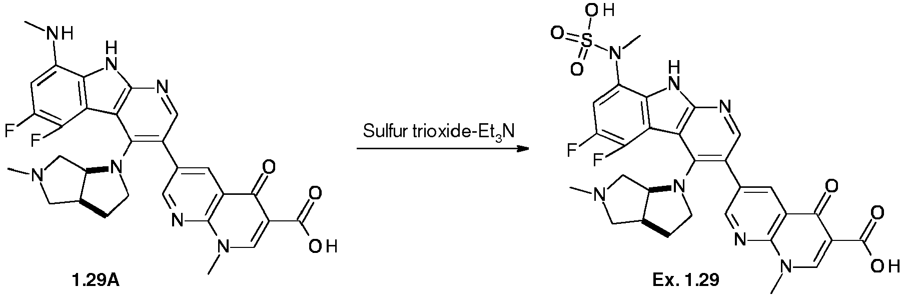

### Step (a) Preparation of 6-[5,6-difluoro-8-(methylamino)-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.29A)

In analogy to the synthesis of compound 1.26E, *cis*-5-methyl-2,3,3a,4,6,6a-hexahydro-1H-pyrrolo[2,3-c]pyrrole (Intermediate C1) was used in the step (a) instead of (3aS,6aS)-5-methyl-2,3,3a,4,6,6a-hexahydro-1H-pyrrolo[2,3-c]pyrrole to give 6-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid after steps b, c, and d as a white solid.MS (ESI): 492.3 ([{³⁵Cl}M+H]⁺).

### Step (b) Preparation of 6-[5,6-difluoro-8-[methyl(sulfo)amino]-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.29)

In analogy to the synthesis of Example 1.27, 6-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (30 mg, 0.054 mmol) was used instead of 6-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.26E) to give 6-[5,6-difluoro-8-[methyl(sulfo)amino]-4-[*cis*-5-methyl-2,3,3 a,4,6,6a-hexahydropyrrolo [2,3-c]pyrrol-1-yl]-9H-pyrido[2,3-b]indol-3-yl]-1-methyl-4-oxo-1,8-naphthyridine-3-carboxylic acid (7.9 mg, 22% yield) as a white solid after prep-HPLC purification. (formic acid as additive. MS (ESI): 640.2 ([M+H]⁺). ¹H NMR (400 MHz, MeOD-d4) δ9.19 (s, 1H), 9.09 (s, 1H), 8.90 (d, J = 4Hz, 1H), 8.32 (s, 1H), 7.67 (m, 1H), 4.26 (s, 3H), 3.69 (m, 1H), 3.45 (m, 2H), 3.25 (s, 3H), 3.01-3.15 (m, 3H), 2.91 (s, 2H), 2.76 (s, 3H), 2.30 (m, 1H), 1.94 (m, 1H).

### Example 1.30

### 6-[8-[[2-[(2-Aminoacetyl)amino]acetyl]-methyl-amino]-5,6-difluoro-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

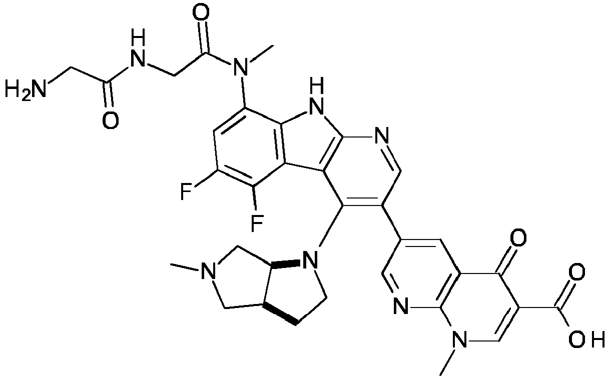

The titled compound was synthesized according to the following scheme:

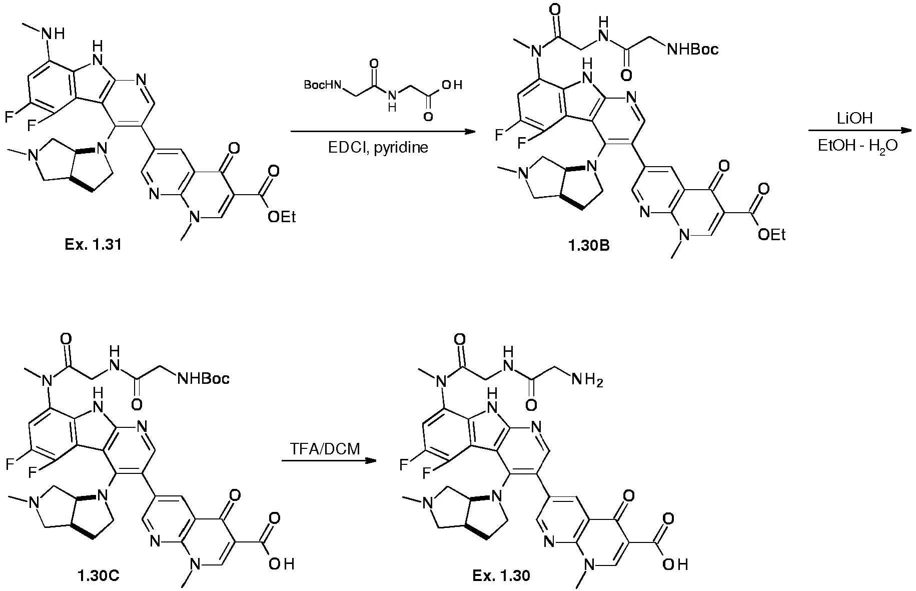

### Step (a) Preparation of ethyl 6-[8-[[2-[[2-(tert-butoxycarbonylamino)acetyl]amino]acetyl]-methyl-amino]-5,6-difluoro-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.30B)

A mixture solution of ethyl 6-[5,6-difluoro-8-(methylamino)-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (200 mg, 0.34 mmol, Example 1.31), 2-[[2-(*tert-*butoxycarbonylamino)acetyl]amino]acetic acid (200 mg, 0.86 mmol), and EDCI (200 mg, 1.04 mmol) in pyridine (5 mL) was stirred at 80 °C for 90 h. After LC-MS showed the starting material was consumed completely, the reaction mixture was cooled back to r.t. and concentrated *in vacuo.* The crude product was then subject to prep-HPLC (TFA as additive) purification to give ethyl 6-[8-[[2-[[2-(*tert*-butoxycarbonylamino)acetyl]amino]acetyl]-methyl-amino]-5,6-difluoro-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (55 mg, 20.2% yield) as a yellow solid. MS (ESI): 802.6 ([M+H]⁺).

### Step (b) Preparation of 6-[8-[[2-[[2-(tert-butoxycarbonylamino)acetyl]amino]acetyl]-methyl-amino]-5,6-difluoro-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.30C)

To a solution of ethyl 6-[8-[[2-[[2-(*tert*-butoxycarbonylamino)acetyl]amino]acetyl]-methyl-amino]-5,6-difluoro-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (50.0 mg, 0.06 mmol) in EtOH (2 mL) and water (2 mL) was added lithium hydroxide (10.0 mg, 0.420 mmol), and the resulting reaction mixture was stirred at 20 °C for 1 h. After LC-MS showed the starting material was consumed, the solution was adjusted to pH = 5-6 with aq. HCl solution (1N) and concentrated *in vacuo* to give a crude product of 6-[8-[[2-[[2-(tert-butoxycarbonylamino)acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (40 mg, 82.9% yield) as a yellow solid. MS (ESI): 774.2 ([M+H]⁺). It was used directly in the next step without further purification.

### Step (c) Preparation of 6-[8-[[2-[(2-aminoacetyl)amino]acetyl]-methyl-amino]-5,6-difluoro-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Example 1.30)

To a solution of 6-[8-[[2-[[2-(tert-butoxycarbonylamino)acetyl]amino]acetyl]-methyl-amino]-5,6-difluoro-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (40.0 mg, 0.05 mmol) in DCM (1 mL) was added TFA (0.2 mL, 2.6 mmol), and the resulting reaction mixture was stirred at 10 °C for 1 h until LC-MS indicated the starting material was consumed. The mixture solution was concentrated *in vacuo* to give a crude product, which was purified by prep-HPLC (NH₃.H₂O as additive) to give 6-[8-[[2-[(2-aminoacetyl)amino]acetyl]-methyl-amino]-5,6-difluoro-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (13.1 mg, 35.8% yield) as an off-white solid. MS (ESI): 674.1 ([M+H]⁺). ¹H NMR (400 MHz, DMSO-*d*₆) *δ* ppm: 9.26 (m, 1H), 9.13 (m, 1H), 8.73 (m, 1H), 8.22 (m, 1H), 8.15 (m, 1H), 7.81 (m, 1H), 7.58 (m, 1H), 4.47 (m, 1H), 4.35 (m, 1H), 4.19 (s, 3H), 4.05 (m, 1H), 3.80 (m, 1H), 3.61 (m, 1H), 3.11 (s, 3H), 3.00 (m, 2H), 2.85 (m, 1H), 2.62 (m, 2H), 2.49 (m, 2H), 2.15 (m, 4H), 2.03 (m, 1H), 1.90 (m, 1H), 1.69 (m, 1H).

### Example 1.31

### Ethyl 6-[5,6-difluoro-8-(methylamino)-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate

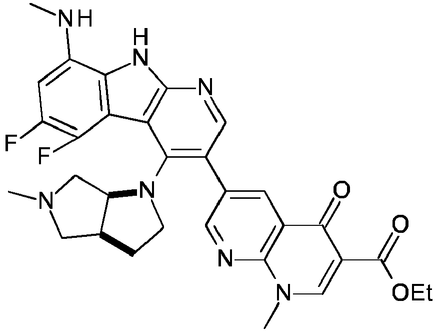

The titled compound was synthesized according to the following scheme:

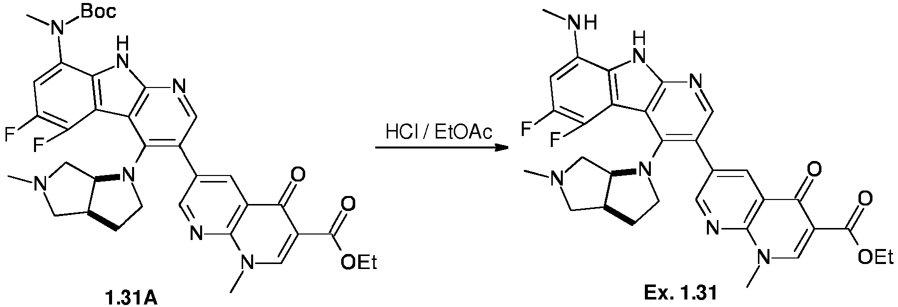

### Step (a) Preparation of ethyl 6-[8-[tert-butoxycarbonyl(methyl)amino]-5,6-difluoro-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.31A)

In analogy to the synthesis of compound 1.16C, cis-5-methyl-2,3,3a,4,6,6a-hexahydro-1H-pyrrolo[2,3-c]pyrrole (CAS: 876130-70-0) was used instead of 5-methyl-1H-hexahydropyrrolo[2,3-c]pyrrole dihydrochloride in step (a) to give ethyl 6-[8-[tert-butoxycarbonyl(methyl)amino]-5,6-difluoro-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (59.6% yield) as a yellow solid after step (b). MS (ESI): 688.2 ([M+H]⁺).

### Step (b) Preparation of ethyl 6-[5,6-difluoro-8-(methylamino)-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (Example 1.31)

To a solution of ethyl 6-[8-[tert-butoxycarbonyl(methyl)amino]-5,6-difluoro-4-[cis-5-methyl-2,3,3a,4,6,6a-hexahydropyrrolo [2,3-c]pyrrol-1-yl]-9H-pyrido [2,3-b]indol-3-yl]-1-methyl-4-oxo-1,8-naphthyridine-3-carboxylate (250 mg, 0.36 mmol) in EtOAc (5 mL) was added HCl in EtOAc solution (1.0 mL, 4 N), and the resulting reaction mixture was stirred at 15 °C for 1 h. After LC-MS showed the staring material was consumed completely, the mixture solution was concentrated *in vacuo* to give a crude product of ethyl 6-[5,6-difluoro-8-(methylamino)-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (210 mg, 98.3% yield) as a yellow solid. MS (ESI): 588.2 ([M+H]⁺).

### Example 1.32

### 7-[5,6-Difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid

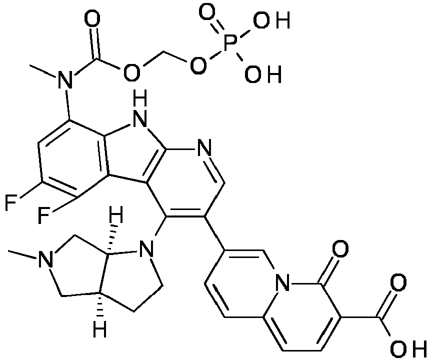

The titled compound was synthesized according to the following scheme:

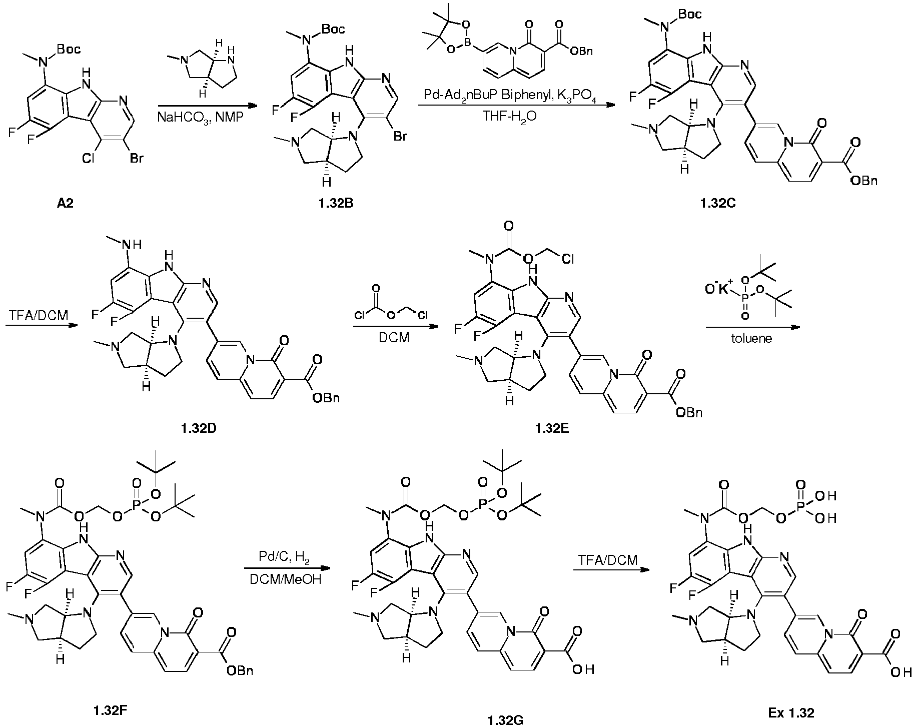

### Step (a) Preparation of tert-butyl N-[3-bromo-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.32B)

In analogy to the synthesis of compound 1.01B, *tert*-butyl N-(3-bromo-4-chloro-5,6-difluoro-9H-pyrido[2,3-b]indol-8-yl)-N-methyl-carbamate (Intermediate A2) was used instead of *tert*-butyl N-(3,4-dichloro-5-cyano-6-fluoro-9H-pyrido[2,3-b]indol-8-yl)-N-methyl-carbamate (Intermediate A3) to give *tert*-butyl N-[3-bromo-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methylcarbamate as a light yellow solid (88.3% yield) after silica gel flash chromatograph purification (petroleum ether:EtOAc = 10: 1 ~ 1:1, 2% Et₃N as additive). MS (ESI): 536.2 ([{⁷⁹Br}M+H]⁺).

### Step (b) Preparation of benzyl 7-[8-[tert-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32C)

In analogy to the synthesis of compound 1.01C, *tert*-butyl N-[3-bromo-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.32B) and benzyl 1-methyl-4-oxo-6-(4,4,5,5-tetramethyl-1,3,2-dioxaborolan-2-yl)-1,8-naphthyridine-3-carboxylate (Intermediate B4) were used instead of *tert*-butyl N-[3-chloro-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.01B) and benzyl 1-methyl-4-oxo-6-(4,4,5,5-tetramethyl-1,3,2-dioxaborolan-2-yl)-1,8-naphthyridine-3-carboxylate (Intermediate B3) to give benzyl 7-[8-[*tert-*butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (77.0% yield) as a yellow solid after prep-HPLC purification (TFA as additive). MS (ESI): 735.3 ([M+H]⁺).

### Step (c) Preparation of benzyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32D)

In analogy to the synthesis of compound 1.01D, benzyl 7-[8-[*tert-*butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32C) was used instead of benzyl 6-[8-[*tert-*butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01C) to give benzyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (97.5%) as a yellow solid after prep-HPLC purification (TFA as additive). MS (ESI): 635.2 ([M+H]⁺).

### Step (d) Preparation of benzyl 7-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32E)

In analogy to the synthesis of compound 1.01E, benzyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32D) was used instead of benzyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01D) to give benzyl 7-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yll-4-oxo-quinolizine-3-carboxylate as a yellow solid (53.6% yield) after prep-HPLC purification (TFA as additive) and subsequent work up. MS (ESI): 727.2 ([{³⁵Cl}M+H]⁺).

### Step (e) Preparation of benzyl 7-[8-[ditert butoxyphosphoryloxymethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32F)

In analogy to the synthesis of compound 1.01F, benzyl 7-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32E) was used instead of benzyl 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01E) to give benzyl 7-[8-[ditert butoxyphosphoryloxymethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate as a light yellow solid (57.9% yield) after prep-HPLC purification (TFA as additive) and subsequent work up. MS (ESI): 901.3 ([M+H]⁺).

### Step (f) Preparation of 7-[8-[ditert-butoxyphosphoryloxymethoxycarbonyl(methyl)aminol-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.32G)

In analogy to the synthesis of compound 1.01G, benzyl 7-[8-[ditert butoxyphosphoryloxymethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32F) was used instead of benzyl 6-[5-cyano-8-[di-*tert-*butoxyphosphoryloxymethoxycarbonyl(methyl)amino]-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01F) to give 7-[8-[di*tert-*butoxyphosphoryloxymethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (87.8% yield) as a crude product of light yellow solid. MS (ESI): 811.3 ([M+H]⁺).

### Step (g) Preparation of 7-[5,6-difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (Example 1.32)

In analogy to the synthesis of Example 1.01, 7-[8-[ditert-butoxyphosphoryloxymethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.32G) was used instead of 6-[5-cyano-8-[di-*tert-*butoxyphosphoryloxymethoxycarbonyl(methyl)amino]-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.01G) to give 7-[5,6-difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid as a light yellow solid (49.8% yield) after prep-HPLC purification (NH₄HCO₃ as additive). MS (ESI): 699.1 ([M+H]⁺). ¹H NMR (400 MHz, DMSO-d6) δ ppm 12.88 - 13.65 (m, 1 H), 9.18 (s, 1 H), 8.38 - 8.50 (m, 1 H), 8.07 - 8.30 (m, 3 H), 7.58 (br dd, J=11.5, 6.6 Hz, 1 H), 7.36 (br d, J=8.6 Hz, 1 H), 5.49 (br s, 1 H), 4.50 (br s, 1 H), 3.26 (br s, 6 H), 3.17 (br s, 2 H), 2.97 (br s, 1 H), 2.73 (br s, 1 H), 2.63 (br s, 1 H), 2.40 (br s, 2 H), 2.01 (br s, 1 H), 1.73 (br s, 1 H).

### Example 1.33

### 7-[5,6-Difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid

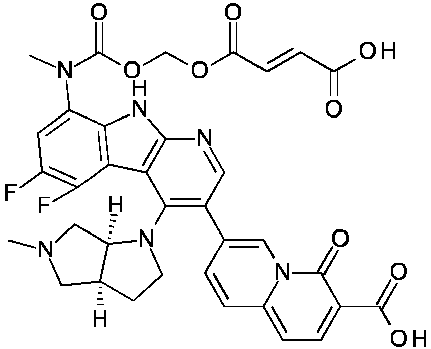

The titled compound was synthesized according to the following scheme:

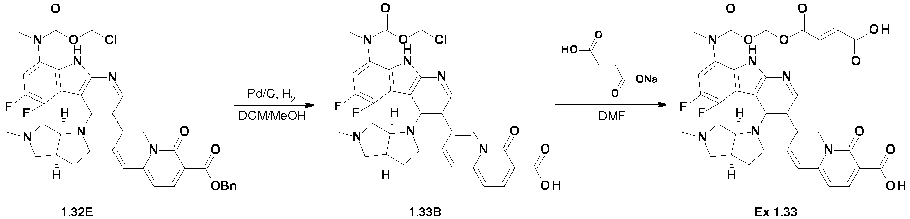

### Step (a) Preparation of 7-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.33B)

In analogy to the synthesis of compound 1.02B, benzyl 7-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.32E) was used instead of benzyl 6-[8-[chloromethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.01E) to give a crude product of 7-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (93.3% yield) as a light yellow solid. MS (ESI): 637.2 ([M+H]⁺).

### Step (b) Preparation of 7-[5,6-difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (Example 1.33)

In analogy to the synthesis of Example 1.02, 7-[8-[chloromethoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.33B) was used instead of 6-[4-[(3aS,6aS)-5-methyl-2,3,3a,4,6,6a-hexahydropyrrolo[2,3-c]pyrrol-1-yl]-8-[chloromethoxycarbonyl(methyl)amino]-5-cyano-6-fluoro-9H-pyrido[2,3-b]indol-3-yl]-1-methyl-4-oxo-1,8-naphthyridine-3-carboxylic acid (compound 1.02B) to give 7-[5,6-difluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (42% yield) as a yellow solid after prep-HPLC purification (NH₄HCO₃ as additive). MS (ESI): 717.1 ([M+H]⁺). ¹H NMR (400 MHz, DMSO-d6) δ ppm 12.52 (br s, 1 H), 9.22 (s, 1 H), 8.45 (d, J=8.6 Hz, 1 H), 8.16 - 8.34 (m, 3 H), 7.61 - 7.76 (m, 1 H), 7.36 (br d, J=8.6 Hz, 1 H), 6.37 - 6.87 (m, 2 H), 5.60 - 5.96 (m, 2 H), 4.36 - 4.53 (m, 1 H), 3.25 - 3.29 (m, 3 H), 3.17 - 3.19 (m, 1 H), 3.12 (br s, 1 H), 2.90 (br s, 2 H), 2.56 - 2.64 (m, 2 H), 2.37 (br d, J=9.3 Hz, 1 H), 2.18 (s, 3 H), 1.88 - 2.05 (m, 2 H), 1.71 (br d, J=4.4 Hz, 1 H).

### Example 1.34

### 7-[8-[[2-[[2-[(2-Aminoacetyl)amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid

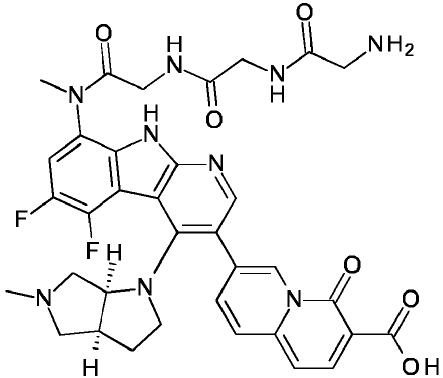

The titled compound was synthesized according to the following scheme:

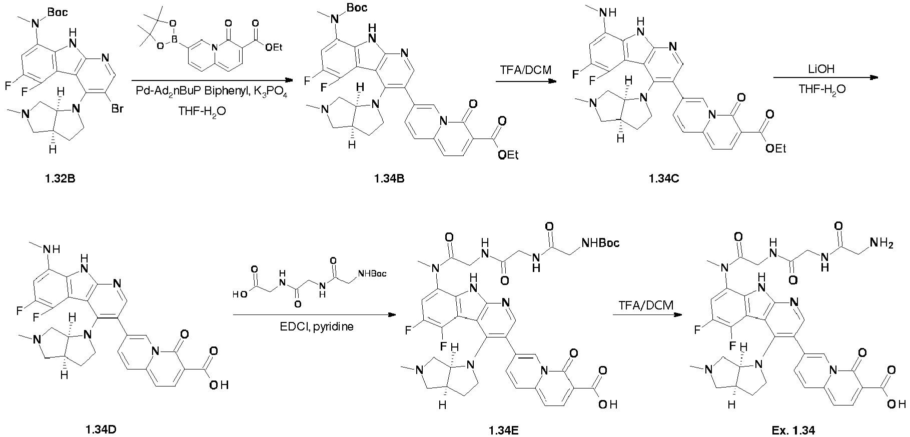

### Step (a) Preparation of ethyl 7-[8-[tert-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.34B)

In analogy to the synthesis of compound 1.03B, *tert*-butyl N-[3-bromo-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.32B)was used instead of *tert*-butyl N-[3-chloro-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (20.0 g, 40.1 mmol, compound 1.01B) to give ethyl 7-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate as a yellow solid (73.0% yield) after prep-HPLC purification (TFA as additive). MS (ESI): 673.3 ([M+H]⁺).

### Step (b) Preparation of ethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.34C)

In analogy to the synthesis of compound 1.03D, ethyl 7-[8-[*tert-*butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.34B) was used instead of 6-[8-[tert-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.03C) to give a crude product of ethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (93.2% yield) after prep-HPLC purification (NH₃.H₂O as additive) as a yellow solid. MS (ESI): 573.3 ([M+H]⁺).

### Step (c) Preparation of 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.34D)

In analogy to the synthesis of compound 1.03C, ethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.34C) was used instead of ethyl 6-[8-[*tert-*butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.03B) to give a crude product of 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (96% yield) as a yellow solid. MS (ESI): 545.1.

### Step (d) Preparation of 7-[8-[[2-[[2-[[2-(tert-butoxycarbonylamino)acetyl]amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.34E)

A mixture solution of 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (200 mg, 0.37 mmol), 2-[[2-[[2-(*tert-*butoxycarbonylamino)acetyl]amino]acetyl]amino]acetic acid (200 mg, 0.69 mmol), and EDCI (200 mg, 1.04 mmol) in pyridine (2 mL) was stirred at 80 °C for 16 h until LC-MS showed the starting material was consumed. The mixture solution was cooled back to r.t., concentrated *in vacuo* to give a crude product, which was purified by prep-HPLC (NH₃.H₂O as additive) to give 7-[8-[[2-[[2-[[2-(*tert*-butoxycarbonylamino)acetyl]amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (80 mg, 26.7% yield) as a yellow solid. MS (ESI): 816.6 ([M+H]⁺).

### Step (e) Preparation of 7-[8-[[2-[[2-[(2-aminoacetyl)amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (Example 1.34)

To a solution of 7-[8-[[2-[[2-[[2-(*tert-*butoxycarbonylamino)acetyl]amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (80 mg, 0.10 mmol) in DCM (5 mL) was added TFA (1.0 mL, 12.98 mmol), and the resulting reaction mixture was stirred at 20 °C for 1 h. The reaction mixture was then concentrated *in vacuo* to give a crude product, which was purified by prep-HPLC (NH₃.H₂O as additive) to give 7-[8-[[2-[[2-[(2-aminoacetyl)amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (45 mg, 61.9% yield) as a yellow solid. MS (ESI): 716.3 ([M+H]⁺). ¹H NMR (400 MHz, DMSO-*d_{6,}* T=80) δ ppm: 9.24 (s, 1H), 8.47 (d, *J*=8.4 Hz, 1H), 8.31 (s, 1H), 8.15 (m, 2H), 7.70 (m, 1H), 7.63 (m, 1H), 7.32 (m, 1H), 4.44 (m, 1H), 3.76 (m, 2H), 3.31 (m, 4H), 3.26(m, 2H), 3.16 (m, 3H), 3.00 (m, 1H), 2.45 (m, 2H), 2.25 (m, 1H), 2.10 (s, 3H), 2.05 (m, 1H), 1.90 (m, 1H), 1.74 (m, 1H).

### Example 1.35

### 7-[8-[[2-[(2-Aminoacetyl)amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid

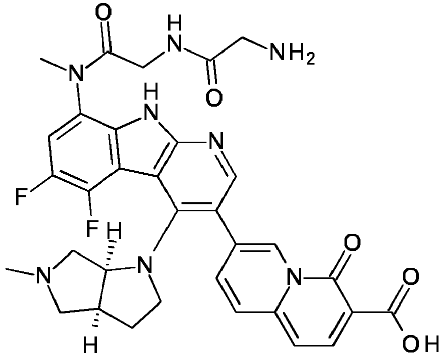

The titled compound was synthesized according to the following scheme:

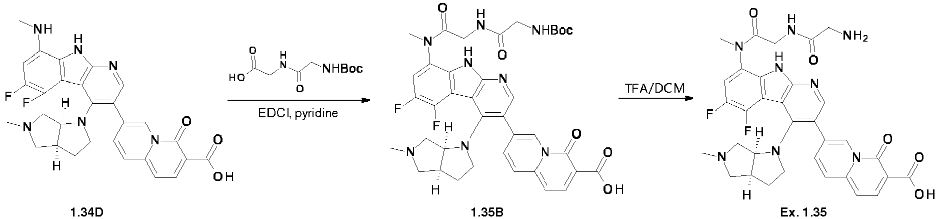

### Step (a) Preparation of 7-[8-[[2-[[2-(tert-butoxycarbonylamino)acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.35B)

In analogy to the synthesis of compound 1.34E, 2-[[2-(*tert-*butoxycarbonylamino)acetyl]amino]acetic acid was used instead of 2-[[2-[[2-(*tert-*butoxycarbonylamino)acetyl]amino]acetyl]amino]acetic acid to give 7-[8-[[2-[[2-(*tert-*butoxycarbonylamino)acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (32.3% yield) as a yellow solid after prep-HPLC purification (NH₃.H₂O as additive). MS (ESI): 759.6 ([M+H]⁺).

### Step (b) Preparation of 7-[8-[[2-[(2-aminoacetyl)amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (Example 1.35)

In analogy to the synthesis of Example 1.34, 7-[8-[[2-[[2-(*tert-*butoxycarbonylamino)acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid was used instead of 7-[8-[[2-[[2-[[2-(*tert-*butoxycarbonylamino)acetyl]amino]acetyl]amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.34E) to give 7-[8-[[2-[(2-aminoacetyl)amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (59.3% yield) as a yellow solid after prep-HPLC purification (TFA as additive). MS (ESI): 659.2 ([M+H]⁺). ¹H NMR (400 MHz, DMSO-*d_{6,}* T=80) δ ppm: 12.65 (m, 1H), 9.24 (m, 1H), 8.52 (d, *J*=8.4 Hz, 1H), 8.37 (m, 2H), 8.22 (d, *J*=8.4 Hz, 1H), 8.05 (m, 1H), 7.94 (m, 1H), 7.75 (m, 1H), 7.39 (d, *J*=8.4 Hz, 1H), 4.32 (m, 1H), 3.85 (m 4H), 3.63 (m, 2H), 3.51 (m, 2H), 3.36 (m, 3H), 2.73 (s, 3H), 2.55 (m, 3H), 2.40(m, 1H), 2.18 (m, 1H), 1.93 (m, 1H).

### Example 1.36

### 7-[5,6-Difluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid

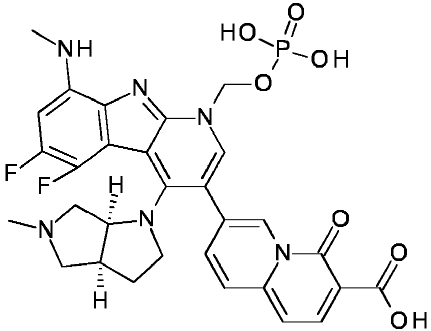

The titled compound was synthesized according to the following scheme:

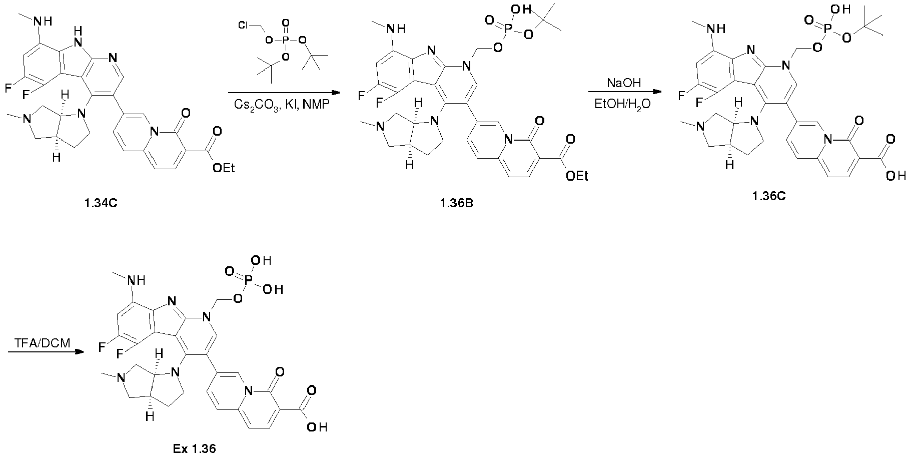

### Step (a) Preparation of ethyl 7-[1-[[tert-butoxy(hydroxy)phosphoryl]oxymethyl]-5,6-difluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylate (compound 1.36B)

In analogy to the synthesis of compound 1.04C, ethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.34C) was used instead of ethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.04B) to give ethyl 7-[1-[[*tert*-butoxy(hydroxy)phosphoryl]oxymethyl]-5,6-difluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylate (42.0% yield) as a yellow solid after prep-HPLC purification (NH₄HCO₃ as additive). MS (ESI): 739.3 ([M+H]⁺).

### Step (b) Preparation of 7-[1-[[tert-butoxy(hydroxy)phosporyl]oxymethyl]-5,6-difluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid (compound 1.36C)

In analogy to the synthesis of compound 1.04D, ethyl 7-[1-[[*tert-*butoxy(hydroxy)phosphorylloxymethyl]-5,6-difluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylate (compound 1.36B) was used instead of ethyl 6-[1-[[*tert-*butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylate (compound 1.04C) to give a crude product of 7-[1-[[*tert-*butoxy(hydroxy)phosphoryl]oxymethyl]-5,6-difluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid (95.3% yield) as a yellow solid. MS (ESI): 711.3 ([M+H]⁺).

### Step (c) Preparation of 7-[5,6-difluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid (Example 1.36)

In analogy to the synthesis of Example 1.04, 7-[1-[[*tert-*butoxy(hydroxy)phosphorylloxymethyl]-5,6-difluoro-8-(methylamino)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid (compound 1.36C) was used instead of ethyl 6-[1-[[*tert-*butoxy(hydroxy)phosphoryl]oxymethyl]-5-cyano-6-fluoro-8-(methylamino)-4-[(3aS,6aS)-5-methyl-2,3,3a,4,6,6a-hexahydropyrrolo [2,3-c]pyrrol-1-yl]pyrido [2,3-b]indol-3-yl]-1-methyl-4-oxo-1,8-naphthyridine-3-carboxylate (compound 1.04C) to give 7-[5,6-difluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid (9.2% yield) as a yellow solid after prep-HPLC purification (TFA as additive). MS (ESI): 655.3 ([M+H]⁺). ¹H NMR (400 MHz, DMSO-d6) δ ppm 14.14 (s, 1 H), 9.37 (br s, 1 H), 8.54 (br s, 1 H), 8.49 (br d, J=8.25 Hz, 1 H), 8.24 (br d, J=12.51 Hz, 2 H,) 7.40 (br d, J=8.25 Hz, 1 H), 6.70 (br dd, J=12.51, 6.00 Hz, 1 H), 6.54 (br s, 1 H), 6.08 (br d, J=9.88 Hz, 2 H), 4.99 (br s, 2 H), 3.12 (br s, 5 H), 2.92 (br s, 3 H), 2.68 (br s, 2 H), 1.90-2.06 (m, 2 H), 1.76 (s, 1 H) 1.20-1.32 (m, 3H).

### Example 1.37

### 2-[(2S)-2-Amino-3-methyl-butanoyl]oxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate

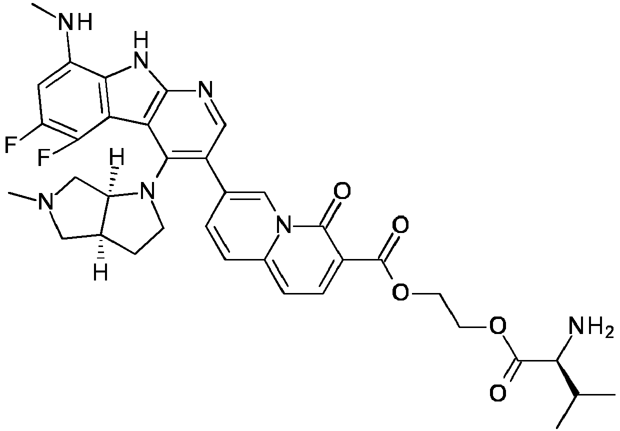

The titled compound was synthesized according to the following scheme:

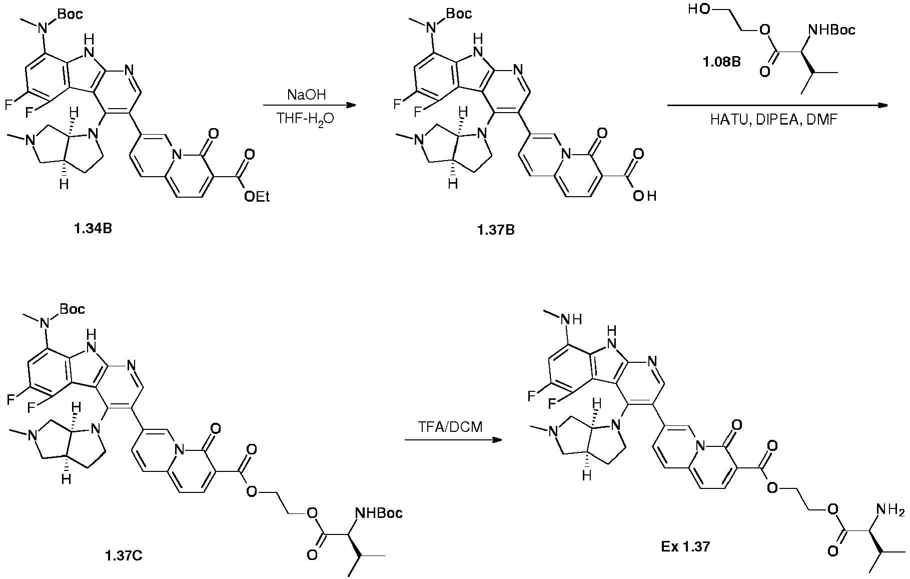

### Step (a) Preparation of 7-[8-[tert-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.37B)

In analogy to the synthesis of compound 1.03C, ethyl 7-[8-[*tert-*butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.34B) was used instead of ethyl 6-[8-[*tert-*butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.03B) to give a crude product of 7-[8-[*tert-*butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (78.3% yield) as a yellow solid. MS (ESI): 650.2 ([M+H]⁺).

### Step (b) Preparation of 2-[(2S)-2-(tert-butoxycarbonylamino)-3-methyl-butanoyl]oxyethyl 7-[8-[tert-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.37C)

In analogy to the synthesis of compound 1.08C, 7-[8-[*tert-*butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (compound 1.37B) was used instead of 6-[8-[*tert-*butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.03C) to give 2-[(2S)-2-(*tert-*butoxycarbonylamino)-3-methyl-butanoyl]oxyethyl 7-[8-[*tert*-butoxycarbonyl(methyl)amino]-5,6-difluoro-4-[(3 aS,6aS)-5-methyl-2,3,3 a,4,6,6a-hexahydropyrrolo [2,3-c]pyrrol-1-yl]-9H-pyrido[2,3-b]indol-3-yl]-4-oxo-quinolizine-3-carboxylate (25.4% yield) as a yellow solid after prep-HPLC purification (NH₄HCO₃ as additive). MS (ESI): 888.5 ([M+H]⁺).

### Step (c) Preparation of 2-[(2S)-2-amino-3-methyl-butanoyl]oxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (Example 1.37)

In analogy to the synthesis of Example 1.08, 2-[(2S)-2-(*tert*-butoxycarbonylamino)-3-methyl-butanoyl]oxyethyl 7-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (compound 1.37C) was used instead of 2-[(2S)-2-(*tert-*butoxycarbonylamino)-3-methyl-butanoyl]oxyethyl 6-[8-[*tert*-butoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.08C) to give 2-[(2S)-2-amino-3-methyl-butanoyl]oxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (49.5% yield) as a yellow solid after prep-HPLC purification (NH₄CO₃ as additive). MS (ESI): 688.4 ([M+H]⁺). ¹H NMR (400 MHz, DMSO-*d*₆) δ ppm: ¹H NMR (400MHz, DMSO-*d*₆) δ = 11.76 (br s, 1H), 9.14 (s, 1H), 8.27 (d, *J*=8.6 Hz, 1H), 8.19 (s, 1H), 8.10 - 8.04 (m, 1H), 8.01 - 7.96 (m, 1H), 6.96 (d, *J*=8.6 Hz, 1H), 6.58 (dd, *J*=6.2, 13.6 Hz, 1H), 5.71 (br d, *J*=4.0 Hz, 1H), 4.52 - 4.33 (m, 5H), 3.17 - 3.03 (m, 3H), 2.89 (d, *J*=4.9 Hz, 4H), 2.45 (br d, *J*=10.5 Hz, 2H), 2.21 (br t, *J*=8.3 Hz, 1H), 2.13 - 2.07 (m, 3H), 2.02 - 1.89 (m, 2H), 1.83 (br dd, *J*=5.1, 9.6 Hz, 1H), 1.66 (br s, 1H), 0.86 (dd, 7=6.8, 10.3 Hz, 6H).

### Example 1.38

### 2-[[2-[[7-[5,6-Difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetic acid

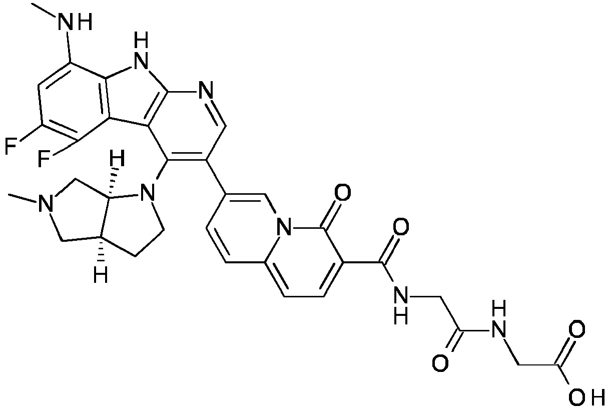

The titled compound was synthesized according to the following scheme:

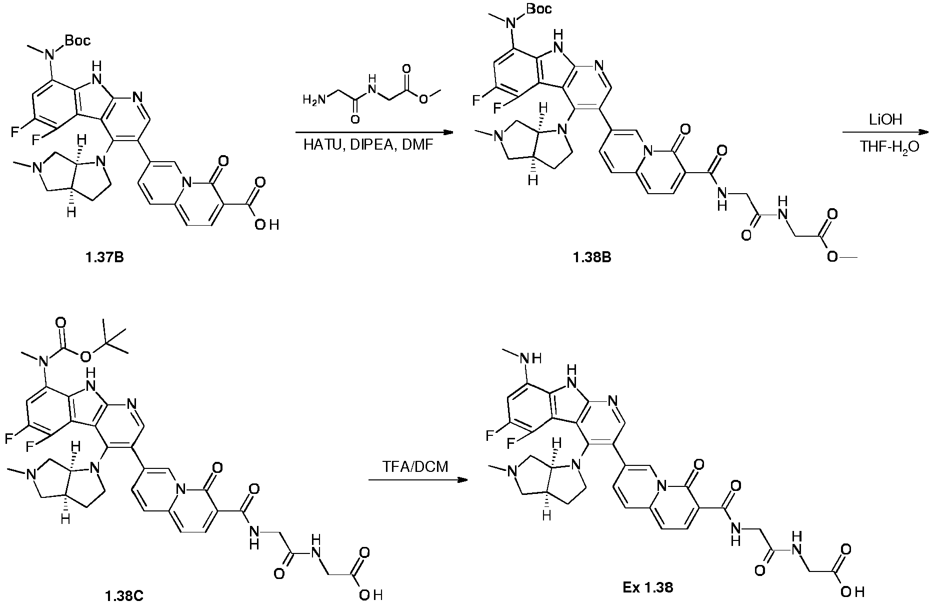

### Step (a) Preparation of methyl 2-[[2-[[7-[8-[tert-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetate (compound 1.38B)

A mixture solution of 7-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (40.0 mg, 0.06 mmol, compound 1.37B), HATU (47.2 mg, 0.12 mmol) and DIPEA (0.03 mL, 0.190 mmol) in DMF (2 mL) was stirred at 20 °C for 0.5 h before methyl 2-[(2-aminoacetyl)amino]acetate (18.1 mg, 0.12 mmol) was added, and the resulting reaction mixture was stirred at 25 °C for another 16 h. After LC-MS showed the starting material was consumed, the mixture solution was concentrated *in vacuo* to give a crude product, which was further purified by prep-HPLC (TFA as additive) to give methyl 2-[[2-[[7-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl] amino] acetyl] amino] acetate (40 mg, 83.4% yield) as a yellow solid. MS (ESI): 773.4 ([M+H]⁺).

### Step (b) Preparation of 2-[[2-[[7-[8-[tert-butoxycarbonyl(methyl)aminol-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetic acid (compound 1.38C)

To a solution of methyl 2-[[2-[[7-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetate (38.0 mg, 0.05 mmol) in THF (5 mL) was added aq. lithium hydroxide solution (1 N, 0.25 mL, 0.25 mmol), and the resulting reaction mixture was stirred at 40 °C for 2 h. After LC-MS showed the starting material was consumed, the mixture solution was concentrated *in vacuo* to give a cruder product of 2-[[2-[[7-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetic acid (37 mg, 97.2% yield) as a yellow solid. MS (ESI): 759.4 ([M+H]⁺). It was used directly in the next step without further purification.

### Step (c) Preparation of 2-ΓΓ2-ΓΓ7-Γ5,6-Difluoro-8-(methylamino)-4-Γ(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetic acid (Example 1.38)

To a solution of 2-[[2-[[7-[8-[*tert*-butoxycarbonyl(methyl)amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetic acid_(37.0 mg, 0.05 mmol) in DCM (5.09 mL) was added TFA (0.19 mL, 2.44 mmol), and the resulting reaction mixture was stirred at 25 °C for 1 h. After LC-MS showed the starting material was consumed, the mixture solution was concentrated *in vacuo* to give a crude product, which was further purified by prep-HPLC (TFA as additive) to give 2-[[2-[[7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acetyl]amino]acetic acid (22.6 mg, 51.5% yield) as a yellow solid. MS (ESI): 659.3 ([M+H]⁺). ¹H NMR (400 MHz, DMSO-d6) δ ppm 11.85 - 12.03 (m, 1 H), 9.88 - 10.05 (m, 1 H), 9.11 - 9.65 (m, 2 H), 8.57 (d, J=8.38 Hz, 1 H), 8.40 (t, J=5.25 Hz, 1 H), 8.25 - 8.37 (m, 1 H), 8.11 (d, J=8.88 Hz, 1 H), 7.94 (d, J=8.88 Hz, 1 H), 7.23 (dd, J=8.44, 3.44 Hz, 1 H), 6.54 - 6.75 (m, 1 H), 4.32 (s, 1 H), 4.10 (d, J=4.88 Hz, 2 H), 3.81 (d, J=5.75 Hz, 1 H), 3.35 (dd, J=17.45, 8.82 Hz, 3 H), 3.10 - 3.21 (m, 1 H), 2.91 (s, 4 H), 2.68 - 2.75 (m, 3 H), 2.52 - 2.55 (m, 2 H), 2.18 (s, 1 H), 1.99 - 2.11 (m, 1 H), 1.75 - 1.95 (m, 1 H).

### Example 1.39

### 1-Isopropoxycarbonyloxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate

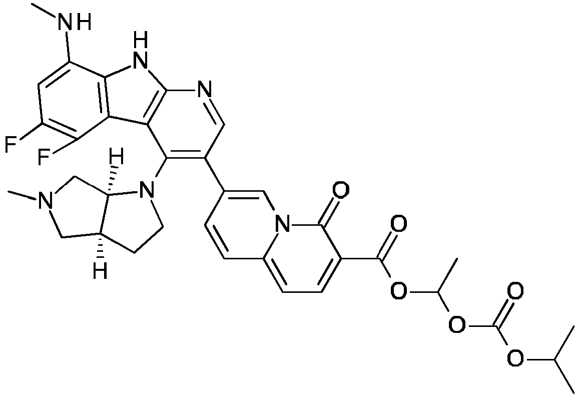

To a solution of 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (100 mg, 0.180 mmol, compound 1.34D) in DMSO (5 mL) was added K₂CO₃ (50 mg, 0.360 mmol), and the reaction mixture was stirred at 20 °C for 0.5 h before 1-chloroethyl isopropyl carbonate (70.0 mg, 0.420 mmol) was added. The resulting reaction mixture was stirred at 20 °C for another 15.5 h before it was concentrated under the reduced pressure. The residue was subject to prep-HPLC (TFA as additive) purification to give 1-Isopropoxycarbonyloxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (132.2 mg, 90.4% yield) as a yellow solid. MS (ESI): 675.4 ([M+H]⁺). ¹H NMR (400 MHz, DMSO-*d₆*) δ ppm: 11.77 (r s, 1H), 9.23 (s, 1H), 8.30 (m, 2H), 8.03 (m, 1H), 7.95 (m, 1H), 6.98 (d, *J*=8.4 Hz, 1H), 6.87 (m, 1H), 6.61 (m, 1H), 4.82 (m, 1H), 4.33 (m, 1H), 3.64 (m, 1H), 3.12 (m, 2H), 2.94 (s, 3H), 2.71 (s, 3H), 2.54 (m, 1H), 2.51 (m, 3H), 2.16 (m, 1H), 1.89 (m, 1H), 1.58 (d, *J*=5.6 Hz, 3H), 1.26 (d, *J*=6.4 Hz, 6H).

### Example 1.40

### 1-Acetoxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate

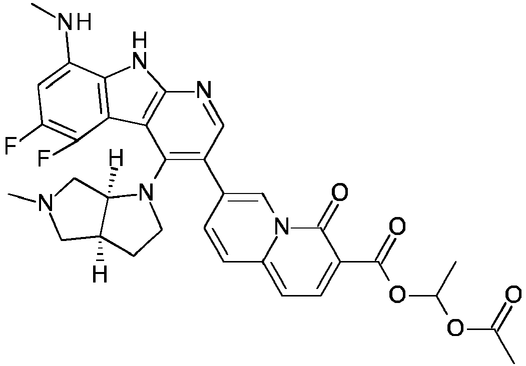

To a solution of 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (200 mg, 0.37 mmol, compound 1.34D) in DMF (5 mL) was added K₂CO₃ (100 mg, 0.72 mmol), and the mixture was stirred at 20 °C for 0.5 h before 1-bromoethyl acetate (100 mg, 0.60 mmol) was added. The resulting mixture was stirred at 20 °C for another 15.5 h before it was concentrated under reduce pressure. The residue was subject to prep-HPLC (TFA as additive) purification to give 1-acetoxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate (15 mg, 4.9% yield) as a yellow solid. MS (ESI): 631.3 ([M+H]⁺). ¹H NMR (400 MHz, DMSO-*d₆*) δ ppm: 11.75 (ms, 1H), 9.23 (s, 1H), 8.31 (d, *J*=8.4 Hz, 2H), 8.031 (m, 1H), 7.94 (m, 1H), 7.00 (m, 2H), 6.62 (m, 1H), 4.33 (m, 1H), 3.64 (m, 2H), 3.41 (m, 1H), 2.94 (s, 3H), 2.72 (s, 3H), 2.54 (m, 2H), 2.13 (m, 2H), 2.07 (s, 3H), 1.90 (m, 2H), 1.56 (d, J=5.6 Hz, 3H).

### Example 1.41

### 7-[5,6-Difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-N-methylsulfonyl-4-oxo-quinolizine-3-carboxamide

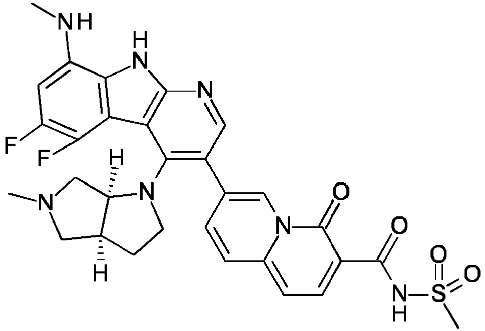

To a solution of 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid (100 mg, 0.18 mmol, compound 1.34D) in DCM (5 mL) was added thionyl chloride (43.7 mg, 0.37 mmol) at 0°C. After the addition, the mixture solution was allowed to warm-up and stirred at 25° C for 2 h until LC-MS showed the starting material was consumed completely. The mixture solution was then added to a solution of methanesulfonamide (87.3 mg, 0.92 mmol) in DCM (5 mL) and the resulting solution was stirred at 25° C for another 16 h. The reaction mixture was concentrated *in vacuo* to give a crude product, which was purified by prep-HPLC (NH₄HCO₃ as additive) to give 7-[5,6-difluoro-8-(methylamino)-4-[(3aS,6aS)-5-methyl-2,3,3 a,4,6, 6a-hexahydropyrrolo [2,3-c]pyrrol-1-yl]-9H-pyrido[2,3-b]indol-3-yl]-N-methylsulfonyl-4-oxo-quinolizine-3-carboxamide (7.5 mg, 6.2% yield) as a yellow solid. MS (ESI): 622.1 ([M+H]⁺). ¹H NMR (400 MHz, DMSO-*d*₆) δ ppm: 11.82 (br s, 1 H), 9.26 (s, 1 H), 8.48 (d, *J*=8.6 Hz, 1 H), 8.20 - 8.23 (m, 1 H), 8.13 - 8.18 (m, 1 H), 7.30 (br d, *J*=8.6 Hz, 1 H), 6.58 (br dd, *J*=13.5, 6.2 Hz, 1 H), 5.75 (br d, *J*=4.4 Hz, 1 H), 4.45 (br s, 1 H), 4.16 (br d, *J*=7.0 Hz, 1 H), 3.17 (s, 3 H), 2.89 (br d, *J*=4.6 Hz, 3 H), 2.70 (s, 1 H), 2.26 (br s, 1 H), 2.15 - 2.21 (m, 2 H), 2.13 (br s, 3 H), 1.97 - 2.05 (m, 1 H), 1.90 (dt, *J*=15.1, 7.5 Hz, 2 H), 1.68 (br s, 1 H).

### Example 1.42

### 6-[5,6-Difluoro-8-(methylamino)-4-[cis-5-methyl-2,3,3a,4,6,6a-hexahydropyrrolo[2,3-c]pyrrol-1-yl]-9H-pyrido[2,3-b]indol-3-yl]-1-methyl-N-methylsulfonyl-4-oxo-1,8-naphthyridine-3-carboxamide

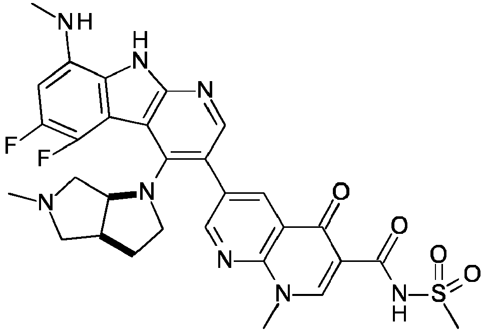

In analogy to the synthesis of Example 1.10, 6-[5,6-difluoro-8-(methylamino)-4-[cis-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.29A) was used instead of 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (compound 1.03D) to give 6-[5,6-Difluoro-8-(methylamino)-4-[*cis*-5-methyl-2,3,3a,4,6,6a-hexahydropyrrolo[2,3-c]pyrrol-1-yl]-9H-pyrido[2,3-b]indol-3-yl]-1-methyl-N-methylsulfonyl-4-oxo-1,8-naphthyridine-3-carboxamide (8% yield) as a yellow solid after prep-HPLC purification (NH₃·H₂O as additive). MS (ESI): 637.1 ([M+H]⁺). ¹H NMR (400 MHz, DMSO-*d*₆) *δ* ppm: 12.97 (d, 1H), 11.93 (d, 1H), 9.47 (m, 1H), 9.30 (s, 1H), 9.19 (m, 1H), 9.10 (m, 1H), 8.68 (s, 1H), 8.26 (d, 1H), 6.64 (m, 1H), 4.18 (d, 3H), 4.12 and 3.98 (m, 1H), 3.66 (m, 1H), 3.44 (s, 3H), 3.10 (m, 1H), 3.02 (m, 1H), 2.91 (s, 3H), 2.82 (m, 2H), 2.65 (m, 5H), 2.17 (m, 1H), 2.06 (m, 1H), 1.82 (m, 1H).

### Example 1.43

### 6-[5,6-Difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

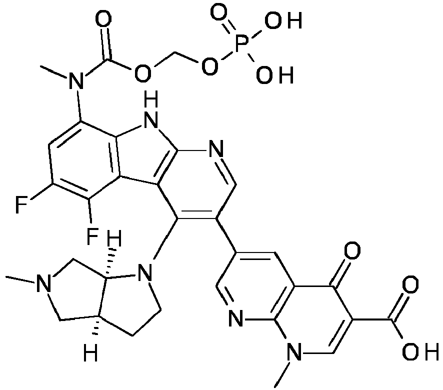

In analogy to the synthesis of Example 1.18, *tert*-butyl N-[3-chloro-5,6-difluoro-4-[(3aS,6aS)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.26B) was used instead of *tert*-butyl N-[3-chloro-5,6-difluoro-4-[(3aR,6aR)-5-methyl-2,3,3a,4,6,6a-hexahydropyrrolo[2,3-c]pyrrol-1-yl]-9H-pyrido[2,3-b]indol-8-yl]-N-methyl-carbamate (compound 1.16B) in step a to afford 6-[5,6-difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid after steps b, c, d, e, and f as a yellow solid. MS (ESI): 714.0 ([M+H]⁺). ¹H NMR (400 MHz, DMSO-*d*₆) δ ppm: 12.87 - 13.85 (m, 1 H), 9.25 (br s, 1 H), 9.10 (br s, 1 H), 8.67 (br s, 1 H), 8.14 (br d, *J*=7.9 Hz, 1 H), 7.59 (br d, *J*=6.7 Hz, 1 H), 5.37 - 5.62 (m, 1 H), 4.34 (br s, 1 H), 4.16 (s, 3 H), 3.30 (br s, 3 H), 3.26 (br s, 3 H), 3.07 (br s, 1 H), 2.88 (br s, 2 H), 2.56 - 2.64 (m, 2 H), 2.18 - 2.38 (m, 2 H), 1.97 (br s, 1 H), 1.67 (br s, 1 H).

### Example 44

### 6-[4-(Dimethylamino)-5,6-difluoro-8-(methylamino)-1-(phosphonooxymethyl)pyrido[2,3-b]indol-3-yl]-1-methyl-4-oxo-1,8-naphthyridine-3-carboxylic acid

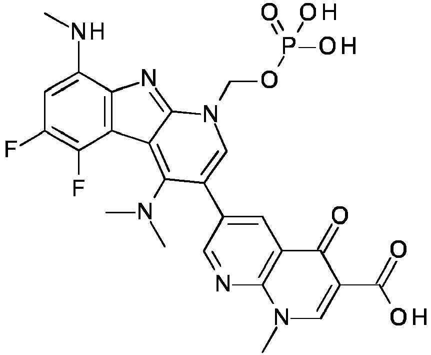

The titled compound was synthesized according to the following scheme:

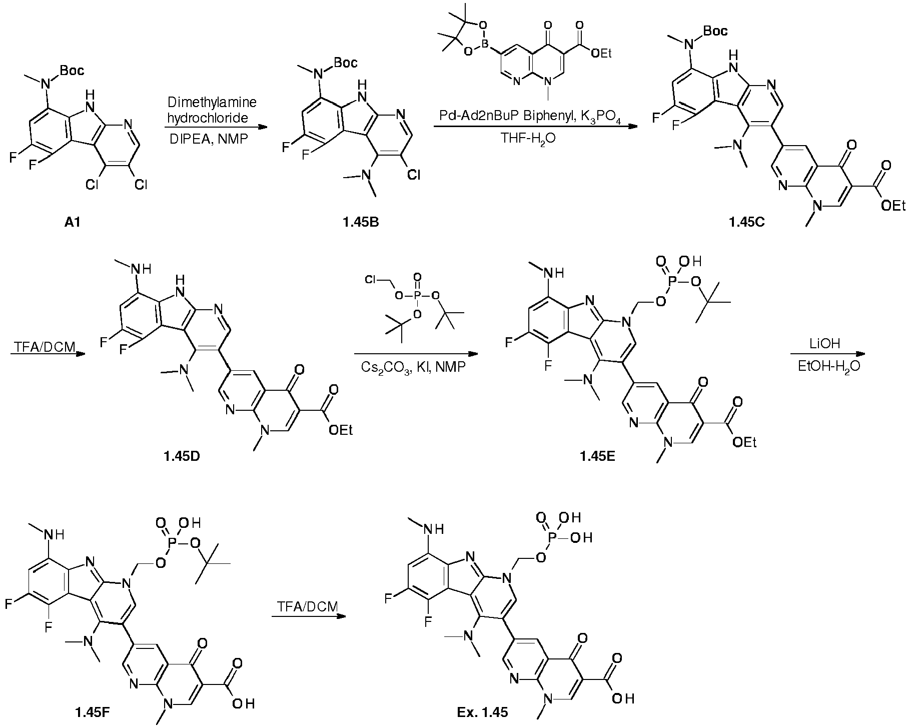

### Step (a) Preparation of tert-butyl N-[3-chloro-4-(dimethylamino)-5,6-difluoro-9H-pyrido[2,3-b]indol-8-yl]-N-methyl-carbamate (compound 1.45B)

A mixture solution of tert-butyl N-(3,4-dichloro-5,6-difluoro-9H-pyrido[2,3-b]indol-8-yl)-N-methyl-carbamate (300.0 mg, 0.75 mmol, Intermediate A1), dimethylamine hydrochloride (150.0 mg, 1.8 mmol), and DIPEA (290.0 mg, 2.2 mmol) in anhydrous NMP (5 mL) was stirred at 100 °C for 12 h until LCMS showed the starting material was consumed. The reaction mixture was cooled back to r.t., diluted with EtOAc (100 mL), poured into water (100 mL), and extracted with EtOAc (100 mL). The organic layer was washed with brine (100 mL), dried over anhy. Na₂SO₄, filtered, and concentrated to give a crude product, which was purified by prep-TLC (petroleum ether:EtOAc = 2:1) to give *tert*-butyl N-[3-chloro-4-(dimethylamino)-5,6-difluoro-9H-pyrido[2,3-b]indol-8-yl]-N-methyl-carbamate (320 mg, 94.0% yield) as a yellow solid. MS (ESI): 411.2 ([M+H]⁺).

### Step (b) Preparation of ethyl 6-[8-[tert-butoxycarbonyl(methyl)aminol-4-(dimethylamino)-5,6-difluoro-9H-pyrido[2,3-b]indol-3-yl]-1-methyl-4-oxo-1,8-naphthyridine-3-carboxylate (compound 1.45C)

To a mixture of *tert*-butyl N-[3-chloro-4-(dimethylamino)-5,6-difluoro-9H-pyrido[2,3-b]indol-8-yl]-N-methyl-carbamate (0.32 g, 0.78 mmol), ethyl 1-methyl-4-oxo-6-(4,4,5,5-tetramethyl-1,3,2-dioxaborolan-2-yl)-1,8-naphthyridine-3-carboxylate (0.44 g, 1.22 mmol, Intermediate B1), and K₃PO₄ (0.5 g, 2.34 mmol) in THF (10 mL) and water (2 mL) was added Pd-Ad2nBuP Biphenyl (29.1 mg, 0.04 mmol, cataCXium A-Pd-G2, CAS: 1375477-29-4) in glovebox under Ar. The reaction mixture was stirred at 70 °C for 2 h until LCMS showed the starting material was consumed. The mixture solution was cooled back to r.t., diluted with EtOAc (100 mL), poured into water (80 mL), and extracted with EtOAc (100 mL). The organic layer was washed with brine (100 mL), filtered, and concentrated in vacuo to give a crude product, which was purified by prep-HPLC (TFA as additive) to give ethyl 6-[8-[*tert-*butoxycarbonyl(methyl)amino]-4-(dimethylamino)-5,6-difluoro-9H-pyrido[2,3-b]indol-3-yl]-1-methyl-4-oxo-1,8-naphthyridine-3-carboxylate (400 mg, 84.7% yield) as a yellow solid. MS (ESI): 564.3 ([M+H]⁺).

### Step (c) Preparation of ethyl 6-[4-(dimethylamino)-5,6-difluoro-8-(methylamino)-9H-pyrido[2,3-b]indol-3-yl]-1-methyl-4-oxo-1,8-naphthyridine-3-carboxylate (compound 1.45D)

To a solution of ethyl 6-[8-[tert-butoxycarbonyl(methyl)amino]-4-(dimethylamino)-5,6-difluoro-9H-pyrido[2,3-b]indol-3-yl]-1-methyl-4-oxo-1,8-naphthyridine-3-carboxylate (400.0 mg, 0.66 mmol) in DCM (3.0 mL) was added TFA (1.0 mL, 13.0 mmol), and the resulting reaction mixture was stirred at 20 °C for 1 h. After LC-MS showed the starting material was consumed, the mixture was then concentrated in vacuo to give a crude product, which was purified by prep-HPLC (TFA as additive) to give ethyl 6-[4-(dimethylamino)-5,6-difluoro-8-(methylamino)-9H-pyrido[2,3-b]indol-3-yl]-1-methyl-4-oxo-1,8-naphthyridine-3-carboxylate (330 mg, 98.8% yield) as a yellow solid. MS (ESI): 507.3 ([M+H]⁺).

### Step (d) Preparation of ethyl 6-[1-[[tert-butoxy(hydroxy)phosphoryl]oxymethyl]-4-(dimethylamino)-5,6-difluoro-8-(methylamino)pyrido[2,3-b]indol-3-yl]-1-methyl-4-oxo-1,8-naphthyridine-3-carboxylate (compound 1.45E)

A mixture solution of ethyl 6-[4-(dimethylamino)-5,6-difluoro-8-(methylamino)-9H-pyrido[2,3-b]indol-3-yl]-1-methyl-4-oxo-1,8-naphthyridine-3-carboxylate (120.0 mg, 0.24 mmol), di-*tert*-butyl chloromethyl phosphate (224.0 mg, 0.87 mmol), cesium carbonate (232.0 mg, 0.71 mmol), and potassium iodide (120.0 mg, 0.72 mmol) in NMP (6.0 mL) was stirred at 20 °C for 12 h. After LC-MS showed the starting material was consumed, the mixture was poured into water (50.0 mL), and extracted with EtOAc (50.0 mL). The organic layer was dried over anhy. Na₂SO₄, filtered, and concentrated in vacuo to give a crude product, which was purified by prep-HPLC (NH₄HCO₃ as additive) to give ethyl 6-[1-[[*tert-*butoxy(hydroxy)phosphoryl]oxymethyl]-4-(dimethylamino)-5,6-difluoro-8-(methylamino)pyrido[2,3-b]indol-3-yl]-1-methyl-4-oxo-1,8-naphthyridine-3-carboxylate (20 mg, 75.3% yield) as colorless oil. MS (ESI): 673.3 ([M+H]⁺).

### Step (e) Preparation of 6-[1-[[tert-butoxy(hydroxy)phosphoryl]oxymethyl]-4-(dimethylamino)-5,6-difluoro-8-(methylamino)pyrido[2,3-b]indol-3-yl]-1-methyl-4-oxo-1,8-naphthyridine-3-carboxylic acid (compound 1.45F)

To a solution of ethyl 6-[1-[[*tert*-butoxy(hydroxy)phosphoryl]oxymethyl]-4-(dimethylamino)-5,6-difluoro-8-(methylamino)pyrido[2,3-b]indol-3-yl]-1-methyl-4-oxo-1,8-naphthyridine-3-carboxylate_4 (20.0 mg, 0.03 mmol) in EtOH (5.0 mL) and water (1.0 mL) was added LiOH.H₂O (12.5 mg, 0.3 mmol), and the resulting reaction mixture was stirred at 20 °C for 2 h. After LC-MS showed the starting material was consumed, the mixture solution was adjusted to pH = 5 with aq. HCl solution (1N). It was concentrated in vacuo to give a crude product, which was purified by prep-HPLC (TFA as additive) to give 6-[1-[[*tert-*butoxy(hydroxy)phosphoryl]oxymethyl]-4-(dimethylamino)-5,6-difluoro-8-(methylamino)pyrido[2,3-b]indol-3-yl]-1-methyl-4-oxo-1,8-naphthyridine-3-carboxylic acid (10 mg, 52.2% yield) as a yellow solid. MS (ESI): 645.2 ([M+H]⁺).

### Step (f) Preparation of 6-[4-(dimethylamino)-5,6-difluoro-8-(methylamino)-1-(phosphonooxymethyl)pyrido[2,3-b]indol-3-yl]-1-methyl-4-oxo-1,8-naphthyridine-3-carboxylic acid (Ex. 1.45)

To a solution of 6-[1-[[*tert*-butoxy(hydroxy)phosphoryl]oxymethyl]-4-(dimethylamino)-5,6-difluoro-8-(methylamino)pyrido[2,3-b]indol-3-yl]-1-methyl-4-oxo-1,8-naphthyridine-3-carboxylic acid (10.0 mg, 0.02 mmol) in DCM (0.6 mL) was added TFA/DCM (v:v=1:4, 0.5 mL, 0.02 mmol), and the resulting reaction mixture was stirred at 20 °C for 1 h. After LC-MS showed the starting material was consumed, the mixture was concentrated in vacuo to give a crude product of 6-[4-(dimethylamino)-5,6-difluoro-8-(methylamino)-1-(phosphonooxymethyl)pyrido[2,3-b]indol-3-yl]-1-methyl-4-oxo-1,8-naphthyridine-3-carboxylic acid. MS (ESI): 589.2 ([M+H]⁺).

### Example 45

### 6-[5-Cyano-6-fluoro-8-[methyl-[1-[(2S)-2-amino-3-methyl-butanoyl]oxyethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid

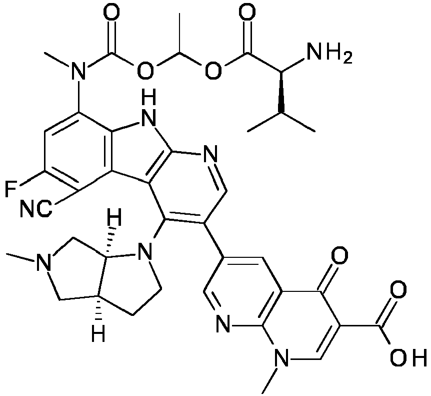

The titled compound was synthesized according to the following scheme:

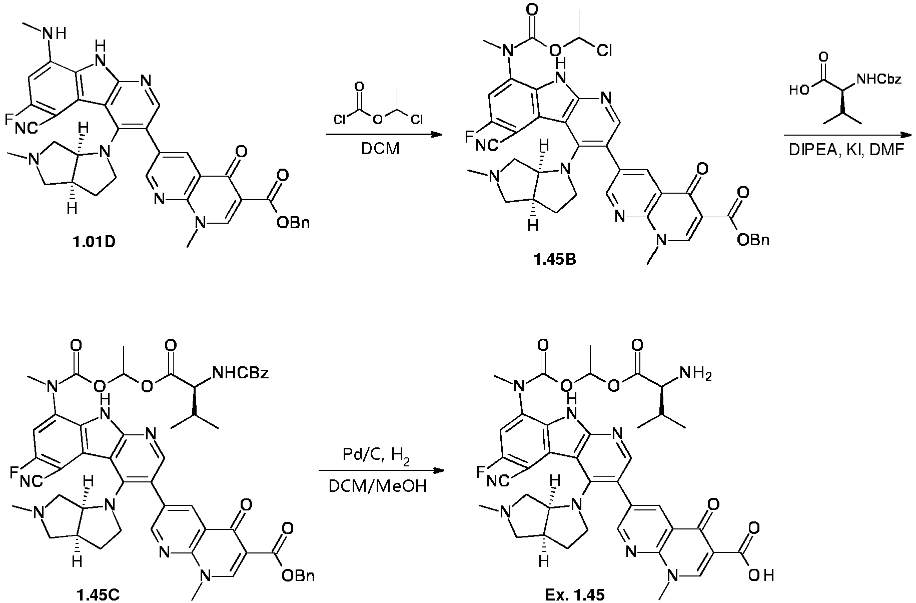

### Step (a) Preparation of benzyl 6-[8-[1-chloroethoxycarbonyl(methyl)amino]-5-cyano-6-fluoro-4-[(3aS,6aS)-5-methyl-2,3,3a,4,6,6a-hexahvdropyrrolo[2,3-c]pyrrol-1-yl]-9H-pyrido[2,3-b]indol-3-yl]-1-methyl-4-oxo-1,8-naphthyridine-3-carboxylate (compound 1.45B)

In analogy to the synthesis of 1.01E, 1-chloroethyl chloroformate was used instead of chloromethyl chloroformate in step d to give a crude product of benzyl 6-[8-[1-chloroethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (77.4% yield) as a light yellow solid. MS (ESI): 763.2 ([M+H]⁺).

### Step (b) Preparation of benzyl 6-[5-cyano-6-fluoro-8-[methyl-[1-[(2S)-2-(benzyloxycarbonylamino)-3-methyl-butanoyl]oxyethoxycarbonyl]aminol-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.45C)

In analogy to the synthesis of 1.14B, (2S)-2-(benzyloxycarbonylamino)-3-methylbutanoic acid was used instead of N-benzyloxycarbonylglycine to give benzyl 6-[5-cyano-6-fluoro-8-[methyl-[1-[(2S)-2-(benzyloxycarbonylamino)-3-methyl-butanoyl]oxyethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (44.3% yield) as a light yellow solid after prep-HPLC purification (formic acid as additive). MS (ESI): 978.4 (([M+H]⁺).

### Step (c) Preparation of 6-[5-cyano-6-fluoro-8-[methyl-[1-[(2S)-2-amino-3-methyl-butanoyl]oxvethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (Ex. 1.45)

In analogy to the synthesis of Example 1.14, benzyl 6-[5-cyano-6-fluoro-8-[methyl-[1-[(2S)-2-(benzyloxycarbonylamino)-3-methyl-butanoyl]oxyethoxycarbonyl]amino]-4-[(3aS,6aS)-5-methyl-2,3,3 a,4, 6, 6a-hexahydropyrrolo [2,3-c]pyrrol-1-yl]-9H-pyrido [2,3-b]indol-3-yl]-1-methyl-4-oxo-1,8-naphthyridine-3-carboxylate (compound 1.45C) was used instead of benzyl 6-[8-[[2-(benzyloxycarbonylamino)acetyl]oxymethoxycarbonyl-methyl-amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate (compound 1.14B) to give 6-[5-cyano-6-fluoro-8-[methyl-[1-[(2S)-2-amino-3-methyl-butanoyl]oxyethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid (45.7% yield) as a yellow solid after prep-HPLC purification (NH₄HCO₃ as additive). MS (ESI): 754.4 ([M+H]⁺).

### Example 46

### Lyophilisation Solubility Assay (LYSA):

The solubility of compounds of present invention was assessed by LYSA assay. Samples were prepared in duplicate from 10 mM DMSO stock solutions (20µL) diluted in 30µL pure DMSO. After evaporation of DMSO with a centrifugal vacuum evaporator, the residue was dissolved in 0.05 M phosphate buffer (150µL, pH 6.5), which was stirred for one hour and shook for two hours. After one night, the solution was filtered using a microtiter filter plate. Then the filtrate and its 1/10 dilution were analyzed by HPLC-UV. In addition, a four-point calibration curve was prepared from the 10 mM stock solutions and used for the solubility determination of the compounds. The results were in µg/mL and summarized in **Table 1**. Particular compounds of the present invention were found to have much improved solubility (LYSA @ pH 6.5) compared to the parent molecules (1.03D, 1.26E and 1.34D).

**Table 1: Solubility data of Examples**

| **Example No. of parent molecule** | **Lysa (µg/mL)** | **Example No. of corresponding prodrugs** | **Lysa (µg/mL)** |
|---|---|---|---|
| **1.03D** | **194** | **1.01** | **>595** |
| | | **1.02** | **>895** |
| | | **1.03** | **>730** |
| | | **1.04** | **>950** |
| | | **1.07** | **>1060** |
| | | **1.08** | **480** |
| **1.26E** | **137** | **1.26** | **>783** |
| | | **1.27** | **>685** |
| | | **1.28** | **>945** |
| | | **1.43** | **595** |
| **1.34D** | **54** | **1.32** | **>875** |
| | | **1.33** | **680** |
| | | **1.34** | **>930** |
| | | **1.35** | **>915** |
| | | **1.36** | **>1000** |
| | | **1.37** | **315** |

### Example 47

### Plasma stability assay

The degradation of prodrug and formation of active drug of present invention were monitored by current plasma stability assay. The assay is conducted in human plasma (purchased from Bioreclamation, Lot No. BRH1380385) or mouse plasma (prepared in-house using CD-1 mouse purchased from Vital River). Plasma is spiked with the test prodrug (10 µM final concentration) dissolved in DMSO (or water) and incubated at 37 °C. Aliquots are removed at 0, 5, 15 and 30 minutes of incubation time, quenched with appropriate volume of internal standard solution (I.S., 0.2 µM of tolbutamide, prepared in ACN and water (1:1, v/v)), according to the mass spectrometer intensity of both analytes. Samples are centrifuged, the supernatant are removed and analyzed by LC-MS/MS.

The degradation of prodrug was illustrated by calculation of percent remaining of prodrug at 30 min compared to that at 0 min (**Table 2**).

**Table 2: Plasma stability data of Examples**

| **Example No.** | **Percent Remaining (%) H/M** |
|---|---|
| **1.02** | **88.2/70.2** |
| **1.04** | **95.9/79.1** |
| **1.08** | **22.3/1.3** |
| **1.13** | **1.9/0.6** |
| **1.15** | **50.5/4.7** |
| **1.18** | **67.2/72.0** |
| **1.21** | **88.7/56.0** |
| **1.39** | **98.4/0.1** |

### Example 48

### 50% Growth Inhibitory Concentration (IC₅₀) Determination Assay:

The in vitro antimicrobial activity of the compounds against *S*. *aureus* (ATCC29213), *K*. *pneumoniae* (ATCC10031), and A. *baumannii* (ATCC17978), was determined according to the following procedure:
The assay used a 10-points Iso-Sensitest broth medium to measure quantitatively the in vitro activity of the compounds against *S*. *aureus* ATCC29213, *K. pneumoniae* ATCC 10031, and *A*. *baumannii* ATCC17978.

Stock compounds in DMSO were serially two-fold diluted (range from 50 to 0.097 µM final concentration) in 384 wells microtiter plates and inoculated with 49 µL the bacterial suspension in Iso-Sensitest broth medium to have a final cell concentration of ~ 5×10⁵ CFU/mL in a final volume/well of 50 µL/well. Microtiter plates were incubated at 35 ± 2 °C.

Bacterial cell growth was determined with the measurement of optical density at λ=600nm each 20 minutes over a time course of 16 h.

Growth inhibition was calculated during the logarithmic growth of the bacterial cells with determination of the concentration inhibiting 50% (IC₅₀) of the growth.

The parent compounds of the present invention were tested for their concentration inhibiting 50% (IC₅₀). The data of IC₅₀ over *S*. *aureus* (ATCC29213), *K. pneumoniae* (ATCC10031), and A. *baumannii* (ATCC17978) are illustrated in Table 6. Particular compounds of the present invention were found to have IC₅₀ ≤ 1 µM.

**Table 3: IC₅₀ values of the parent compounds of this invention against S. aureus, K. pneumoniae and A. baumannii**

| **Example No.** | **IC50 (µM)** | | |
|---|---|---|---|
| | *S*. *aureus* ATCC29213 | *K. pneumoniae* ATCC10031 | *A. baumannii* ATCC17978 |
| **1.03D** | <0.098* | <0.098* | 0.045 |
| **1.26E** | <0.098* | <0.098* | 0.039 |
| **1.34D** | <0.098* | <0.098* | <0.098* |

## Claims

1. A compound of formula (I), 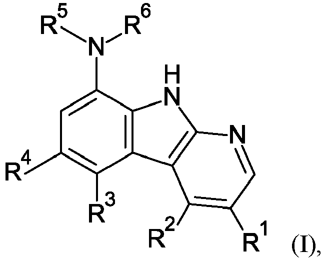 wherein
R¹ is 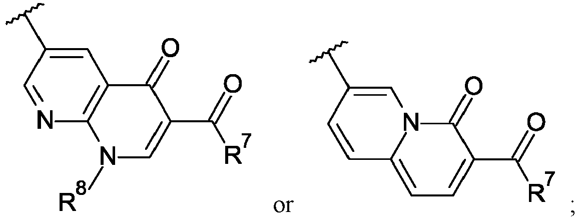 wherein R⁷ is selected from the group consisting of OH; (C₁₋₆alkyl-2-oxo-1,3-dioxol-4-yl)C₁₋₆alkoxy; aminoC₁₋₆alkylcarbonyloxyC₁₋₆alkoxy; C₁₋₆alkoxy; C₁₋₆alkoxycarbonyloxyC₁₋₆alkoxy; C₁₋₆alkylcarbonyloxyC₁₋₆alkoxy; C₁₋₆alkylsulfonylamino; carboxyC₁₋₆alkylaminocarbonylC₁₋₆alkylamino and phenylC₁₋₆alkoxy;
R⁸ is C₁₋₆alkyl;
R² is selected from 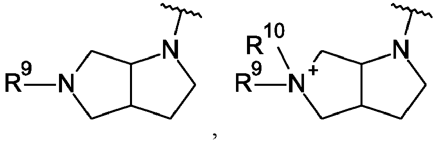 and -N(R⁹)₂; wherein R⁹ is C₁₋₆alkyl;
R¹⁰ is selected from the group consisting of C₁₋₆alkyl-2-oxo-1,3-dioxol-4-yl; C₁₋₆alkoxy(hydroxy)phosphoryloxyC₁₋₆alkyl and phosphonooxyC₁₋₆alkyl;
R³ is halogen or cyano;
R⁴ is halogen;
R⁵ is selected from the group consisting of H; ((aminoC₁₋₆alkylcarbonyl)aminoC₁₋₆alkylcarbonyl; ((aminoC₁₋₆alkylcarbonyl)aminoC₁₋₆alkylcarbonyl)aminoC₁₋₆alkylcarbonyl; (C₁₋₆alkoxy(hydroxy)phosphoryl)C₁₋₆alkoxycarbonyl; (C₁-₆alkoxy)₂phosphoryl; aminoC₁₋₆alkylcarbonyloxyC₁₋₆alkoxycarbonyl; C₁₋₆alkylcarbonyloxyC₁₋₆alkoxycarbonyl; carboxyC₂₋₆alkenylcarbonyloxyC₁₋₆alkoxycarbonyl; formyl; phenylC₁₋₆alkoxy(hydroxy)phosphoryl; phosphonooxyC₁₋₆alkoxycarbonyl and sulfo;
R⁶ is C₁₋₆alkyl;
with the proviso that when R⁵ is H and R⁷ is OH, R² is not 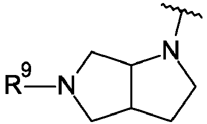 or -N(R⁹)₂;
or a pharmaceutically acceptable salt thereof.

2. A compound of formula (I) according to claim 1, wherein
R¹ is 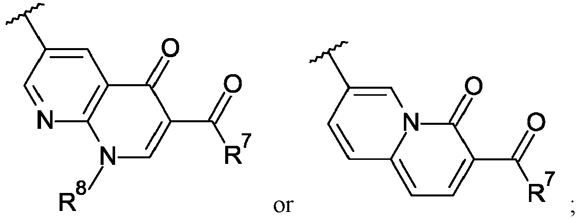 wherein R⁷ is selected from the group consisting of OH; (5-methyl-2-oxo-1,3-dioxol-4-yl)methoxy; acetoxyethoxy; amino(methyl)butanoyloxyethoxy; benzyloxy; carboxymethylaminocarbonylmethylamino; ethoxy; isopropoxycarbonyloxyethoxy and methylsulfonylamino;
R⁸ is methyl;
R² is selected from 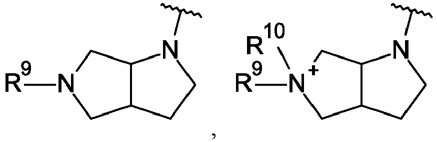 and -N(R⁹)₂; wherein R⁹ is methyl;
R¹⁰ is selected from the group consisting of 5-methyl-2-oxo-1,3-dioxol-4-ylmethyl; *tert*-butoxy(hydroxy)phosphoryloxymethyl and phosphonooxymethyl;
R³ is fluoro or cyano;
R⁴ is fluoro;
R⁵ is selected from the group consisting of H; ((aminoacetyl)aminoacetyl)aminoacetyl; (aminoacetyl)aminoacetyl; (tert-butoxy(hydroxy)phosphoryloxy)methoxycarbonyl; acetoxyethoxycarbonyl; aminoacetyloxymethoxycarbonyl; amino(methyl)butanoyloxyethoxycarbony; benzyloxy(hydroxy)phosphoryl; carboxypropenoyloxymethoxycarbonyl; diethoxyphosphoryl; formyl; phosphonooxymethoxycarbonyl and sulfo;
R⁶ is methyl;
with the proviso that when R⁵ is H and R⁷ is OH, R² is not 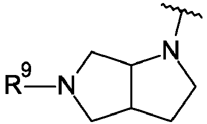 or -N(R⁹)₂;
or a pharmaceutically acceptable salt thereof.

3. A compound according to claim 1, wherein
R¹ is 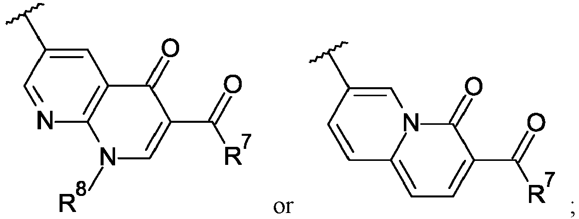 wherein R⁷ is selected from the group consisting of (C₁₋₆alkyl-2-oxo-1,3-dioxol-4-yl)C₁₋₆alkoxy; aminoC₁₋₆alkylcarbonyloxyC₁₋₆alkoxy; C₁₋₆alkoxy; C₁₋₆alkoxycarbonyloxyC₁₋₆alkoxy; C₁₋₆alkylcarbonylC₁₋₆alkoxy; C₁₋₆alkylsulfonylamino; carboxyC₁₋₆alkylaminocarbonylC₁₋₆alkylamino and phenylC₁₋₆alkoxy;
R⁸ is C₁₋₆alkyl;
R² is 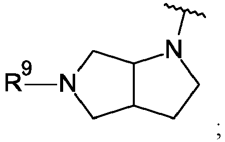 wherein R⁹ is C₁₋₆alkyl;
R³ is halogen or cyano;
R⁴ is halogen;
R⁵ is H;
R⁶ is C₁₋₆alkyl;
or a pharmaceutically acceptable salt thereof.

4. A compound according to claim 3, or a pharmaceutically acceptable salt thereof, wherein R⁷ is selected from the group consisting of (C₁₋₆alkyl-2-oxo-1,3-dioxol-4-yl)C₁₋₆alkoxy; aminoC₁₋₆alkylcarbonyloxyC₁₋₆alkoxy; C₁₋₆alkoxycarbonyloxyC₁₋₆alkoxy and C₁₋₆alkylcarbonyloxyC₁₋₆alkoxy.

5. A compound according to claim 1, wherein
R¹ is 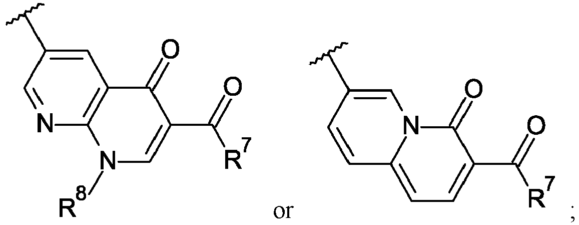 wherein R⁷ is OH;
R⁸ is C₁₋₆alkyl;
R² is 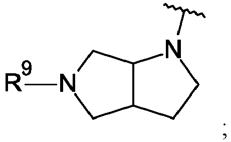 wherein R⁹ is C₁₋₆alkyl;
R³ is halogen or cyano;
R⁴ is halogen;
R⁵ is selected from the group consisting of ((aminoC₁₋₆alkylcarbonyl)aminoC₁₋₆alkylcarbonyl; ((aminoC₁₋₆alkylcarbonyl)aminoC₁₋₆alkylcarbonyl)aminoC₁₋₆alkylcarbonyl; (C₁₋₆alkoxy(hydroxy)phosphoryl)C₁₋₆alkoxycarbonyl; (C₁₋₆alkoxy)zphosphoryl; aminoC₁₋₆alkylcarbonyloxyC₁₋₆alkoxycarbonyl; C₁₋₆alkylcarbonyloxyC₁₋₆alkoxycarbonyl; carboxyC₂₋₆alkenylcarbonyloxyC₁₋₆alkoxycarbonyl; formyl; phenylC₁₋₆alkoxy(hydroxy)phosphoryl; phosphonooxyC₁₋₆alkoxycarbonyl and sulfo;
R⁶ is C₁₋₆alkyl;
or a pharmaceutically acceptable salt thereof.

6. A compound according to claim 5, or a pharmaceutically acceptable salt thereof, wherein R⁵ is selected from the group consisting of C₁₋₆alkylcarbonyloxyC₁₋₆alkoxycarbonyl; carboxyC₂₋₆alkenylcarbonyloxyC₁₋₆alkoxycarbonyl and phosphonooxyC₁₋₆alkoxycarbonyl.

7. A compound of formula (II), 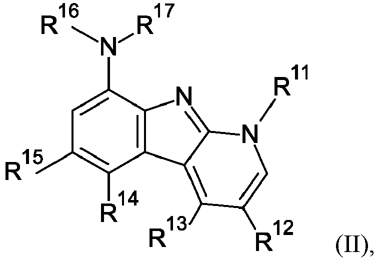 wherein
R¹¹ is selected from the group consisting of C₁₋₆alkyl-2-oxo-1,3-dioxol-4-yl; C₁₋₆alkoxy(hydroxy)phosphoryloxyC₁₋₆alkyl and phosphonooxyC₁₋₆alkyl;
R¹² is 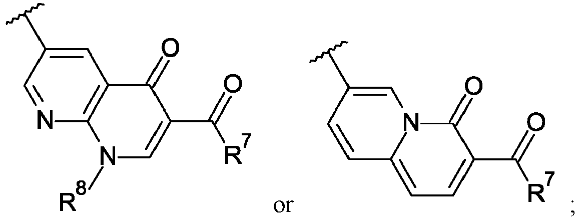 wherein R⁷ is OH;
R⁸ is C₁₋₆alkyl;
R¹³ is 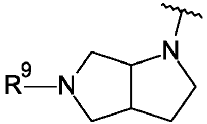 or -N(R⁹)₂; wherein R⁹ is C₁₋₆alkyl;
R¹⁴ is halogen or cyano;
R¹⁵ is halogen;
R¹⁶ is H;
R¹⁷ is C₁₋₆alkyl;
or a pharmaceutically acceptable salt thereof.

8. A compound according to claim 7, wherein
R¹¹ is selected from the group consisting of 5-methyl-2-oxo-1,3-dioxol-4-ylmethyl; *tert-*butoxy(hydroxy)phosphoryloxymethyl and phosphonooxymethyl;
R¹² is 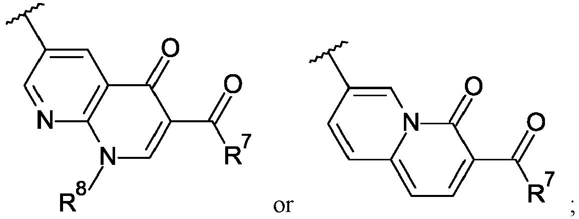 wherein R⁷ is OH; R⁸ is methyl;
R¹³ is 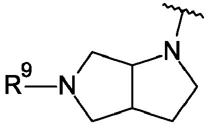 or -N(R⁹)₂; wherein R⁹ is methyl;
R¹⁴ is fluoro or cyano;
R¹⁵ is fluoro;
R¹⁶ is H;
R¹⁷ is methyl;
or a pharmaceutically acceptable salt thereof.

9. A compound according to claims 1 or 7 selected from
6-[5-Cyano-6-fluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3 -carboxylic acid;
6-[5-Cyano-6-fluoro-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5-Cyano-6-fluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5-Cyano-6-fluoro-8-(methylamino)-1-(phosphonooxymethyl)-4-[(3 aS, 6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5-Cyano-6-fluoro-8-(methylamino)-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5-Cyano-6-fluoro-8-(methylamino)-4-[(3aS,6aS)-5-methyl-5-[(5-methyl-2-oxo-1,3-dioxol-4-yl)methyl] -2,3,3 a,4, 6, 6a-hexahydropyrrolo [2,3 -c]pyrrol-5 -ium-1-yl]-9H-pyrido[2,3-b]indol-3-yl]-1-methyl-4-oxo-1,8-naphthyridine-3-carboxylic acid;
6-[5-Cyano-6-fluoro-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid;
2-[(2S)-2-Amino-3-methyl-butanoyl]oxyethyl 6-[5-cyano-6-fluoro-8-(methylamino)-4-[(3 aS, 6aS)-5-methyl-2,3,3 a,4, 6, 6a-hexahydropyrrolo [2,3 -c]pyrrol-1-yl] -9H-pyrido [2,3-b]indol-3-yl]-1-methyl-4-oxo-1,8-naphthyridine-3-carboxylate;
2-[[2-[[6-[5-Cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3 -carbonyl] amino] acetyl] amino] acetic acid;
6-[5-Cyano-6-fluoro-8-(methylamino)-4-[(3 aS, 6aS)-5-methyl-2,3,3 a,4, 6,6a-hexahydropyrrolo[2,3-c]pyrrol-1-yl]-9H-pyrido[2,3-b]indol-3-yl]-1-methyl-N-methylsulfonyl-4-oxo-1, 8-naphthyridine-3-carboxamide;
1-Acetoxyethyl 6-[5-cyano-6-fluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate;
1-Isopropoxycarbonyloxyethyl 6-[6-fluoro-5-methyl-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylate;
(5-Methyl-2-oxo-1,3-dioxol-4-yl)methyl 6-[5-cyano-6-fluoro-8-(methylamino)-4-[(3 aS, 6aS)-5-methyl-2,3,3 a,4, 6, 6a-hexahydropyrrolo [2,3 -c]pyrrol-1-yl] -9H-pyrido [2,3-b]indol-3-yl]-1-methyl-4-oxo-1,8-naphthyridine-3-carboxylate;
6-[8-[(2-Aminoacetyl)oxymethoxycarbonyl-methyl-amino]-5-cyano-6-fluoro-4-[(3 aS, 6aS)-5-methyl-2,3,3 a,4, 6, 6a-hexahydropyrrolo [2,3 -c]pyrrol-1-yl] -9H-pyrido [2,3-b]indol-3-yl]-1-methyl-4-oxo-1,8-naphthyridine-3-carboxylic acid;
6-[8-[1-Acetoxyethoxycarbonyl(methyl)amino]-5-cyano-6-fluoro-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3 -carboxylic acid;
6-[8-[Diethoxyphosphoryl(methyl)amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[8-[[Benzyloxy(hydroxy)phosphoryl]-methyl-amino]-5,6-difluoro-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3 -carboxylic acid;
6-[5,6-Difluoro-8-[methyl(phosphonooxymethoxycarbonyl)amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3 -carboxylic acid;
6-[8-[[tert-Butoxy(hydroxy)phosphoryl]oxymethoxycarbonyl-methyl-amino]-5,6-difluoro-4- [(3 aR, 6aR)-5-methyl-2,3,3 a,4, 6, 6a-hexahydropyrrolo [2,3 -c]pyrrol-1-yl]-9H-pyrido[2,3-b]indol-3-yl]-1-methyl-4-oxo-1,8-naphthyridine-3-carboxylic acid;
(E)-4-[[[3-(6-Benzyloxycarbonyl-8-methyl-5-oxo-1,8-naphthyridin-3-yl)-5,6-difluoro-4-[(3 aR, 6aR)-5-methyl-2,3,3 a,4, 6, 6a-hexahydropyrrolo [2,3 -c]pyrrol-1-yl] -9H-pyrido [2,3-b]indol-8-yl]-methyl-carbamoyl]oxymethoxy]-4-oxo-but-2-enoic acid;
6-[5,6-Difluoro-8-[methyl-[[(E)-3-carboxyprop-2-enoyl]oxymethoxycarbonyl]amino]-4-[(3 aR, 6aR)-5-methyl-2,3,3 a,4, 6, 6a-hexahydropyrrolo [2,3 -c]pyrrol-1-yl] -9H-pyrido [2,3-b]indol-3-yl]-1-methyl-4-oxo-1,8-naphthyridine-3-carboxylic acid;
6-[1-[[*tert*-Butoxy(hydroxy)phosphoryl]oxymethyl]-5,6-difluoro-8-(methylamino)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5,6-Difluoro-8-(methylamino)-1-(phosphonooxymethyl)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridine-3-carboxylic acid;
6-[5,6-Difluoro-8-(methylamino)-4-[(3aR,6aR)-5-[[*tert-*butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3-carboxylic acid;
6-[5,6-Difluoro-8-(methylamino)-4-[(3aR,6aR)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridine-3 -carboxylic acid;
6-[5,6-Difluoro-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5,6-Difluoro-8-[methyl(sulfo)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[5,6-Difluoro-8-[methyl-[[(E)-3-carboxyprop-2-enoyl]oxymethoxycarbonyl]amino]-4-[(3 aS, 6aS)-5-methyl-2,3,3 a,4, 6, 6a-hexahydropyrrolo [2,3 -c]pyrrol-1-yl] -9H-pyrido [2,3-b]indol-3-yl]-1-methyl-4-oxo-1,8-naphthyridine-3-carboxylic acid;
6-[5,6-Difluoro-8-[methyl(sulfo)amino]-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
6-[8-[[2-[(2-Aminoacetyl)amino]acetyl]-methyl-amino]-5,6-difluoro-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
Ethyl 6-[5,6-difluoro-8-(methylamino)-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3 -carboxylate;
7-[5,6-Difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid;
7-[5,6-Difluoro-8-[methyl-[[(E)-3-carboxyprop-2-enoyl]oxymethoxycarbonyl]amino]-4-[(3 aS, 6aS)-5-methyl-2,3,3 a,4, 6, 6a-hexahydropyrrolo [2,3 -c]pyrrol-1-yl] -9H-pyrido [2,3-b]indol-3-yl]-4-oxo-quinolizine-3-carboxylic acid;
7-[8-[[2-[[2-[(2-Aminoacetyl)amino]acetyl]amino]acetyl]-methyl-amino]-5,6-difluoro-4-[(3 aS, 6aS)-5-methyl-2,3,3 a,4, 6, 6a-hexahydropyrrolo [2,3 -c]pyrrol-1-yl] -9H-pyrido [2,3-b]indol-3-yl]-4-oxo-quinolizine-3-carboxylicacid;
7-[8-[[2-[(2-Aminoacetyl)amino]acetyl]-methyl-amino]-5,6-difluoro-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylic acid;
7-[5,6-Difluoro-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-4-oxo-quinolizine-3-carboxylic acid;
2-[(2S)-2-Amino-3-methyl-butanoyl]oxyethyl 7-[5,6-difluoro-8-(methylamino)-4-[(3 aS, 6aS)-5-methyl-2,3,3 a,4, 6, 6a-hexahydropyrrolo [2,3 -c]pyrrol-1-yl] -9H-pyrido [2,3-b]indol-3-yl]-4-oxo-quinolizine-3-carboxylate;
2-[[2-[[7-[5,6-Difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carbonyl] amino] acetyl] amino] acetic acid;
1-Isopropoxycarbonyloxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3 -carboxylate;
1-Acetoxyethyl 7-[5,6-difluoro-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxo-quinolizine-3-carboxylate;
7-[5, 6-Difluoro-8-(methylamino)-4-[(3 aS, 6aS)-5-methyl-2,3,3 a,4, 6,6a-hexahydropyrrolo[2,3-c]pyrrol-1-yl]-9H-pyrido[2,3-b]indol-3-yl]-N-methylsulfonyl-4-oxo-quinolizine-3-carboxamide;
6-[5,6-Difluoro-8-(methylamino)-4-[*cis*-5-methyl-2,3,3a,4,6,6a-hexahydropyrrolo[2,3-c]pyrrol-1-yl]-9H-pyrido [2,3-b]indol-3-yl]-1-methyl-N-methylsulfonyl-4-oxo-1, 8-naphthyridine-3-carboxamide;
6-[5,6-Difluoro-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3 -carboxylic acid;
6-[4-(Dimethylamino)-5,6-difluoro-8-(methylamino)-1-(phosphonooxymethyl)pyrido[2,3-b]indol-3-yl]-1-methyl-4-oxo-1,8-naphthyridine-3-carboxylic acid; and
6-[5-Cyano-6-fluoro-8-[methyl-[1-[(2S)-2-amino-3-methyl-butanoyl]oxyethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridine-3-carboxylic acid;
or a pharmaceutically acceptable salt.

10. A process for the preparation of a compound according to any one of claims 1 to 9 comprising any of the following reactions:
a) the coupling reaction of compound of formula (Ij), 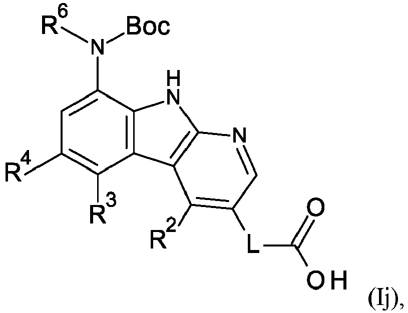 with an alcohol or amine in the presence of a coupling reagent followed by the deprotection of Boc group with an acid;
b) reaction of compound of formula (Ik), 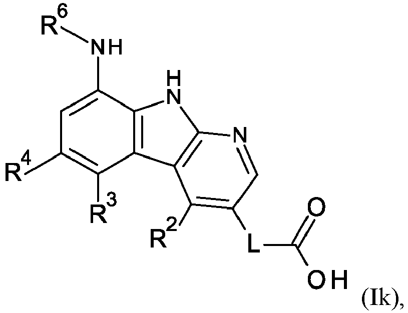 with acylating or sulfonylating reagents;
c) hydrolysis or hydrogentation of compound of formula (In), 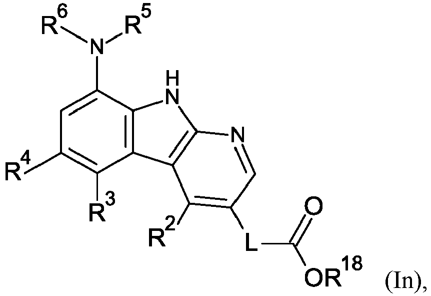 in the presence of a base or a catalyst;
d) hydrolysis or hydrogentation of compound of formula (Ip), 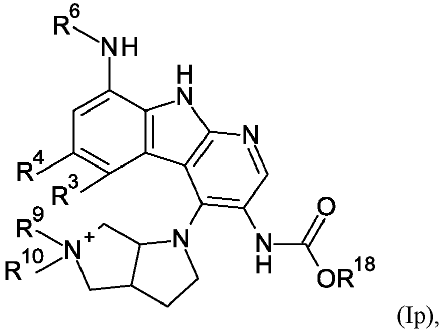 in the presence of a base or a catalyst;
e) hydrolysis or hydrogentation of compound of formula (Iq), 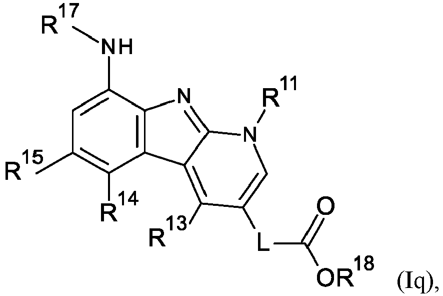
in the presence of a base or a catalyst;
wherein R¹ to R¹⁷ are defined above; L is unsubstituted or substituted 4-oxo-1,8-naphthyridinyl, or 4-oxoquinolizinyl; R¹⁸ is C₁₋₆alkyl or Bn;
in step a), the coupling reagent is HATU; the acid is TFA;
in step b), the acylating reagent is formic acid; the sulfonylating reagent is sulfur trioxide;
in step c), d) and e), the base is NaOH or LiOH; the catalyst is Pd/C.

11. A compound according to any one of claims 1 to 9 for use as therapeutically active substance.

12. A pharmaceutical composition comprising a compound in accordance with any one of claims 1 to 9 and a therapeutically inert carrier.

13. A compound according to any one of claims 1 to 9 for use in the treatment or prophylaxis of bacterial infection.

14. The compound for use according to claim 13, wherein the bacterial infection is caused by *Streptococcus pneumoniae, Streptococcus pyogenes, Enterococcus faecalis, Enterococcus faecium, Enterobacter spp.* species, *Proteus spp.* species, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides spp.* species *Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii, E. coli* or *Mycobacterium ulcerans.*

15. The compound for use according to claim 14, wherein the bacterial infection is caused by *Acinetobacter baumannii, Klebsiella pneumoniae, Pseudomonas aeruginosa, Staphylococcus aureus* or *E. coli.*

## Patentansprüche

1. Verbindung der Formel (I) 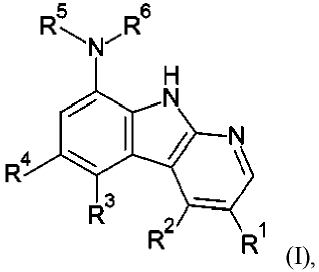 wobei
R¹ 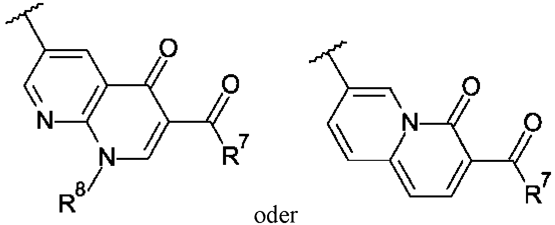 ist,
wobei R⁷ aus der Gruppe ausgewählt ist, die aus OH; (C₁₋₆-Alkyl-2-oxo-1,3-dioxol-4-yl)-C₁₋₆-alkoxy; Amino-C₁₋₆-alkylcarbonyloxy-C₁₋₆-alkoxy; C₁₋₆-Alkoxy; C₁₋₆-Alkoxycarbonyloxy-C₁₋₆-alkoxy; C₁₋₆-Alkylcarbonyloxy-C₁₋₆-alkoxy; C₁₋₆-Alkylsulfonylamino; Carboxy-C₁₋₆-alkylaminocarbonyl-C₁₋₆-alkylamino und Phenyl-C₁₋₆-alkoxy besteht;
R⁸ C₁₋₆-Alkyl ist;
R² aus 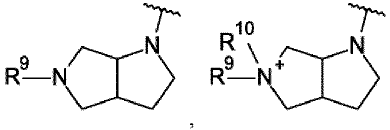 und -N(R⁹)₂ ausgewählt ist;
wobei R⁹ C₁₋₆-Alkyl ist;
R¹⁰ aus der Gruppe ausgewählt ist, die aus C₁₋₆-Alkyl-2-oxo-1,3-dioxol-4-yl; C₁₋₆-Alkoxy(hydroxy)phosphoryloxy-C₁₋₆-alkyl und Phosphonooxy-C₁₋₆-alkyl besteht;
R³ Halogen oder Cyano ist;
R⁴ Halogen ist;
R⁵ aus der Gruppe ausgewählt ist, die aus H; ((Amino-C₁₋₆-alkylcarbonyl)amino-C₁₋₆-alkylcarbonyl; ((Amino-C₁₋₆-alkylcarbonyl)amino-C₁₋₆-alkylcarbonyl)amino-C₁₋₆-alkylcarbonyl; (C₁₋₆-Alkoxy(hydroxy)phosphoryl)-C₁₋₆-alkoxycarbonyl; (C₁₋₆-Alkoxy)₂-phosphoryl; Amino-C₁₋₆-alkylcarbonyloxy-C₁₋₆-alkoxycarbonyl; C₁₋₆-Alkylcarbonyloxy-C₁₋₆-alkoxycarbonyl; Carboxy-C₂₋₆-alkenylcarbonyloxy-C₁₋₆-alkoxycarbonyl; Formyl; Phenyl-C₁₋₆-alkoxy(hydroxy)phosphoryl; Phosphonooxy-C₁₋₆-alkoxycarbonyl und Sulfo besteht;
R⁶ C₁₋₆-Alkyl ist;
mit der Maßgabe, dass, wenn R⁵ H ist und R⁷ OH ist, R² nicht 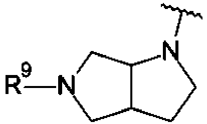 oder -N(R⁹)₂ ist;
oder ein pharmazeutisch unbedenkliches Salz davon.

2. Verbindung der Formel (I) nach Anspruch 1, wobei
R¹ 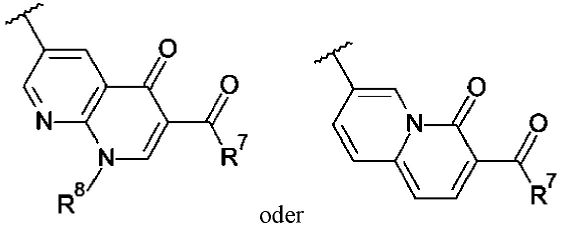 ist;
wobei R⁷ aus der Gruppe ausgewählt ist, die aus OH; (5-Methyl-2-oxo-1,3-dioxol-4-yl)methoxy; Acetoxyethoxy; Amino(methyl)butanoyloxyethoxy; Benzyloxy; Carboxymethylaminocarbonylmethylamino; Ethoxy; Isopropoxycarbonyloxyethoxy und Methylsulfonylamino besteht;
R⁸ Methyl ist;
R² aus 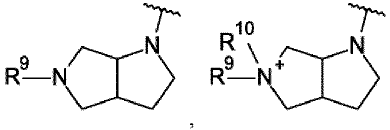 und -N(R⁹)₂ ausgewählt ist;
wobei R⁹ Methyl ist;
R¹⁰ aus der Gruppe ausgewählt ist, die aus 5-Methyl-2-oxo-1,3-dioxol-4-ylmethyl; *tert-*Butoxy(hydroxy)phosphoryloxymethyl und Phosphonooxymethyl besteht;
R³ Fluor oder Cyano ist;
R⁴ Fluor ist;
R⁵ aus der Gruppe ausgewählt ist, die aus H; ((Aminoacetyl)aminoacetyl)aminoacetyl; (Aminoacetyl)aminoacetyl; (tert-Butoxy(hydroxy)phosphoryloxy)methoxycarbonyl; Acetoxyethoxycarbonyl; Aminoacetyloxymethoxycarbonyl; Amino(methyl)butanoyloxyethoxycarbony; Benzyloxy(hydroxy)phosphoryl; Carboxypropenoyloxymethoxycarbonyl; Diethoxyphosphoryl; Formyl; Phosphonooxymethoxycarbonyl und Sulfo besteht;
R⁶ Methyl ist;
mit der Maßgabe, dass, wenn R⁵ H ist und R⁷ OH ist, R² nicht 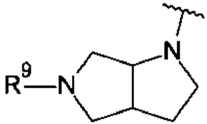 oder -N(R⁹)₂ ist;
oder ein pharmazeutisch unbedenkliches Salz davon.

3. Verbindung nach Anspruch 1, wobei
R¹ 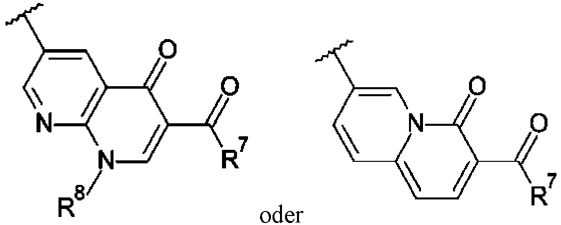 ist;
wobei R⁷ aus der Gruppe ausgewählt ist, die aus (C₁₋₆-Alkyl-2-oxo-1,3-dioxol-4-yl)-C₁₋₆-alkoxy; Amino-C₁₋₆-alkylcarbonyloxy-C₁₋₆-alkoxy; C₁₋₆-Alkoxy; C₁₋₆-Alkoxycarbonyloxy-C₁₋₆-alkoxy; C₁₋₆-Alkylcarbonyl-C₁₋₆-alkoxy; C₁₋₆-Alkylsulfonylamino; Carboxy-C₁₋₆-alkylaminocarbonyl-C₁₋₆-alkylamino und Phenyl-C₁₋₆-alkoxy besteht;
R⁸ C₁₋₆-Alkyl ist;
R² 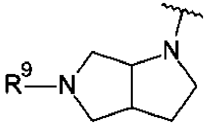 ist; wobei R⁹ C₁₋₆-Alkyl ist;
R³ Halogen oder Cyano ist;
R⁴ Halogen ist;
R⁵ H ist;
R⁶ C₁₋₆-Alkyl ist;
oder ein pharmazeutisch unbedenkliches Salz davon.

4. Verbindung nach Anspruch 3 oder ein pharmazeutisch unbedenkliches Salz davon, wobei R⁷ aus der Gruppe ausgewählt ist, die aus (C₁₋₆-Alkyl-2-oxo-1,3-dioxol-4-yl)-C₁₋₆-alkoxy; Amino-C₁₋₆-alkylcarbonyloxy-C₁₋₆-alkoxy; C₁₋₆-Alkoxycarbonyloxy-C₁₋₆-alkoxy und C₁₋₆-Alkylcarbonyloxy-C₁₋₆-alkoxy besteht.

5. Verbindung nach Anspruch 1, wobei
R¹ 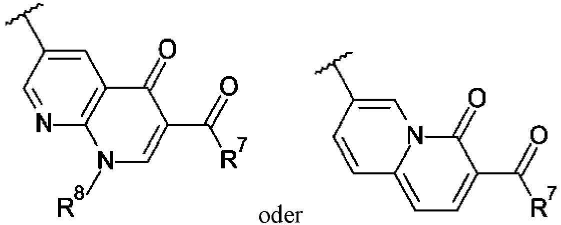 ist;
wobei R⁷ OH ist;
R⁸ C₁₋₆-Alkyl ist;
R² 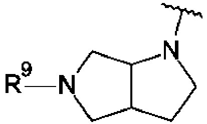 ist; wobei R⁹ C₁₋₆-Alkyl ist;
R³ Halogen oder Cyano ist;
R⁴ Halogen ist;
R⁵ aus der Gruppe ausgewählt ist, die aus ((Amino-C₁₋₆-alkylcarbonyl)amino-C₁₋₆-alkylcarbonyl; ((Amino-C₁₋₆-alkylcarbonyl)amino-C₁₋₆-alkylcarbonyl)amino-C₁₋₆-alkylcarbonyl; (C₁₋₆-Alkoxy(hydroxy)phosphoryl)-C₁₋₆-alkoxycarbonyl; (C₁₋₆-Alkoxy)₂-phosphoryl; Amino-C₁₋₆-alkylcarbonyloxy-C₁₋₆-alkoxycarbonyl; C₁₋₆-Alkylcarbonyloxy-C₁₋₆-alkoxycarbonyl; Carboxy-C₂₋₆-alkenylcarbonyloxy-C₁₋₆-alkoxycarbonyl; Formyl; Phenyl-C₁₋₆-alkoxy(hydroxy)phosphoryl; Phosphonooxy-C₁₋₆-alkoxycarbonyl und Sulfo besteht;
R⁶ C₁₋₆-Alkyl ist;
oder ein pharmazeutisch unbedenkliches Salz davon.

6. Verbindung nach Anspruch 5 oder ein pharmazeutisch unbedenkliches Salz davon, wobei R⁵ aus der Gruppe ausgewählt ist, die aus C₁₋₆-Alkylcarbonyloxy-C₁₋₆-alkoxycarbonyl; Carboxy-C₂₋₆-alkenylcarbonyloxy-C₁₋₆-alkoxycarbonyl und Phosphonooxy-C₁₋₆-alkoxycarbonyl besteht.

7. Verbindung der Formel (II) 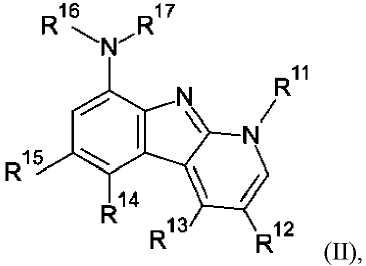 wobei
R¹¹ aus der Gruppe ausgewählt ist, die aus C₁₋₆-Alkyl-2-oxo-1,3-dioxol-4-yl; C₁₋₆-Alkoxy(hydroxy)phosphoryloxy-C₁₋₆-alkyl und Phosphonooxy-C₁₋₆-alkyl besteht;
R¹² 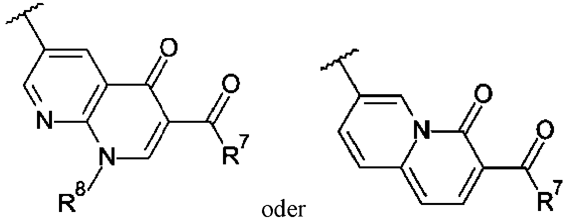 ist;
wobei R⁷ OH ist;
R⁸ C₁₋₆-Alkyl ist;
R¹³ 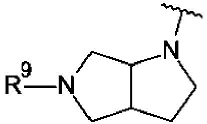 oder -N(R⁹)₂ ist; wobei R⁹ C₁₋₆-Alkyl ist;
R¹⁴ Halogen oder Cyano ist;
R¹⁵ Halogen ist;
R¹⁶ H ist;
R¹⁷ C₁₋₆-Alkyl ist;
oder ein pharmazeutisch unbedenkliches Salz davon.

8. Verbindung nach Anspruch 7, wobei
R¹¹ aus der Gruppe ausgewählt ist, die aus 5-Methyl-2-oxo-1,3-dioxol-4-ylmethyl; *tert-*Butoxy(hydroxy)phosphoryloxymethyl und Phosphonooxymethyl besteht;
R¹² 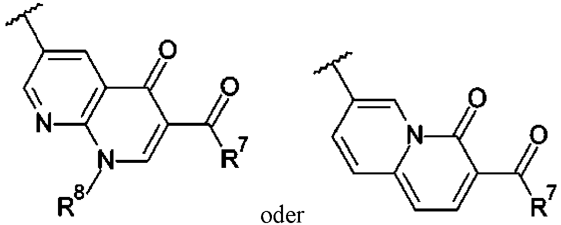 ist;
wobei R⁷ OH ist; R⁸ Methyl ist;
R¹³ 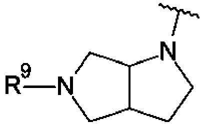 oder -N(R⁹)₂ ist; wobei R⁹ Methyl ist;
R¹⁴ Fluor oder Cyano ist;
R¹⁵ Fluor ist;
R¹⁶ H ist;
R¹⁷ Methyl ist;
oder ein pharmazeutisch unbedenkliches Salz davon.

9. Verbindung nach den Ansprüchen 1 oder 7, ausgewählt aus:
6-[5-Cyano-6-fluor-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[5-Cyano-6-fluor-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[5-Cyano-6-fluor-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[5-Cyano-6-fluor-8-(methylamino)-1-(phosphonooxymethyl)-4-[(3 aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridin-3-carbonsäure;
6-[5-Cyano-6-fluor-8-(methylamino)-1-[(5-methyl-2-oxo-1,3-dioxol-4-yl)methyl]-4-[(3aS,6aS)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridin-3 -carbonsäure;
6-[5-Cyano-6-fluor-8-(methylamino)-4-[(3aS,6aS)-5-methyl-5-[(5-methyl-2-oxo-1,3-dioxol-4-yl)methyl]-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridin-3-carbonsäure;
6-[5-Cyano-6-fluor-8-(methylamino)-4-[(3aS,6aS)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1, 8-naphthyri din-3 -carbonsäure;
2-[(2S)-2-Amino-3-methylbutanoyl]oxyethyl-6-[5-cyano-6-fluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carboxylat;
2-[[2-[[6-[5-Cyano-6-fluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonyl]amino]acetyl]amino]essigsäure;
6-[5-Cyano-6-fluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-N-methylsulfonyl-4-oxo-1,8-naphthyridin-3-carboxamid;
1-Acetoxyethyl-6-[5-cyano-6-fluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3 -carboxylat;
1-Isopropoxycarbonyloxyethyl-6-[6-fluor-5-methyl-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carboxylat;
(5-Methyl-2-oxo-1,3-dioxol-4-yl)methyl-6-[5-cyano-6-fluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1, 8-naphthyri din-3 -carboxylat;
6-[8-[(2-Aminoacetyl)oxymethoxycarbonylmethylamino]-5-cyano-6-fluor-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1, 8-naphthyri din-3 -carbonsäure;
6-[8-[1-Acetoxyethoxycarbonyl(methyl)amino]-5-cyano-6-fluor-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[8-[Diethoxyphosphoryl(methyl)amino]-5,6-difluor-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3 -carbonsäure;
6-[8-[[Benzyloxy(hydroxy)phosphoryl]-methylamino]-5,6-difluor-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[5,6-Difluor-8-[methyl(phosphonooxymethoxycarbonyl)amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[8-[[*tert*-Butoxy(hydroxy)phosphoryl]oxymethoxycarbonylmethylamino]-5,6-difluor-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
(E)-4-[[[3-(6-Benzyloxycarbonyl-8-methyl-5-oxo-1,8-naphthyridin-3-yl)-5,6-difluor-4-[(3aR,6aR)-5-methyl-2,3,3a,4,6,6a-hexahydropyrrolo[2,3-c]pyrrol-1-yl]-9H-pyrido[2,3-b]indol-8-yl]-methylcarbamoyl]oxymethoxy]-4-oxo-but-2-ensäure;
6-[5,6-Difluor-8-[methyl-[[(E)-3-carboxyprop-2-enoyl]oxymethoxycarbonyl]amino]-4-[(3aR,6aR)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[1-[[*tert*-Butoxy(hydroxy)phosphoryl]oxymethyl]-5,6-difluor-8-(methylamino)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridin-3 -carbonsäure;
6-[5,6-Difluor-8-(methylamino)-1-(phosphonooxymethyl)-4-[(3aR,6aR)-5-methyl-2,3,3a,4,6,6a-hexahydropyrrolo[2,3-c]pyrrol-1-yl]pyrido[2,3-b]indol-3-yl]-1-methyl-4-oxo-1,8-naphthyridin-3-carbonsäure;
6-[5,6-Difluor-8-(methylamino)-4-[(3aR,6aR)-5-[[tert-butoxy(hydroxy)phosphoryl]oxymethyl]-5-methyl-2,3,3a,4,6,6a-hexahydropyrrolo[2,3-c]pyrrol-5-ium-1-yl]-9H-pyrido[2,3-b]indol-3-yl]-1-methyl-4-oxo-1,8-naphthyridin-3-carbonsäure;
6-[5,6-Difluor-8-(methylamino)-4-[(3aR,6aR)-5-methyl-5-(phosphonooxymethyl)-2,3,3a,4,6,6a-hexahydropyrrolo[2,3-c]pyrrol-5-ium-1-yl]-9H-pyrido[2,3-b]indol-3-yl]-1-methyl-4-oxo-1, 8-naphthyri din-3 -carbonsäure;
6-[5,6-Difluor-8-[formyl(methyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3 -carbonsäure;
6-[5,6-Difluor-8-[methyl(sulfo)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3 -carbonsäure;
6-[5,6-Difluor-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[5,6-Difluor-8-[methyl(sulfo)amino]-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[8-[[2-[(2-Aminoacetyl)amino]acetyl]-methylamino]-5,6-difluor-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
Ethyl-6-[5,6-difluor-8-(methylamino)-4-[*cis*-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carboxylat;
7-[5,6-Difluor-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxochinolizin-3 -carbonsäure;
7-[5,6-Difluor-8-[methyl-[[(E)-3-carboxyprop-2-enoyl]oxymethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3 -yl] -4-oxochinolizin-3 -carbonsäure;
7-[8-[[2-[[2-[(2-Aminoacetyl)amino]acetyl]amino]acetyl]-methylamino]-5,6-difluor-4-[(3aS,6aS)-5-methyl-2,3,3a,4,6,6a-hexahydropyrrolo[2,3-c]pyrrol-1-yl]-9H-pyrido[2,3-b]indol-3 -yl] -4-oxochinolizin-3 -carbonsäure;
7-[8-[[2-[(2-Aminoacetyl)amino]acetyl]-methylamino]-5,6-difluor-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxochinolizin-3 -carbonsäure;
7-[5,6-Difluor-8-(methylamino)-1-(phosphonooxymethyl)-4-[(3aS,6aS)-5-methyl-2,3,3a,4,6,6a-hexahydropyrrolo[2,3-c]pyrrol-1-yl]pyrido[2,3-b]indol-3-yl]-4-oxochinolizin-3-carbonsäure;
2-[(2S)-2-Amino-3-methylbutanoyl]oxyethyl-7-[5,6-difluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxochinolizin-3 -carboxyl at;
2-[[2-[[7-[5,6-Difluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxochinolizin-3-carbonyl]amino]acetyl]amino]essigsäure;
1-Isopropoxycarbonyloxyethyl-7-[5,6-difluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxochinolizin-3 -carboxyl at;
1-Acetoxyethyl-7-[5,6-difluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-4-oxochinolizin-3-carboxylat;
7-[5,6-Difluor-8-(methylamino)-4-[(3aS,6aS)-5-methyl-2,3,3a,4,6,6a-hexahydropyrrolo[2,3-c]pyrrol-1-yl]-9H-pyrido[2,3-b]indol-3-yl]-N-methylsulfonyl-4-oxochinolizin-3-carboxamid;
6-[5,6-Difluor-8-(methylamino)-4-[*cis*-5-methyl-2,3,3a,4,6,6a-hexahydropyrrolo[2,3-c]pyrrol-1-yl]-9H-pyrido[2,3-b]indol-3-yl]-1-methyl-N-methylsulfonyl-4-oxo-1,8-naphthyridin-3 -carboxamid;
6-[5,6-Difluor-8-[methyl(phosphonooxymethoxycarbonyl)amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3-carbonsäure;
6-[4-(Dimethylamino)-5,6-difluor-8-(methylamino)-1-(phosphonooxymethyl)pyrido[2,3-b]indol-3-yl]-1-methyl-4-oxo-1,8-naphthyridin-3-carbonsäure; und
6-[5-Cyano-6-fluor-8-[methyl-[1-[(2S)-2-amino-3-methylbutanoyl]oxyethoxycarbonyl]amino]-4-[(3aS,6aS)-5-methyl-2,3,3a,4,6,6a-hexahydropyrrolo[2,3-c]pyrrol-1-yl]-9H-pyrido[2,3-b]indol-3-yl]-1-methyl-4-oxo-1,8-naphthyridin-3 -carbonsäure;
oder ein pharmazeutisch unbedenkliches Salz.

10. Verfahren zur Herstellung einer Verbindung nach einem der Ansprüche 1 bis 9, umfassend eine der folgenden Reaktionen:
a) die Kopplungsreaktion einer Verbindung der Formel (Ij) 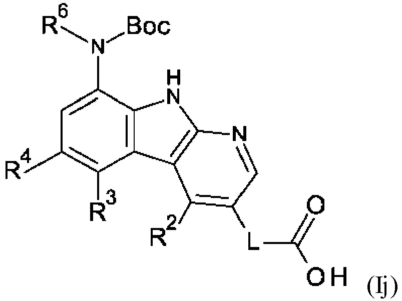 mit einem Alkohol oder Amin in der Gegenwart eines Kopplungsreagens, gefolgt von der Entschützung der Boc-Gruppe mit einer Säure;
b) Reaktion einer Verbindung der Formel (Ik) 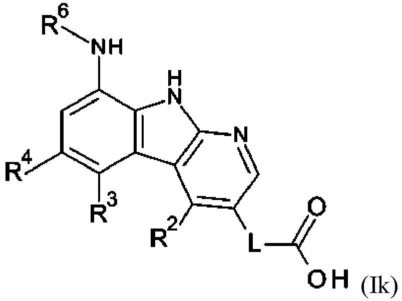 mit Acylierungs- oder Sulfonylierungsreagenzien;
c) Hydrolyse oder Hydrierung einer Verbindung der Formel (In) 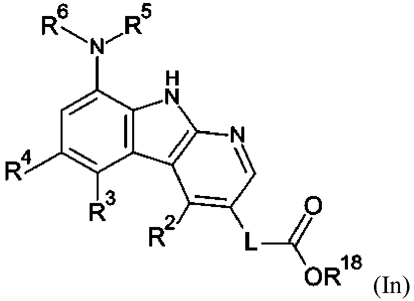 in der Gegenwart einer Base oder eines Katalysators;
d) Hydrolyse oder Hydrierung einer Verbindung der Formel (Ip) 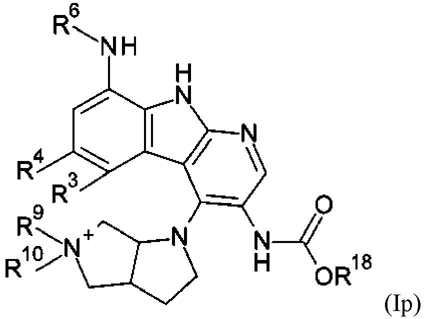 in der Gegenwart einer Base oder eines Katalysators;
e) Hydrolyse oder Hydrierung einer Verbindung der Formel (Iq) 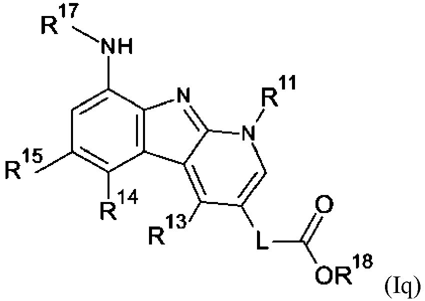
in der Gegenwart einer Base oder eines Katalysators;
wobei R¹ bis R¹⁷ oben definiert sind; L unsubstituiertes oder substituiertes 4-Oxo-1,8-naphthyridinyl oder 4-Oxochinolizinyl ist; R¹⁸ C₁₋₆-Alkyl oder Bn ist;
in Schritt a) das Kopplungsreagens HATU ist; die Säure TFA ist;
in Schritt b) das Acylierungsreagens Ameisensäure ist; das Sulfonylierungsreagens Schwefeltrioxid ist;
in Schritt c), d) und e) die Base NaOH oder LiOH ist; der Katalysator Pd/C ist.

11. Verbindung nach einem der Ansprüche 1 bis 9 zur Verwendung als therapeutisch wirksame Substanz.

12. Pharmazeutische Zusammensetzung, umfassend eine Verbindung nach einem der Ansprüche 1 bis 9 und einen therapeutisch inerten Träger.

13. Verbindung nach einem der Ansprüche 1 bis 9 zur Verwendung bei der Behandlung oder Prophylaxe einer bakteriellen Infektion.

14. Verbindung zur Verwendung nach Anspruch 13, wobei die bakterielle Infektion von *Streptococcus pneumoniae, Streptococcus pyogenes, Enterococcus faecalis, Enterococcus faecium, Enterobacter* spp.-Spezies, *Proteus* spp.-Spezies, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides spp*.-Spezies, *Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii, E. coli* oder *Mycobacterium ulcerans* verursacht wird.

15. Verbindung zur Verwendung nach Anspruch 14, wobei die bakterielle Infektion von *Acinetobacter baumannii, Klebsiella pneumoniae, Pseudomonas aeruginosa, Staphylococcus aureus* oder *E. coli* verursacht wird.

## Revendications

1. Composé de formule (I), 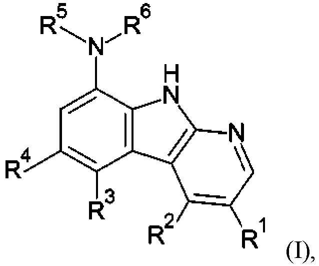 dans lequel
R¹ représente 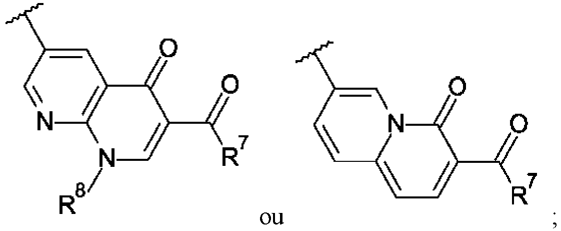 dans lequel R⁷ est choisi dans le groupe constitué par OH ; un (alkyle en C₁₋₆-2-oxo-1,3-dioxol-4-yl)alcoxy en C₁₋₆ ; un aminoalkyle en C₁₋₆-carbonyloxyalcoxy en C₁₋₆ ; un alcoxy en C₁₋₆ ; un alcoxy en C₁₋₆-carbonyloxyalcoxy en C₁₋₆ ; un alkyle en C₁₋₆-carbonyloxyalcoxy en C₁₋₆ ; un alkyle en C₁₋₆-sulfonylamino ; un carboxyalkyle en C₁₋₆-aminocarbonylalkyle en C₁₋₆-amino et un phénylalcoxy en C₁₋₆ ;
R⁸ représente un alkyle en C₁₋₆ ;
R² est choisi parmi 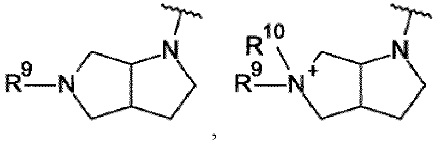 et -N(R⁹)₂ ; dans lequel R⁹ représente un alkyle en C₁₋₆ ;
R¹⁰ est choisi dans le groupe constitué par un alkyle en C₁₋₆-2-oxo-1,3-dioxol-4-yle ; un alcoxy en C₁₋₆(hydroxy)phosphoryloxyalkyle en C₁₋₆ et un phosphonooxyalkyle en C₁₋₆ ;
R³ représente un halogène ou un cyano ;
R⁴ représente un halogène ;
R⁵ est choisi dans le groupe constitué par H ; un ((aminoalkyle en C₁₋₆-carbonyl)aminoalkyle en C₁₋₆-carbonyle ; un ((aminoalkyle en C₁₋₆-carbonyl)aminoalkyle en C₁₋₆-carbonyl)aminoalkyle en C₁₋₆-carbonyle ; un (alcoxy en C₁₋₆(hydroxy)phosphoryl)alcoxy en C₁₋₆-carbonyle ; un (alcoxy en C₁₋₆)₂phosphoryle ; un aminoalkyle en C₁₋₆-carbonyloxyalcoxy en C₁₋₆-carbonyle ; un alkyle en C₁₋₆-carbonyloxyalcoxy en C₁₋₆-carbonyle ; un carboxyalcényle en C₂₋₆-carbonyloxyalcoxy en C₁₋₆-carbonyle ; un formyle ; un phénylalcoxy en C₁₋₆(hydroxy)phosphoryle ; un phosphonooxyalcoxy en C₁₋₆-carbonyle et un sulfo ;
R⁶ représente un alkyle en C₁₋₆ ;
à condition que lorsque R⁵ représente H et R⁷ représente OH, R² ne représente pas 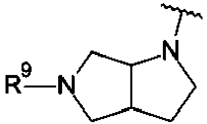 ou -N(R⁹)₂ ;
ou un sel pharmaceutiquement acceptable de celui-ci.

2. Composé de formule (I) selon la revendication 1, dans lequel
R¹ représente 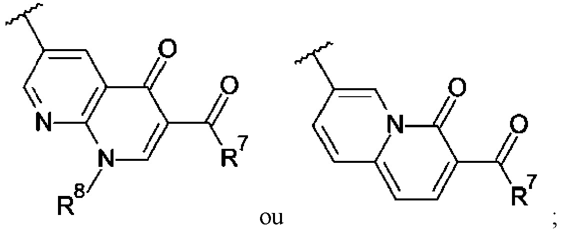 dans lequel R⁷ est choisi dans le groupe constitué par OH; un (5-méthyl-2-oxo-1,3-dioxol-4-yl)méthoxy ; un acétoxyéthoxy ; un amino(méthyl)butanoyloxyéthoxy ; un benzyloxy ; un carboxyméthylaminocarbonylméthylamino ; un éthoxy ; un isopropoxycarbonyloxyéthoxy et un méthylsulfonylamino ;
R⁸ représente un méthyle ;
R² est choisi parmi 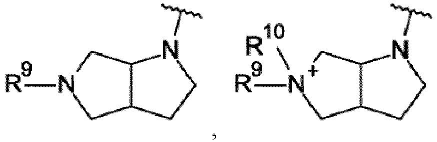 et -N(R⁹)₂ ; dans lequel R⁹ représente un méthyle ;
R¹⁰ est choisi dans le groupe constitué par un 5-méthyl-2-oxo-1,3-dioxol-4-ylméthyle ; un tert-butoxy(hydroxy)phosphoryloxyméthyle et un phosphonooxyméthyle ;
R³ représente un fluor ou un cyano ;
R⁴ représente un fluor ;
R⁵ est choisi dans le groupe constitué par H ; un ((aminoacétyl)aminoacétyl)aminoacétyle ; un (aminoacétyl)aminoacétyle ; un (tert-butoxy(hydroxy)phosphoryloxy)méthoxycarbonyle ; un acétoxyéthoxycarbonyle ; un aminoacétyloxyméthoxycarbonyle ; un amino(méthyl)butanoyloxyéthoxycarbony ; un benzyloxy(hydroxy)phosphoryle ; un carboxypropénoyloxyméthoxycarbonyle ; un diéthoxyphosphoryle ; un formyle ; un phosphonooxyméthoxycarbonyle et un sulfo ;
R⁶ représente un méthyle ;
à condition que lorsque R⁵ représente H et R⁷ représente OH, R² ne représente pas 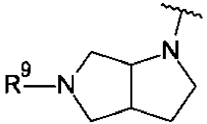 ou -N(R⁹)₂ ;
ou un sel pharmaceutiquement acceptable de celui-ci.

3. Composé selon la revendication 1, dans lequel
R¹ représente 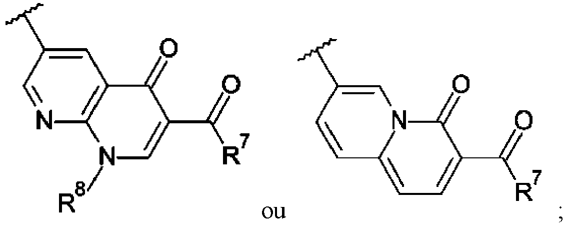 dans lequel R⁷ est choisi dans le groupe constitué par un (alkyle en C₁₋₆-2-oxo-1,3-dioxol-4-yl)alcoxy en C₁₋₆ ; un aminoalkyle en C₁₋₆-carbonyloxyalcoxy en C₁₋₆ ; un alcoxy en C₁₋₆ ; un alcoxy en C₁₋₆-carbonyloxyalcoxy en C₁₋₆ ; un alkyle en C₁₋₆-carbonylalcoxy en C₁₋₆ ; un alkyle en C₁₋₆-sulfonylamino ; un carboxyalkyle en C₁₋₆-aminocarbonylalkyle en C₁₋₆-amino et un phénylalcoxy en C₁₋₆ ;
R⁸ représente un alkyle en C₁₋₆ ;
R² représente 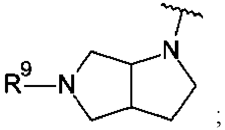 dans lequel R⁹ représente un alkyle en C₁₋₆ ;
R³ représente un halogène ou un cyano ;
R⁴ représente un halogène ;
R⁵ représente H ;
R⁶ représente un alkyle en C₁₋₆ ;
ou un sel pharmaceutiquement acceptable de celui-ci.

4. Composé selon la revendication 3, ou un sel pharmaceutiquement acceptable de celui-ci, dans lequel R⁷ est choisi dans le groupe constitué par un (alkyle en C₁₋₆-2-oxo-1,3-dioxol-4-yl)alcoxy en C₁₋₆ ; un aminoalkyle en C₁₋₆-carbonyloxyalcoxy en C₁₋₆ ; un alcoxy en C₁₋₆-carbonyloxyalcoxy en C₁₋₆ et un alkyle en C₁₋₆-carbonyloxyalcoxy en C₁₋₆.

5. Composé selon la revendication 1, dans lequel
R¹ représente 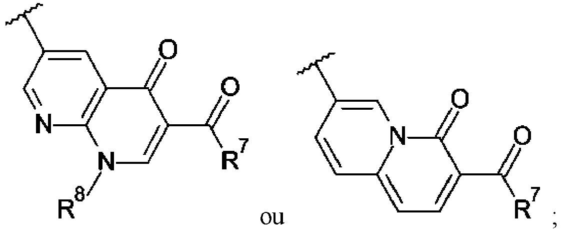 dans lequel R⁷ représente OH ;
R⁸ représente un alkyle en C₁₋₆ ;
R² représente 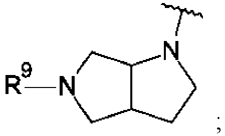 dans lequel R⁹ représente un alkyle en C₁₋₆ ;
R³ représente un halogène ou un cyano ;
R⁴ représente un halogène ;
R⁵ est choisi dans le groupe constitué par un ((aminoalkyle en C₁₋₆-carbonyl)aminoalkyle en C₁₋₆-carbonyle ; un ((aminoalkyle en C₁₋₆-carbonyl)aminoalkyle en C₁₋₆-carbonyl)aminoalkyle en C₁₋₆-carbonyle ; un (alcoxy en C₁₋₆(hydroxy)phosphoryl)alcoxy en C₁₋₆-carbonyle ; un (alcoxy en C₁₋₆)₂phosphoryle ; un aminoalkyle en C₁₋₆-carbonyloxyalcoxy en C₁₋₆-carbonyle ; un alkyle en C₁₋₆-carbonyloxyalcoxy en C₁₋₆-carbonyle ; un carboxyalcényle en C₂₋₆-carbonyloxyalcoxy en C₁₋₆-carbonyle ; un formyle ; un phénylalcoxy en C₁₋₆(hydroxy)phosphoryle ; un phosphonooxyalcoxy en C₁₋₆-carbonyle et un sulfo ;
R⁶ représente un alkyle en C₁₋₆ ;
ou un sel pharmaceutiquement acceptable de celui-ci.

6. Composé selon la revendication 5, ou un sel pharmaceutiquement acceptable de celui-ci, dans lequel R⁵ est choisi dans le groupe constitué par un alkyle en C₁₋₆-carbonyloxyalcoxy en C₁₋₆-carbonyle ; un carboxyalcényle en C₂₋₆-carbonyloxyalcoxy en C₁₋₆-carbonyle et un phosphonooxyalcoxy en C₁₋₆-carbonyle.

7. Composé de formule (II), 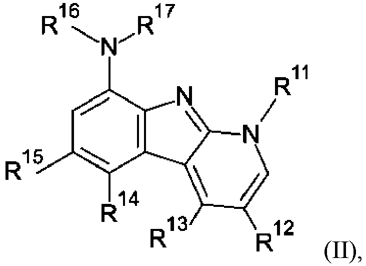 dans lequel
R¹¹ est choisi dans le groupe constitué par un alkyle en C₁₋₆-2-oxo-1,3-dioxol-4-yle ; un alcoxy en C₁₋₆(hydroxy)phosphoryloxyalkyle en C₁₋₆ et un phosphonooxyalkyle en C₁₋₆ ;
R¹² représente 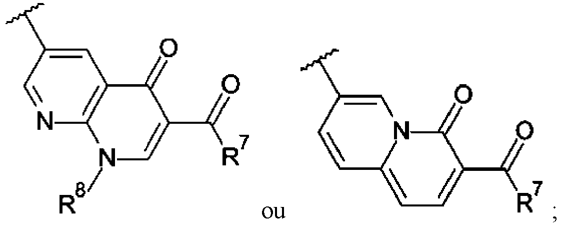 dans lequel R⁷ représente OH ;
R⁸ représente un alkyle en C₁₋₆ ;
R¹³ représente 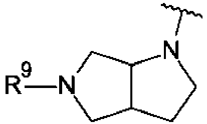 ou -N(R⁹)₂ ; dans lequel R⁹ représente un alkyle en C₁₋₆ ;
R¹⁴ représente un halogène ou un cyano ;
R¹⁵ représente un halogène ;
R¹⁶ représente H ;
R¹⁷ représente un alkyle en C₁₋₆ ;
ou un sel pharmaceutiquement acceptable de celui-ci.

8. Composé selon la revendication 7, dans lequel
R¹¹ est choisi dans le groupe constitué par un 5-méthyl-2-oxo-1,3-dioxol-4-ylméthyle ; un *tert-*butoxy(hydroxy)phosphoryloxyméthyle et un phosphonooxyméthyle ;
R¹² représente 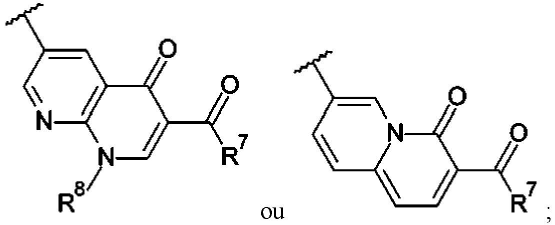 dans laquelle R⁷ représente OH ; R⁸ représente un méthyle ;
R¹³ représente 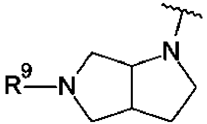 ou -N(R⁹)₂ ; dans lequel R⁹ représente un méthyle ;
R¹⁴ représente un fluor ou un cyano ;
R¹⁵ représente un fluor ;
R¹⁶ représente H ;
R¹⁷ représente un méthyle ;
ou un sel pharmaceutiquement acceptable de celui-ci.

9. Composé selon les revendications 1 ou 7, choisi parmi :
l'acide 6-[5-cyano-6-fluoro-8-[méthyl(phosphonooxyméthoxycarbonyl)amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[5-cyano-6-fluoro-8-[méthyl-[[(E)-3-carboxyprop-2-énoyl]oxyméthoxycarbonyl]amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[5-cyano-6-fluoro-8-[formyl(méthyl)amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[5-cyano-6-fluoro-8-(méthylamino)-1-(phosphonooxyméthyl)-4-[(3aS,6aS)-5-méthyl-2,3,3a,4,6,6a-hexahydropyrrolo[2,3-c]pyrrol-1-yl]pyrido[2,3-b]indol-3-yl]-1-méthyl-4-oxo-1,8-naphtyridine-3-carboxylique ;
l'acide 6-[5-cyano-6-fluoro-8-(méthylamino)-1-[(5-méthyl-2-oxo-1,3-dioxol-4-yl)méthyl]-4-[(3aS,6aS)-5-méthyl-2,3,3a,4,6,6a-hexahydropyrrolo[2,3-c]pyrrol-1-yl]pyrido[2,3-b]indol-3-yl]-1-méthyl-4-oxo-1,8-naphtyridine-3-carboxylique ;
l'acide 6-[5-cyano-6-fluoro-8-(méthylamino)-4-[(3aS,6aS)-5-méthyl-5-[(5-méthyl-2-oxo-1,3-dioxol-4-yl)méthyl]-2,3,3a,4,6,6a-hexahydropyrrolo[2,3-c]pyrrol-5-ium-1-yl]-9H-pyrido[2,3-b]indol-3-yl]-1-méthyl-4-oxo-1,8-naphtyridine-3-carboxylique ;
l'acide 6-[5-cyano-6-fluoro-8-(méthylamino)-4-[(3aS,6aS)-5-méthyl-5-(phosphonooxyméthyl)-2,3,3a,4,6,6a-hexahydropyrrolo[2,3-c]pyrrol-5-ium-1-yl]-9H-pyrido[2,3-b]indol-3-yl]-1-méthyl-4-oxo-1,8-naphtyridine-3-carboxylique ;
le 6-[5-cyano-6-fluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylate de 2-[(2S)-2-amino-3-méthyl-butanoyl]oxyéthyle ;
l'acide 2-[[2-[[6-[5-cyano-6-fluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carbonyl]amino]acétyl]amino]acétique ;
le 6-[5-cyano-6-fluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-N-méthylsulfonyl-4-oxo-1,8-naphtyridine-3-carboxamide ;
le 6-[5-cyano-6-fluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylate de 1-acétoxyéthyle ;
le 6-[6-fluoro-5-méthyl-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylate de 1-isopropoxycarbonyloxyéthyle ;
le 6-[5-cyano-6-fluoro-8-(méthylamino)-4-[(3 aS,6aS)-5-méthyl-2,3,3 a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylate de (5-méthyl-2-oxo-1,3-dioxol-4-yl)méthyle ;
l'acide 6-[8-[(2-aminoacétyl)oxyméthoxycarbonyl-méthyl-amino]-5-cyano-6-fluoro-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[8-[1-acétoxyéthoxycarbonyl(méthyl)amino]-5-cyano-6-fluoro-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[8-[diéthoxyphosphoryl(méthyl)amino]-5,6-difluoro-4-[(3aR,6aR)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3 -carboxylique ;
l'acide 6-[8-[[benzyloxy(hydroxy)phosphoryl]-méthyl-amino]-5,6-difluoro-4-[(3aR,6aR)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[5,6-difluoro-8-[méthyl(phosphonooxyméthoxycarbonyl)amino]-4-[(3aR,6aR)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[8-[[tert-butoxy(hydroxy)phosphoryl]oxyméthoxycarbonyl-méthyl-amino]-5,6-difluoro-4-[(3aR,6aR)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide (E)-4-[[[3-(6-benzyloxycarbonyl-8-méthyl-5-oxo-1,8-naphtyridin-3-yl)-5,6-difluoro-4-[(3aR,6aR)-5-méthyl-2,3,3a,4,6,6a-hexahydropyrrolo[2,3-c]pyrrol-1-yl]-9H-pyrido[2,3-b]indol-8-yl]-méthyl-carbamoyl]oxyméthoxy]-4-oxo-but-2-énoïque ;
l'acide 6-[5,6-difluoro-8-[méthyl-[[(E)-3-carboxyprop-2-énoyl]oxyméthoxycarbonyl]amino]-4-[(3aR,6aR)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[1-[[*tert*-butoxy(hydroxy)phosphoryl]oxyméthyl]-5,6-difluoro-8-(méthylamino)-4-[(3aR,6aR)-5-méthyl-2,3,3a,4,6,6a-hexahydropyrrolo[2,3-c]pyrrol-1-yl]pyrido[2,3-b]indol-3-yl]-1-méthyl-4-oxo-1,8-naphtyridine-3-carboxylique ;
l'acide 6-[5,6-difluoro-8-(méthylamino)-1-(phosphonooxyméthyl)-4-[(3aR,6aR)-5-méthyl-2,3,3a,4,6,6a-hexahydropyrrolo[2,3-c]pyrrol-1-yl]pyrido[2,3-b]indol-3-yl]-1-méthyl-4-oxo-1,8-naphtyridine-3-carboxylique ;
l'acide 6-[5,6-difluoro-8-(méthylamino)-4-[(3aR,6aR)-5-[[tert-butoxy(hydroxy)phosphoryl]oxyméthyl]-5-méthyl-2,3,3a,4,6,6a-hexahydropyrrolo[2,3-c]pyrrol-5-ium-1-yl]-9H-pyrido[2,3-b]indol-3-yl]-1-méthyl-4-oxo-1, 8-naphtyridine-3 - carboxylique ;
l'acide 6-[5,6-difluoro-8-(méthylamino)-4-[(3aR,6aR)-5-méthyl-5-(phosphonooxyméthyl)-2,3,3a,4,6,6a-hexahydropyrrolo[2,3-c]pyrrol-5-ium-1-yl]-9H-pyrido[2,3-b]indol-3-yl]-1-méthyl-4-oxo-1,8-naphtyridine-3-carboxylique ;
l'acide 6-[5,6-difluoro-8-[formyl(méthyl)amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[5,6-difluoro-8-[méthyl(sulfo)amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[5,6-difluoro-8-[méthyl-[[(E)-3-carboxyprop-2-énoyl]oxyméthoxycarbonyl]amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[5,6-difluoro-8-[méthyl(sulfo)amino]-4-[cis-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[8-[[2-[(2-aminoacétyl)amino]acétyl]-méthyl-amino]-5,6-difluoro-4-[cis-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
le 6-[5,6-difluoro-8-(méthylamino)-4-[cis-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylate d'éthyle ;
l'acide 7-[5,6-difluoro-8-[méthyl(phosphonooxyméthoxycarbonyl)amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carboxylique ;
l'acide 7-[5,6-difluoro-8-[méthyl-[[(E)-3-carboxyprop-2-énoyl]oxyméthoxycarbonyl]amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carboxylique ;
l'acide 7-[8-[[2-[[2-[(2-aminoacétyl)amino]acétyl]amino]acétyl]-méthyl-amino]-5,6-difluoro-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carboxylique ;
l'acide 7-[8-[[2-[(2-aminoacétyl)amino]acétyl]-méthyl-amino]-5,6-difluoro-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carboxylique ;
l'acide 7-[5,6-difluoro-8-(méthylamino)-1-(phosphonooxyméthyl)-4-[(3aS,6aS)-5-méthyl-2,3,3a,4,6,6a-hexahydropyrrolo[2,3-c]pyrrol-1-yl]pyrido[2,3-b]indol-3-yl]-4-oxo-quinolizine-3-carboxylique ;
le 7-[5,6-difluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carboxylate de 2-[(2S)-2-amino-3-méthyl-butanoyl]oxyéthyle ;
l'acide 2-[[2-[[7-[5,6-difluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carbonyl]amino]acétyl]amino]acétique ;
le 7-[5,6-difluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carboxylate de 1-isopropoxycarbonyloxyéthyle ;
le 7-[5,6-difluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-4-oxo-quinolizine-3-carboxylate de 1-acétoxyéthyle ;
le 7-[5,6-difluoro-8-(méthylamino)-4-[(3aS,6aS)-5-méthyl-2,3,3a,4,6,6a-hexahydropyrrolo[2,3-c]pyrrol-1-yl]-9H-pyrido[2,3-b]indol-3-yl]-N-méthylsulfonyl-4-oxo-quinolizine-3-carboxamide ;
le 6-[5,6-difluoro-8-(méthylamino)-4-[cis-5-méthyl-2,3,3a,4,6,6a-hexahydropyrrolo[2,3-c]pyrrol-1-yl]-9H-pyrido[2,3-b]indol-3-yl]-1-méthyl-N-méthylsulfonyl-4-oxo-1,8-naphtyridine-3-carboxamide ;
l'acide 6-[5,6-difluoro-8-[méthyl(phosphonooxyméthoxycarbonyl)amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
l'acide 6-[4-(diméthylamino)-5,6-difluoro-8-(méthylamino)-1-(phosphonooxyméthyl)pyrido[2,3-b]indol-3-yl]-1-méthyl-4-oxo-1,8-naphtyridine-3-carboxylique ; et
l'acide 6-[5-cyano-6-fluoro-8-[méthyl-[1-[(2S)-2-amino-3-méthyl-butanoyl]oxyéthoxycarbonyl]amino]-4-[(3aS,6aS)-5-méthyl-2,3,3a,4,6,6a-hexahydropyrrolo[2,3-c]pyrrol-1-yl]-9H-pyrido[2,3-b]indol-3-yl]-1-méthyl-4-oxo-1,8-naphtyridine-3-carboxylique ;
ou un sel pharmaceutiquement acceptable.

10. Procédé de préparation d'un composé selon l'une quelconque des revendications 1 à 9 comprenant l'une quelconque des réactions suivantes :
a) la réaction de couplage d'un composé de formule (Ij), 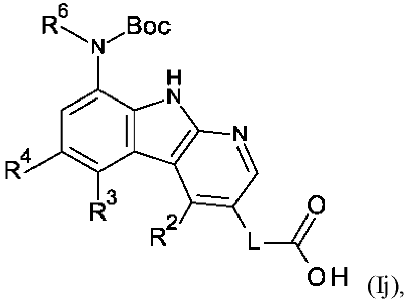 avec un alcool ou une amine en présence d'un réactif de couplage suivie de la déprotection du groupe Boc avec un acide ;
b) la réaction d'un composé de formule (Ik), 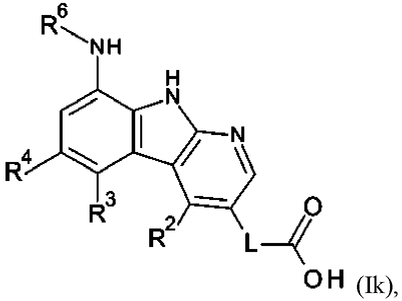 avec des réactifs d'acylation ou de sulfonylation ;
c) l'hydrolyse ou l'hydrogénation d'un composé de formule (In), 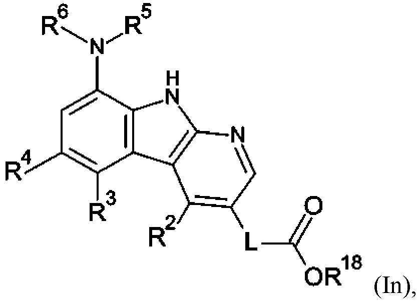 en présence d'une base ou d'un catalyseur ;
d) l'hydrolyse ou l'hydrogénation d'un composé de formule (Ip), 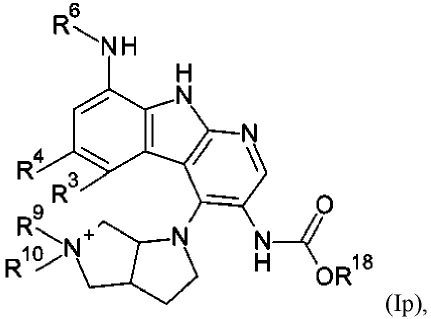 en présence d'une base ou d'un catalyseur ;
e) l'hydrolyse ou l'hydrogénation d'un composé de formule (Iq), 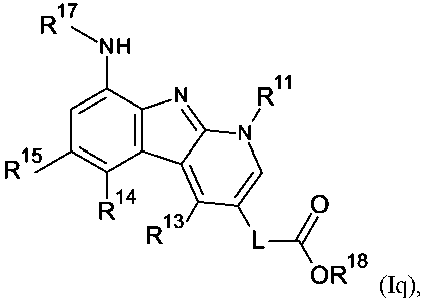
en présence d'une base ou d'un catalyseur ;
dans lequel R¹ à R¹⁷ sont tels que définis ci-dessus ; L représente un 4-oxo-1,8-naphtyridinyle ou un 4-oxoquinolizinyle non substitué ou substitué ; R¹⁸ représente un alkyle en C₁₋₆ ou Bn ;
dans l'étape a), le réactif de couplage est HATU ; l'acide est TFA ;
dans l'étape b), le réactif d'acylation est l'acide formique ; le réactif de sulfonylation est le trioxyde de soufre ;
dans l'étape c), d) et e), la base est NaOH ou LiOH ; le catalyseur est Pd/C.

11. Composé selon l'une quelconque des revendications 1 à 9 pour une utilisation comme substance thérapeutiquement active.

12. Composition pharmaceutique comprenant un composé selon l'une quelconque des revendications 1 à 9 et un véhicule thérapeutiquement inerte.

13. Composé selon l'une quelconque des revendications 1 à 9 pour une utilisation dans le traitement ou la prophylaxie d'une infection bactérienne.

14. Composé pour une utilisation selon la revendication 13, dans lequel l'infection bactérienne est causée par *Streptococcus pneumoniae, Streptococcus pyogenes, Enterococcus faecalis, Enterococcus faecium,* les espèces *Enterobacter spp.,* les espèces *Proteus spp., Serratia marcescens, Staphylococcus aureus, Staphylococci* à coagulase négative, *Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile,* les espèces *Bacteroides spp., Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii, E. coli* ou *Mycobacterium ulcerans.*

15. Composé pour une utilisation selon la revendication 14, dans lequel l'infection bactérienne est causée par *Acinetobacter baumannii, Klebsiella pneumoniae, Pseudomonas aeruginosa, Staphylococcus aureus* ou *E. coli.*
